(19) Europäisches Patentamt
European Patent Office
Office européen des brevets

(11) EP 3 515 414 B1

(12) EUROPEAN PATENT SPECIFICATION

(45) Date of publication and mention of the grant of the patent:
**30.11.2022 Bulletin 2022/48**

(21) Application number: **17851730.6**

(22) Date of filing: **18.09.2017**

(51) International Patent Classification (IPC):
***A61K 31/496*** *(2006.01)* ***A61K 31/5377*** *(2006.01)*
***A61K 31/519*** *(2006.01)* ***A61K 31/635*** *(2006.01)*
***A61K 45/06*** *(2006.01)* ***A61P 35/00*** *(2006.01)*

(52) Cooperative Patent Classification (CPC):
(C-Sets available)
**A61K 31/5377; A61K 31/496; A61K 31/519; A61K 31/635; A61K 45/06; A61P 35/00** (Cont.)

(86) International application number:
**PCT/US2017/052086**

(87) International publication number:
**WO 2018/053437 (22.03.2018 Gazette 2018/12)**

# (54) COMBINATION THERAPY

KOMBINATIONSTHERAPIE

POLYTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.09.2016 US 201662396734 P**
**25.10.2016 US 201662412720 P**

(43) Date of publication of application:
**31.07.2019 Bulletin 2019/31**

(73) Proprietor: **MEI Pharma, Inc.**
**San Diego, CA 92130 (US)**

(72) Inventor: **GOLD, Daniel P.**
**Del Mar, California 92014 (US)**

(74) Representative: **Mewburn Ellis LLP**
**Aurora Building**
**Counterslip**
**Bristol BS1 6BX (GB)**

(56) References cited:
**WO-A1-2014/055647**
**WO-A1-2015/083008**
**US-A1- 2012 165 309**
**US-A1- 2012 252 802**
**US-A1- 2012 252 802**
**US-A1- 2013 150 364**
**US-A1- 2014 088 102**
**US-A1- 2016 193 211**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/519, A61K 2300/00;**
**A61K 31/5377, A61K 2300/00;**
**A61K 31/635, A61K 2300/00**

## Description

### FIELD

The present invention relates to compounds (see the appended claims for the generic definition of these compounds) for use in a combination therapy for treatment of hematological cancer. In certain embodiments, the methods comprise administering an effective amount of a phosphoinositide-3-kinase (PI3K) inhibitor, as defined herein, and an effective amount of a Bruton tyrosine kinase (BTK) inhibitor, a Bcl-2 inhibitor, an EZH2 inhibitor, or a combination thereof to a patient.

### BACKGROUND

Phosphoinositide-3-kinases (PI3Ks) play a variety of roles in normal tissue physiology (Foukas & Shepherd, Biochem. Soc. Trans. 2004, 32, 330; Shepherd, Acta Physiol. Scand. 2005, 183, 3), with p110$\alpha$ having a specific role in cancer growth, p110$\beta$ in thrombus formation mediated by integrin $\alpha_{\Pi}\beta_3$ (Jackson et al., Nat. Med. 2005, 11, 507), and p110$\gamma$ in inflammation, rheumatoid arthritis, and other chronic inflammation states (Barber et al., Nat. Med. 2005, 11, 933; Camps et al., Nat. Med. 2005, 11, 936; Rommel et al., Nat. Rev. 2007, 7, 191; and Ito, et al., J. Pharm. Exp. Therap. 2007, 321, 1). Inhibitors of PI3K have therapeutic potential in the treatment of various proliferative diseases, including cancer.

The Bruton's tyrosine kinase (BTK) inhibitors are a class of drugs that inhibit Bruton tyrosine kinase (BTK), a member of the Tec family of kinases with a very distinct role in B-cell antigen receptor (SCR) signaling.

There continues to be a need for development of effective dosages and dosing regimens for administering PI3K inhibitors and BTK inhibitors in combination with a second agent in treating, preventing and managing various proliferative diseases, including cancers, autoimmune diseases and/or inflammatory diseases.

WO 2015/083008 A1 (11 June 2015) describes a therapeutic combination of a phosphoinositide 3-kinase (PI3K) inhibitor and a Bruton's tyrosine kinase (BTK) inhibitor and its use in methods of treatment of solid tumor cancers.

US 2012/0252802 A1 (04 October 2012) describes (alpha-substituted aralkylamino or heteroarylalkylamino) pyrimidinyl and 1,3,5-triazinyl benzimidazoles and their as agents or drugs for treating proliferative diseases.

US 2016/0193211 A1 (07 July 2016) describes therapies for the effective treatment of colon and colorectal carcinomas and a pharmaceutical kit, comprising combinations of a BTK inhibitor and fluorouracil, for the treatment of colon and colorectal carcinomas.

WO 2014/055647 A1 (10 April 2014) describes (sulfmyl and sulfonyl benzimidazolyl) pyrimidines and triazines and their use for treating proliferative diseases.

US 2014/0088102 A1 (27 March 2014) describes (alpha-substituted cycloalkylamino or heterocyclylamino) pyrimidinyl and 1,3,5-triazinyl benzimidazoles and their use as agents or drugs for treating proliferative diseases.

US 2013/0150364 A1 (13 June 2013) describes a compound having a PI3K$\delta$ selective inhibitory action and/or an IL-2 production inhibitory action and/or a B cell proliferation inhibitory action (including an activation inhibitory action).

US 2012/0165309 A1 (28 June 2012) describes a 3-substituted triazine or 3-substituted pyrimidine derivatives as agents for preventing or treating rejection in the transplantation of various organs, allergy diseases (asthma, atopic dermatitis, etc.), autoimmune diseases (rheumatoid arthritis, psoriasis, ulcerative colitis, Crohn's disease, systemic lupus erythematosus, etc.), hematologic tumor (leukemia etc.).

### SUMMARY OF THE INVENTION

The present invention is a compound of Formula (I) for use in a method of treating a hematological cancer in combination with a second agent selected from a BTK inhibitor, a Bcl-2 inhibitor, an EZH2 inhibitor, and any combination thereof:

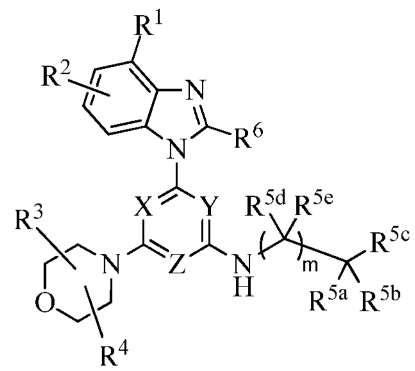

Formula (I),

or an enantiomer, a mixture of enantiomers, a mixture of two or more diastereomers, or an isotopic variant thereof; or a pharmaceutically acceptable salt, solvate, or hydrate thereof; wherein:

X, Y, and Z are each independently N or $CR^X$, with the proviso that at least two of X, Y, and Z are nitrogen atoms; where $R^X$ is hydrogen or $C_{1-6}$ alkyl;

$R^1$ and $R^2$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; wherein each $R^{1a}$, $R^{1b}$, $R^{1c}$, and $R^{1d}$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, - $C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, - $OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, - $NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, - $NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or - $S(O)_2NR^{1b}R^{1c}$;

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, - $OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, - $OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, - $NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, - $SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl, heteroaryl, and heterocyclyl in $R^1$, $R^2$, $R^3$, $R^4$, $R^6$, $R^X$, $R^{1a}$, $R^{1b}$, $R^{1c}$, $R^{1d}$, $R^{5a}$, $R^{5b}$, $R^{5c}$, $R^{5d}$, $R^{5e}$, $R^{5f}$, and $R^{5g}$ is optionally substituted with one, two, three, four, or five substituents Q, wherein each substituent Q is independently selected from (a) oxo, cyano, halo, and nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl, each of which is further optionally substituted with one, two, three, or four, substituents $Q^a$; and (c)-C(O)$R^a$, -C(O)O$R^a$, -C(O)N$R^bR^c$, -C(N$R^a$)N$R^bR^c$, -O$R^a$, -OC(O)$R^a$, -OC(O)O$R^a$, -OC(O)N$R^bR^c$,-OC(=N$R^a$)N$R^bR^c$, -OS(O)$R^a$, -OS(O)$_2$R, -OS(O)N$R^bR^c$, -OS(O)$_2$N$R^bR^c$, -N$R^bR^c$, -N$R^a$C(O)$R^d$, -N$R^a$C(O)O$R^d$, -N$R^a$C(O)N$R^bR^c$, -N$R^a$C(=N$R^d$)N$R^bR^c$, -N$R^a$S(O)$R^d$, -N$R^a$S(O)$_2R^d$, -N$R^a$S(O)N$R^bR^c$, -N$R^a$S(O)$_2$N$R^bR^c$, -S$R^a$, -S(O)$R^a$, -S(O)$_2R^a$, -S(O)N$R^bR^c$, and -S(O)$_2$N$R^bR^c$, wherein each $R^a$, $R^b$, $R^c$, and $R^d$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is further optionally substituted with one, two, three, or four, substituents $Q^a$; or (iii) $R^b$ and $R^c$ together with the N atom to which they are attached form heterocyclyl, which is further optionally substituted with one, two, three, or four, substituents $Q^a$;

wherein each $Q^a$ is independently selected from the group consisting of (a) oxo, cyano, halo, and nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl; and (c) -C(O)$R^e$, -C(O)O$R^e$, -C(O)N$R^fR^g$, -C(N$R^e$)N$R^fR^g$, -O$R^e$, -OC(O)$R^e$, - OC(O)O$R^e$, -OC(O)N$R^fR^g$, -OC(=N$R^e$)N$R^fR^g$, -OS(O)$R^e$, -OS(O)$_2R^e$, -OS(O)N$R^fR^g$, - OS(O)$_2$N$R^fR^g$, -N$R^fR^g$, -N$R^e$C(O)$R^h$, -N$R^e$C(O)O$R^h$, -N$R^e$C(O)N$R^fR^g$, -N$R^e$C(=N$R^h$)N$R^fR^g$, - N$R^e$S(O)$R^h$, -N$R^e$S(O)$_2R^h$, -N$R^e$S(O)N$R^fR^g$, -N$R^e$S(O)$_2$N$R^fR^g$, -S$R^e$, -S(O)$R^e$, -S(O)$_2R^e$, - S(O)N$R^fR^g$, and -S(O)$_2$N$R^fR^g$; wherein each $R^e$, $R^f$, $R^g$, and $R^h$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^f$ and $R^g$ together with the N atom to which they are attached form heterocyclyl;

wherein two substituents Q that are adjacent to each other optionally form a $C_{3-10}$ cycloalkenyl, $C_{6-14}$ aryl, heteroaryl, or heterocyclyl, each optionally substituted with one, two, three, or four substituents $Q^a$.

In some embodiments, $R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, or heteroaryl; or (c) -C(O)$R^{1a}$, -C(O)O$R^{1a}$, -C(O)N$R^{1b}R^{1c}$, - C(N$R^{1a}$)N$R^{1b}R^{1c}$, -O$R^{1a}$, -OC(O)$R^{1a}$, -OC(O)O$R^{1a}$, -OC(O)N$R^{1b}R^{1c}$, -OC(=N$R^{1a}$)N$R^{1b}R^{1c}$, - OS(O)$R^{1a}$, -OS(O)$_2R^{1a}$, -OS(O)N$R^{1b}R^{1c}$, -S(O)$_2$N$R^{1b}R^{1c}$, -N$R^{1b}R^{1c}$, -N$R^{1a}$C(O)$R^{1d}$, -N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, -N$R^{1a}$S(O)$R^{1d}$, -N$R^{1a}$S(O)$_2R^{1d}$, -N$R^{1a}$S(O)N$R^{1b}R^{1c}$, - N$R^{1a}$S(O)$_2$N$R^{1b}R^{1c}$, -S$R^{1a}$, -S(O)$R^{1a}$, -S(O)$_2R^{1a}$, -S(O)N$R^{1b}R^{1c}$, or -S(O)$_2$N$R^{1b}R^{1c}$.

In some embodiments, $R^{5a}$ and $R^{5b}$ are each independently (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - C(O)$R^{1a}$, -C(O)O$R^{1a}$, -C(O)N$R^{1b}R^{1c}$, -C(N$R^{1a}$)N$R^{1b}R^{1c}$, -O$R^{1a}$, -OC(O)$R^{1a}$, -OC(O)O$R^{1a}$, - OC(O)N$R^{1b}R^{1c}$, -OC(=N$R^{1a}$)N$R^{1b}R^{1c}$, -OS(O)$R^{1a}$, -OS(O)$_2R^{1a}$, -OS(O)N$R^{1b}R^{1c}$, -OS(O)$_2$N$R^{1b}R^{1c}$, - N$R^{1b}R^{1c}$, -N$R^{1a}$C(O)$R^{1d}$, -N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, - N$R^{1a}$S(O)$R^{1d}$, -N$R^{1a}$S(O)$_2R^{1d}$, -N$R^{1a}$S(O)N$R^{1b}R^{1c}$, -N$R^{1a}$S(O)$_2$N$R^{1b}R^{1c}$, -S$R^{1a}$, -S(O)$R^{1a}$, -S(O)$_2R^{1a}$, -S(O)N$R^{1b}R^{1c}$, or -S(O)$_2$N$R^{1b}R^{1c}$.

In some embodiments, $R^{5a}$ and $R^{5b}$ are each methyl, optionally substituted with one, two, or three halo(s). In some embodiments, n is 1. In some embodiments, n is 1 and $R^{5f}$ and $R^{5g}$ are each hydrogen. In some embodiments, n is 0. In some embodiments, m is 0.

Synthesis of compounds of Formula (I) is described in US Patent No. 9,056,852 B2.

In some embodiments, the compound of Formula (I) is of Formula (XI):

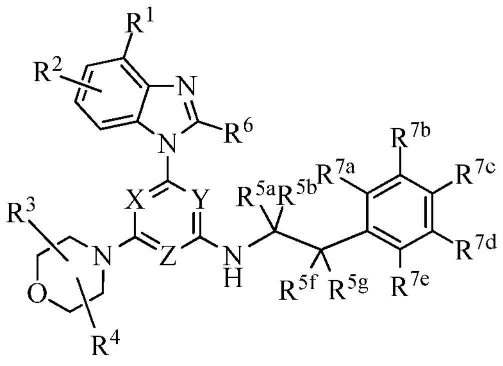

Formula (XI)

or an enantiomer, a mixture of enantiomers, a mixture of two or more diastereomers, or an isotopic variant thereof; or a pharmaceutically acceptable salt, solvate, or hydrate thereof; wherein:

$R^{7a}$, $R^{7b}$, $R^{7c}$, $R^{7d}$, and $R^{7e}$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is optionally substituted with one, two, three, or four substituents $Q^a$; or (c) $-C(O)R^a$, $- C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $- OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $- NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$, or $-S(O)_2NR^bR^c$; or

two of $R^{7a}$, $R^{7h}$, $R^{7c}$, $R^{7d}$, and $R^{7e}$ that are adjacent to each other form $C_{3-10}$ cycloalkenyl, $C_{6-14}$ aryl, heteroaryl, or heterocyclyl, each optionally substituted with one, two, three, or four substituents $Q^a$.

Synthesis of compounds of Formula (XI) is described in US Patent No. 9,056,852 B2.

In some embodiments, the compound of Formula (I) is Compound I:

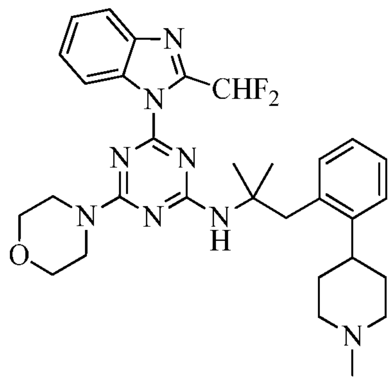

or an isotopic variant thereof, or a pharmaceutically acceptable salt, solvate, or hydrate thereof.

In some embodiments, the compound of Formula (I) is Compound II:

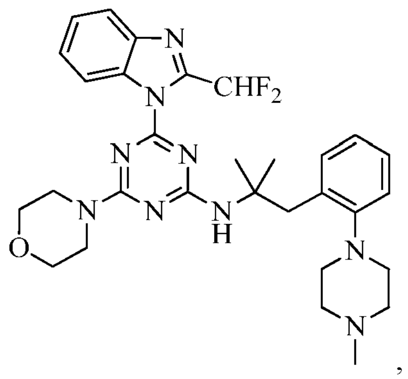

Compound II

or an isotopic variant thereof, or a pharmaceutically acceptable salt, solvate, or hydrate thereof.

In some embodiments, the compound of Formula (I) is Compound III:

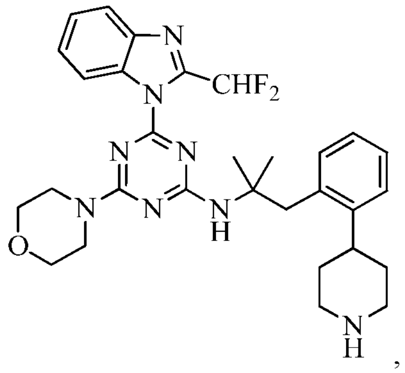

Compound III

or an isotopic variant thereof, or a pharmaceutically acceptable salt, solvate, or hydrate thereof.

In some embodiments, the compound of Formula (I) is Compound IV:

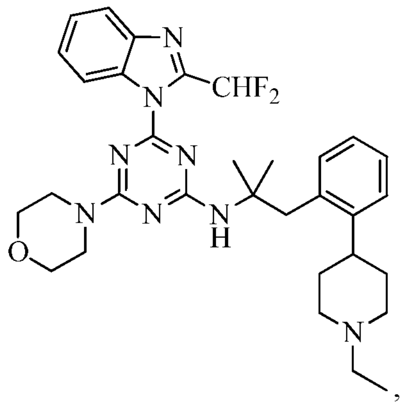

Compound IV

or an isotopic variant thereof, or a pharmaceutically acceptable salt, solvate, or hydrate thereof.

In some embodiments, the compound of Formula (I) is Compound V:

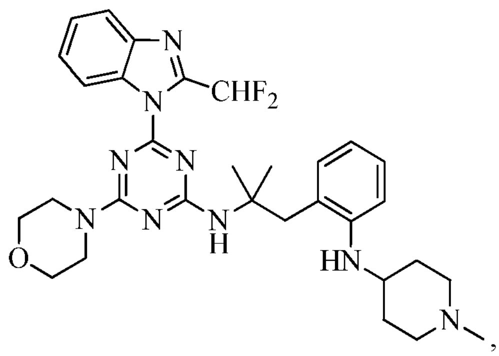

Compound V

or an isotopic variant thereof, or a pharmaceutically acceptable salt, solvate, or hydrate thereof.

In some embodiments, the compound of Formula (I) is Compound VI:

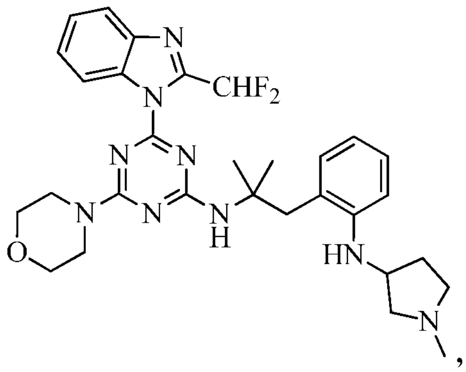

Compound VI

or an isotopic variant thereof, or a pharmaceutically acceptable salt, solvate, or hydrate thereof.

In some embodiments, the compound of Formula (I) is Compound VII:

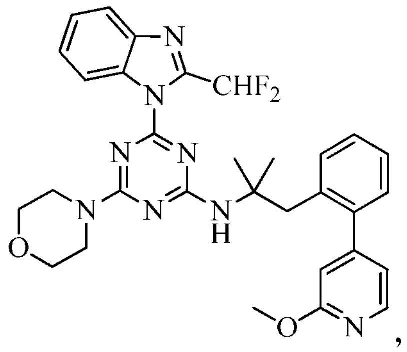

Compound VII

or an isotopic variant thereof, or a pharmaceutically acceptable salt, solvate, or hydrate thereof.

In some embodiments, the compound of Formula (I) is Compound VIII:

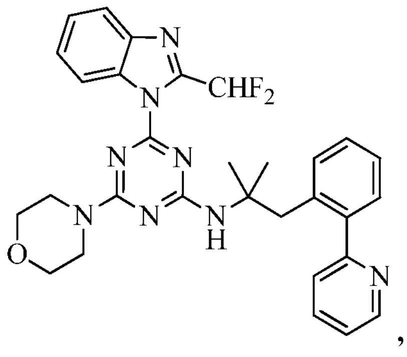

Compound VIII

or an isotopic variant thereof, or a pharmaceutically acceptable salt, solvate, or hydrate thereof.

In some embodiments, the compound of Formula (I) is Compound IX:

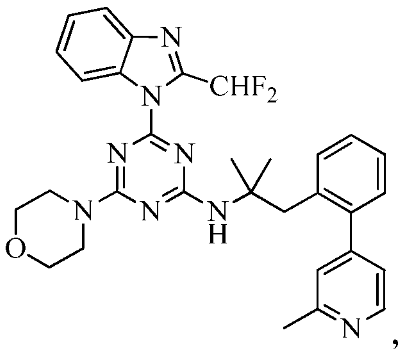

Compound IX

or an isotopic variant thereof, or a pharmaceutically acceptable salt, solvate, or hydrate thereof.

In some embodiments, the compound of Formula (I) is Compound X:

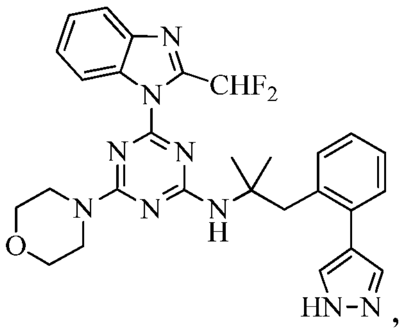

Compound X

or an isotopic variant thereof, or a pharmaceutically acceptable salt, solvate, or hydrate thereof.

In some embodiments, the second agent is a BTK inhibitor. In some embodiments, the second agent is a BTK inhibitor selected from ibrutinib, BGB3111, CC-292, ACP 196, CNX-774, CGI1746, LFM-A13, CNX-774, ONO-4059, RN486 CPI-0610, DUAL946, GSK525762, I-BET151, JQ1, OTX015, PFI-1, RVX-208, RVX2135, TEN-010, and a combination thereof. In some embodiments, the second agent is a BTK inhibitor selected from ibrutinib and BGB3111. In another embodiment, the second agent is the BTK inhibitor, ibrutinib. In yet another embodiment, the second agent is the BTK inhibitor BGB3111. In some embodiments, the second agent is a Bc1-2 inhibitor. In some embodiments, the second agent is a Bc1-2 inhibitor selected from venetoclax and PNT2258. In some embodiments, the second agent is an EZH2 inhibitor. In some embodiments, the second agent is the EZH2 inhibitor tazemetostat.

In some embodiments, the hematological cancer being treated is chronic lymphocytic leukemia or non-Hodgkin's lymphoma. In some embodiments, the hematological cancer being treated is non-Hodgkin's lymphoma and the non-Hodgkin's lymphoma is diffuse large B-cell lymphoma (DLBCL). In some embodiments, the diffuse large B-cell lymphoma is of activated B-cells (ABC DLBCL) or Germinal center B-cells (GCB DLBCL).

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**FIG. 1** illustrates % growth inhibition vs. concentration of compound I in DB cells as measured in ATPLite assay.
**FIG. 2** illustrates % growth inhibition vs. concentration of compound I in DOHH-2 cells as measured in ATPLite assay.
**FIG. 3** illustrates % growth inhibition vs. concentration of compound I in HT cells as measured in ATPLite assay.
**FIG. 4** illustrates % growth inhibition vs. concentration of compound I in NU-DHL-1 cells as measured in ATPLite assay.
**FIG. 5** illustrates % growth inhibition vs. concentration of compound I in OCI-Ly19 cells as measured in ATPLite assay.

**FIG. 6** illustrates % growth inhibition vs. concentration of compound I in OCI- Ly3 cells as measured in ATPLite assay.
**FIG. 7** illustrates % growth inhibition vs. concentration of compound I in Pfeiffer cells as measured in ATPLite assay.
**FIG. 8** illustrates % growth inhibition vs. concentration of compound I in SU-DHL-10 cells as measured in ATPLite assay.
**FIG. 9** illustrates growth inhibition ($GI_{50}$) for compound I in the tested cell lines.
**FIG. 10** illustrates % growth inhibition for compound I in the tested cell lines.

## DETAILED DESCRIPTION

Described herein are pharmaceutical compositions comprising i) a PI3K inhibitor; and ii) a BTK inhibitor, a Bcl-2 inhibitor, an EZH2 inhibitor, or any combination thereof. The pharmaceutical compositions described herein are useful for treating hematological cancer. Also described herein are methods of treating hematological cancer with a combination of i) a PI3K inhibitor, as described herein, and; ii) a BTK inhibitor, a Bcl-2 inhibitor, an EZH2 inhibitor, or any combination thereof.
To facilitate understanding of the disclosure set forth herein, a number of terms are defined below.
Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs.

## DEFINITIONS

The term "subject" refers to an animal, including, but not limited to, a primate (e.g., human), cow, pig, sheep, goat, horse, dog, cat, rabbit, rat, or mouse. The terms "subject" and "patient" are used interchangeably herein in reference, for example, to a mammalian subject, such as a human subject, in one embodiment, a human.
The terms "treat," "treating," and "treatment" are meant to include alleviating or abrogating a disorder, disease, or condition, or one or more of the symptoms associated with the disorder, disease, or condition; or alleviating or eradicating the cause(s) of the disorder, disease, or condition itself.
The terms "prevent," "preventing," and "prevention" are meant to include a method of delaying and/or precluding the onset of a disorder, disease, or condition, and/or its attendant symptoms; barring a subject from acquiring a disorder, disease, or condition; or reducing a subject's risk of acquiring a disorder, disease, or condition.
The terms "therapeutically effective amount" and "effective amount" are meant to include the amount of a compound that, when administered, is sufficient to prevent development of, or alleviate to some extent, one or more of the symptoms of the disorder, disease, or condition being treated. The terms "therapeutically effective amount" or "effective amount" also refer to the amount of a compound that is sufficient to elicit the biological or medical response of a biological molecule (e.g., a protein, enzyme, RNA, or DNA), cell, tissue, system, animal, or human, which is being sought by a researcher, veterinarian, medical doctor, or clinician.
The terms "pharmaceutically acceptable carrier," "pharmaceutically acceptable excipient," "physiologically acceptable carrier," and "physiologically acceptable excipient" refer to a pharmaceutically-acceptable material, composition, or vehicle, such as a liquid or solid filler, diluent, solvent, or encapsulating material. In one embodiment, each component is "pharmaceutically acceptable" in the sense of being compatible with other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See,* Remington: The Science and Practice of Pharmacy, 21st Edition, Lippincott Williams & Wilkins: Philadelphia, PA, 2005; Handbook of Pharmaceutical Excipients, 5th Edition, Rowe et al., Eds., The Pharmaceutical Press and the American Pharmaceutical Association: 2005; and Handbook of Pharmaceutical Additives, 3rd Edition, Ash and Ash Eds., Gower Publishing Company: 2007; Pharmaceutical Preformulation and Formulation, 2nd Edition, Gibson Ed., CRC Press LLC: Boca Raton, FL, 2009.
The terms "about" and "approximately" mean an acceptable error for a particular value as determined by one of ordinary skill in the art, which depends in part on how the value is measured or determined. In certain embodiments, the terms "about" and "approximately" mean within 1, 2, 3, or 4 standard deviations. In certain embodiments, the term "about" or "approximately" means within 50%, 20%, 15%, 10%, 9%, 8%, 7%, 6%, 5%, 4%, 3%, 2%, 1%, 0.5%, or 0.05% of a given value or range.
The terms "active ingredient" and "active substance" refer to a compound, which is administered, alone or in combination with one or more pharmaceutically acceptable excipients, to a subject for treating, preventing, or ameliorating one or more symptoms of a disorder, disease, or condition. As used herein, "active ingredient" and "active substance" may be an optically active isomer of a compound described herein.
The terms "drug," "therapeutic agent," and "chemotherapeutic agent" refer to a compound, or a pharmaceutical

composition thereof, which is administered to a subject for treating, preventing, or ameliorating one or more symptoms of a disorder, disease, or condition.

The terms "naturally occurring" and "native" when used in connection with biological materials such as nucleic acid molecules, polypeptides, host cells, and the like, refer to materials which are found in nature and are not manipulated by man. Similarly, "non-naturally occurring" or "non-native" refers to a material that is not found in nature or that has been structurally modified or synthesized by man.

The term "PI3K" refers to a phosphoinositide 3-kinase or variant thereof, which is capable of phosphorylating the inositol ring of PI in the D-3 position. The term "PI3K variant" is intended to include proteins substantially homologous to a native PI3K, i.e., proteins having one or more naturally or non-naturally occurring amino acid deletions, insertions, or substitutions (e.g., PI3K derivatives, homologs, and fragments), as compared to the amino acid sequence of a native PI3K. The amino acid sequence of a PI3K variant is at least about 80% identical, at least about 90% identical, or at least about 95% identical to a native PI3K. Examples of PI3K include, but are not limited to, p110$\alpha$, p110$\beta$, p110$\delta$, p110$\gamma$, PI3K-C2$\alpha$, PI3K-C2$\beta$, PI3K-C2$\gamma$, Vps34, mTOR, ATM, ATR, and DNA-PK. *See,* Fry, Biochem. Biophys. Acta 1994, 1226, 237-268; Vanhaesebroeck and Waterfield, Exp. Cell. Res. 1999, 253, 239-254; and Fry, Breast Cancer Res. 2001, 3, 304-312. PI3Ks are classified into at least four classes. Class I includes p110$\alpha$, p110$\beta$, p110$\delta$, and p110$\gamma$. Class II includes PI3K-C2$\alpha$, PI3K-C2$\beta$, and PI3K-C2$\gamma$. Class III includes Vps34. Class IV includes mTOR, ATM, ATR, and DNA-PK. In certain embodiments, the PI3K is a Class I kinase. In certain embodiments, the PI3K is p110$\alpha$, p110$\beta$, p110$\delta$, or p110$\gamma$. In certain embodiments, the PI3K is a variant of a Class I kinase. In certain embodiments, the PI3K is a p110$\alpha$ mutant. Examples of p110$\alpha$ mutants include, but are not limited to, R38H, G106V, K111N, K227E, N345K, C420R, P539R, E542K, E545A, E545G, E545K, Q546K, Q546P, E453Q, H710P, I800L, T1025S, M10431, M1043V, H1047L, H1047R, and H1047Y (Ikenoue et al., Cancer Res. 2005, 65, 4562-4567; Gymnopoulos et al., Proc. Natl. Acad Sci., 2007, 104, 5569-5574). In certain embodiments, the PI3K is a Class II kinase. In certain embodiments, the PI3K is PI3K-C2$\alpha$, PI3K- C2$\beta$, or PI3K-C2$\gamma$. In certain embodiments, the PI3K is a Class III kinase. In certain embodiments, the PI3K is Vps34. In certain embodiments, the PI3K is a Class IV kinase. In certain embodiments, the PI3K is mTOR, ATM, ATR, or DNA-PK.

As used herein, "BTK" refers to Bruton's tyrosine kinase. BTK belongs to the Tec tyrosine kinase family (Vetrie et al., Nature 361: 226-233, 1993; Bradshaw, Cell Signal. 22: 1175-84, 2010). BTK is primarily expressed in most hematopoietic cells such as B cells, mast cells and macrophages (Smith et al., J. Immunol. 152: 557-565, 1994) and is localized in bone marrow, spleen and lymph node tissue. BTK plays important roles in B-cell receptor (BCR) and FcR signaling pathways, which involve in B-cell development, differentiation (Khan, Immunol. Res. 23: 147, 2001. The BTK inhibitor may be selected from the compounds disclosed in US Patent Nos. 8,084,620B2; 7,514,444B2; 7,718,662B1; and 7,393,848B1; US Publication Nos. 2016/0083392A1; 2015/0005277A1; 2015/0259354A1; 2012/053189A1; 2010/254905A1; 2008/0139582A1; 2012/0077832A1; 2012/0232054A1; 2012/082702A1; 2010/0160303A1, 2012/129852A1; 2006/0178367A1; 2006/0183746A1; 2012/040961A1; 2010/144705A1; 2012/0028981A1; 2012/058996A1; 2009/0318448A1; 2010/0016301A1; 2009/105209A1; 2010/0222325A1; and 2010/0004231 A1; International Publication Nos. WO 2011/153514; WO 2011/046964; WO 2010/009342; WO 2008/121742; WO 2008/054827; WO 2007/087068; WO 2011/090760; WO 2010/028236; WO 2009/158571; WO 2009/051822, WO 2010/123870; WO 2010/126960; WO 2011/162515; WO 2012/135801; WO 2011/152351; WO 2007/136790A2; WO 2002/050071; WO 2008/116064; WO 2010/011837; WO 2011/159857,. WO 2011/019780, WO 2011/029043; WO 2011/029046; WO 2005/005429; WO 2005/014599; WO 2005/047290; WO 2006/053121; WO 2008/033834; WO 2008/033858; WO 2006/099075; WO 2008/033854; WO 2008/033857; WO 2009/039397, WO 2009/137596; WO 2010/056875; WO 2010/068788; WO 2010/068806; WO 2010/068810, WO 2011/140488; WO 2012/030990; WO 2012/031004; WO 2005/011597; WO 2008/045627; WO 2008/144253, WO 2007/140222, WO 2013/008095, WO 2012/170976A2, WO 2012/135944A1; WO 2010/065898A2; WO 2012/158795A1; WO 2012/158764A1; WO 2012/158810A1; WO 2012156334A1; WO 2012020008; WO 2010122038; WO 2010006970; WO 2010006947; WO 2010000633; WO 2009077334; WO 2009098144, WO 2006065946; WO 2007027594; WO 2007027729 and EP2068849. The BTK inhibitor may also be selected from ibrutinib, BGB3111, CC-292 (AVL-292), ACP 196 (Acalabrutinib), CNX-774, CGI1746, LFM-A13, CNX-774, ONO-4059, RN486 CPI-0610, DUAL946, GSK525762, I-BET151, JQ1, OTX015, PFI-1, RVX-208, RVX2135, TEN-010, and a combination thereof. In one embodiment, the BTK inhibitor is ibrutinib or BGB3111.

As used herein "Bcl-2" refers to B-cell lymphoma 2, an anti-apoptotic protein that has been implicated in numerous types of cancers including chronic lymphocytic leukemia, melanoma, breast, prostate and lung carcinomas. In one embodiment, the Bcl-2 inhibitor is ABT-199 (venetoclax), ABT-737, ABT-263 (navitoclax), or PNT2258. In another embodiment, the Bcl-2 inhibitor is venetoclax or PNT2258.

As used herein "EZH2" refers to enhancer of zeste homolog 2, which is a histone-lysine N-methyltransferase enzyme (EC 2.1.1.43) encoded by EZH2 gene, that participates in DNA methylation and, ultimately, transcriptional repression. Mutation or over-expression of EZH2 has been linked to many to forms of cancer. In one embodiment, the EZH2 inhibitor is EPZ-6438 (tazemetostat, E7438), DNZeP (3-Deazaneplanocin), GSK2816126, EPZ005687 or Ell. In another embodiment, the EZH2 inhibitor is tazemetostat (EPZ-6438).

The terms "synergy," "synergism," and "synergistic" as used herein refer to a combination of therapies (e.g., use of a PI3K inhibitor of Formula (I) and a BTK inhibitor, a Bcl-2 inhibitor, an EZH2 inhibitor or any combination thereof) that is more effective than the expected additive effects of any two or more single therapies. For example, a synergistic effect of a combination of therapies permits the use of lower dosages of one or more of the therapies and/or less frequent administration of said therapies to a subject. The ability to utilize lower dosages of therapies and/or to administer the therapies less frequently reduces the toxicity associated with the administration of the therapies to a subject without reducing the efficacy of said therapies in the prevention, management, treatment, or amelioration of a given disease, such as an autoimmune disease, inflammatory disease, or cancer including, but not limited to, chronic lymphocytic leukemia or non-Hodgkin's lymphoma. In addition, a synergistic effect can result in improved efficacy of therapies in the prevention, management, treatment, or amelioration of a given disease, such an autoimmune disease, inflammatory disease, or cancer i, but not limited to, chronic lymphocytic leukemia or non-Hodgkin's lymphoma. Finally, synergistic effects of a combination of therapies may avoid or reduce adverse or unwanted side effects associated with the use of any single therapy. The "synergy," "synergism," or "synergistic" effect of a combination may be determined herein by the methods of Chou et al., and/or Clarke et al. See Ting-Chao Chou, Theoretical Basis, Experimental Design, and Computerized Simulation of Synergism and Antagonism in Drug Combination Studies, Pharmacol Rev 58:621-681 (2006), and Clarke et al., Issues in experimental design and endpoint analysis in the study of experimental cytotoxic agents in vivo in breast cancer and other models, Breast Cancer Research and Treatment 46:255-278 (1997).

The term "isotopic variant" refers to a compound that contains an unnatural proportion of an isotope at one or more of the atoms that constitute such a compound. In certain embodiments, an "isotopic variant" of a compound contains unnatural proportions of one or more isotopes, including, but not limited to, hydrogen ($^1H$). deuterium ($^2H$), tritium ($^3H$), carbon-11 ($^{11}C$), carbon-12 ($^{12}C$), carbon-13 ($^{13}C$), carbon-14 ($^{14}C$), nitrogen-13 ($^{13}N$), nitrogen-14 ($^{14}N$), nitrogen-15 ($^{15}N$), oxygen-14 ($^{14}O$), oxygen-15 ($^{15}O$), oxygen-16 ($^{16}O$), oxygen-17 ($^{17}O$), oxygen-18 ($^{18}O$), fluorine-17 ($^{17}F$), fluorine-18 ($^{18}F$), phosphorus-31 ($^{31}P$), phosphorus-32 ($^{32}P$), phosphorus-33 ($^{33}P$), sulfur-32 ($^{32}S$), sulfur-33 ($^{33}S$), sulfur-34 ($^{34}S$), sulfur-35 ($^{35}S$), sulfur-36 ($^{36}S$), chlorine-35 ($^{35}Cl$), chlorine-36 ($^{36}Cl$), chlorine-37 ($^{37}Cl$), bromine-79 ($^{79}Br$), bromine-81 ($^{81}Br$), iodine-123 ($^{123}I$), iodine-125 ($^{125}I$), iodine-127 ($^{127}I$), iodine-129 ($^{129}I$), and iodine-131 ($^{131}I$). In certain embodiments, an "isotopic variant" of a compound is in a stable form, that is, non-radioactive. In certain embodiments, an "isotopic variant" of a compound contains unnatural proportions of one or more isotopes, including, but not limited to, hydrogen ($^1H$). deuterium ($^2H$), carbon-12 ($^{12}C$), carbon-13 ($^{13}C$), nitrogen-14 ($^{14}N$), nitrogen-15 ($^{15}N$), oxygen-16 ($^{16}O$), oxygen-17 ($^{17}O$), oxygen-18 ($^{18}O$), fluorine-17 ($^{17}F$), phosphorus-31 ($^{31}P$), sulfur-32 ($^{32}S$), sulfur-33 ($^{33}S$), sulfur-34 ($^{34}S$), sulfur-36 ($^{36}S$), chlorine-35 ($^{35}Cl$), chlorine-37 ($^{37}Cl$), bromine-79 ($^{79}Br$), bromine-81 ($^{81}Br$), and iodine-127 ($^{127}I$). In certain embodiments, an "isotopic variant" of a compound is in an unstable form, that is, radioactive. In certain embodiments, an "isotopic variant" of a compound contains unnatural proportions of one or more isotopes, including, but not limited to, tritium ($^3H$), carbon-11 ($^{11}C$), carbon-14 ($^{14}C$), nitrogen-13 ($^{13}N$), oxygen-14 ($^{14}O$), oxygen-15 ($^{15}O$), fluorine-18 ($^{18}F$), phosphorus-32 ($^{32}P$), phosphorus-33 ($^{33}P$), sulfur-35 ($^{35}S$), chlorine-36 ($^{36}Cl$), iodine-123 ($^{123}I$), iodine-125 ($^{125}I$), iodine-129 ($^{129}I$), and iodine-131 ($^{131}I$). It will be understood that, in a compound as described herein, any hydrogen can be $^2H$, for example, or any carbon can be $^{13}C$, for example, or any nitrogen can be $^{15}N$, for example, or any oxygen can be $^{18}O$, for example, where feasible according to the judgment of one of skill. In certain embodiments, an "isotopic variant" of a compound contains unnatural proportions of deuterium (D).

The term "alkyl" refers to a linear or branched saturated monovalent hydrocarbon radical, wherein the alkylene may optionally be substituted with one or more substituents Q as described herein. The term "alkyl" also encompasses both linear and branched alkyl, unless otherwise specified. In certain embodiments, the alkyl is a linear saturated monovalent hydrocarbon radical that has 1 to 20 ($C_{1-20}$), 1 to 15 ($C_{1-15}$), 1 to 10 ($C_{1-10}$), or 1 to 6 ($C_{1-6}$) carbon atoms, or branched saturated monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. As used herein, linear $C_{1-6}$ and branched $C_{3-6}$ alkyl groups are also referred as "lower alkyl." Examples of alkyl groups include, but are not limited to, methyl, ethyl, propyl (including all isomeric forms), n-propyl, isopropyl, butyl (including all isomeric forms), *n*-butyl, isobutyl, *sec*-butyl, *t*-butyl, pentyl (including all isomeric forms), and hexyl (including all isomeric forms). For example, $C_{1-6}$ alkyl refers to a linear saturated monovalent hydrocarbon radical of 1 to 6 carbon atoms or a branched saturated monovalent hydrocarbon radical of 3 to 6 carbon atoms.

The term "alkylene" refers to a linear or branched saturated divalent hydrocarbon radical, wherein the alkylene may optionally be substituted with one or more substituents Q as described herein. The term "alkylene" encompasses both linear and branched alkylene, unless otherwise specified. In certain embodiments, the alkylene is a linear saturated divalent hydrocarbon radical that has 1 to 20 ($C_{1-20}$), 1 to 15 ($C_{1-15}$), 1 to 10 ($C_{1-10}$), or 1 to 6 ($C_{1-6}$) carbon atoms, or branched saturated divalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. As used herein, linear $C_{1-6}$ and branched $C_{3-6}$ alkylene groups are also referred as "lower alkylene." Examples of alkylene groups include, but are not limited to, methylene, ethylene, propylene (including all isomeric forms), *n*-propylene, isopropylene, butylene (including all isomeric forms), *n*-butylene, isobutylene, *t*-butylene, pentylene (including all isomeric forms), and hexylene (including all isomeric forms). For example, $C_{1-6}$ alkylene refers to a linear saturated divalent hydrocarbon radical of 1 to 6 carbon atoms or a branched saturated divalent hydrocarbon radical of 3 to 6 carbon

atoms.

The term "heteroalkylene" refers to a linear or branched saturated divalent hydrocarbon radical that contains one or more heteroatoms each independently selected from O, S, and N in the hydrocarbon chain. For example, $C_{1-6}$ heteroalkylene refers to a linear saturated divalent hydrocarbon radical of 1 to 6 carbon atoms or a branched saturated divalent hydrocarbon radical of 3 to 6 carbon atoms. In certain embodiments, the heteroalkylene is a linear saturated divalent hydrocarbon radical that has 1 to 20 ($C_{1-20}$), 1 to 15 ($C_{1-15}$), 1 to 10 ($C_{1-10}$), or 1 to 6 ($C_{1-6}$) carbon atoms, or branched saturated divalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. As used herein, linear $C_{1-6}$ and branched $C_{3-6}$ heteroalkylene groups are also referred as "lower heteroalkylene." Examples of heteroalkylene groups include, but are not limited to, $-CH_2O-$, - $CH_2OCH_2-$, $-CH_2CH_2O-$, $-CH_2NH-$, $-CH_2NHCH_2-$, $-CH_2CH_2NH-$, $-CH_2S-$, $-CH_2SCH_2-$, and - $CH_2CH_2S-$. In certain embodiments, heteroalkylene may also be optionally substituted with one or more substituents Q as described herein.

The term "alkenyl" refers to a linear or branched monovalent hydrocarbon radical, which contains one or more, in one embodiment, one, two, three, four, or five, in another embodiment, one, carbon-carbon double bond(s). The alkenyl may be optionally substituted with one or more substituents Q as described herein. The term "alkenyl" also embraces radicals having "*cis*" and *"trans"* configurations, or alternatively, "Z" and "E" configurations, as appreciated by those of ordinary skill in the art. As used herein, the term "alkenyl" encompasses both linear and branched alkenyl, unless otherwise specified. For example, $C_{2-6}$ alkenyl refers to a linear unsaturated monovalent hydrocarbon radical of 2 to 6 carbon atoms or a branched unsaturated monovalent hydrocarbon radical of 3 to 6 carbon atoms. In certain embodiments, the alkenyl is a linear monovalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of alkenyl groups include, but are not limited to, ethenyl, propen-1-yl, propen-2-yl, allyl, butenyl, and 4-methylbutenyl.

The term "alkenylene" refers to a linear or branched divalent hydrocarbon radical, which contains one or more, in one embodiment, one, two, three, four, or five, in another embodiment, one, carbon-carbon double bond(s). The alkenylene may be optionally substituted with one or more substituents Q as described herein. Similarly, the term "alkenylene" also embraces radicals having *"cis"* and *"trans"* configurations, or alternatively, "E" and "Z" configurations. As used herein, the term "alkenylene" encompasses both linear and branched alkenylene, unless otherwise specified. For example, $C_{2-6}$ alkenylene refers to a linear unsaturated divalent hydrocarbon radical of 2 to 6 carbon atoms or a branched unsaturated divalent hydrocarbon radical of 3 to 6 carbon atoms. In certain embodiments, the alkenylene is a linear divalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched divalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of alkenylene groups include, but are not limited to, ethenylene, allylene, propenylene, butenylene, and 4-methylbutenylene.

The term "heteroalkenylene" refers to a linear or branched divalent hydrocarbon radical, which contains one or more, in one embodiment, one, two, three, four, or five, in another embodiment, one, carbon-carbon double bond(s), and which contains one or more heteroatoms each independently selected from O, S, and N in the hydrocarbon chain. The heteroalkenylene may be optionally substituted with one or more substituents Q as described herein. The term "heteroalkenylene" embraces radicals having a *"cis"* or *"trans"* configuration or a mixture thereof, or alternatively, a "Z" or "E" configuration or a mixture thereof, as appreciated by those of ordinary skill in the art. For example, $C_{2-6}$ heteroalkenylene refers to a linear unsaturated divalent hydrocarbon radical of 2 to 6 carbon atoms or a branched unsaturated divalent hydrocarbon radical of 3 to 6 carbon atoms. In certain embodiments, the heteroalkenylene is a linear divalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched divalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of heteroalkenylene groups include, but are not limited to, $-CH=CHO-$, $-CH=CHOCH_2-$, - $CH=CHCH_2O-$, $-CH=CHS-$, $-CH=CHSCH_2-$, $-CH=CHCH_2S-$, or $-CH=CHCH_2NH-$.

The term "alkynyl" refers to a linear or branched monovalent hydrocarbon radical, which contains one or more, in one embodiment, one, two, three, four, or five, in another embodiment, one, carbon-carbon triple bond(s). The alkynyl may be optionally substituted with one or more substituents Q as described herein. The term "alkynyl" also encompasses both linear and branched alkynyl, unless otherwise specified. In certain embodiments, the alkynyl is a linear monovalent hydrocarbon radical of 2 to 20 ($C_{2-20}$), 2 to 15 ($C_{2-15}$), 2 to 10 ($C_{2-10}$), or 2 to 6 ($C_{2-6}$) carbon atoms, or a branched monovalent hydrocarbon radical of 3 to 20 ($C_{3-20}$), 3 to 15 ($C_{3-15}$), 3 to 10 ($C_{3-10}$), or 3 to 6 ($C_{3-6}$) carbon atoms. Examples of alkynyl groups include, but are not limited to, ethynyl ($-C=CH$) and propargyl ($-CH_2C\equiv CH$). For example, $C_{2-6}$ alkynyl refers to a linear unsaturated monovalent hydrocarbon radical of 2 to 6 carbon atoms or a branched unsaturated monovalent hydrocarbon radical of 3 to 6 carbon atoms.

The term "cycloalkyl" refers to a cyclic saturated bridged and/or non-bridged monovalent hydrocarbon radical, which may be optionally substituted with one or more substituents Q as described herein. In certain embodiments, the cycloalkyl has from 3 to 20 ($C_{3-20}$), from 3 to 15 ($C_{3-15}$), from 3 to 10 ($C_{3-10}$), or from 3 to 7 ($C_{3-7}$) carbon atoms. Examples of cycloalkyl groups include, but are not limited to, cyclopropyl, cyclobutyl, cyclopentyl, cyclohexyl, cycloheptyl, bicyclo[2.1.1]hexyl, bicyclo[2.2.1]heptyl, decalinyl, and adamantyl.

The term "cycloalkenyl" refers to a cyclic unsaturated, nonaromatic bridged and/or non-bridged monovalent hydrocarbon radical, which may be optionally substituted with one or more substituents Q as described herein. In certain embodiments, the cycloalkenyl has from 3 to 20 ($C_{3-20}$), from 3 to 15 ($C_{3-15}$), from 3 to 10 ($C_{3-10}$), or from 3 to 7 ($C_{3-7}$) carbon atoms. Examples of cycloalkyl groups include, but are not limited to, cyclobutenyl, cyclopentenyl, cyclohexenyl, or cycloheptenyl,

The term "aryl" refers to a monocyclic aromatic group and/or multicyclic monovalent aromatic group that contain at least one aromatic hydrocarbon ring. In certain embodiments, the aryl has from 6 to 20 ($C_{6-20}$), from 6 to 15 ($C_{6-15}$), or from 6 to 10 ($C_{6-10}$) ring atoms. Examples of aryl groups include, but are not limited to, phenyl, naphthyl, fluorenyl, azulenyl, anthryl, phenanthryl, pyrenyl, biphenyl, and terphenyl. Aryl also refers to bicyclic or tricyclic carbon rings, where one of the rings is aromatic and the others of which may be saturated, partially unsaturated, or aromatic, for example, dihydronaphthyl, indenyl, indanyl, or tetrahydronaphthyl (tetralinyl). In certain embodiments, aryl may be optionally substituted with one or more substituents Q as described herein.

The terms "aralkyl" and "arylalkyl" refer to a monovalent alkyl group substituted with one or more aryl groups. In certain embodiments, the aralkyl has from 7 to 30 ($C_{7-30}$), from 7 to 20 ($C_{7-20}$), or from 7 to 16 ($C_{7-16}$) carbon atoms. Examples of aralkyl groups include, but are not limited to, benzyl, 2-phenylethyl, and 3-phenylpropyl. In certain embodiments, the aralkyl are optionally substituted with one or more substituents Q as described herein.

The term "heteroaryl" refers to a monovalent monocyclic aromatic group or monovalent polycyclic aromatic group that contain at least one aromatic ring, wherein at least one aromatic ring contains one or more heteroatoms independently selected from O, S, N, and P in the ring. A heteroaryl group is bonded to the rest of a molecule through its aromatic ring. Each ring of a heteroaryl group can contain one or two O atoms, one or two S atoms, one to four N atoms, and/or one or two P atoms, provided that the total number of heteroatoms in each ring is four or less and each ring contains at least one carbon atom. In certain embodiments, the heteroaryl has from 5 to 20, from 5 to 15, or from 5 to 10 ring atoms. Examples of monocyclic heteroaryl groups include, but are not limited to, furanyl, imidazolyl, isothiazolyl, isoxazolyl, oxadiazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl, and triazolyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl, and xanthenyl. In certain embodiments, the heteroaryl may also be optionally substituted with one or more substituents Q as described herein as described herein.

The terms "heterocyclyl" and "heterocyclic" refer to a monovalent monocyclic non-aromatic ring system or monovalent polycyclic ring system that contains at least one non-aromatic ring, wherein one or more of the non-aromatic ring atoms are heteroatoms independently selected from O, S, N, and P; and the remaining ring atoms are carbon atoms. In certain embodiments, the heterocyclyl or heterocyclic group has from 3 to 20, from 3 to 15, from 3 to 10, from 3 to 8, from 4 to 7, or from 5 to 6 ring atoms. A heterocyclyl group is bonded to the rest of a molecule through its non-aromatic ring. In certain embodiments, the heterocyclyl is a monocyclic, bicyclic, tricyclic, or tetracyclic ring system, which may be spiro, fused, or bridged, and in which nitrogen or sulfur atoms may be optionally oxidized, nitrogen atoms may be optionally quaternized, and some rings may be partially or fully saturated, or aromatic. The heterocyclyl may be attached to the main structure at any heteroatom or carbon atom which results in the creation of a stable compound. Examples of such heterocyclic groups include, but are not limited to, azepinyl, benzodioxanyl, benzodioxolyl, benzofuranonyl, benzopyranonyl, benzopyranyl, benzotetrahydrofuranyl, benzotetrahydrothienyl, benzothiopyranyl, benzoxazinyl, β-carbolinyl, chromanyl, chromonyl, cinnolinyl, coumarinyl, decahydroisoquinolinyl, dihydrobenzisothiazinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, imidazolinyl, indolinyl, isobenzotetrahydrofuranyl, isobenzotetrahydrothienyl, isochromanyl, isocoumarinyl, isoindolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, tetrahydroquinolinyl, and 1,3,5-trithianyl. In certain embodiments, the heterocyclyl may also be optionally substituted with one or more substituents Q as described herein.

The terms "halogen", "halide" and "halo" refer to fluorine, chlorine, bromine, and/or iodine.

The term "optionally substituted" is intended to mean that a group or substituent, such as an alkyl, alkylene, heteroalkylene, alkenyl, alkenylene, heteroalkenylene, alkynyl, cycloalkyl, cycloalkenyl, aryl, aralkyl, heteroaryl, heteroaryl-$C_{1-6}$ alkyl, and heterocyclyl group, may be substituted with one or more substituents Q, each of which is independently selected from, e.g., (a) oxo (=O), halo, cyano (-CN), and nitro ($-NO_2$); (b) $C_{1-6}$ alkyl, $C_{2-6}$ alkenyl, $C_{2-6}$ alkynyl, $C_{3-10}$

cycloalkyl, $C_{6-14}$ aryl, $C_{7-15}$ aralkyl, heteroaryl, and heterocyclyl, each of which is further optionally substituted with one or more, in one embodiment, one, two, three, four, or five, substituents $Q^a$; and (c) $-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^eS(O)_2R^h$, $-NR^aS(O)NR^bR^c$, $-NR^aS(O)_2NR^bR^c$, $-P(O)R^aR^d$, $-P(O)(OR^a)R^d$, $-P(O)(OR^a)(OR^d)$, $-SR^a$, $-S(O)R^a$, $-S(O)_2R^a$, $-S(O)NR^bR^c$, and $-S(O)_2NR^bR^c$, wherein each $R^a$, $R^b$, $R^c$, and $R^d$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is optionally substituted with one or more, in one embodiment, one, two, three, or four substituents $Q^a$; or (iii) $R^b$ and $R^c$ together with the N atom to which they are attached form heteroaryl or heterocyclyl, optionally substituted with one or more, in one embodiment, one, two, three, or four, substituents $Q^a$. As used herein, all groups that can be substituted are "optionally substituted," unless otherwise specified.

In one embodiment, each substituent $Q^a$ is independently selected from the group consisting of (a) oxo, cyano, halo, and nitro; and (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl; and (c) $-C(O)R^e$, $-C(O)OR^e$, $-C(O)NR^fR^g$, $-C(NR^e)NR^fR^g$, $-OR^e$, $-OC(O)R^e$, $-OC(O)OR^e$, $-OC(O)NR^fR^g$, $-OC(=NR^e)NR^fR^g$, $-OS(O)R^e$, $-OS(O)_2R^e$, $-OS(O)NR^fR^g$, $-OS(O)_2NR^fR^g$, $-NR^fR^g$, $-NR^eC(O)R^h$, $-NR^eC(O)OR^h$, $-NR^eC(O)NR^fR^g$, $-NR^eC(=NR^h)NR^fR^g$, $-NR^eS(O)R^h$, $-NR^eS(O)_2R^h$, $-NR^eS(O)NR^fR^g$, $-NR^eS(O)_2NR^fR^g$, $-P(O)R^eR^h$, $-P(O)(OR^e)R^h$, $-P(O)(OR^e)(OR^h)$, $-SR^e$, $-S(O)R^e$, $-S(O)_2R^e$, $-S(O)NR^fR^g$, and $-S(O)_2NR^fR^g$; wherein each $R^e$, $R^f$, $R^g$, and $R^h$ is independently (i) hydrogen, $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (ii) $R^f$ and $R^g$ together with the N atom to which they are attached form heteroaryl or heterocyclyl.

In certain embodiments, "optically active" and "enantiomerically active" refer to a collection of molecules, which has an enantiomeric excess of no less than about 50%, no less than about 70%, no less than about 80%, no less than about 90%, no less than about 91%, no less than about 92%, no less than about 93%, no less than about 94%, no less than about 95%, no less than about 96%, no less than about 97%, no less than about 98%, no less than about 99%, no less than about 99.5%, or no less than about 99.8%. In certain embodiments, the compound comprises about 95% or more of the desired enantiomer and about 5% or less of the less preferred enantiomer based on the total weight of the racemate in question.

In describing an optically active compound, the prefixes R and S are used to denote the absolute configuration of the molecule about its chiral center(s). The (+) and (-) are used to denote the optical rotation of the compound, that is, the direction in which a plane of polarized light is rotated by the optically active compound. The (-) prefix indicates that the compound is levorotatory, that is, the compound rotates the plane of polarized light to the left or counterclockwise. The (+) prefix indicates that the compound is dextrorotatory, that is, the compound rotates the plane of polarized light to the right or clockwise. However, the sign of optical rotation, (+) and (-), is not related to the absolute configuration of the molecule, R and S.

The phrase "an enantiomer, a mixture of enantiomers, a mixture of two or more diastereomers, or an isotopic variant thereof; or a pharmaceutically acceptable salt, solvate, or hydrate thereof" has the same meaning as the phrase "an enantiomer, a mixture of enantiomers, a mixture of two or more diastereomers, or an isotopic variant of the compound referenced therein; or a pharmaceutically acceptable salt, solvate, or hydrate of the compound referenced therein; or a pharmaceutically acceptable salt, solvate, or hydrate of an enantiomer, a mixture of enantiomers, a mixture of two or more diastereomers, or an isotopic variant of the compound referenced therein."

The term "solvate" refers to a complex or aggregate formed by one or more molecules of a solute, e.g., a compound described herein, and one or more molecules of a solvent, which present in a stoichiometric or non-stoichiometric amount. Suitable solvents include, but are not limited to, water, methanol, ethanol, *n*-propanol, isopropanol, and acetic acid. In certain embodiments, the solvent is pharmaceutically acceptable. In one embodiment, the complex or aggregate is in a crystalline form. In another embodiment, the complex or aggregate is in a noncrystalline form. Where the solvent is water, the solvate is a hydrate. Examples of hydrates include, but are not limited to, a hemihydrate, monohydrate, dihydrate, trihydrate, tetrahydrate, and pentahydrate.

COMPOUNDS

Disclosed herein are PI3K inhibitors of Formula (I):

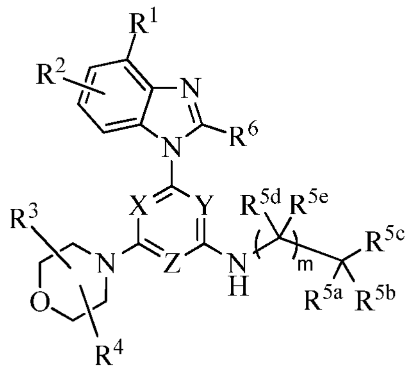

Formula (I)

or an enantiomer, a mixture of enantiomers, a mixture of two or more diastereomers, or an isotopic variant thereof; or a pharmaceutically acceptable salt, solvate, or hydrate thereof; wherein:

X, Y, and Z are each independently N or $CR^X$, with the proviso that at least two of X, Y, and Z are nitrogen atoms; where $R^X$ is hydrogen or $C_{1-6}$ alkyl;

$R^1$ and $R^Z$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; wherein each $R^{1a}$, $R^{1b}$, $R^{1c}$, and $R^{1d}$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, - $C(NR^{1a})NR^{1b}R^{1c}$, $-OR^{1a}$, $-OC(O)R^{1a}$, $-OC(O)OR^{1a}$, $-C(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, - $NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, - $NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, - $OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$,$-OS(O)_2R^{1a}$, - $OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, - $NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, - $SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$,$-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$,$-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $_S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl, heteroaryl, and heterocyclyl in $R^1$, $R^2$, $R^3$, $R^4$, $R^6$, $R^X$, $R^{1a}$, $R^{1b}$, $R^{1c}$, $R^{1d}$, $R^{5a}$, $R^{5b}$, $R^{5c}$, $R^{5d}$, $R^{5e}$, $R^{5f}$, and $R^{5g}$ is optionally substituted with one or more, in one embodiment, one, two, three, four, or five substituents Q, wherein each substituent Q is independently selected from (a) oxo, cyano, halo, and nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl, each of which is further optionally substituted with one or more, in one embodiment, one, two, three, or four, substituents $Q^a$; and (c) -C(O)$R^a$, -C(O)O$R^a$, - C(O)N$R^bR^c$, -C(N$R^a$)N$R^bR^c$, -O$R^a$, -OC(O)$R^a$, -OC(O)O$R^a$, -OC(O)N$R^bR^c$, -OC(=N$R^a$)N$R^bR^c$, - OS(O)$R^a$, -OS(O)$_2R^a$, -OS(O)N$R^bR^c$, -OS(O)$_2$N$R^bR^c$, -N$R^bR^c$, -N$R^a$C(O)$R^d$, -N$R^a$C(O)O$R^d$, - N$R^a$C(O)N$R^bR^c$, -N$R^a$C(=N$R^d$)N$R^bR^c$, -N$R^a$S(O)$R^d$, -N$R^a$S(O)$_2R^d$, -N$R^a$S(O)N$R^bR^c$, - N$R^a$S(O)$_2$N$R^bR^c$, -S$R^a$, -S(O)$R^a$, -S(O)$_2R^a$, -S(O)N$R^bR^c$, and -S(O)$_2$N$R^bR^c$, wherein each $R^a$, $R^b$, $R^c$, and $R^d$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is further optionally substituted with one or more, in one embodiment, one, two, three, or four, substituents $Q^a$; or (iii) $R^b$ and $R^c$ together with the N atom to which they are attached form heterocyclyl, which is further optionally substituted with one or more, in one embodiment, one, two, three, or four substituents $Q^a$;

wherein each $Q^a$ is independently selected from the group consisting of (a) oxo, cyano, halo, and nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl; and (c) -C(O)$R^e$, -C(O)O$R^e$, -C(O)N$R^fR^g$, -C(N$R^e$)N$R^fR^g$, -O$R^e$, -OC(O)$R^e$, - OC(O)O$R^e$, -OC(O)N$R^fR^g$, -OC(=N$R^e$)N$R^fR^g$, -OS(O)$R^e$, -OS(O)$_2R^e$, -OS(O)N$R^fR^g$, - OS(O)$_2$N$R^fR^g$, -N$R^fR^g$, -N$R^e$C(O)$R^h$, -N$R^e$C(O)O$R^h$, -N$R^e$C(O)N$R^fR^g$, -N$R^e$C(=N$R^h$)N$R^fR^g$, - N$R^e$S(O)$R^h$, -N$R^e$S(O)$_2R^h$, -N$R^e$S(O)N$R^fR^g$, -N$R^e$S(O)$_2$N$R^fR^g$, -S$R^e$, -S(O)$R^e$, -S(O)$_2R^e$, - S(O)N$R^fR^g$, and -S(O)$_2$N$R^fR^g$; wherein each $R^e$, $R^f$, $R^g$, and $R^h$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^f$ and $R^g$ together with the N atom to which they are attached form heterocyclyl; or wherein two substituents Q that are adjacent to each other optionally form a $C_{3-10}$ cycloalkenyl, $C_{6-14}$ aryl, heteroaryl, or heterocyclyl, each optionally substituted with one, two, three, or four substituents $Q^a$.

In one embodiment of a compound of Formula (I),

X, Y, and Z are each independently N or $CR^{x,}$ with the proviso that at least two of X, Y, and Z are nitrogen atoms; where $R^X$ is hydrogen or $C_{1-6}$ alkyl;

$R^1$ and $R^2$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - C(O)$R^{1a}$, -C(O)O$R^{1a}$, -C(O)N$R^{1b}R^{1c}$, -C(N$R^{1a}$)N$R^{1b}R^{1c}$, -O$R^{1a}$ , -OC(O)$R^{1a}$, -OC(O)O$R^{1a}$, - OC(O)N$R^{1b}R^{1c}$, -OC(=N$R^{1a}$)N$R^{1b}R^{1c}$, -OS(O)$R^{1a}$, -OS(O)$_2R^{1a}$, -OS(O)N$R^{1b}R^{1c}$, -OS(O)$_2$N$R^{1b}R^{1c}$, - N$R^{1b}R^{1c}$, -N$R^{1a}$C(O)$R^{1d}$, -N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, - N$R^{1a}$S(O)$R^{1d}$, -N$R^{1a}$S(O)$_2R^{1d}$, -N$R^{1a}$S(O)N$R^{1b}R^{1c}$, -N$R^{1a}$S(O)$_2$N$R^{1b}R^{1c}$, -S$R^{1a}$, -S(O)$R^{1a}$, -S(O)$_2R^{1a}$, -S(O)N$R^{1b}R^{1c}$, or -S(O)$_2$N$R^{1b}R^{1c}$; wherein each $R^{1a}$, $R^{1b}$, $R^{1c}$, and $R^{1d}$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) -C(O)$R^{1a}$, -C(O)O$R^{1a}$, -C(O)N$R^{1b}R^{1c}$, - C(N$R^{1a}$)N$R^{1b}R^{1c}$, -O$R^{1a}$, -OC(O)$R^{1a}$, -OC(O)O$R^{1a}$, -OC(O)N$R^{1b}R^{1c}$, -OC(=N$R^{1a}$)N$R^{1b}R^{1c}$, - OS(O)$R^{1a}$, -OS(O)$_2R^{1a}$, -OS(O)N$R^{1b}R^{1c}$, -OS(O)$_2$N$R^{1b}R^{1c}$, -N$R^{1b}R^{1c}$, -N$R^{1a}$C(O)$R^{1d}$, - N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, -N$R^{1a}$S(O)$R^{1d}$, -N$R^{1a}$S(O)$_2R^{1d}$, - N$R^{1a}$S(O)N$R^{1b}R^{1c}$, -N$R^{1a}$S(O)$_2$N$R^{1b}R^{1c}$, -S$R^{1a}$, -S(O)$R^{1a}$, -S(O)$_2R^{1a}$, -S(O)N$R^{1b}R^{1c}$, or - S(O)$_2$N$R^{1b}R^{1c}$,

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) -C(O)$R^{1a}$, -C(O)O$R^{1a}$, -C(O)N$R^{1b}R^{1c}$, -C(N$R^{1a}$)N$R^{1b}R^{1c}$, - O$R^{1a}$, -OC(O)$R^{1a}$, -OC(O)O$R^{1a}$, -OC(O)N$R^{1b}R^{1c}$, -OC(=N$R^{1a}$)N$R^{1b}R^{1c}$, -OS(O)$R^{1a}$, -OS(O)$_2R^{1a}$, - OS(O)N$R^{1b}R^{1c}$, -OS(O)$_2$N$R^{1b}R^{1c}$, -N$R^{1b}R^{1c}$, -N$R^{1a}$C(O)$R^{1d}$, -N$R^{1a}$C(O)O$R^{1d}$, -N$R^{1a}$C(O)N$R^{1b}R^{1c}$, - N$R^{1a}$C(=N$R^{1d}$)N$R^{1b}R^{1c}$, -N$R^{1a}$S(O)$R^{1d}$, -N$R^{1a}$S(O)$_2R^{1d}$, -N$R^{1a}$S(O)N$R^{1b}R^{1c}$, -N$R^{1a}$S(O)$_2$N$R^{1b}R^{1c}$, - S$R^{1a}$, -S(O)$R^{1a}$, -S(O)$_2R^{1a}$, -S(O)N$R^{1b}R^{1c}$, or -S(O)$_2$N$R^{1b}R^{1c}$;

$R^{5c}$ is -($CR^{5f}R^{5g}$),($C_{6-14}$ aryl) or -($CR^{5f}R^{5g}$)$_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) -C(O)$R^{1a}$, - C(O)O$R^{1a}$, -C(O)N$R^{1b}R^{1c}$, -C(N$R^{1a}$)N$R^{1b}R^{1c}$, -O$R^{1a}$, -OC(O)$R^{1a}$, -OC(O)O$R^{1a}$, -OC(O)N$R^{1b}R^{1c}$, - OC(=N$R^{1a}$)N$R^{1b}R^{1c}$, -OS(O)$R^{1a}$,-OS(O)$_2R^{1a}$, -OS(O)N$R^{1b}R^{1c}$, -OS(O)$_2$N$R^{1b}R^{1c}$, -N$R^{1b}R^{1c}$, - N$R^{1a}$C(O)$R^{1d}$, -N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, -N$R^{1a}$S(O)$R^{1d}$, - N$R^{1a}$S(O)$_2R^{1d}$, -N$R^{1a}$S(O)N$R^{1b}R^{1c}$, -N$R^{1a}$S(O)$_2$N$R^{1b}R^{1c}$, -S$R^{1a}$, -S(O)$R^{1a}$, -S(O)$_2R^{1a}$, -S(O)N$R^{1b}R^{1c}$, or -S(O)$_2$N$R^{1b}R^{1c}$; $R^{5f}$ and $R^{5g}$ are each independently (a) hydrogen or halo; (b) $C_{1-6}$ alkyl, $C_{2-6}$

alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$,$-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl, heteroaryl, and heterocyclyl is optionally substituted with one or more, in one embodiment, one, two, three, four, or five substituents Q as defined herein.

In another embodiment of a compound of Formula (I),

X, Y, and Z are each independently N or $CR^X$, with the proviso that at least two of X, Y, and Z are nitrogen atoms; where $R^X$ is hydrogen or $C_{1-6}$ alkyl;

$R^1$ and $R^2$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; wherein each $R^{1a}$, $R^{1b}$, $R^{1c}$, and $R^{1d}$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, - $C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, - $OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, - $NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, - $NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or - $S(O)_2NR^{1b}R^{1c}$,

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, - $OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, - $OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, - $NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, - $SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$,$-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl,

heteroaryl, and heterocyclyl is optionally substituted with one or more, in one embodiment, one, two, three, four, or five substituents Q as defined herein.

In yet another embodiment of a compound of Formula (I),

X, Y, and Z are each independently N or $CR^X$, with the proviso that at least two of X, Y, and Z are nitrogen atoms; where $R^X$ is hydrogen or $C_{1-6}$ alkyl;

$R^1$ and $R^2$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; wherein each $R^{1a}$, $R^{1b}$, $R^{1c}$, and $R^{1d}$ is independently (i) hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, - $OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, - $OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, - $NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, - $SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, - $OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, - $OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, - $NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, - $SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, - $C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, - $OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, - $NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, - $NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl, heteroaryl, and heterocyclyl is optionally substituted with one or more, in one embodiment, one, two, three, four, or five substituents Q as defined herein.

In still another embodiment of a compound of Formula (I),

X, Y, and Z are N;

$R^1$ and $R^2$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) - $C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, - $OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, - $NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, - $NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; wherein each $R^{1a}$, $R^{1b}$, $R^{1c}$, and $R^{1d}$ is independently (i)

hydrogen; (ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (iii) $R^{1b}$ and $R^{1c}$ together with the N atom to which they are attached form heterocyclyl;

$R^3$ and $R^4$ are each independently hydrogen or $C_{1-6}$ alkyl; or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;

$R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$,

$R^{5b}$ is (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, or heteroaryl; or (c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;

$R^{5d}$ and $R^{5e}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or (d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;

m is 0 or 1; and

n is 0, 1, 2, 3, or 4;

wherein each alkyl, alkylene, heteroalkylene, alkenyl, alkenylene, heteroalkenylene, alkynyl, cycloalkyl, aryl, aralkyl, heteroaryl, and heterocyclyl is optionally substituted with one or more, in one embodiment, one, two, three, four, or five substituents Q as defined herein.

Synthesis of compounds of Formula (I) is described in US Patent No. 9,056,852 B2.

Also described herein is a compound of Formula (IX):

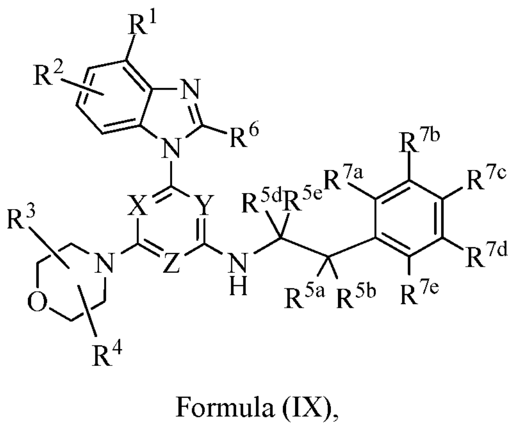

Formula (IX),

or an enantiomer, a mixture of enantiomers, a mixture of two or more diastereomers, or an isotopic variant thereof; or a pharmaceutically acceptable salt, solvate, or hydrate thereof; wherein:

$R^{7a}$, $R^{7b}$, $R^{7c}$, $R^{7d}$, and $R^{7e}$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is optionally substituted with one, two, three, or four substituents $Q^a$; or (c) $-C(O)R^a$,$-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$,$-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$,$-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$, or $-S(O)_2NR^bR^c$; or

two of $R^{7a}$, $R^{7b}$, $R^{7c}$, $R^{7d}$, and $R^{7e}$ that are adjacent to each other form $C_{3-10}$ cycloalkenyl, $C_{6-14}$ aryl, heteroaryl, or heterocyclyl, each optionally substituted with one, two, three, or four substituents $Q^a$; and

$R^1$, $R^2$, $R^3$, $R^4$, $R^6$, $R^{1a}$, $R^{1b}$, $R^{1c}$, $R^{1d}$, $R^{5a}$, $R^{5b}$, $R^{5d}$, $R^{5e}$, X, Y, and Z are each as defined herein.

Synthesis of compounds Formula (IX) is described in US Patent No. 9,056,852 B2.

In one embodiment, the compound of Formula (IX) has the structure of Formula (IXa):

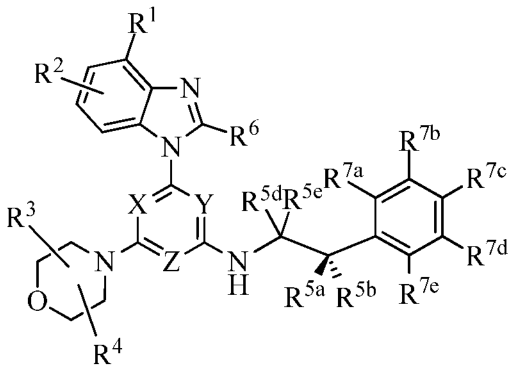

Formula (IXa),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5d}$, $R^{5e}$, $R^{7a}$, $R^{7b}$, $R^{7c}$, $R^{7d}$, $R^{7e}$, X, Y, and Z are each as defined herein.

Synthesis of compounds of Formula (IXa) is described in US Patent No. 9,056,852 B2.

In another embodiment, the compound of Formula (IX) has the structure of Formula (IXb):

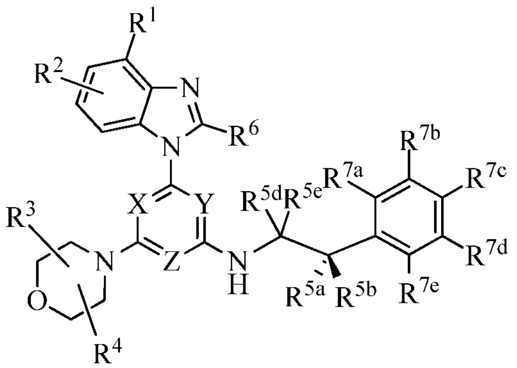

Formula (IXb),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5d}$, $R^{5e}$, $R^{7a}$, $R^{7b}$, $R^{7c}$, $R^{7d}$, $R^{7e}$, X, Y, and Z are each as defined herein.

Synthesis of compounds of Formula (IXb) is described in US Patent No. 9,056,852 B2.

In certain embodiments of compounds of Formula (IX), (IXa), or (IXb), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; in certain embodiments, one of $R^{7a}$, $R^{7b}$, $R^{7c}$, $R^{7d}$, and $R^{7e}$ is $C_{6-14}$ aryl, e.g., phenyl, optionally substituted with one, two, three, or four substituents $Q^a$; in certain embodiments, one of $R^{7a}$, $R^{7b}$, $R^{7c}$, $R^{7d}$, and $R^{7e}$ is heteroaryl, e.g., 5-membered or 6-membered heteroaryl, optionally substituted with one, two, three, or four substituents $Q^a$; in certain embodiments, one of $R^{7a}$, $R^{7b}$, $R^{7c}$, $R^{7d}$, and $R^{7e}$ is heterocyclyl, e.g., 5-membered or 6-membered heterocyclyl, optionally substituted with one, two, three, or four substituents $Q^a$; in certain embodiments, one of $R^{7a}$, $R^{7b}$, $R^{7c}$, $R^{7d}$, and $R^{7e}$ is

phenyl, imidazolyl, pyrozolyl, pyridinyl, piperidinyl, or piperazinyl, each optionally substituted with one, two, three, or four substituents $Q^a$; in certain embodiments, one of $R^{7a}$, $R^{7b}$, $R^{7c}$, $R^{7d}$, and $R^{7e}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compounds of Formula (IX), (IXa), or (IXb), $R^{7a}$ is $C_{6-14}$ aryl, heteroaryl, or heterocyclyl, each of which is optionally substituted with one, two, three, or four substituents $Q^a$; in certain embodiments, $R^{7a}$ is $C_{6-14}$ aryl, e.g., phenyl, optionally substituted with one, two, three, or four substituents $Q^a$; in certain embodiments, $R^{7a}$ is heteroaryl, e.g., 5-membered or 6-membered heteroaryl, optionally substituted with one, two, three, or four substituents $Q^a$; in certain embodiments, $R^{7a}$ is heterocyclyl, e.g., 5-membered or 6-membered heterocyclyl, optionally substituted with one, two, three, or four substituents $Q^a$; in certain embodiments, $R^{7a}$ is phenyl, imidazolyl, pyrozolyl, pyridinyl, piperidinyl, or piperazinyl, each optionally substituted with one, two, three, or four substituents $Q^a$; in certain embodiments, $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; in certain embodiments, $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compounds of Formula (IX), (IXa), or (IXb),

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently hydrogen, halo, $C_{1-6}$ alkyl, optionally substituted with one, two, three, four, or five substituents Q;
$R^{5d}$ and $R^{5e}$ are each independently $C_{1-6}$ alkyl, optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or $CR^x$, with the proviso that at least two of X, Y, and Z are N; where $R^x$ is a hydrogen or $C_{1-6}$ alkyl, optionally substituted with one, two, three, or four substituents $Q^a$.

In certain embodiments of compounds of Formula (IX), (IXa), or (IXb),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are hydrogen;
$R^{5d}$ and $R^{5e}$ are each independently $C_{1-6}$ alkyl;

$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compounds of Formula (IX), (IXa), or (IXb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is $C_{6-14}$ aryl, monocyclic heteroaryl, or monocyclic heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compounds of Formula (IX), (IXa), or (IXb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, 5- or 6-membered heteroaryl, or 5- or 6-membered heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (IX), (IXa), or (IXb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (IX), (IXa), or (IXb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, imidazolyl, pyrozolyl, pyridinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

Also described herein is a compound of Formula (X):

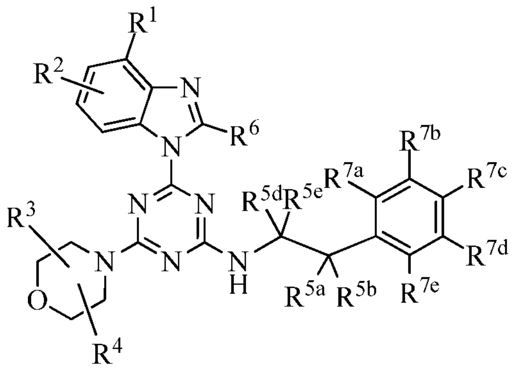

Formula (X),

or an enantiomer, a mixture of enantiomers, a mixture of two or more diastereomers, or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5d}$, $R^{5e}$, $R^{7a}$, $R^{7b}$, $R^{7c}$, $R^{7d}$, and $R^{7e}$ are each as defined herein.

Synthesis of compounds of Formula (X) is described in US Patent No. 9,056,852 B2.

In one embodiment, the compound of Formula (X) has the structure of Formula (Xa):

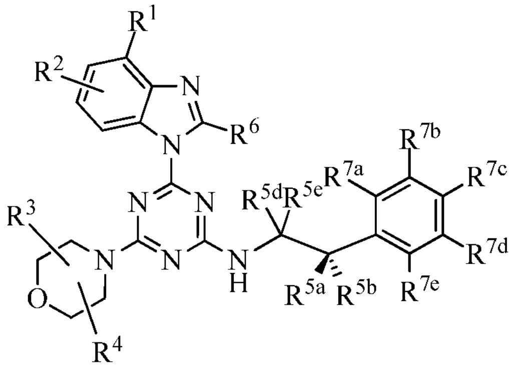

Formula (Xa),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5d}$, $R^{5e}$, $R^{7a}$, $R^{7b}$, $R^{7c}$, $R^{7d}$, and $R^{7e}$ are each as defined herein.

Synthesis of compounds of Formula (Xa) is described in US Patent No. 9,056,852 B2.

In another embodiment, the compound of Formula (X) has the structure of Formula (Xb):

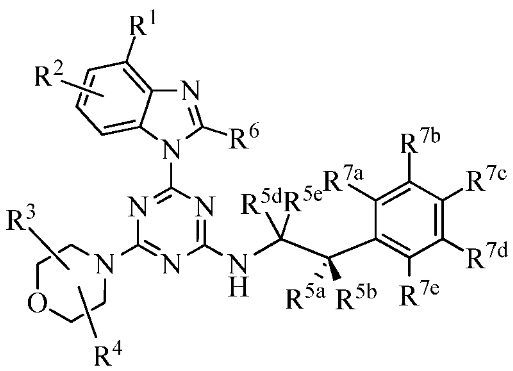

Formula (Xb),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5d}$, $R^{5e}$, $R^{7a}$, $R^{7b}$, $R^{7c}$, $R^{7d}$, and $R^{7e}$ are each as defined herein.

Synthesis of compounds of Formula (Xb) is described in US Patent No. 9,056,852 B2.

In certain embodiments of compound of Formula (X), (Xa), or (Xb), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; in certain embodiments, one of $R^{7a}$, $R^{7b}$, $R^{7c}$, $R^{7d}$, and $R^{7e}$ is $C_{6-14}$ aryl, e.g., phenyl, optionally substituted with one, two, three, or four substituents $Q^a$; in certain embodiments, one of $R^{7a}$, $R^{7b}$, $R^{7c}$, $R^{7d}$, and $R^{7e}$ is heteroaryl, e.g., 5-membered or 6-membered heteroaryl, optionally substituted with one, two, three, or four substituents $Q^a$; in certain embodiments, one of $R^{7a}$, $R^{7b}$, $R^{7c}$, $R^{7d}$, and $R^{7e}$ is heterocyclyl, e.g., 5-membered or 6-membered heterocyclyl, optionally substituted with one, two, three, or four substituents $Q^a$; in certain embodiments, one of $R^{7a}$, $R^{7b}$, $R^{7c}$, $R^{7d}$, and $R^{7e}$ is phenyl, imidazolyl, pyrozolyl, pyridinyl, piperidinyl, or piperazinyl, each optionally substituted with one, two, three, or four substituents $Q^a$; in certain embodiments, one of $R^{7a}$, $R^{7b}$, $R^{7c}$, $R^{7d}$, and $R^{7e}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compound of Formula (X), (Xa), or (Xb), $R^{7a}$ is $C_{6-14}$ aryl, heteroaryl, or heterocyclyl, each of which is optionally substituted with one or more substituents $Q^a$; in certain embodiments, $R^{7a}$ is $C_{6-14}$ aryl, e.g., phenyl, optionally substituted with one, two, three, or four substituents $Q^a$; in certain embodiments, $R^{7a}$ is heteroaryl, e.g., 5-membered or 6-membered heteroaryl, optionally substituted with one, two, three, or four substituents $Q^a$; in certain embodiments, $R^{7a}$ is heterocyclyl, e.g., 5-membered or 6-membered heterocyclyl, optionally substituted with one, two, three, or four substituents $Q^a$; in certain embodiments, $R^{7a}$ is phenyl, imidazolyl, pyrozolyl, pyridinyl, piperidinyl, or piperazinyl, each optionally substituted with one, two, three, or four substituents $Q^a$; in certain embodiments, $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; in certain embodiments, $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compound of Formula (X), (Xa), or (Xb),

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently hydrogen, halo, $C_{1-6}$ alkyl, optionally substituted with one, two, three, four, or five substituents Q;
$R^{5d}$ and $R^{5e}$ are each independently $C_{1-6}$ alkyl, optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In certain embodiments of compound of Formula (X), (Xa), or (Xb),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are hydrogen;
$R^{5d}$ and $R^{5e}$ are each independently $C_{1-6}$ alkyl;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In certain embodiments of compound of Formula (X), (Xa), or (Xb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is $C_{6-14}$ aryl, monocyclic heteroaryl, or monocyclic heterocyclyl, each of which is optionally substituted with one, two, three, or four substituents $Q^a$; and
$R^{7b}$, $R^{7c}$, $R^{7d}$, and $R^{7e}$ are hydrogen.

In certain embodiments of compound of Formula (X), (Xa), or (Xb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, 5- or 6-membered heteroaryl, or 5- or 6-membered heterocyclyl, each of which is optionally substituted with one, two, three, or four substituents $Q^a$; and
$R^{7b}$, $R^{7c}$, $R^{7d}$, and $R^{7e}$ are hydrogen.

In certain embodiments of compound of Formula (X), (Xa), or (Xb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

In certain embodiments of compound of Formula (X), (Xa), or (Xb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are hydrogen;
$R^{5d}$ and $R^{5e}$ are methyl;
$R^{7a}$ is phenyl, imidazolyl, pyrozolyl, pyridinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

Also described herein is a compound of Formula (XI):

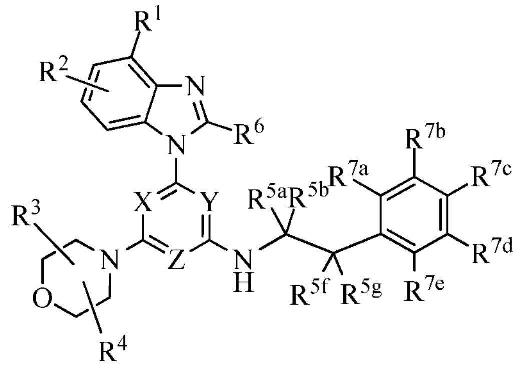

Formula (XI),

or an enantiomer, a mixture of enantiomers, a mixture of two or more diastereomers, or an isotopic variant thereof; or a pharmaceutically acceptable salt, solvate, or hydrate thereof; wherein:

$R^{7a}$, $R^{7b}$, $R^{7c}$, $R^{7d}$, and $R^{7e}$ are each independently (a) hydrogen, cyano, halo, or nitro; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is optionally substituted with one, two, three, or four substituents $Q^a$; or (c) $-C(O)R^a$,$-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$,$-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$,$-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$, or $-S(O)_2NR^bR^c$; or two of $R^{7a}$, $R^{7b}$, $R^{7c}$, $R^{7d}$, and $R^{7e}$ that are adjacent to each other form $C_{3-10}$ cycloalkenyl, $C_{6-14}$ aryl, heteroaryl, or heterocyclyl, each optionally substituted with one, two, three, or four substituents $Q^a$; and

$R^1$, $R^2$, $R^3$, $R^4$, $R^6$, $R^{1a}$, $R^{1b}$, $R^{1c}$, $R^{1d}$, $R^{5a}$, $R^{5b}$, $R^{5f}$, $R^{5g}$, X, Y, and Z are each as defined herein.

Synthesis of compounds of Formula (XI) is described in US Patent No. 9,056,852 B2.

In one embodiment, the compound of Formula (XI) has the structure of Formula (XIa):

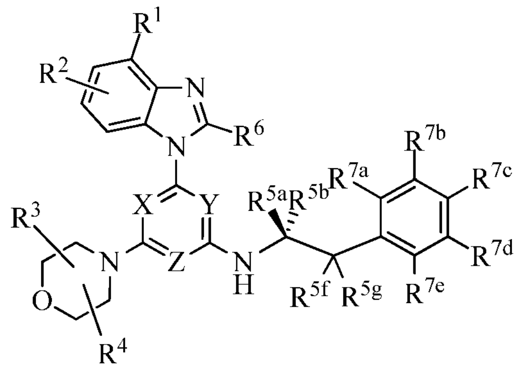

Formula (XIa),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5f}$, $R^{5g}$, $R^{7a}$, $R^{7b}$, $R^{7c}$, $R^{7d}$, $R^{7e}$, X, Y, and Z are each as defined herein.

Synthesis of compounds of Formula (XIa) is described in US Patent No. 9,056,852 B2.

In another embodiment, the compound of Formula (XI) has the structure of Formula (XIb):

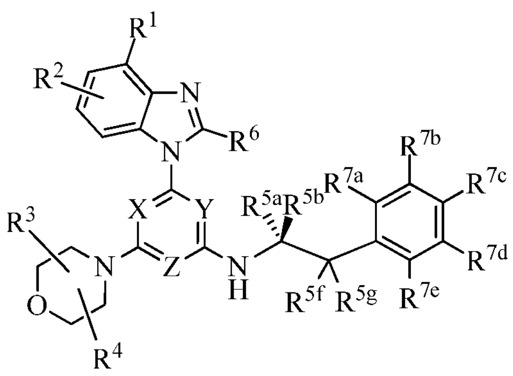

Formula (XIb),

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{5f}$, $R^{5g}$, $R^{7a}$, $R^{7b}$, $R^{7c}$, $R^{7d}$, $R^{7e}$, X, Y, and Z are each as defined herein.

Synthesis of compounds of Formula (XIb) is described in US Patent No. 9,056,852 B2.

In certain embodiments of compound of Formula (XI), (XIa), or (XIb), $R^{5a}$ and $R^{5b}$ are each independently (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$,$-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$,$-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$,$-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$,$-SR^{1a}$, $-S(O)R^{1a}$, $-S(O)_2R^{1a}$, $-S(O)NR^{1b}R^{1c}$, or $-S(O)_2NR^{1b}R^{1c}$; and $R^1$, $R^2$, $R^3$, $R^4$, $R^{5f}$, $R^{5g}$, $R^6$, $R^{7a}$, $R^{7b}$, $R^{7c}$, $R^{7d}$, $R^{7e}$, X, Y, Z, $R^{1a}$, $R^{1b}$, $R^{1c}$, and $R^{1d}$ are defined herein elsewhere.

In certain embodiments of compound of Formula (XI), (XIa), or (XIb), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; in certain embodiments, one of $R^{7a}$, $R^{7b}$, $R^{7c}$, $R^{7d}$, and $R^{7e}$ is $C_{6-14}$ aryl, e.g., phenyl, optionally substituted with one, two, three, or four substituents $Q^a$; in certain embodiments, one of $R^{7a}$, $R^{7b}$, $R^{7c}$, $R^{7d}$, and $R^{7e}$ is heteroaryl, e.g., 5-membered or 6-membered heteroaryl, optionally substituted with one, two, three, or four substituents $Q^a$; in certain embodiments, one of $R^{7a}$, $R^{7b}$, $R^{7c}$, $R^{7d}$, and $R^{7e}$ is heterocyclyl, e.g., 5-membered or 6-membered heterocyclyl, optionally substituted with one, two, three, or four substituents $Q^a$; in certain embodiments, one of $R^{7a}$, $R^{7b}$, $R^{7c}$, $R^{7d}$, and $R^{7e}$ is phenyl, imidazolyl, pyrozolyl, pyridinyl, piperidinyl, or piperazinyl, each optionally substituted with one, two, three, or four substituents $Q^a$; in certain embodiments, one of $R^{7a}$, $R^{7b}$, $R^{7c}$, $R^{7d}$, and $R^{7e}$ is phenyl, imidazolyl, pyrozolyl, pyridinyl, pyrimidinyl, pyrrolidinyl, piperidinyl, or piperazinyl, each optionally substituted with one or more substituents $Q^a$; in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compound of Formula (XI), (XIa), or (XIb), $R^{7a}$ is $C_{6-14}$ aryl, heteroaryl, or heterocyclyl, each of which is optionally substituted with one, two, three, or four substituents $Q^a$; in certain embodiments, $R^{7a}$ is $C_{6-14}$ aryl, e.g., phenyl, optionally substituted with one, two, three, or four substituents $Q^a$; in certain embodiments, $R^{7a}$ is heteroaryl, e.g., 5-membered or 6-membered heteroaryl, optionally substituted with one, two, three, or four substituents $Q^a$; in certain embodiments, $R^{7a}$ is heterocyclyl, e.g., 5-membered or 6-membered heterocyclyl, optionally substituted with one, two, three, or four substituents $Q^a$; in certain embodiments, $R^{7a}$ is phenyl, imidazolyl, pyrozolyl, pyridinyl, piperidinyl, or piperazinyl, each optionally substituted with one, two, three, or four substituents $Q^a$; in certain embodiments, $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; in certain embodiments, $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and in certain embodiments, $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments of compound of Formula (XI), (XIa), or (XIb),

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently $C_{1-6}$ alkyl, optionally substituted with one, two, three, four, or five substituents Q;
$R^{5f}$ and $R^{5g}$ are each independently hydrogen, halo, $C_{1-6}$ alkyl, optionally substituted with one, two, three, four, or five substituents Q; or $R^{5f}$ and $R^{5g}$ together with the carbon atom to which they are attached form $C_{1-10}$ cycloalkyl or heterocyclyl, each of which is optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or $CR^x$, with the proviso that at least two of X, Y, and Z are N; where $R^x$ is a hydrogen or $C_{1-6}$ alkyl, optionally substituted with one, two, three, or four substituents $Q^a$.

In certain embodiments of compound of Formula (XI), (XIa), or (XIb),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are each independently $C_{1-6}$ alkyl;
$R^{5f}$ and $R^{5g}$ are each independently hydrogen or $C_{1-6}$ alkyl; or $R^{5f}$ and $R^{5g}$ together with the carbon atom to which they are attached form $C_{1-10}$ cycloalkyl;
$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (XI), (XIa), or (XIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{5f}$ and $R^{5g}$ are hydrogen; or $R^{5f}$ and $R^{5g}$ together with the carbon atom to which they are attached form cyclopropyl, cyclobutyl, cyclopentyl, or cyclohexyl;
$R^{7a}$ is $C_{6-14}$ aryl, monocyclic heteroaryl, or monocyclic heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (XI), (XIa), or (XIb),

$R^1$ is hydrogen or methoxy;
$R^2$ is hydrogen;
$R^3$ and $R^4$ are hydrogen;

$R^6$ is difluoromethyl;
$R^{5a}$ and $R^{5b}$ are methyl;
$R^{5f}$ and $R^{5g}$ are hydrogen; or $R^{5f}$ and $R^{5g}$ together with the carbon atom to which they are attached form cyclopropyl, cyclobutyl, cyclopentyl, or cyclohexyl;
$R^{7a}$ is phenyl, 5- or 6-membered heteroaryl, or 5- or 6-membered heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (XI), (XIa), or (XIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{5f}$ and $R^{5g}$ are hydrogen; or $R^{5f}$ and $R^{5g}$ together with the carbon atom to which they are attached form cyclopropyl, cyclobutyl, cyclopentyl, or cyclohexyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In certain embodiments of compound of Formula (XI), (XIa), or (XIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{5f}$ and $R^{5g}$ are hydrogen; or $R^{5f}$ and $R^{5g}$ together with the carbon atom to which they are attached form cyclopropyl, cyclobutyl, cyclopentyl, or cyclohexyl;
$R^{7a}$ is phenyl, imidazolyl, pyrozolyl, pyridinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

Also described herein is a compound of Formula (XVI):

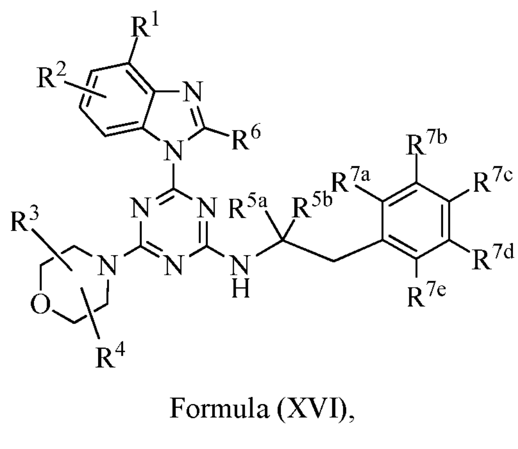

Formula (XVI),

or an enantiomer, a mixture of enantiomers, a mixture of two or more diastereomers, or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{7a}$, $R^{7b}$, $R^{7c}$, $R^{7d}$, and $R^{7e}$ are each as defined herein.
Synthesis of compounds of Formula (XVI) is described in US Patent No. 9,056,852 B2.

In one embodiment of a compound of Formula (XVI), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In another embodiment of a compound of Formula (XVI), one of $R^{7a}$, $R^{7b}$, $R^{7c}$, $R^{7d}$, and $R^{7e}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is heteroaryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is 5-membered or 6-membered heteroaryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In yet another embodiment of a compound of Formula (XVI), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In still another embodiment of a compound of Formula (XVI), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVI), $R^{7a}$ is $C_{6-14}$ aryl, heteroaryl, or heterocyclyl, each of which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In another embodiment of a compound of Formula (XVI), $R^{7a}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is 5-membered or 6-membered heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is heterocyclyl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In yet another embodiment of a compound of Formula (XVI), $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In yet another embodiment of a compound of Formula (XVI), $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVI),

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently $C_{1-6}$ alkyl, optionally substituted with one, two, three, four, or five substituents Q;
$R^{7a}$ is $C_{6-14}$ aryl, heteroaryl, or heterocyclyl, each of which is optionally substituted with one or more substituents $Q^a$; and
$R^{7b}$, $R^{7c}$, $R^{7d}$, and $R^{7e}$ are hydrogen.

In one embodiment of a compound of Formula (XVI),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are each independently $C_{1-6}$ alkyl;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVI),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{7a}$ is $C_{6-14}$ aryl, monocyclic heteroaryl, or monocyclic heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVI),

$R^1$ is hydrogen or methoxy;
$R^2$ is hydrogen;
$R^3$ and $R^4$ are hydrogen;
$R^6$ is difluoromethyl;

$R^{5a}$ and $R^{5b}$ are methyl;
$R^{7a}$ is phenyl, 5- or 6-membered heteroaryl, or 5- or 6-membered heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVI),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, four, or five substituents Q; and
$R^{7b}$, $R^{7c}$, $R^{7d}$, and $R^{7e}$ are hydrogen.

In one embodiment of a compound of Formula (XVI),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are methyl;
$R^{7a}$ is phenyl, imidazolyl, pyrozolyl, pyridinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

In one embodiment, the compound of Formula (XVI) has the structure of Formula (XVIa):

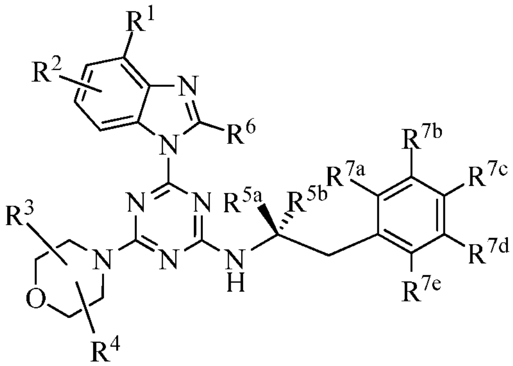

Formula (XVIa)

or an isotopic variant thereof; or a pharmaceutically acceptable salt, s solvate, or hydrate thereof; wherein $R^1$, $R^2$, $R^3$, $R^4$, $R^6$, $R^{5a}$, $R^{5b}$, $R^{7a}$, $R^{7b}$, $R^{7c}$, $R^{7d}$, and $R^{7e}$ are each as defined herein.

Synthesis of compounds of Formula (XVIa) is described in US Patent No. 9,056,852 B2.

In one embodiment of a compound of Formula (XVIa), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is heteroaryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is 5-membered or 6-membered heteroaryl, which is optionally substituted with one, two, three, or four substituents $Q^a$; and $R^1$, $R^2$, $R^3$, $R^4$,

$R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In one embodiment of a compound of Formula (XVIa), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIa), $R^{7a}$ is $C_{6-14}$ aryl, heteroaryl, or heterocyclyl, each of which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is 5-membered or 6-membered heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is heterocyclyl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa), $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl,

1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In one embodiment of a compound of Formula (XVIa), $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIa),

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently hydrogen or $C_{1-6}$ alkyl, optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIa),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are each independently hydrogen or $C_{1-6}$ alkyl;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIa),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is $C_{6-14}$ aryl, monocyclic heteroaryl, or monocyclic heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIa),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, 5- or 6-membered heteroaryl, or 5- or 6-membered heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIa),

$R^1$ is hydrogen or methoxy;
$R^2$ is hydrogen;
$R^3$ and $R^4$ are hydrogen;
$R^6$ is difluoromethyl;

$R^{5a}$ and $R^{5b}$ are each independently hydrogen or $C_{1-6}$ alkyl;
$R^{7a}$ is phenyl, imidazolyl, pyrozolyl, pyridinyl, pyrrolidinyl, piperidinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIa),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, imidazolyl, pyrozolyl, pyridinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

Synthesis of compounds of Formula (XVIa) is described in US Patent No. 9,056,852 B2.
In another embodiment, the compound of Formula (XVI) has the structure of Formula (XVIb) :

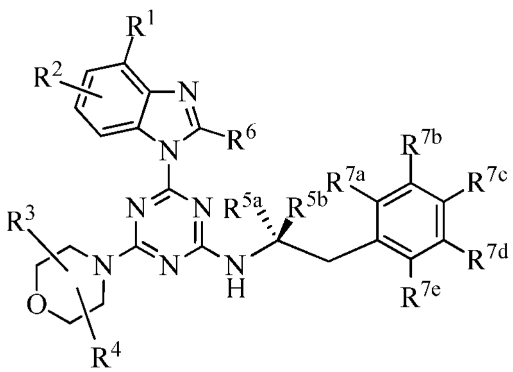

Formula (XVIb)

or an isotopic variant thereof; or a pharmaceutically acceptable salt, solvate, or hydrate thereof; wherein $R^1$, $R^2$, $R^3$, $R^4$, $R^6$, $R^{5a}$, $R^{5b}$, $R^{7a}$, $R^{7b}$, $R^{7c}$, $R^{7d}$, and $R^{7e}$ are each as defined herein.
In one embodiment of a compound of Formula (XVIb), one of $R^{7a}$, $R^{7b}$, $R^{7c}$, $R^{7d}$, and $R^{7e}$ is $C_{6-14}$ aryl, heteroaryl, or heterocyclyl, each of which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is heteroaryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is 5-membered or 6-membered heteroaryl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.
In one embodiment of a compound of Formula (XVIb), one of $R^{7a}$, $R^{7b}$, $R^{7c}$, $R^{7d}$, and $R^{7e}$ is phenyl, imidazolyl,

pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIb), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In one embodiment of a compound of Formula (XVIb), one of $R^{7a}$, $R^{7b}$, $R^{7c}$, $R^{7d}$, and $R^{7e}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, the remaining of $R^{7a}$, $R^{7b}$, $R^{7c}$, $R^{7d}$, and $R^{7e}$, X, Y, and Z are each as defined herein.

In one embodiment of a compound of Formula (XVIb), $R^{7a}$ is $C_{6-4}$ aryl, heteroaryl, or heterocyclyl, each of which is optionally substituted with one, two, three, four, or five substituents Q; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is $C_{6-14}$ aryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is 5-membered or 6-membered heteroaryl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is heterocyclyl, which is optionally substituted with one, two, three, or four substituents $Q^a$; and $R^1$, $R^2$, $R^3$, $R^4$, $R^6$, $R^{5a}$, $R^{5b}$, $R:^{7b}$, $R^{7c}$, $R^{7d}$, $R^{7e}$, X, Y, and Z are each as defined herein.

In one embodiment of a compound of Formula (XVIb), $R^{7a}$ is 5-membered or 6-membered heterocyclyl, which is optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is phenyl, imidazolyl, pyrozolyl, pyridinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb), $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In one embodiment of a compound of Formula (XVIb), $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-methylpyridin-4-yl, 2-methoxypyridin-4-yl, 1-methylpiperidin-4-yl, or 4-methylpiperazin-1-yl; and $R^1$, $R^2$, $R^3$, $R^4$, $R^6$, $R^{5a}$, $R^{5b}$, $R^{7b}$, $R^{7c}$, $R^{7d}$, $R^{7e}$, X, Y, and Z are each as defined herein.

In one embodiment of a compound of Formula (XVIb),

$R^1$ is hydrogen or $-OR^{1a}$, where $R^{1a}$ is $C_{1-6}$ alkyl, optionally substituted with one, two, three, four, or five substituents Q;
$R^2$ is hydrogen;

$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently hydrogen or $C_{1-6}$ alkyl, optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIb),

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are each independently hydrogen or $C_{1-6}$ alkyl;
$R^{7a}$ is $C_{6-14}$ aryl, heteroaryl, or heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{-6}$ alkyl;
$R^{7a}$ is $C_{6-14}$ aryl, monocyclic heteroaryl, or monocyclic heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, 5- or 6-membered heteroaryl, or 5- or 6-membered heterocyclyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIb),

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVIb),

$R^1$ is hydrogen or methoxy;
$R^2$ is hydrogen;
$R^3$ and $R^4$ are hydrogen;
$R^6$ is difluoromethyl;

$R^{5a}$ and $R^{5b}$ are each independently hydrogen or $C_{1-6}$ alkyl;
$R^{7a}$ is phenyl, imidazolyl, pyrozolyl, pyridinyl, piperidinyl, or piperazinyl, each of which is optionally substituted with one, two, three, or four substituents $Q^a$; and
$R^{7b}$, $R^{7c}$, $R^{7d}$, and $R^{7e}$ are hydrogen.

In one embodiment of a compound of Formula (XVI), (XVIa), or (XVIb), $R^{5a}$ and $R^{5b}$ are each independently (a) halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or (c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$; and $R^1$, $R^2$, $R^3$, $R^4$, $R^6$, $R^{7a}$, $R^{7b}$, $R^{7c}$, $R^{7d}$, $R^{7e}$, $R^{1a}$, $R^{1b}$, $R^{1c}$, and $R^{1d}$ are defined herein elsewhere.

In one embodiment of any of the formulae described herein,

$R^1$ is hydrogen or $-OR^{1a}$, where $R^{1a}$ is $C_{1-6}$ alkyl, optionally substituted with one, two, three, four, or five substituents Q;
$R^2$ is hydrogen;
$R^3$ and $R^4$ are hydrogen;
$R^6$ is $C_{1-6}$ alkyl, optionally substituted with one, two, three, four, or five substituents Q;
$R^{5a}$ and $R^{5b}$ are each independently hydrogen or $C_{1-6}$ alkyl optionally substituted with one, two, three, four, or five substituents Q;
$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or $CR^x$, with the proviso that at least two of X, Y, and Z are N; where $R^x$ is a hydrogen or $C_{1-6}$ alkyl, optionally substituted with one, two, three, or four substituents $Q^a$.

In one embodiment of any of the formulae described herein,

$R^1$ is hydrogen or methoxy;
$R^2$ is hydrogen;
$R^3$ and $R^4$ are hydrogen;
$R^6$ is $C_{1-6}$ alkyl, optionally substituted with one or more halo;
$R^{5a}$ and $R^{5b}$ are each independently hydrogen or $C_{1-6}$ alkyl;
$R^{7a}$ is $C_{6-14}$ aryl, heteroaryl, or heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH..

In one embodiment of any of the formulae described herein,

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is $C_{6-14}$ aryl, monocyclic heteroaryl, or monocyclic heterocyclyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In one embodiment of any of the formulae described herein,

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, 5- or 6-membered heteroaryl, or 5- or 6-membered heterocyclyl, each of which is optionally substituted

with one, two, three, or four substituents $Q^a$;
$R^{7b}$, $R^{7c}$, $R^{7d}$, and $R^{7e}$ are hydrogen; and
X, Y, and Z are each independently N or CH.

In one embodiment of any of the formulae described herein,

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, imidazolyl, pyrozolyl, pyridinyl, pyrimidinyl, pyrrolidinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

In one embodiment of any of the formulae described herein,

$R^1$ is hydrogen or methoxy;
$R^2$ is hydrogen;
$R^3$ and $R^4$ are hydrogen;
$R^6$ is difluoromethyl;
$R^{5a}$ and $R^{5b}$ are each independently hydrogen or $C_{1-6}$ alkyl;
$R^{7a}$ is phenyl, imidazolyl, pyrozolyl, pyridinyl, piperidinyl, or piperazinyl, each of which is optionally substituted with one, two, three, or four substituents $Q^a$;
$R^{7b}$, $R^{7c}$, $R^{7d}$, and $R^{7e}$ are hydrogen; and
X, Y, and Z are each independently N or CH.

The groups or variables, $R^1$, $R^2$, $R^3$, $R^4$, $R^6$, $R^{5a}$, $R^{5b}$, $R^{5c}$, $R^{5d}$, $R^{5e}$, $R^{5f}$, $R^{5g}$, $R^{7a}$, $R^{7b}$, $R^{7c}$, $R^{7d}$, $R^{7e}$, m, n, X, Y, and Z in Formulae described herein, e.g., Formulae (I), (IX), (X), (XI), (XVI), (Ia), (IXa), (Xa), (XIa), (XVIa), (Ib), (IXb), (Xb), (XIb), (XVIb) are further defined in the embodiments described herein. All combinations of the embodiments described herein for such groups and/or variables are within the scope of this disclosure.

In certain embodiments, $R^1$ is hydrogen. In certain embodiments, $R^1$ is cyano. In certain embodiments, $R^1$ is halo. In certain embodiments, $R^1$ is fluoro, chloro, bromo, or iodo. In certain embodiments, $R^1$ is nitro. In certain embodiments, $R^1$ is $C_{1-6}$ alkyl, optionally substituted with one, two, three, four, or five substituents Q as described herein. In certain embodiments, $R^1$ is $C_{2-6}$ alkenyl, optionally substituted with one, two, three, four, or five substituents Q as described herein. In certain embodiments, $R^1$ is $C_{2-6}$ alkynyl, optionally substituted with one, two, three, four, or five substituents Q as described herein. In certain embodiments, $R^1$ is $C_{3-10}$ cycloalkyl, optionally substituted with one, two, three, four, or five substituents Q as described herein. In certain embodiments, $R^1$ is $C_{6-14}$ aryl, optionally substituted with one, two, three, four, or five substituents Q as described herein. In certain embodiments, $R^1$ is $C_{7-15}$ aralkyl, optionally substituted with one, two, three, four, or five substituents Q as described herein. In certain embodiments, $R^1$ is heteroaryl, optionally substituted with one, two, three, four, or five substituents Q as described herein. In certain embodiments, $R^1$ is heterocyclyl, optionally substituted with one, two, three, four, or five substituents Q as described herein.

In certain embodiments, $R^1$ is $-C(O)R^{1a}$, wherein $R^{1a}$ is as defined herein. In certain embodiments, $R^1$ is $-C(O)OR^{1a}$, wherein $R^{1a}$ is as defined herein. In certain embodiments, $R^1$ is - $C(O)NR^{1b}R^{1c}$, wherein $R^{1b}$ and $R^{1c}$ are each as defined herein. In certain embodiments, $R^1$ is - $C(NR^{1a})NR^{1b}R^{1c}$, wherein $R^{1a}$, $R^{1b}$, and $R^{1c}$ are each as defined herein. In certain embodiments, $R^1$ is - $OR^{1a}$, wherein $R^{1a}$ is as defined herein. In certain embodiments, $R^1$ is $-O-C_{1-6}$ alkyl, wherein the alkyl is optionally substituted with one, two, three, four, or five substituents Q as described herein. In certain embodiments, $R^1$ is methoxy, ethoxy, propoxy, isopropoxy, or 3-dimethylaminopropoxy. In certain embodiments, $R^1$ is $-OC(O)R^{1a}$, wherein $R^{1a}$ is as defined herein. In certain embodiments, $R^1$ is $-OC(O)OR^{1a}$, wherein $R^{1a}$ is as defined herein. In certain embodiments, $R^1$ is $-OC(O)NR^{1b}R^{1c}$, wherein $R^{1b}$ and $R^{1c}$ are each as defined herein. In certain embodiments, $R^1$ is $-OC(=NR^{1a})NR^{1b}R^{1c}$, wherein $R^{1a}$, $R^{1b}$, and $R^{1c}$ are each as defined herein. In certain embodiments, $R^1$ is $-OS(O)R^{1a}$, wherein $R^{1a}$ is as defined herein. In certain embodiments, $R^1$ is $-OS(O)_2R^{1a}$, wherein $R^{1a}$ is as defined herein. In certain embodiments, $R^1$ is $-OS(O)NR^{1b}R^{1c}$, wherein $R^{1b}$ and $R^{1c}$ are each as defined herein. In certain embodiments, $R^1$ is $-OS(O)_2NR^{1b}R^{1c}$, wherein $R^{1b}$ and $R^{1c}$ are each as defined herein. In certain embodiments, $R^1$ is $-NR^{1b}R^{1c}$, wherein $R^{1b}$ and $R^{1c}$ are each as defined herein. In certain embodiments, $R^1$ is $-NR^{1a}C(O)R^{1d}$, wherein $R^{1a}$ and $R^{1d}$ are each as defined herein. In certain embodiments, $R^1$ is $-NR^{1a}C(O)OR^{1d}$, wherein $R^{1a}$ and $R^{1d}$ are each as defined herein. In certain embodiments, $R^1$ is $-NR^{1a}C(O)NR^{1b}R^{1c}$, wherein $R^{1a}$, $R^{1b}$, and $R^{1c}$ are each as defined herein.

In certain embodiments, $R^1$ is $-NR^{1a}C(=NR^{1d})NR^{1b}R^{1c}$, wherein $R^{1a}$, $R^{1b}$, $R^{1c}$, and $R^{1d}$ are each as defined herein. In certain embodiments, $R^1$ is $-NR^{1a}S(O)R^{1d}$, wherein $R^{1a}$ and $R^{1d}$ are each as defined herein. In certain embodiments, $R^1$ is $-NR^{1a}S(O)_2R^{1d}$, wherein $R^{1a}$ and $R^{1d}$ are each as defined herein. In certain embodiments, $R^1$ is $-NR^{1a}S(O)NR^{1b}R^{1c}$, wherein $R^{1a}$, $R^{1b}$, and $R^{1c}$ are each as defined herein. In certain embodiments, $R^1$ is $-NR^{1a}S(O)_2NR^{1b}R^{1c}$, wherein $R^{1a}$, $R^{1b}$, and $R^{1c}$ are each as defined herein. In certain embodiments, $R^1$ is $-SR^{1a}$, wherein $R^{1a}$ is as defined herein. In certain embodiments, $R^1$ is $-S(O)R^{1a}$, wherein $R^{1a}$ is as defined herein. In certain embodiments, $R^1$ is $-S(O)_2R^{1a}$, wherein $R^{1a}$ is as defined herein. In certain embodiments, $R^1$ is $-S(O)NR^{1b}R^{1c}$, wherein $R^{1b}$ and $R^{1c}$ are each as defined herein. In certain embodiments, $R^1$ is $-S(O)_2NR^{1b}R^{1c}$; wherein $R^{1b}$ and $R^{1c}$ are each as defined herein.

In certain embodiments, $R^2$ is hydrogen. In certain embodiments, $R^2$ is cyano. In certain embodiments, $R^2$ is halo. In certain embodiments, $R^2$ is fluoro, chloro, bromo, or iodo. In certain embodiments, $R^2$ is nitro. In certain embodiments, $R^2$ is $C_{1-6}$ alkyl, optionally substituted with one, two, three, four, or five substituents Q as described herein. In certain embodiments, $R^2$ is $C_{2-6}$ alkenyl, optionally substituted with one, two, three, four, or five substituents Q as described herein. In certain embodiments, $R^2$ is $C_{2-6}$ alkynyl, optionally substituted with one, two, three, four, or five substituents Q as described herein. In certain embodiments, $R^2$ is $C_{3-10}$ cycloalkyl, optionally substituted with one, two, three, four, or five substituents Q as described herein. In certain embodiments, $R^2$ is $C_{3-7}$ cycloalkyl, optionally substituted with one, two, three, four, or five substituents Q as described herein. In certain embodiments, $R^2$ is $C_{6-14}$ aryl, optionally substituted with one, two, three, four, or five substituents Q as described herein. In certain embodiments, $R^2$ is $C_{7-15}$ aralkyl, optionally substituted with one, two, three, four, or five substituents Q as described herein. In certain embodiments, $R^2$ is heteroaryl, optionally substituted with one, two, three, four, or five substituents Q as described herein. In certain embodiments, $R^2$ is heterocyclyl, optionally substituted with one, two, three, four, or five substituents Q as described herein.

In certain embodiments, $R^2$ is $-C(O)R^{1a}$, wherein $R^{1a}$ is as defined herein. In certain embodiments, $R^2$ is $-C(O)OR^{1a}$, wherein $R^{1a}$ is as defined herein. In certain embodiments, $R^2$ is $- C(O)NR^{1b}R^{1c}$, wherein $R^{1b}$ and $R^{1c}$ are each as defined herein. In certain embodiments, $R^2$ is $- C(NR^{1a})NR^{1b}R^{1c}$, wherein $R^{1a}$, $R^{1b}$, and $R^{1c}$ are each as defined herein. In certain embodiments, $R^2$ is $- OR^{1a}$, wherein $R^{1a}$ is as defined herein. In certain embodiments, $R^1$ is $-O-C_{1-6}$ alkyl, wherein the alkyl is optionally substituted with one, two, three, four, or five substituents Q as described herein.. In certain embodiments, $R^1$ is methoxy, ethoxy, propoxy, isopropoxy, or 3-dimethylaminopropoxy. In certain embodiments, $R^2$ is $-OC(O)R^{1a}$, wherein $R^{1a}$ is as defined herein. In certain embodiments, $R^2$ is $-OC(O)OR^{1a}$, wherein $R^{1a}$ is as defined herein. In certain embodiments, $R^2$ is $-OC(O)NR^{1b}R^{1c}$, wherein $R^{1b}$ and $R^{1c}$ are each as defined herein. In certain embodiments, $R^2$ is $-OC(=NR^{1a})NR^{1b}R^{1c}$, wherein $R^{1a}$, $R^{1b}$, and $R^{1c}$ are each as defined herein. In certain embodiments, $R^2$ is $-OS(O)R^{1a}$, wherein $R^{1a}$ is as defined herein. In certain embodiments, $R^2$ is $-OS(O)_2R^{1a}$, wherein $R^{1a}$ is as defined herein. In certain embodiments, $R^2$ is $-OS(O)NR^{1b}R^{1c}$, wherein $R^{1b}$ and $R^{1c}$ are each as defined herein. In certain embodiments, $R^2$ is $-OS(O)_2NR^{1b}R^{1c}$, wherein $R^{1b}$ and $R^{1c}$ are each as defined herein. In certain embodiments, $R^2$ is $-NR^{1b}R^{1c}$, wherein $R^{1b}$ and $R^{1c}$ are each as defined herein. In certain embodiments, $R^2$ is amino ($-NH_2$). In certain embodiments, $R^2$ is $-NR^{1a}C(O)R^{1d}$, wherein $R^{1a}$ and $R^{1d}$ are each as defined herein. In certain embodiments, $R^2$ is $-NR^{1a}C(O)OR^{1d}$, wherein $R^{1a}$ and $R^{1d}$ are each as defined herein. In certain embodiments, $R^2$ is $-NR^{1a}C(O)NR^{1b}R^{1c}$, wherein $R^{1a}$, $R^{1b}$, and $R^{1c}$ are each as defined herein. In certain embodiments, $R^2$ is $-NR^{1a}C(=NR^{1d})NR^{1b}R^{1c}$, wherein $R^{1a}$, $R^{1b}$, $R^{1c}$, and $R^{1d}$ are each as defined herein. In certain embodiments, $R^2$ is $-NR^{1a}S(O)R^{1d}$, wherein $R^{1a}$ and $R^{1d}$ are each as defined herein. In certain embodiments, $R^2$ is $-NR^{1a}S(O)_2R^{1d}$, wherein $R^{1a}$ and $R^{1d}$ are each as defined herein. In certain embodiments, $R^2$ is $-NR^{1a}S(O)NR^{1b}R^{1c}$, wherein $R^{1a}$, $R^{1b}$, and $R^{1c}$ are each as defined herein. In certain embodiments, $R^2$ is $-NR^{1a}S(O)_2NR^{1b}R^{1c}$, wherein $R^{1a}$, $R^{1b}$, and $R^{1c}$ are each as defined herein. In certain embodiments, $R^2$ is $-SR^{1a}$, wherein $R^{1a}$ is as defined herein. In certain embodiments, $R^2$ is $-S(O)R^{1a}$, wherein $R^{1a}$ is as defined herein. In certain embodiments, $R^2$ is $-S(O)_2R^{1a}$, wherein $R^{1a}$ is as defined herein. In certain embodiments, $R^2$ is $-S(O)NR^{1b}R^{1c}$, wherein $R^{1b}$ and $R^{1c}$ are each as defined herein. In certain embodiments, $R^2$ is $-S(O)_2NR^{1b}R^{1c}$; wherein $R^{1b}$ and $R^{1c}$ are each as defined herein.

In certain embodiments, $R^3$ is hydrogen. In certain embodiments, $R^3$ is $C_{1-6}$ alkyl, optionally substituted with one, two, three, four, or five substituents Q as described herein. In certain embodiments, $R^3$ is hydrogen, methyl, ethyl, or propyl (e.g., n-propyl, isopropyl, or 2-isopropyl).

In certain embodiments, $R^4$ is hydrogen. In certain embodiments, $R^4$ is $C_{1-6}$ alkyl, optionally substituted with one, two, three, four, or five substituents Q as described herein. In certain embodiments, $R^4$ is hydrogen, methyl, ethyl, or propyl (e.g., n-propyl, isopropyl, or 2-isopropyl).

In certain embodiments, $R^3$ and $R^4$ are linked together to form a bond. In certain embodiments, $R^3$ and $R^4$ are linked together to form $C_{1-6}$ alkylene, optionally substituted with one, two, three, four, or five substituents Q as described herein. In certain embodiments, $R^3$ and $R^4$ are linked together to form methylene, ethylene, or propylene, each optionally substituted with one, two, three, four, or five substituents Q as described herein. In certain embodiments, $R^3$ and $R^4$ are linked together to form $C_{1-6}$ heteroalkylene, optionally substituted with one, two, three, four, or five substituents Q as described herein. In certain embodiments, $R^3$ and $R^4$ are linked together to form $C_{2-6}$ alkenylene, optionally substituted with one, two, three, four, or five substituents Q as described herein. In certain embodiments, $R^3$ and $R^4$ are linked

together to form $C_{2-6}$ heteroalkenylene, optionally substituted with one, two, three, four, or five substituents Q as described herein.

In certain embodiments, $R^6$ is hydrogen. In certain embodiments, $R^6$ is $C_{1-6}$ alkyl, optionally substituted with one, two, three, four, or five substituents Q as described herein. In certain embodiments, $R^6$ is $C_{1-6}$ alkyl, optionally substituted with one or more, in one embodiment, one, two, or three, halo. In certain embodiments, $R^6$ is $C_{1-6}$ alkyl, optionally substituted with one or more, in one embodiment, one, two, or three, fluoro. In certain embodiments, $R^6$ is methyl, fluoromethyl, difluoromethyl, or trifluoromethyl. In certain embodiments, $R^6$ is difluoromethyl. In certain embodiments, $R^6$ is -S-$C_{1-6}$ alkyl, wherein the alkyl is optionally substituted with one, two, three, four, or five substituents Q as described herein. In certain embodiments, $R^6$ is -S(O)-$C_{1-6}$ alkyl, wherein the alkyl is optionally substituted with one, two, three, four, or five substituents Q as described herein. In certain embodiments, $R^6$ is -$SO_2$-$C_{1-6}$ alkyl, wherein the alkyl is optionally substituted with one, two, three, four, or five substituents Q as described herein.

In certain embodiments, $R^{5a}$ is hydrogen. In certain embodiments, $R^{5a}$ is not hydrogen. In certain embodiments, $R^{5a}$ is halo. In certain embodiments, $R^{5a}$ is fluoro, chloro, bromo, or iodo. In certain embodiments, $R^{5a}$ is $C_{1-6}$ alkyl, optionally substituted with one, two, three, four, or five substituents Q as described herein. In certain embodiments, $R^{5a}$ is methyl, ethyl, propyl, or butyl, each optionally substituted with one, two, three, four, or five substituents Q as described herein. In certain embodiments, $R^{5a}$ is methyl, ethyl, *n*-propyl, isopropyl, *n*-butyl, isobutyl, or *t*-butyl. In certain embodiments, $R^{5a}$ is methyl. In certain embodiments, $R^{5a}$ is $C_{2-6}$ alkenyl, optionally substituted with one, two, three, four, or five substituents Q as described herein. In certain embodiments, $R^{5a}$ is $C_{2-6}$ alkynyl, optionally substituted with one, two, three, four, or five substituents Q as described herein. In certain embodiments, $R^{5a}$ is $C_{3-10}$ cycloalkyl, optionally substituted with one, two, three, four, or five substituents Q as described herein. In certain embodiments, $R^{5a}$ is $C_{3-7}$ cycloalkyl, optionally substituted with one, two, three, four, or five substituents Q as described herein. In certain embodiments, $R^{5a}$ is $C_{6-14}$ aryl, optionally substituted with one, two, three, four, or five substituents Q as described herein. In certain embodiments, $R^{5a}$ is $C_{7-15}$ aralkyl, optionally substituted with one, two, three, four, or five substituents Q as described herein. In certain embodiments, $R^{5a}$ is heteroaryl, optionally substituted with one, two, three, four, or five substituents Q as described herein. In certain embodiments, $R^{5a}$ is heterocyclyl, optionally substituted with one, two, three, four, or five substituents Q as described herein.

In certain embodiments, $R^{5a}$ is -C(O)$R^{1a}$, wherein $R^{1a}$ is as defined herein. In certain embodiments, $R^{5a}$ is -C(O)O$R^{1a}$, wherein $R^{1a}$ is as defined herein. In certain embodiments, $R^{5a}$ is - C(O)O$R^{1a}$, wherein $R^{1a}$ is $C_{1-6}$ alkyl, optionally substituted with one, two, three, four, or five substituents Q as described herein. In certain embodiments, $R^{5a}$ is -C(O)O$CH_3$. In certain embodiments, $R^{5a}$ is -C(O)N$R^{1b}R^{1c}$, wherein $R^{1b}$ and $R^{1c}$ are each as defined herein. In certain embodiments, $R^{5a}$ is -C(N$R^{1a}$)N$R^{1b}R^{1c}$, wherein $R^{1a}$, $R^{1b}$, and $R^{1c}$ are each as defined herein. In certain embodiments, $R^{5a}$ is -O$R^{1a}$, wherein $R^{1a}$ is as defined herein. In certain embodiments, $R^{5a}$ is - OC(O)$R^{1a}$, wherein $R^{1a}$ is as defined herein. In certain embodiments, $R^{5a}$ is -OC(O)O$R^{1a}$, wherein $R^{1a}$ is as defined herein. In certain embodiments, $R^{5a}$ is -OC(O)N$R^{1b}R^{1c}$, wherein $R^{1b}$ and $R^{1c}$ are each as defined herein. In certain embodiments, $R^{5a}$ is -OC(=N$R^{1a}$)N$R^{1b}R^{1c}$, wherein $R^{1a}$, $R^{1b}$, and $R^{1c}$ are each as defined herein. In certain embodiments, $R^{5a}$ is -OS(O)$R^{1a}$, wherein $R^{1a}$ is as defined herein. In certain embodiments, $R^{5a}$ is -OS$(O)_2R^{1a}$, wherein $R^{1a}$ is as defined herein. In certain embodiments, $R^{5a}$ is -OS(O)N$R^{1b}R^{1c}$, wherein $R^{1b}$ and $R^{1c}$ are each as defined herein. In certain embodiments, $R^{5a}$ is - OS$(O)_2$N$R^{1b}R^{1c}$, wherein $R^{1b}$ and $R^{1c}$ are each as defined herein. In certain embodiments, $R^{5a}$ is - N$R^{1b}R^{1c}$, wherein $R^{1b}$ and $R^{1c}$ are each as defined herein. In certain embodiments, $R^{5a}$ is amino (-$NH_2$). In certain embodiments, $R^{5a}$ is -N$R^{1a}$C(O)$R^{1d}$, wherein $R^{1a}$ and $R^{1d}$ are each as defined herein. In certain embodiments, $R^{5a}$ is
-N$R^{1a}$C(O)O$R^{1d}$, wherein $R^{1a}$ and $R^{1d}$ are each as defined herein. In certain embodiments, $R^{5a}$ is - N$R^{1a}$C(O)N$R^{1b}R^{1c}$, wherein $R^{1a}$, $R^{1b}$, and $R^{1c}$ are each as defined herein. In certain embodiments, $R^{5a}$ is -N$R^{1a}$C(=N$R^{1d}$)N$R^{1b}R^{1c}$, wherein $R^{1a}$, $R^{1b}$, $R^{1c}$, and $R^{1d}$ are each as defined herein. In certain embodiments, $R^{5a}$ is -N$R^{1a}$S(O)$R^{1d}$, wherein $R^{1a}$ and $R^{1d}$ are each as defined herein. In certain embodiments, $R^{5a}$ is -N$R^{1a}$S$(O)_2R^{1d}$, wherein $R^{1a}$ and $R^{1d}$ are each as defined herein. In certain embodiments, $R^{5a}$ is -N$R^{1a}$S(O)N$R^{1b}R^{1c}$, wherein $R^{1a}$, $R^{1b}$, and $R^{1c}$ are each as defined herein. In certain embodiments, $R^{5a}$ is -N$R^{1a}$S$(O)_2$N$R^{1b}R^{1c}$, wherein $R^{1a}$, $R^{1b}$, and $R^{1c}$ are each as defined herein. In certain embodiments, $R^{5a}$ is -S$R^{1a}$, wherein $R^{1a}$ is as defined herein. In certain embodiments, $R^{5a}$ is -S(O)$R^{1a}$, wherein $R^{1a}$ is as defined herein. In certain embodiments, $R^{5a}$ is -S$(O)_2R^{1a}$, wherein $R^{1a}$ is as defined herein. In certain embodiments, $R^{5a}$ is -S(O)N$R^{1b}R^{1c}$, wherein $R^{1b}$ and $R^{1c}$ are each as defined herein. In certain embodiments, $R^{5a}$ is -S$(O)_2$N$R^{1b}R^{1c}$; wherein $R^{1b}$ and $R^{1c}$ are each as defined herein.

In certain embodiments, $R^{5a}$ is (a) hydrogen or halo; (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, or heteroaryl, each of which is optionally substituted with one, two, three, four, or five substituents Q; or (c) -C(O)$R^{1a}$, -C(O)O$R^{1a}$, -C(O)N$R^{1b}R^{1c}$, - C(N$R^{1a}$)N$R^{1b}R^{1c}$, -O$R^{1a}$, -OC(O)$R^{1a}$, -OC(O)O$R^{1a}$, -OC(O)N$R^{1b}R^{1c}$, -OC(=N$R^{1a}$)N$R^{1b}R^{1c}$, - OS(O)$R^{1a}$, -OS$(O)_2R^{1a}$, -OS(O)N$R^{1b}R^{1c}$, -OS$(O)_2$N$R^{1b}R^{1c}$, -N$R^{1b}R^{1c}$, -N$R^{1a}$C(O)$R^{1d}$, -N$R^{1a}$C(O)O$R^{1d}$, -N$R^{1a}$C(O)N$R^{1b}R^{1c}$, -N$R^{1a}$C(=N$R^{1d}$)N$R^{1b}R^{1c}$, -N$R^{1a}$S(O)$R^{1d}$, -N$R^{1a}$S$(O)_2R^{1d}$, - N$R^{1a}$S(O)N$R^{1b}R^{1c}$, -N$R^{1a}$S$(O)_2$N$R^{1b}R^{1c}$, -S$R^{1a}$, -S(O)$R^{1a}$, -S$(O)_2R^{1a}$, -S(O)N$R^{1b}R^{1c}$, or - S$(O)_2$N$R^{1b}R^{1c}$. In certain embodiments, $R^{5a}$ is (a) hydrogen or halo; or (b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, or heteroaryl, each of which is optionally substituted with one, two, three, four, or five substituents Q.

In certain embodiments, $R^{5b}$ is halo. In certain embodiments, $R^{5b}$ is fluoro, chloro, bromo, or iodo. In certain embodiments, $R^{5b}$ is $C_{1-6}$ alkyl, optionally substituted with one, two, three, four, or five substituents Q as described herein. In certain embodiments, $R^{5b}$ is methyl, ethyl, propyl, or butyl, each optionally substituted with one, two, three, four, or five substituents Q as described herein. In certain embodiments, $R^{5b}$ is methyl, ethyl, *n*-propyl, isopropyl, *n*-butyl, isobutyl, or *t*-butyl. In certain embodiments, $R^{5b}$ is methyl. In certain embodiments, $R^{5b}$ is $C_{2-6}$ alkenyl, optionally substituted with one, two, three, four, or five substituents Q as described herein. In certain embodiments, $R^{5b}$ is $C_{2-6}$ alkynyl, optionally substituted with one, two, three, four, or five substituents Q as described herein. In certain embodiments, $R^{5b}$ is $C_{3-10}$ cycloalkyl, optionally substituted with one, two, three, four, or five substituents Q as described herein. In certain embodiments, $R^{5b}$ is $C_{3-7}$ cycloalkyl, optionally substituted with one, two, three, four, or five substituents Q as described herein. In certain embodiments, $R^{5b}$ is $C_{6-14}$ aryl, optionally substituted with one, two, three, four, or five substituents Q as described herein. In certain embodiments, $R^{5b}$ is $C_{7-15}$ aralkyl, optionally substituted with one, two, three, four, or five substituents Q as described herein. In certain embodiments, $R^{5b}$ is heteroaryl, optionally substituted with one, two, three, four, or five substituents Q as described herein. In certain embodiments, $R^{5b}$ is heterocyclyl, optionally substituted with one, two, three, four, or five substituents Q as described herein. In certain embodiments, $R^{5b}$ is not heterocyclyl.

In certain embodiments, $R^{5b}$ is $-C(O)R^{1a}$, wherein $R^{1a}$ is as defined herein. In certain embodiments, $R^{5b}$ is $-C(O)OR^{1a}$, wherein $R^{1a}$ is as defined herein. In certain embodiments, $R^{5b}$ is - $C(O)OR^{1a}$, wherein $R^{1a}$ is $C_{1-6}$ alkyl, optionally substituted with one, two, three, four, or five substituents Q as described herein. In certain embodiments, $R^{5b}$ is $-C(O)OCH_3$. In certain embodiments, $R^{5b}$ is $-C(O)NR^{1b}R^{1c}$, wherein $R^{1b}$ and $R^{1c}$ are each as defined herein. In certain embodiments, $R^{5b}$ is $-C(NR^{1a})NR^{1b}R^{1c}$, wherein $R^{1a}$, $R^{1b}$, and $R^{1c}$ are each as defined herein. In certain embodiments, $R^{5b}$ is $-OR^{1a}$, wherein $R^{1a}$ is as defined herein. In certain embodiments, $R^{5b}$ is - $OC(O)R^{1a}$, wherein $R^{1a}$ is as defined herein. In certain embodiments, $R^{5b}$ is $-OC(O)OR^{1a}$, wherein $R^{1a}$ is as defined herein. In certain embodiments, $R^{5b}$ is $-OC(O)NR^{1b}R^{1c}$, wherein $R^{1b}$ and $R^{1c}$ are each as defined herein. In certain embodiments, $R^{5b}$ is $-OC(=NR^{1a})NR^{1b}R^{1c}$, wherein $R^{1a}$, $R^{1b}$, and $R^{1c}$ are each as defined herein. In certain embodiments, $R^{5b}$ is $-OS(O)R^{1a}$, wherein $R^{1a}$ is as defined herein. In certain embodiments, $R^{5b}$ is $-OS(O)_2R^{1a}$, wherein $R^{1a}$ is as defined herein. In certain embodiments, $R^{5b}$ is $-OS(O)NR^{1b}R^{1c}$, wherein $R^{1b}$ and $R^{1c}$ are each as defined herein. In certain embodiments, $R^{5b}$ is $-OS(O)_2NR^{1b}R^{1c}$, wherein $R^{1b}$ and $R^{1c}$ are each as defined herein. In certain embodiments, $R^{5b}$ is - $NR^{1b}R^{1c}$, wherein $R^{1b}$ and $R^{1c}$ are each as defined herein. In certain embodiments, $R^{5b}$ is amino ($-NH_2$). In certain embodiments, $R^{5b}$ is $-NR^{1a}C(O)R^{1d}$, wherein $R^{1a}$ and $R^{1d}$ are each as defined herein. In certain embodiments, $R^{5b}$ is $-NR^{1a}C(O)OR^{1d}$, wherein $R^{1a}$ and $R^{1d}$ are each as defined herein. In certain embodiments, $R^{5b}$ is $-NR^{1a}C(O)NR^{1b}R^{1c}$, wherein $R^{1a}$, $R^{1b}$, and $R^{1c}$ are each as defined herein. In certain embodiments, $R^{5b}$ is $-NR^{1a}C(=NR^{1d})NR^{1b}R^{1c}$, wherein $R^{1a}$, $R^{1b}$, $R^{1c}$, and $R^{1d}$ are each as defined herein. In certain embodiments, $R^{5b}$ is $-NR^{1a}S(O)R^{1d}$, wherein $R^{1a}$ and $R^{1d}$ are each as defined herein. In certain embodiments, $R^{5b}$ is $-NR^{1a}S(O)_2R^{1d}$, wherein $R^{1a}$ and $R^{1d}$ are each as defined herein. In certain embodiments, $R^{5b}$ is $-NR^{1a}S(O)NR^{1b}R^{1c}$, wherein $R^{1a}$, $R^{1b}$, and $R^{1c}$ are each as defined herein. In certain embodiments, $R^{5b}$ is $-NR^{1a}S(O)_2NR^{1b}R^{1c}$, wherein $R^{1a}$, $R^{1b}$, and $R^{1c}$ are each as defined herein. In certain embodiments, $R^{5b}$ is $-SR^{1a}$, wherein $R^{1a}$ is as defined herein. In certain embodiments, $R^{5b}$ is $-S(O)R^{1a}$, wherein $R^{1a}$ is as defined herein. In certain embodiments, $R^{5b}$ is - $S(O)_2R^{1a}$, wherein $R^{1a}$ is as defined herein. In certain embodiments, $R^{5b}$ is $-S(O)NR^{1b}R^{1c}$, wherein $R^{1b}$ and $R^{1c}$ are each as defined herein. In certain embodiments, $R^{5b}$ is $-S(O)_2NR^{1b}R^{1c}$; wherein $R^{1b}$ and $R^{1c}$ are each as defined herein.

In certain embodiments, $R^{5a}$ and $R^{5b}$ are each independently methyl, ethyl, *n*-propyl, isopropyl, *n*-butyl, isobutyl, or *t*-butyl, each optionally substituted with one, two, three, four, or five substituents Q as described herein. In certain embodiments, $R^{5a}$ and $R^{5b}$ are each independently methyl, ethyl, *n*-propyl, isopropyl, *n*-butyl, isobutyl, or *t*-butyl, each optionally substituted with one or more halo. In certain embodiments, $R^{5a}$ and $R^{5b}$ are each independently methyl, ethyl, *n*-propyl, isopropyl, *n*-butyl, isobutyl, or *t*-butyl. In certain embodiments, $R^{5a}$ and $R^{5b}$ are each methyl.

In certain embodiments, $R^{5c}$ is $C_{6-14}$ aryl, optionally substituted with one, two, three, four, or five substituents Q as described herein. In certain embodiments, $R^{5b}$ is $C_{6-14}$ aryl substituted at the 2-position with one substituent Q as described herein. In certain embodiments, $R^{5c}$ is phenyl or naphthyl, each optionally substituted with one, two, three, four, or five substituents Q as described herein. In certain embodiments, $R^{5c}$ is phenyl, naphtha-1-yl, or naphtha-2-yl, each optionally substituted with one, two, three, four, or five substituents Q as described herein. In certain embodiments, $R^{5c}$ is phenyl, 4-chlorophenyl, 4-methoxyphenyl, or naphtha-2-yl. In certain embodiments, $R^{5c}$ is heteroaryl, optionally substituted with one, two, three, four, or five substituents Q as described herein. In certain embodiments, $R^{5c}$ is monocyclic heteroaryl, optionally substituted with one, two, three, four, or five substituents Q as described herein. In certain embodiments, $R^{5c}$ is 5- or 6-membered heteroaryl, optionally substituted with one, two, three, four, or five substituents Q as described herein. In certain embodiments, $R^{5c}$ is bicyclic heteroaryl, optionally substituted with one, two, three, four, or five substituents Q as described herein.

In certain embodiments, $R^{5c}$ is $-(CR^{5f}R^{5g})_n-(C_{6-14}$ aryl), wherein the $C_{6-14}$ aryl is optionally substituted with one, two, three, four, or five substituents Q as described herein. In certain embodiments, $R^{5c}$ is benzyl, 2-phenethyl, 3-phenylpropyl, or 4-phenylbutyl, wherein each of the phenyl moiety is optionally substituted with one, two, three, four, or five substituents Q as described herein. In certain embodiments, $R^{5c}$ is benzyl, 2-phenethyl, 3-phenylpropyl, or 4-phenylbutyl. In certain embodiments, $R^{5c}$ is benzyl, fluorobenzyl, chlorobenzyl, bromobenzyl, cyanobenzyl, methylbenzyl, or methoxybenzyl. In certain embodiments, $R^{5c}$ is (naphthalen-1-yl)methyl, (naphthalen-2-yl)methyl 2-(naphthalen-1-yl)ethyl, 2-(naphthalen-2-yl)ethyl, 3-(naphthalen-1-yl)propyl, 3-(naphthalen-2-yl)propyl, 4-(naphthalen-1-yl)butyl, or 4-(naphthalen-2-yl)butyl, wherein each of the naphthyl moiety is optionally substituted with one, two, three, four, or five substituents Q as described herein. In certain embodiments, n is 0 or 1. In one embodiment, n is 1. In one embodiment, n is 1, 2, 3, or 4. In certain embodiments, $R^{5c}$ is -$CH_2$-($C_{6-14}$ aryl), wherein the $C_{6-14}$ aryl is optionally substituted with one, two, three, four, or five substituents Q as described herein. In certain embodiments, $R^{5c}$ is -$C(CH_3)_2$-($C_{6-14}$ aryl), wherein the $C_{6-14}$ aryl is optionally substituted with one, two, three, four, or five substituents Q as described herein. In certain embodiments, $R^{5c}$ is -$CH_2$-phenyl or -$CH_2$-naphthyl, wherein the phenyl or naphthyl is each optionally substituted with one, two, three, four, or five substituents Q as described herein, such as, e.g., optionally substituted with one or more F, Cl, Br, I, -CN, -$CH_3$, -$CF_3$, -$OCH_3$, or -$OCF_3$. In certain embodiments, $R^{5c}$ is -$CH_2$-phenyl, -$CH_2$-naphtha-1-yl, or -$CH_2$-naphtha-2-yl, wherein the phenyl or naphthyl is each optionally substituted with one, two, three, four, or five substituents Q as described herein, such as, e.g., optionally substituted with one or more F, Cl, Br, I, -CN, -$CH_3$, -$CF_3$, -$OCH_3$, or -$OCF_3$. In certain embodiments, $R^{5c}$ is -$CH_2$-phenyl, -$CH_2$-naphtha-1-yl, or -$CH_2$-naphtha-2-yl, wherein the phenyl or naphthyl is each optionally substituted with one or more F, Cl, Br, I, -CN, -$CH_3$, -$CF_3$, -$OCH_3$,-$OCF_3$. In other embodiments, $R^{5c}$ is -$CH_2$-phenyl, -$CH_2$-naphtha-1-yl, or -$CH_2$-naphtha-2-yl, wherein the phenyl or naphthyl is each optionally substituted with one or more F, Cl, Br, I, -CN,-$CH_3$, -$CF_3$, -$OCH_3$, -$OCF_3$, -O-($C_{1-4}$ alkylene)-N-($C_{1-4}$ alkyl)$_2$ (e.g., -O-$CH_2CH_2$-N($CH_3$)$_2$), -O-heterocyclyl (e.g., -O-(N-methylpiperidinyl) or -O-piperidinyl), -O-heteroaryl (e.g., -O-pyridyl), - NH-heterocyclyl (e.g., -NH-(N-methylpiperidinyl), -NH-(N-methylpyrrolidinyl), -NH-piperidinyl, or -NH-pyrrolidinyl), -NH-heteroaryl (e.g., -NH-pyridyl), -$NCH_3$-heterocyclyl (e.g., -$NCH_3$-(N-methylpiperidinyl), -$NCH_3$-(N-methylpyrrolidinyl), -$NCH_3$-piperidinyl, or -$NCH_3$-pyrrolidinyl),-$NCH_3$-heteroaryl (e.g., -$NCH_3$-pyridyl), heterocyclyl (e.g., piperidinyl, piperazinyl, N-methylpiperidinyl, or N-methylpiperazinyl), or heteroaryl (e.g., pyridyl or imidazolyl). In certain embodiments, $R^{5c}$ is -$CH_2$-phenyl, -$C(CH_3)_2$-phenyl, -$CH_2$-(2-methylphenyl), -$CH_2$-(2-methoxylphenyl), -$CH_2$-(2-fluorophenyl), -$CH_2$-(2-chlorophenyl), -$CH_2$-(2-bromophenyl), -$CH_2$-(3-methylphenyl), -$CH_2$-(3-methoxylphenyl), -$CH_2$-(3-fluorophenyl), -$CH_2$-(3-chlorophenyl),-$CH_2$-(3-bromophenyl), -$CH_2$-(4-methylphenyl), -$CH_2$-(4-methoxylphenyl), -$CH_2$-(4-fluorophenyl), -$CH_2$-(4-chlorophenyl), -$CH_2$-(4-bromophenyl), -$CH_2$-naphtha-1-yl, or -$CH_2$-naphtha-2-yl.

In certain embodiments, $R^{5c}$ is -($CR^{5f}R^{5g}$)-($C_{6-14}$ aryl), wherein the $C_{6-14}$ aryl is optionally substituted with one, two, three, four, or five substituents Q as described herein, and wherein $R^{5f}$ and $R^{5g}$ together with the carbon atom to which they are attached form a 3- to 6-membered cycloalkyl or heterocyclyl. In one embodiment, $R^{5c}$ is -cyclopropyl-phenyl. In one embodiment, $R^{5c}$ is -cyclobutyl-phenyl. In one embodiment, $R^{5c}$ is -cyclopentyl-phenyl. In one embodiment, $R^{5c}$ is -cyclohexylphenyl.

In certain embodiments, $R^{5c}$ is -($CR^{5f}R^{5g}$)$_n$-heteroaryl, wherein the heteroaryl is optionally substituted with one, two, three, four, or five substituents Q as described herein, wherein n is defined herein elsewhere. In certain embodiments, $R^{5c}$ is -$CH_2$-(monocyclic heteroaryl), wherein the heteroaryl is optionally substituted with one, two, three, four, or five substituents Q as described herein. In certain embodiments, $R^{5c}$ is -$CH_2$-(5- or 6-membered heteroaryl), wherein the heteroaryl is optionally substituted with one, two, three, four, or five substituents Q as described herein. In certain embodiments, $R^{5c}$ is -$CH_2$-(bicyclic heteroaryl), wherein the heteroaryl is optionally substituted with one, two, three, four, or five substituents as described herein.

In certain embodiments, $R^{5d}$ is hydrogen. In certain embodiments, $R^{5d}$ is halo. In certain embodiments, $R^{5d}$ is fluoro, chloro, bromo, or iodo. In certain embodiments, $R^{5d}$ is $C_{1-6}$ alkyl, optionally substituted with one, two, three, four, or five substituents Q as described herein. In certain embodiments, $R^{5d}$ is methyl, optionally substituted with one, two, three, four, or five substituents Q as described herein. In certain embodiments, $R^{5d}$ is methyl. In certain embodiments, $R^{5d}$ is methyl, ethyl, propyl, or butyl, each optionally substituted with one, two, three, four, or five substituents Q as described herein. In certain embodiments, $R^{5d}$ is methyl, ethyl, *n*-propyl, isopropyl, *n*-butyl, isobutyl, or *t*-butyl. In certain embodiments, $R^{5d}$ is $C_{2-6}$ alkenyl, optionally substituted with one, two, three, four, or five substituents Q as described herein. In certain embodiments, $R^{5d}$ is $C_{2-6}$ alkynyl, optionally substituted with one, two, three, four, or five substituents Q as described herein. In certain embodiments, $R^{5d}$ is $C_{3-10}$ cycloalkyl, optionally substituted with one, two, three, four, or five substituents Q as described herein. In certain embodiments, $R^{5d}$ is $C_{6-14}$ aryl, optionally substituted with one, two, three, four, or five substituents Q as described herein. In certain embodiments, $R^{5d}$ is $C_{7-15}$ aralkyl, optionally substituted with one, two, three, four, or five substituents Q as described herein. In certain embodiments, $R^{5d}$ is heteroaryl, optionally substituted with one, two, three, four, or five substituents Q as described herein. In certain embodiments, $R^{5d}$ is heterocyclyl, optionally substituted with one, two, three, four, or five substituents Q as described herein.

In certain embodiments, $R^{5d}$ is -C(O)$R^{1a}$, wherein $R^{1a}$ is as defined herein. In certain embodiments, $R^{5d}$ is -C(O)O$R^{1a}$, wherein $R^{1a}$ is as defined herein. In certain embodiments, $R^{5d}$ is-C(O)O$R^{1a}$, wherein $R^{1a}$ is $C_{1-6}$ alkyl, optionally substituted with one, two, three, four, or five substituents Q as described herein. In certain embodiments, $R^{5d}$ is -C(O)$OCH_3$. In certain embodiments, $R^{5d}$ is -C(O)N$R^{1b}R^{1c}$, wherein $R^{1b}$ and $R^{1c}$ are each as defined herein. In certain embodiments, $R^{5d}$ is -C(N$R^{1a}$)N$R^{1b}R^{1c}$, wherein $R^{1a}$, $R^{1b}$, and $R^{1c}$ are each as defined herein. In certain embodiments, $R^{5d}$ is -O$R^{1a}$, wherein $R^{1a}$ is as defined herein. In certain embodiments, $R^{5d}$ is - OC(O)$R^{1a}$, wherein $R^{1a}$ is as defined

herein. In certain embodiments, $R^{5d}$ is -OC(O)O$R^{1a}$, wherein $R^{1a}$ is as defined herein. In certain embodiments, $R^{5d}$ is -OC(O)N$R^{1b}R^{1c}$, wherein $R^{1b}$ and $R^{1c}$ are each as defined herein. In certain embodiments, $R^{5d}$ is -OC(=N$R^{1a}$)N$R^{1b}R^{1c}$, wherein $R^{1a}$, $R^{1b}$, and $R^{1c}$ are each as defined herein. In certain embodiments, $R^{5d}$ is -OS(O)$R^{1a}$, wherein $R^{1a}$ is as defined herein. In certain embodiments, $R^{5d}$ is -OS$(O)_2R^{1a}$, wherein $R^{1a}$ is as defined herein. In certain embodiments, $R^{5d}$ is -OS(O)N$R^{1b}R^{1c}$, wherein $R^{1b}$ and $R^{1c}$ are each as defined herein. In certain embodiments, $R^{5d}$ is -OS$(O)_2$N$R^{1b}R^{1c}$, wherein $R^{1b}$ and $R^{1c}$ are each as defined herein. In certain embodiments, $R^{5d}$ is - N$R^{1b}R^{1c}$, wherein $R^{1b}$ and $R^{1c}$ are each as defined herein. In certain embodiments, $R^{5d}$ is amino (-$NH_2$). In certain embodiments, $R^{5d}$ is -N$R^{1a}$C(O)$R^{1d}$, wherein $R^{1a}$ and $R^{1d}$ are each as defined herein. In certain embodiments, $R^{5d}$ is -N$R^{1a}$C(O)O$R^{1d}$, wherein $R^{1a}$ and $R^{1d}$ are each as defined herein. In certain embodiments, $R^{5d}$ is -N$R^{1a}$C(O)N$R^{1b}R^{1c}$, wherein $R^{1a}$, $R^{1b}$, and $R^{1c}$ are each as defined herein. In certain embodiments, $R^{5d}$ is -N$R^{1a}$C(=N$R^{1d}$)N$R^{1b}R^{1c}$, wherein $R^{1a}$, $R^{1b}$, $R^{1c}$ , and $R^{1d}$ are each as defined herein. In certain embodiments, $R^{5d}$ is -N$R^{1a}$S(O)$R^{1d}$, wherein $R^{1a}$ and $R^{1d}$ are each as defined herein. In certain embodiments, $R^{5d}$ is -N$R^{1a}$S$(O)_2R^{1d}$, wherein $R^{1a}$ and $R^{1d}$ are each as defined herein. In certain embodiments, $R^{5d}$ is -N$R^{1a}$S(O)N$R^{1b}R^{1c}$, wherein $R^{1a}$, $R^{1b}$, and $R^{1c}$ are each as defined herein. In certain embodiments, $R^{5d}$ is -N$R^{1a}$S$(O)_2$N$R^{1b}R^{1c}$, wherein $R^{1a}$, $R^{1b}$, and $R^{1c}$ are each as defined herein. In certain embodiments, $R^{5d}$ is -S$R^{1a}$, wherein $R^{1a}$ is as defined herein. In certain embodiments, $R^{5d}$ is -S(O)$R^{1a}$, wherein $R^{1a}$ is as defined herein. In certain embodiments, $R^{5d}$ is - S$(O)_2R^{1a}$, wherein $R^{1a}$ is as defined herein. In certain embodiments, $R^{5d}$ is -S(O)N$R^{1b}R^{1c}$, wherein $R^{1b}$ and $R^{1c}$ are each as defined herein. In certain embodiments, $R^{5d}$ is -S$(O)_2$N$R^{1b}R^{1c}$; wherein $R^{1b}$ and $R^{1c}$ are each as defined herein.

In certain embodiments, $R^{5e}$ is hydrogen. In certain embodiments, $R^{5e}$ is halo. In certain embodiments, $R^{5e}$ is fluoro, chloro, bromo, or iodo. In certain embodiments, $R^{5e}$ is $C_{1-6}$ alkyl, optionally substituted with one, two, three, four, or five substituents Q as described herein. In certain embodiments, $R^{5e}$ is methyl, optionally substituted with one, two, three, four, or five substituents Q as described herein. In certain embodiments, $R^{5e}$ is methyl. In certain embodiments, $R^{5e}$ is methyl, ethyl, propyl, or butyl, each optionally substituted with one, two, three, four, or five substituents Q as described herein. In certain embodiments, $R^{5e}$ is methyl, ethyl, *n*-propyl, isopropyl, *n*-butyl, isobutyl, or *t*-butyl. In certain embodiments, $R^{5e}$ is $C_{2-6}$ alkenyl, optionally substituted with one, two, three, four, or five substituents Q as described herein. In certain embodiments, $R^{5e}$ is $C_{2-6}$ alkynyl, optionally substituted with one, two, three, four, or five substituents Q as described herein. In certain embodiments, $R^{5e}$ is $C_{3-10}$ cycloalkyl, optionally substituted with one, two, three, four, or five substituents Q as described herein. In certain embodiments, $R^{5e}$ is $C_{6-14}$ aryl, optionally substituted with one, two, three, four, or five substituents Q as described herein. In certain embodiments, $R^{5e}$ is $C_{7-15}$ aralkyl, optionally substituted with one, two, three, four, or five substituents Q as described herein. In certain embodiments, $R^{5e}$ is heteroaryl, optionally substituted with one, two, three, four, or five substituents Q as described herein. In certain embodiments, $R^{5e}$ is heterocyclyl, optionally substituted with one, two, three, four, or five substituents Q as described herein.

In certain embodiments, $R^{5e}$ is -C(O)$R^{1a}$, wherein $R^{1a}$ is as defined herein. In certain embodiments, $R^{5e}$ is -C(O)O$R^{1a}$, wherein $R^{1a}$ is as defined herein. In certain embodiments, $R^{5e}$ is - C(O)O$R^{1a}$, wherein $R^{1a}$ is $C_{1-6}$ alkyl, optionally substituted with one, two, three, four, or five substituents Q as described herein. In certain embodiments, $R^{5e}$ is -C(O)O$CH_3$. In certain embodiments, $R^{5e}$ is -C(O)N$R^{1b}R^{1c}$, wherein $R^{1b}$ and $R^{1c}$ are each as defined herein. In certain embodiments, $R^{5e}$ is -C(N$R^{1a}$)N$R^{1b}R^{1c}$, wherein $R^{1a}$, $R^{1b}$, and $R^{1c}$ are each as defined herein. In certain embodiments, $R^{5e}$ is -O$R^{1a}$, wherein $R^{1a}$ is as defined herein. In certain embodiments, $R^{5e}$ is - OC(O)$R^{1a}$, wherein $R^{1a}$ is as defined herein. In certain embodiments, $R^{5e}$ is -OC(O)O$R^{1a}$, wherein $R^{1a}$ is as defined herein. In certain embodiments, $R^{5e}$ is -OC(O)N$R^{1b}R^{1c}$, wherein $R^{1b}$ and $R^{1c}$ are each as defined herein. In certain embodiments, $R^{5e}$ is -OC(=N$R^{1a}$)N$R^{1b}R^{1c}$, wherein $R^{1a}$, $R^{1b}$, and $R^{1c}$ are each as defined herein. In certain embodiments, $R^{5e}$ is -OS(O)$R^{1a}$, wherein $R^{1a}$ is as defined herein. In certain embodiments, $R^{5e}$ is -OS$(O)_2R^{1a}$, wherein $R^{1a}$ is as defined herein. In certain embodiments, $R^{5e}$ is -OS(O)N$R^{1b}R^{1c}$, wherein $R^{1b}$ and $R^{1c}$ are each as defined herein. In certain embodiments, $R^{5e}$ is - OS$(O)_2$N$R^{1b}R^{1c}$, wherein $R^{1b}$ and $R^{1c}$ are each as defined herein. In certain embodiments, $R^{5e}$ is - N$R^{1b}R^{1c}$, wherein $R^{1b}$ and $R^{1c}$ are each as defined herein. In certain embodiments, $R^{5e}$ is amino (-$NH_2$). In certain embodiments, $R^{5e}$ is -N$R^{1a}$C(O)$R^{1d}$, wherein $R^{1a}$ and $R^{1d}$ are each as defined herein. In certain embodiments, $R^{5e}$ is -N$R^{1a}$C(O)O$R^{1d}$, wherein $R^{1a}$ and $R^{1d}$ are each as defined herein. In certain embodiments, $R^{5e}$ is -N$R^{1a}$C(O)N$R^{1b}R^{1c}$, wherein $R^{1a}$, $R^{1b}$, and $R^{1c}$ are each as defined herein. In certain embodiments, $R^{5e}$ is -N$R^{1a}$C(=N$R^{1d}$)N$R^{1b}R^{1c}$, wherein $R^{1a}$, $R^{1b}$, $R^{1c}$ , and $R^{1d}$ are each as defined herein. In certain embodiments, $R^{5e}$ is -N$R^{1a}$S(O)$R^{1d}$, wherein $R^{1a}$ and $R^{1d}$ are each as defined herein. In certain embodiments, $R^{5e}$ is -N$R^{1a}$S$(O)_2R^{1d}$, wherein $R^{1a}$ and $R^{1d}$ are each as defined herein. In certain embodiments, $R^{5e}$ is -N$R^{1a}$S(O)N$R^{1b}R^{1c}$, wherein $R^{1a}$, $R^{1b}$, and $R^{1c}$ are each as defined herein. In certain embodiments, $R^{5e}$ is -N$R^{1a}$S$(O)_2$N$R^{1b}R^{1c}$, wherein $R^{1a}$, $R^{1b}$, and $R^{1c}$ are each as defined herein. In certain embodiments, $R^{5e}$ is -S$R^{1a}$, wherein $R^{1a}$ is as defined herein. In certain embodiments, $R^{5e}$ is -S(O)$R^{1a}$, wherein $R^{1a}$ is as defined herein. In certain embodiments, $R^{5e}$ is-S$(O)_2R^{1a}$, wherein $R^{1a}$ is as defined herein. In certain embodiments, $R^{5e}$ is -S(O)N$R^{1b}R^{1c}$, wherein $R^{1b}$ and $R^{1c}$ are each as defined herein. In certain embodiments, $R^{5e}$ is -S$(O)_2$N$R^{1b}R^{1c}$; wherein $R^{1b}$ and $R^{1c}$ are each as defined herein.

In certain embodiments, $R^{5f}$ is hydrogen. In certain embodiments, $R^{5f}$ is halo. In certain embodiments, $R^{5f}$ is fluoro, chloro, bromo, or iodo. In certain embodiments, $R^{5f}$ is $C_{1-6}$ alkyl, optionally substituted with one, two, three, four,

or five substituents Q as described herein. In certain embodiments, $R^{5f}$ is methyl, optionally substituted with one, two, three, four, or five substituents Q as described herein. In certain embodiments, $R^{5f}$ is methyl. In certain embodiments, $R^{5f}$ is methyl, ethyl, propyl, or butyl, each optionally substituted with one, two, three, four, or five substituents Q as described herein. In certain embodiments, $R^{5f}$ is methyl, ethyl, *n*-propyl, isopropyl, *n*-butyl, isobutyl, or *t*-butyl. In certain embodiments, $R^{5f}$ is $C_{2-6}$ alkenyl, optionally substituted with one, two, three, four, or five substituents Q as described herein. In certain embodiments, $R^{5f}$ is $C_{2-6}$ alkynyl, optionally substituted with one, two, three, four, or five substituents Q as described herein. In certain embodiments, $R^{5f}$ is $C_{3-10}$ cycloalkyl, optionally substituted with one, two, three, four, or five substituents Q as described herein. In certain embodiments, $R^{5f}$ is $C_{6-14}$ aryl, optionally substituted with one, two, three, four, or five substituents Q as described herein. In certain embodiments, $R^{5f}$ is $C_{7-15}$ aralkyl, optionally substituted with one, two, three, four, or five substituents Q as described herein. In certain embodiments, $R^{5f}$ is heteroaryl, optionally substituted with one, two, three, four, or five substituents Q as described herein. In certain embodiments, $R^{5f}$ is heterocyclyl, optionally substituted with one, two, three, four, or five substituents Q as described herein.

In certain embodiments, $R^{5f}$ is $-C(O)R^{1a}$, wherein $R^{1a}$ is as defined herein. In certain embodiments, $R^{5f}$ is $-C(O)OR^{1a}$, wherein $R^{1a}$ is as defined herein. In certain embodiments, $R^{5f}$ is - $C(O)OR^{1a}$, wherein $R^{1a}$ is $C_{1-6}$ alkyl, optionally substituted with one, two, three, four, or five substituents Q as described herein. In certain embodiments, $R^{5f}$ is $-C(O)OCH_3$. In certain embodiments, $R^{5f}$ is $-C(O)NR^{1b}R^{1c}$, wherein $R^{1b}$ and $R^{1c}$ are each as defined herein. In certain embodiments, $R^{5f}$ is $-C(NR^{1a})NR^{1b}R^{1c}$, wherein $R^{1a}$, $R^{1b}$, and $R^{1c}$ are each as defined herein. In certain embodiments, $R^{5f}$ is $-OR^{1a}$, wherein $R^{1a}$ is as defined herein. In certain embodiments, $R^{5f}$ is - $OC(O)R^{1a}$, wherein $R^{1a}$ is as defined herein. In certain embodiments, $R^{5f}$ is $-OC(O)OR^{1a}$, wherein $R^{1a}$ is as defined herein. In certain embodiments, $R^{5f}$ is $-OC(O)NR^{1b}R^{1c}$, wherein $R^{1b}$ and $R^{1c}$ are each as defined herein. In certain embodiments, $R^{5f}$ is $-OC(=NR^{1a})NR^{1b}R^{1c}$, wherein $R^{1a}$, $R^{1b}$, and $R^{1c}$ are each as defined herein. In certain embodiments, $R^{5f}$ is $-OS(O)R^{1a}$, wherein $R^{1a}$ is as defined herein. In certain embodiments, $R^{5f}$ is $-OS(O)_2R^{1a}$, wherein $R^{1a}$ is as defined herein. In certain embodiments, $R^{5f}$ is $-OS(O)NR^{1b}R^{1c}$, wherein $R^{1b}$ and $R^{1c}$ are each as defined herein. In certain embodiments, $R^{5f}$ is - $OS(O)_2NR^{1b}R^{1c}$, wherein $R^{1b}$ and $R^{1c}$ are each as defined herein. In certain embodiments, $R^{5f}$ is - $NR^{1b}R^{1c}$, wherein $R^{1b}$ and $R^{1c}$ are each as defined herein. In certain embodiments, $R^{5f}$ is amino ($-NH_2$). In certain embodiments, $R^{5f}$ is $-NR^{1a}C(O)R^{1d}$, wherein $R^{1a}$ and $R^{1d}$ are each as defined herein. In certain embodiments, $R^{5f}$ is $-NR^{1a}C(O)OR^{1d}$, wherein $R^{1a}$ and $R^{1d}$ are each as defined herein. In certain embodiments, $R^{5f}$ is $-NR^{1a}C(O)NR^{1b}R^{1c}$, wherein $R^{1a}$, $R^{1b}$, and $R^{1c}$ are each as defined herein. In certain embodiments, $R^{5f}$ is $-NR^{1a}C(=NR^{1d})NR^{1b}R^{1c}$, wherein $R^{1a}$, $R^{1b}$, $R^{1c}$ , and $R^{1d}$ are each as defined herein. In certain embodiments, $R^{5f}$ is $-NR^{1a}S(O)R^{1d}$, wherein $R^{1a}$ and $R^{1d}$ are each as defined herein. In certain embodiments, $R^{5f}$ is $-NR^{1a}S(O)_2R^{1d}$, wherein $R^{1a}$ and $R^{1d}$ are each as defined herein. In certain embodiments, $R^{5f}$ is $-NR^{1a}S(O)NR^{1b}R^{1c}$, wherein $R^{1a}$, $R^{1b}$, and $R^{1c}$ are each as defined herein. In certain embodiments, $R^{5f}$ is $-NR^{1a}S(O)_2NR^{1b}R^{1c}$, wherein $R^{1a}$, $R^{1b}$, and $R^{1c}$ are each as defined herein. In certain embodiments, $R^{5f}$ is $-SR^{1a}$, wherein $R^{1a}$ is as defined herein. In certain embodiments, $R^{5f}$ is $-S(O)R^{1a}$, wherein $R^{1a}$ is as defined herein. In certain embodiments, $R^{5f}$ is-$S(O)_2R^{1a}$, wherein $R^{1a}$ is as defined herein. In certain embodiments, $R^{5f}$ is $-S(O)NR^{1b}R^{1c}$, wherein $R^{1b}$ and $R^{1c}$ are each as defined herein. In certain embodiments, $R^{5f}$ is $-S(O)_2NR^{1b}R^{1c}$; wherein $R^{1b}$ and $R^{1c}$ are each as defined herein.

In certain embodiments, $R^{5g}$ is hydrogen. In certain embodiments, $R^{5g}$ is halo. In certain embodiments, $R^{5g}$ is fluoro, chloro, bromo, or iodo. In certain embodiments, $R^{5g}$ is $C_{1-6}$ alkyl, optionally substituted with one, two, three, four, or five substituents Q as described herein. In certain embodiments, $R^{5g}$ is methyl, optionally substituted with one, two, three, four, or five substituents Q as described herein. In certain embodiments, $R^{5g}$ is methyl. In certain embodiments, $R^{5g}$ is methyl, ethyl, propyl, or butyl, each optionally substituted with one, two, three, four, or five substituents Q as described herein. In certain embodiments, $R^{5g}$ is methyl, ethyl, *n*-propyl, isopropyl, *n*-butyl, isobutyl, or *t*-butyl. In certain embodiments, $R^{5g}$ is $C_{2-6}$ alkenyl, optionally substituted with one, two, three, four, or five substituents Q as described herein. In certain embodiments, $R^{5g}$ is $C_{2-6}$ alkynyl, optionally substituted with one, two, three, four, or five substituents Q as described herein. In certain embodiments, $R^{5g}$ is $C_{3-10}$ cycloalkyl, optionally substituted with one, two, three, four, or five substituents Q as described herein. In certain embodiments, $R^{5g}$ is $C_{6-14}$ aryl, optionally substituted with one, two, three, four, or five substituents Q as described herein. In certain embodiments, $R^{5g}$ is $C_{7-15}$ aralkyl, optionally substituted with one, two, three, four, or five substituents Q as described herein. In certain embodiments, $R^{5g}$ is heteroaryl, optionally substituted with one, two, three, four, or five substituents Q as described herein. In certain embodiments, $R^{5g}$ is heterocyclyl, optionally substituted with one, two, three, four, or five substituents Q as described herein.

In certain embodiments, $R^{5g}$ is $-C(O)R^{1a}$, wherein $R^{1a}$ is as defined herein. In certain embodiments, $R^{5g}$ is $-C(O)OR^{1a}$, wherein $R^{1a}$ is as defined herein. In certain embodiments, $R^{5g}$ is - $C(O)OR^{1a}$, wherein $R^{1a}$ is $C_{1-6}$ alkyl, optionally substituted with one, two, three, four, or five substituents Q as described herein. In certain embodiments, $R^{5g}$ is $-C(O)OCH_3$. In certain embodiments, $R^{5g}$ is $-C(O)NR^{1b}R^{1c}$, wherein $R^{1b}$ and $R^{1c}$ are each as defined herein. In certain embodiments, $R^{5g}$ is $-C(NR^{1a})NR^{1b}R^{1c}$, wherein $R^{1a}$, $R^{1b}$, and $R^{1c}$ are each as defined herein. In certain embodiments, $R^{5g}$ is $-OR^{1a}$, wherein $R^{1a}$ is as defined herein. In certain embodiments, $R^{5g}$ is - $OC(O)R^{1a}$, wherein $R^{1a}$ is as defined herein. In certain embodiments, $R^{5g}$ is $-OC(O)OR^{1a}$, wherein $R^{1a}$ is as defined herein. In certain embodiments, $R^{5g}$ is $-OC(O)NR^{1b}R^{1c}$, wherein $R^{1b}$ and $R^{1c}$ are each as defined herein. In certain embodiments, $R^{5g}$ is $-OC(=NR^{1a})NR^{1b}R^{1c}$,

wherein $R^{1a}$, $R^{1b}$, and $R^{1c}$ are each as defined herein. In certain embodiments, $R^{5g}$ is -OS(O)$R^{1a}$, wherein $R^{1a}$ is as defined herein. In certain embodiments, $R^{5g}$ is -OS(O)$_2R^{1a}$, wherein $R^{1a}$ is as defined herein. In certain embodiments, $R^{5g}$ is -OS(O)N$R^{1b}R^{1c}$, wherein $R^{1b}$ and $R^{1c}$ are each as defined herein. In certain embodiments, $R^{5g}$ is -OS(O)$_2$N$R^{1b}R^{1c}$, wherein $R^{1b}$ and $R^{1c}$ are each as defined herein. In certain embodiments, $R^{5g}$ is - N$R^{1b}R^{1c}$, wherein $R^{1b}$ and $R^{1c}$ are each as defined herein. In certain embodiments, $R^{5g}$ is amino (-$NH_2$). In certain embodiments, $R^{5g}$ is -N$R^{1a}$C(O)$R^{1b}$, wherein $R^{1a}$ and $R^{1d}$ are each as defined herein. In certain embodiments, $R^{5g}$ is -N$R^{1a}$C(O)O$R^{1d}$, wherein $R^{1a}$ and $R^{1d}$ are each as defined herein. In certain embodiments, $R^{5g}$ is -N$R^{1a}$C(O)N$R^{1b}R^{1c}$, wherein $R^{1a}$, $R^{1b}$, and $R^{1c}$ are each as defined herein. In certain embodiments, $R^{5g}$ is -N$R^{1a}$C(=N$R^{1d}$)N$R^{1b}R^{1c}$, wherein $R^{1a}$, $R^{1b}$, $R^{1c}$ , and $R^{1d}$ are each as defined herein. In certain embodiments, $R^{5g}$ is -N$R^{1a}$S(O)$R^{1d}$, wherein $R^{1a}$ and $R^{1d}$ are each as defined herein. In certain embodiments, $R^{5g}$ is -N$R^{1a}$S(O)$_2R^{1d}$, wherein $R^{1a}$ and $R^{1d}$ are each as defined herein. In certain embodiments, $R^{5g}$ is -N$R^{1a}$S(O)N$R^{1b}R^{1c}$, wherein $R^{1a}$, $R^{1b}$, and $R^{1c}$ are each as defined herein. In certain embodiments, $R^{5g}$ is -N$R^{1a}$S(O)$_2$N$R^{1b}R^{1c}$, wherein $R^{1a}$, $R^{1b}$, and $R^{1c}$ are each as defined herein. In certain embodiments, $R^{5g}$ is -S$R^{1a}$, wherein $R^{1a}$ is as defined herein. In certain embodiments, $R^{5g}$ is -S(O)$R^{1a}$, wherein $R^{1a}$ is as defined herein. In certain embodiments, $R^{5g}$ is - S(O)$_2R^{1a}$, wherein $R^{1a}$ is as defined herein. In certain embodiments, $R^{5g}$ is -S(O)N$R^{1b}R^{1c}$, wherein $R^{1b}$ and $R^{1c}$ are each as defined herein. In certain embodiments, $R^{5g}$ is -S(O)$_2$N$R^{1b}R^{1c}$; wherein $R^{1b}$ and $R^{1c}$ are each as defined herein.

In certain embodiments, when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a $C_{3-7}$ cycloalkyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cyclopropyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cyclobutyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cyclopentyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cyclohexyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cycloheptyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a cyclopropyl.

In certain embodiments, when one occurrence of $R^{5f}$ and one occurrence of $R^{5g}$ are attached to the same carbon atom, the $R^{5f}$ and $R^{5g}$ together with the carbon atom to which they are attached form a heterocyclyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a 3-membered heterocyclyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a 4-membered heterocyclyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a 5-membered heterocyclyl, optionally substituted with one, two, three, four, or five substituents Q as described herein. In certain embodiments, when one occurrence of $R^{5f}$ and one occurrence of $R^{5g}$ are attached to the same carbon atom, the $R^{5f}$ and $R^{5g}$ together with the carbon atom to which they are attached form a 6-membered heterocyclyl, optionally substituted with one, two, three, four, or five substituents Q as described herein.

In certain embodiments, $R^{7a}$ is hydrogen. In certain embodiments, $R^{7a}$ is cyano. In certain embodiments, $R^{7a}$ is halo. In certain embodiments, $R^{7a}$ is fluoro, chloro, bromo, or iodo. In certain embodiments, $R^{7a}$ is nitro. In certain embodiments, $R^{7a}$ is $C_{1-6}$ alkyl, optionally substituted with one, two, three, or four substituents $Q^a$ as described herein. In certain embodiments, $R^{7a}$ is $C_{2-6}$ alkenyl, optionally substituted with one, two, three, or four substituents $Q^a$ as described herein. In certain embodiments, $R^{7a}$ is $C_{2-6}$ alkynyl, optionally substituted with one, two, three, or four substituents $Q^a$ as described herein. In certain embodiments, $R^{7a}$ is $C_{3-7}$ cycloalkyl, optionally substituted with one, two, three, or four substituents $Q^a$ as described herein. In certain embodiments, $R^{7a}$ is $C_{3-10}$ cycloalkyl, optionally substituted with one, two, three, or four substituents $Q^a$ as described herein. In certain embodiments, $R^{7a}$ is $C_{6-14}$ aryl, optionally

substituted with one, two, three, or four substituents $Q^a$ as described herein. In certain embodiments, $R^{7a}$ is phenyl, optionally substituted with one, two, three, or four substituents $Q^a$ as described herein. In certain embodiments, $R^{7a}$ is phenyl, optionally substituted with one or more substituents, each of which is selected independently from the group consisting of fluoro, chloro, bromo, methyl, and methoxy. In certain embodiments, $R^{7a}$ is phenyl, 2-fluorophenyl, 2-chlorophenyl, 2-bromophenyl, 2-methylphenyl, 2-methoxyphenyl, 3-fluorophenyl, 3-chlorophenyl, 3-methoxyphenyl, 4-fluorophenyl, 4-chlorophenyl, 4-bromophenyl, 4-methoxyphenyl. In certain embodiments, $R^{7a}$ is $C_{7-15}$ aralkyl, optionally substituted with one, two, three, or four substituents $Q^a$ as described herein. In certain embodiments, $R^{7a}$ is heteroaryl, optionally substituted with one, two, three, or four substituents $Q^a$ as described herein. In certain embodiments, $R^{7a}$ is monocyclic heteroaryl, optionally substituted with one, two, three, or four substituents $Q^a$ as described herein. In certain embodiments, $R^{7a}$ is 5-membered heteroaryl, optionally substituted with one, two, three, or four substituents $Q^a$ as described herein. In certain embodiments, $R^{7a}$ is imidazolyl or pyrozolyl, optionally substituted with one, two, three, or four substituents $Q^a$ as described herein. In certain embodiments, $R^{7a}$ is imidazol-1-yl, pyrozol-4-yl, 1-methyl-pyrozol-4-yl, or 2-methylpyrozol-3-yl. In certain embodiments, $R^{7a}$ is 6-membered heteroaryl, optionally substituted with one, two, three, or four substituents $Q^a$ as described herein. In certain embodiments, $R^{7a}$ is pyridinyl, optionally substituted with one, two, three, or four substituents $Q^a$ as described herein. In certain embodiments, $R^{7a}$ is pyridin-2-yl, pyridin-3-yl, pyridin-4-yl, 2-methylpyridin-4-yl, or 2-methoxypyridin-4-yl. In certain embodiments, $R^{7a}$ is heterocyclyl, optionally substituted with one, two, three, or four substituents $Q^a$ as described herein. In certain embodiments, $R^{7a}$ is monocyclic heterocyclyl, optionally substituted with one, two, three, or four substituents $Q^a$ as described herein. In certain embodiments, $R^{7a}$ is 5-membered heterocyclyl, optionally substituted with one, two, three, or four substituents $Q^a$ as described herein. In certain embodiments, $R^{7a}$ is 6-membered heterocyclyl, optionally substituted with one, two, three, or four substituents $Q^a$ as described herein. In certain embodiments, $R^{7a}$ is piperidinyl or piperazinyl, optionally substituted with one, two, three, or four substituents $Q^a$ as described herein. In certain embodiments, $R^{7a}$ is 1-methylpiperidin-4-yl, or 4-methylpiperazin-1-yl.

In certain embodiments, $R^{7a}$ is $-C(O)R^a$, wherein $R^a$ is as defined herein. In certain embodiments, $R^{7a}$ is $-C(O)OR^a$, wherein $R^a$ is as defined herein. In certain embodiments, $R^{7a}$ is $-C(O)NR^bR^c$, wherein $R^b$ and $R^c$ are each as defined herein. In certain embodiments, $R^{7a}$ is $-C(NR^a)NR^bR^c$, wherein $R^a$, $R^b$, and $R^c$ are each as defined herein. In certain embodiments, $R^{7a}$ is $-OR^a$, wherein $R^a$ is as defined herein. In certain embodiments, $R^a$ is $-O-C_{1-6}$ alkyl, wherein the alkyl is optionally substituted with one, two, three, or four substituents $Q^a$ as described herein. In certain embodiments, $R^a$ is methoxy, ethoxy, propoxy, isopropoxy, or 3-dimethylaminopropoxy. In certain embodiments, $R^{7a}$ is $-OC(O)R^a$, wherein $R^a$ is as defined herein. In certain embodiments, $R^{7a}$ is $-OC(O)OR^a$, wherein $R^a$ is as defined herein. In certain embodiments, $R^{7a}$ is $-OC(O)NR^bR^c$, wherein $R^b$ and $R^c$ are each as defined herein. In certain embodiments, $R^{7a}$ is $-OC(=NR^a)NR^bR^c$, wherein $R^a$, $R^b$, and $R^c$ are each as defined herein. In certain embodiments, $R^{7a}$ is $-OS(O)R^a$, wherein $R^a$ is as defined herein. In certain embodiments, $R^{7a}$ is $-OS(O)_2R^a$, wherein $R^a$ is as defined herein. In certain embodiments, $R^{7a}$ is $-OS(O)NR^bR^c$, wherein $R^b$ and $R^c$ are each as defined herein. In certain embodiments, $R^{7a}$ is $-OS(O)_3NR^bR^c$, wherein $R^b$ and $R^c$ are each as defined herein. In certain embodiments, $R^{7a}$ is $-NR^bR^c$, wherein $R^b$ and $R^c$ are each as defined herein. In certain embodiments, $R^{7a}$ is amino ($-NH_2$). In certain embodiments, $R^{7a}$ is $-NR^aC(O)R^d$, wherein $R^a$ and $R^d$ are each as defined herein. In certain embodiments, $R^{7a}$ is $-NR^aC(O)OR^d$, wherein $R^a$ and $R^d$ are each as defined herein. In certain embodiments, $R^{7a}$ is $-NR^aC(O)NR^bR^c$, wherein $R^a$, $R^b$, and $R^c$ are each as defined herein. In certain embodiments, $R^{7a}$ is $-NR^aC(=NR^d)NR^bR^c$, wherein $R^a$, $R^b$, $R^c$, and $R^d$ are each as defined herein. In certain embodiments, $R^{7a}$ is $-NR^aS(O)R^d$, wherein $R^a$ and $R^d$ are each as defined herein. In certain embodiments, $R^{7a}$ is $-NR^aS(O)_2R^d$, wherein $R^a$ and $R^d$ are each as defined herein. In certain embodiments, $R^{7a}$ is $-NR^aS(O)NR^bR^c$, wherein $R^a$, $R^b$, and $R^c$ are each as defined herein. In certain embodiments, $R^{7a}$ is $-NR^aS(O)_2NR^bR^c$, wherein $R^a$, $R^b$, and $R^c$ are each as defined herein. In certain embodiments, $R^{7a}$ is $-SR^a$, wherein $R^a$ is as defined herein. In certain embodiments, $R^{7a}$ is $-S(O)R^a$, wherein $R^a$ is as defined herein. In certain embodiments, $R^{7a}$ is $-S(O)_2R^a$, wherein $R^a$ is as defined herein. In certain embodiments, $R^{7a}$ is $-S(O)NR^bR^c$, wherein $R^b$ and $R^c$ are each as defined herein. In certain embodiments, $R^{7a}$ is $-S(O)_2NR^bR^c$; wherein $R^b$ and $R^c$ are each as defined herein.

In certain embodiments, $R^{7a}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$. In certain embodiments, $R^{7a}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments, $R^{7b}$ is hydrogen. In certain embodiments, $R^{7b}$ is cyano. In certain embodiments, $R^{7b}$ is halo. In certain embodiments, $R^{7b}$ is fluoro, chloro, bromo, or iodo. In certain embodiments, $R^{7b}$ is nitro. In certain embodiments, $R^{7b}$ is $C_{1-6}$ alkyl, optionally substituted with one, two, three, or four substituents $Q^a$ as described herein.

In certain embodiments, $R^{7b}$ is $C_{2-6}$ alkenyl, optionally substituted with one, two, three, or four substituents $Q^a$ as described herein. In certain embodiments, $R^{7b}$ is $C_{2-6}$ alkynyl, optionally substituted with one, two, three, or four substituents $Q^a$ as described herein. In certain embodiments, $R^{7b}$ is $C_{3-10}$ cycloalkyl, optionally substituted with one, two, three, or four substituents $Q^a$ as described herein. In certain embodiments, $R^{7b}$ is $C_{3-7}$ cycloalkyl, optionally substituted with one, two, three, or four substituents $Q^a$ as described herein. In certain embodiments, $R^{7b}$ is $C_{6-14}$ aryl, optionally substituted with one, two, three, or four substituents $Q^a$ as described herein. In certain embodiments, $R^{7b}$ is $C_{7-15}$ aralkyl, optionally substituted with one, two, three, or four substituents $Q^a$ as described herein. In certain embodiments, $R^{7b}$ is heteroaryl, optionally substituted with one, two, three, or four substituents $Q^a$ as described herein. In certain embodiments, $R^{7b}$ is heterocyclyl, optionally substituted with one, two, three, or four substituents $Q^a$ as described herein.

In certain embodiments, $R^{7b}$ is $-C(O)R^a$, wherein $R^a$ is as defined herein. In certain embodiments, $R^{7b}$ is $-C(O)OR^a$, wherein $R^a$ is as defined herein. In certain embodiments, $R^{7b}$ is $-C(O)NR^bR^c$, wherein $R^b$ and $R^c$ are each as defined herein. In certain embodiments, $R^{7b}$ is $-C(NR^a)NR^bR^c$, wherein $R^a$, $R^b$, and $R^c$ are each as defined herein. In certain embodiments, $R^{7b}$ is $-OR^a$, wherein $R^a$ is as defined herein. In certain embodiments, $R^a$ is $-O-C_{1-6}$ alkyl, wherein the alkyl is optionally substituted with one, two, three, or four substituents $Q^a$ as described herein. In certain embodiments, $R^a$ is methoxy, ethoxy, propoxy, isopropoxy, or 3-dimethylaminopropoxy. In certain embodiments, $R^{7b}$ is $-OC(O)R^a$, wherein $R^a$ is as defined herein. In certain embodiments, $R^{7b}$ is $-OC(O)OR^a$, wherein $R^a$ is as defined herein. In certain embodiments, $R^{7b}$ is $-OC(O)NR^bR^c$, wherein $R^b$ and $R^c$ are each as defined herein. In certain embodiments, $R^{7b}$ is $-OC(=NR^a)NR^bR^c$, wherein $R^a$, $R^b$, and $R^c$ are each as defined herein. In certain embodiments, $R^{7b}$ is $-OS(O)R^a$, wherein $R^a$ is as defined herein. In certain embodiments, $R^{7b}$ is $-OS(O)_2R^a$, wherein $R^a$ is as defined herein. In certain embodiments, $R^{7b}$ is $-OS(O)NR^bR^c$, wherein $R^b$ and $R^c$ are each as defined herein. In certain embodiments, $R^{7b}$ is $-OS(O)_2NR^bR^c$, wherein $R^b$ and $R^c$ are each as defined herein. In certain embodiments, $R^{7b}$ is $-NR^bR^c$, wherein $R^b$ and $R^c$ are each as defined herein. In certain embodiments, $R^{7b}$ is amino ($-NH_2$). In certain embodiments, $R^{7b}$ is $-NR^aC(O)R^d$, wherein $R^a$ and $R^d$ are each as defined herein. In certain embodiments, $R^{7b}$ is $-NR^aC(O)OR^d$, wherein $R^a$ and $R^d$ are each as defined herein. In certain embodiments, $R^{7b}$ is $-NR^aC(O)NR^bR^c$, wherein $R^a$, $R^b$, and $R^c$ are each as defined herein. In certain embodiments, $R^{7b}$ is $-NR^aC(=NR^d)NR^bR^c$, wherein $R^a$, $R^b$, $R^c$, and $R^d$ are each as defined herein. In certain embodiments, $R^{7b}$ is $-NR^aS(O)R^d$, wherein $R^a$ and $R^d$ are each as defined herein. In certain embodiments, $R^{7b}$ is $-NR^aS(O)_2R^d$, wherein $R^a$ and $R^d$ are each as defined herein. In certain embodiments, $R^{7b}$ is $-NR^aS(O)NR^bR^c$, wherein $R^a$, $R^b$, and $R^c$ are each as defined herein. In certain embodiments, $R^{7b}$ is $-NR^aS(O)_2NR^bR^c$, wherein $R^a$, $R^b$, and $R^c$ are each as defined herein. In certain embodiments, $R^{7b}$ is $-SR^a$, wherein $R^a$ is as defined herein. In certain embodiments, $R^{7b}$ is $-S(O)R^a$, wherein $R^a$ is as defined herein. In certain embodiments, $R^{7b}$ is $-S(O)_2R^a$, wherein $R^a$ is as defined herein. In certain embodiments, $R^{7b}$ is $-S(O)NR^bR^c$, wherein $R^b$ and $R^c$ are each as defined herein. In certain embodiments, $R^{7b}$ is $-S(O)_2NR^bR^c$; wherein $R^b$ and $R^c$ are each as defined herein.

In certain embodiments, $R^{7b}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$. In certain embodiments, $R^{7b}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments, $R^{7c}$ is hydrogen. In certain embodiments, $R^{7c}$ is cyano. In certain embodiments, $R^{7c}$ is halo. In certain embodiments, $R^{7c}$ is fluoro, chloro, bromo, or iodo. In certain embodiments, $R^{7c}$ is nitro. In certain embodiments, $R^{7c}$ is $C_{1-6}$ alkyl, optionally substituted with one, two, three, or four substituents $Q^a$ as described herein. In certain embodiments, $R^{7c}$ is $C_{2-6}$ alkenyl, optionally substituted with one, two, three, or four substituents $Q^a$ as described herein. In certain embodiments, $R^{7c}$ is $C_{2-6}$ alkynyl, optionally substituted with one, two, three, or four substituents $Q^a$ as described herein. In certain embodiments, $R^{7c}$ is $C_{3-10}$ cycloalkyl, optionally substituted with one, two, three, or four substituents $Q^a$ as described herein. In certain embodiments, $R^{7c}$ is $C_{3-7}$ cycloalkyl, optionally substituted with one, two, three, or four substituents $Q^a$ as described herein. In certain embodiments, $R^{7c}$ is $C_{6-14}$ aryl, optionally substituted with one, two, three, or four substituents $Q^a$ as described herein. In certain embodiments, $R^{7c}$ is $C_{7-15}$ aralkyl, optionally substituted with one, two, three, or four substituents $Q^a$ as described herein. In certain embodiments, $R^{7c}$ is heteroaryl, optionally substituted with one, two, three, or four substituents $Q^a$ as described herein. In certain embodiments, $R^{7c}$ is heterocyclyl, optionally substituted with one, two, three, or four substituents $Q^a$ as described herein.

In certain embodiments, $R^{7c}$ is $-C(O)R^a$, wherein $R^a$ is as defined herein. In certain embodiments, $R^{7c}$ is $-C(O)OR^a$, wherein $R^a$ is as defined herein. In certain embodiments, $R^{7c}$ is $-C(O)NR^bR^c$, wherein $R^b$ and $R^c$ are each as defined herein. In certain embodiments, $R^{7c}$ is $-C(NR^a)NR^bR^c$, wherein $R^a$, $R^b$, and $R^c$ are each as defined herein. In certain embodiments, $R^{7c}$ is $-OR^a$, wherein $R^a$ is as defined herein. In certain embodiments, $R^a$ is $-O-C_{1-6}$ alkyl, wherein the alkyl is optionally substituted with one, two, three, or four substituents $Q^a$ as described herein. In certain

embodiments, $R^a$ is methoxy, ethoxy, propoxy, isopropoxy, or 3-dimethylaminopropoxy. In certain embodiments, $R^{7c}$ is -OC(O)$R^a$, wherein $R^a$ is as defined herein. In certain embodiments, $R^{7c}$ is - OC(O)O$R^a$, wherein $R^a$ is as defined herein. In certain embodiments, $R^{7c}$ is -OC(O)N$R^bR^c$, wherein $R^b$ and $R^c$ are each as defined herein. In certain embodiments, $R^{7c}$ is -OC(=N$R^a$)N$R^bR^c$, wherein $R^a$, $R^b$, and $R^c$ are each as defined herein. In certain embodiments, $R^{7c}$ is -OS(O)$R^a$, wherein $R^a$ is as defined herein. In certain embodiments, $R^{7c}$ is -OS$(O)_2R^a$, wherein $R^a$ is as defined herein. In certain embodiments, $R^{7c}$ is -OS(O)N$R^bR^c$, wherein $R^b$ and $R^c$ are each as defined herein. In certain embodiments, $R^{7c}$ is -OS$(O)_2$N$R^bR^c$, wherein $R^b$ and $R^c$ are each as defined herein. In certain embodiments, $R^{7c}$ is -N$R^bR^c$, wherein $R^b$ and $R^c$ are each as defined herein. In certain embodiments, $R^{7c}$ is amino (-$NH_2$). In certain embodiments, $R^{7c}$ is -N$R^a$C(O)$R^d$, wherein $R^a$ and $R^d$ are each as defined herein. In certain embodiments, $R^{7c}$ is -N$R^a$C(O)O$R^d$, wherein $R^a$ and $R^d$ are each as defined herein. In certain embodiments, $R^{7c}$ is -N$R^a$C(O)N$R^bR^c$, wherein $R^a$, $R^b$, and $R^c$ are each as defined herein. In certain embodiments, $R^{7c}$ is -N$R^a$C(=N$R^d$)N$R^bR^c$, wherein $R^a$, $R^b$, $R^c$, and $R^d$ are each as defined herein. In certain embodiments, $R^{7c}$ is -N$R^a$S(O)$R^d$, wherein $R^a$ and $R^d$ are each as defined herein. In certain embodiments, $R^{7c}$ is -N$R^a$S$(O)_2R^d$, wherein $R^a$ and $R^d$ are each as defined herein. In certain embodiments, $R^{7c}$ is -N$R^a$S(O)N$R^bR^c$, wherein $R^a$, $R^b$, and $R^c$ are each as defined herein. In certain embodiments, $R^{7c}$ is -N$R^a$S$(O)_2$N$R^bR^c$, wherein $R^a$, $R^b$, and $R^c$ are each as defined herein. In certain embodiments, $R^{7c}$ is -S$R^a$, wherein $R^a$ is as defined herein. In certain embodiments, $R^{7c}$ is - S(O)$R^a$, wherein $R^a$ is as defined herein. In certain embodiments, $R^{7c}$ is -S$(O)_2R^a$, wherein $R^a$ is as defined herein. In certain embodiments, $R^{7c}$ is -S(O)N$R^bR^c$, wherein $R^b$ and $R^c$ are each as defined herein. In certain embodiments, $R^{7c}$ is -S$(O)_2$N$R^bR^c$; wherein $R^b$ and $R^c$ are each as defined herein.

In certain embodiments, $R^{7c}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$. In certain embodiments, $R^{7c}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments, $R^{7d}$ is hydrogen. In certain embodiments, $R^{7d}$ is cyano. In certain embodiments, $R^{7d}$ is halo. In certain embodiments, $R^{7d}$ is fluoro, chloro, bromo, or iodo. In certain embodiments, $R^{7d}$ is nitro. In certain embodiments, $R^{7d}$ is $C_{1-6}$ alkyl, optionally substituted with one, two, three, or four substituents $Q^a$ as described herein. In certain embodiments, $R^{7d}$ is $C_{2-6}$ alkenyl, optionally substituted with one, two, three, or four substituents $Q^a$ as described herein. In certain embodiments, $R^{7d}$ is $C_{2-6}$ alkynyl, optionally substituted with one, two, three, or four substituents $Q^a$ as described herein. In certain embodiments, $R^{7d}$ is $C_{3-10}$ cycloalkyl, optionally substituted with one, two, three, or four substituents $Q^a$ as described herein. In certain embodiments, $R^{7d}$ is $C_{3-7}$ cycloalkyl, optionally substituted with one, two, three, or four substituents $Q^a$ as described herein. In certain embodiments, $R^{7d}$ is $C_{6-14}$ aryl, optionally substituted with one, two, three, or four substituents $Q^a$ as described herein. In certain embodiments, $R^{7d}$ is $C_{7-15}$ aralkyl, optionally substituted with one, two, three, or four substituents $Q^a$ as described herein. In certain embodiments, $R^{7d}$ is heteroaryl, optionally substituted with one, two, three, or four substituents $Q^a$ as described herein. In certain embodiments, $R^{7d}$ is heterocyclyl, optionally substituted with one, two, three, or four substituents $Q^a$ as described herein.

In certain embodiments, $R^{7d}$ is -C(O)$R^a$, wherein $R^a$ is as defined herein. In certain embodiments, $R^{7d}$ is -C(O)O$R^a$, wherein $R^a$ is as defined herein. In certain embodiments, $R^{7d}$ is - C(O)N$R^bR^c$, wherein $R^b$ and $R^c$ are each as defined herein. In certain embodiments, $R^{7d}$ is - C(N$R^a$)N$R^bR^c$, wherein $R^a$, $R^b$, and $R^c$ are each as defined herein. In certain embodiments, $R^{7d}$ is - O$R^a$, wherein $R^a$ is as defined herein. In certain embodiments, $R^a$ is -O-$C_{1-6}$ alkyl, wherein the alkyl is optionally substituted with one, two, three, or four substituents $Q^a$ as described herein. In certain embodiments, $R^a$ is methoxy, ethoxy, propoxy, isopropoxy, or 3-dimethylaminopropoxy. In certain embodiments, $R^{7d}$ is -OC(O)$R^a$, wherein $R^a$ is as defined herein. In certain embodiments, $R^{7d}$ is - OC(O)O$R^a$, wherein $R^a$ is as defined herein. In certain embodiments, $R^{7d}$ is -OC(O)N$R^bR^c$, wherein $R^b$ and $R^c$ are each as defined herein. In certain embodiments, $R^{7d}$ is -OC(=N$R^a$)N$R^bR^c$, wherein $R^a$, $R^b$, and $R^c$ are each as defined herein. In certain embodiments, $R^{7d}$ is -OS(O)$R^a$, wherein $R^a$ is as defined herein. In certain embodiments, $R^{7d}$ is -OS$(O)_2R^a$, wherein $R^a$ is as defined herein. In certain embodiments, $R^{7d}$ is -OS(O)N$R^bR^c$, wherein $R^b$ and $R^c$ are each as defined herein. In certain embodiments, $R^{7d}$ is -OS$(O)_2$N$R^bR^c$, wherein $R^b$ and $R^c$ are each as defined herein. In certain embodiments, $R^{7d}$ is -N$R^bR^c$, wherein $R^b$ and $R^c$ are each as defined herein. In certain embodiments, $R^{7d}$ is amino (-$NH_2$). In certain embodiments, $R^{7d}$ is -N$R^a$C(O)$R^d$, wherein $R^a$ and $R^d$ are each as defined herein. In certain embodiments, $R^{7d}$ is -N$R^a$C(O)O$R^d$, wherein $R^a$ and $R^d$ are each as defined herein. In certain embodiments, $R^{7d}$ is -N$R^a$C(O)N$R^bR^c$, wherein $R^a$, $R^b$, and $R^c$ are each as defined herein. In certain embodiments, $R^{7d}$ is -N$R^a$C(=N$R^d$)N$R^bR^c$, wherein $R^a$, $R^b$, $R^c$, and $R^d$ are each as defined herein. In certain embodiments, $R^{7d}$ is -N$R^a$S(O)$R^d$, wherein $R^a$ and $R^d$ are each as defined herein. In certain embodiments, $R^{7d}$ is -N$R^a$S$(O)_2R^d$, wherein $R^a$ and $R^d$ are each as defined herein. In certain embodiments, $R^{7d}$ is -N$R^a$S(O)N$R^bR^c$, wherein $R^a$, $R^b$, and $R^c$ are each as defined herein. In certain embodiments, $R^{7d}$ is -N$R^a$S$(O)_2$N$R^bR^c$, wherein $R^a$, $R^b$, and $R^c$

are each as defined herein. In certain embodiments, $R^{7d}$ is -$SR^a$, wherein $R^a$ is as defined herein. In certain embodiments, $R^{7d}$ is - $S(O)R^a$, wherein $R^a$ is as defined herein. In certain embodiments, $R^{7d}$ is -$S(O)_2R^a$, wherein $R^a$ is as defined herein. In certain embodiments, $R^{7d}$ is -$S(O)NR^bR^c$, wherein $R^b$ and $R^c$ are each as defined herein. In certain embodiments, $R^{7d}$ is -$S(O)_2NR^bR^c$; wherein $R^b$ and $R^c$ are each as defined herein.

In certain embodiments, $R^{7d}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$. In certain embodiments, $R^{7d}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments, $R^{7e}$ is hydrogen. In certain embodiments, $R^{7e}$ is cyano. In certain embodiments, $R^{7e}$ is halo. In certain embodiments, $R^{7e}$ is fluoro, chloro, bromo, or iodo. In certain embodiments, $R^{7e}$ is nitro. In certain embodiments, $R^{7e}$ is $C_{1-6}$ alkyl, optionally substituted with one, two, three, or four substituents $Q^a$ as described herein. In certain embodiments, $R^{7e}$ is $C_{2-6}$ alkenyl, optionally substituted with one, two, three, or four substituents $Q^a$ as described herein. In certain embodiments, $R^{7e}$ is $C_{2-6}$ alkynyl, optionally substituted with one, two, three, or four substituents $Q^a$ as described herein. In certain embodiments, $R^{7e}$ is $C_{3-10}$ cycloalkyl, optionally substituted with one, two, three, or four substituents $Q^a$ as described herein. In certain embodiments, $R^{7e}$ is $C_{3-7}$ cycloalkyl, optionally substituted with one, two, three, or four substituents $Q^a$ as described herein. In certain embodiments, $R^{7e}$ is $C_{6-14}$ aryl, optionally substituted with one, two, three, or four substituents $Q^a$ as described herein. In certain embodiments, $R^{7e}$ is $C_{7-15}$ aralkyl, optionally substituted with one, two, three, or four substituents $Q^a$ as described herein. In certain embodiments, $R^{7e}$ is heteroaryl, optionally substituted with one, two, three, or four substituents $Q^a$ as described herein. In certain embodiments, $R^{7e}$ is heterocyclyl, optionally substituted with one, two, three, or four substituents $Q^a$ as described herein.

In certain embodiments, $R^{7e}$ is -$C(O)R^a$, wherein $R^a$ is as defined herein. In certain embodiments, $R^{7e}$ is -$C(O)OR^a$, wherein $R^a$ is as defined herein. In certain embodiments, $R^{7e}$ is - $C(O)NR^bR^c$, wherein $R^b$ and $R^c$ are each as defined herein. In certain embodiments, $R^{7e}$ is - $C(NR^a)NR^bR^c$, wherein $R^a$, $R^b$, and $R^c$ are each as defined herein. In certain embodiments, $R^{7e}$ is - $OR^a$, wherein $R^a$ is as defined herein. In certain embodiments, $R^a$ is -O-$C_{1-6}$ alkyl, wherein the alkyl is optionally substituted with one, two, three, or four substituents $Q^a$ as described herein. In certain embodiments, $R^a$ is methoxy, ethoxy, propoxy, isopropoxy, or 3-dimethylaminopropoxy. In certain embodiments, $R^{7e}$ is -$OC(O)R^a$, wherein $R^a$ is as defined herein. In certain embodiments, $R^{7e}$ is - $OC(O)OR^a$, wherein $R^a$ is as defined herein. In certain embodiments, $R^{7e}$ is -$OC(O)NR^bR^c$, wherein $R^b$ and $R^c$ are each as defined herein. In certain embodiments, $R^{7e}$ is -$OC(=NR^a)NR^bR^c$, wherein $R^a$, $R^b$, and $R^c$ are each as defined herein. In certain embodiments, $R^{7e}$ is -$OS(O)R^a$, wherein $R^a$ is as defined herein. In certain embodiments, $R^{7e}$ is -$OS(O)_2R^a$, wherein $R^a$ is as defined herein. In certain embodiments, $R^{7e}$ is -$OS(O)NR^bR^c$, wherein $R^b$ and $R^c$ are each as defined herein. In certain embodiments, $R^{7e}$ is -$OS(O)_2NR^bR^c$, wherein $R^b$ and $R^c$ are each as defined herein. In certain embodiments, $R^{7e}$ is -$NR^bR^c$, wherein $R^b$ and $R^c$ are each as defined herein. In certain embodiments, $R^{7e}$ is amino (-$NH_2$). In certain embodiments, $R^{7e}$ is -$NR^aC(O)R^d$, wherein $R^a$ and $R^d$ are each as defined herein. In certain embodiments, $R^{7e}$ is -$NR^aC(O)OR^d$, wherein $R^a$ and $R^d$ are each as defined herein. In certain embodiments, $R^{7e}$ is -$NR^aC(O)NR^bR^c$, wherein $R^a$, $R^b$, and $R^c$ are each as defined herein. In certain embodiments, $R^{7e}$ is -$NR^aC(=NR^d)NR^bR^c$, wherein $R^a$, $R^b$, $R^c$, and $R^d$ are each as defined herein. In certain embodiments, $R^{7e}$ is -$NR^aS(O)R^d$, wherein $R^a$ and $R^d$ are each as defined herein. In certain embodiments, $R^{7e}$ is -$NR^aS(O)_2R^d$, wherein $R^a$ and $R^d$ are each as defined herein. In certain embodiments, $R^{7e}$ is -$NR^aS(O)NR^bR^c$, wherein $R^a$, $R^b$, and $R^c$ are each as defined herein. In certain embodiments, $R^{7e}$ is -$NR^aS(O)_2NR^bR^c$, wherein $R^a$, $R^b$, and $R^c$ are each as defined herein. In certain embodiments, $R^{7e}$ is -$SR^a$, wherein $R^a$ is as defined herein. In certain embodiments, $R^{7e}$ is - $S(O)R^a$, wherein $R^a$ is as defined herein. In certain embodiments, $R^{7e}$ is -$S(O)_2R^a$, wherein $R^a$ is as defined herein. In certain embodiments, $R^{7e}$ is -$S(O)NR^bR^c$, wherein $R^b$ and $R^c$ are each as defined herein. In certain embodiments, $R^{7e}$ is -$S(O)_2NR^bR^c$; wherein $R^b$ and $R^c$ are each as defined herein.

In certain embodiments, $R^{7e}$ is phenyl, imidazolyl, pyrozolyl, pyridinyl, pyrimidinyl, pyrrolidinyl, piperidinyl, or piperazinyl, each optionally substituted with one, two, three, or four substituents $Q^a$. In certain embodiments, $R^{7e}$ is phenyl, 2-fluorophenyl, 2-chlorophenyl, 2-bromophenyl, 2-methylphenyl, 2-(3-dimethylaminopropyl)phenyl, 2-methoxyphenyl, 3-fluorophenyl, 3-chlorophenyl, 3-methylphenyl, 3-methoxyphenyl, 4-fluorophenyl, 4-chlorophenyl, 4-bromophenyl, 4-methoxyphenyl, 2,4-difluorophenyl, 2,6-difluorophenyl, 4-fluoro-3-methoxyphenyl, 3-methoxyphenyl, 4-methoxyphenyl, 3-morpholin-4-ylmethylphenyl, imidazol-1-yl, pyrozol-4-yl, 1-methyl-pyrozol-4-yl, 2-methylpyrozol-3-yl, pyridin-2-yl, pyridin-3-yl, pyridin-4-yl, 2-fluoropyridin-3-yl, 2-methylpyridin-4-yl, 2-(4-methylpiperazin-1-yl)pyridin-4-yl, 2-methoxypyridin-4-yl, pyrimidin-5-yl, pyrrolidin-3-yl, 1-methylpyrrolidin-3-yl, piperidin-4-yl, 1-methylpiperidin-4-yl, 1-ethylpiperidin-4-yl, 1-isopropylpiperidin-4-yl, 1-acetylpiperidin-4-yl, 1-methylsulfonylpiperidin-4-yl, or 4-methylpiperazin-1-yl.

In certain embodiments, $R^{7a}$ and $R^{7b}$ together with the carbon atoms to which they are attached form $C_{3-10}$

cycloalkenyl, $C_{6-14}$ aryl, heteroaryl, or heterocyclyl, each optionally substituted with one, two, three, or four substituents $Q^a$. In certain embodiments, $R^{7a}$ and $R^{7b}$ together with the carbon atoms to which they are attached form $C_{3-10}$ cycloalkenyl, optionally substituted with one, two, three, or four substituents $Q^a$. In certain embodiments, $R^{7a}$ and $R^{7b}$ together with the carbon atoms to which they are attached form cyclohexenyl, optionally substituted with one, two, three, or four substituents $Q^a$. In certain embodiments, $R^{7a}$ and $R^{7b}$ together with the carbon atoms to which they are attached form $C_{6-14}$ aryl, optionally substituted with one, two, three, or four substituents $Q^a$. In certain embodiments, $R^{7a}$ and $R^{7b}$ together with the carbon atoms to which they are attached form phenyl, optionally substituted with one, two, three, or four substituents $Q^a$. In certain embodiments, $R^{7a}$ and $R^{7b}$ together with the carbon atoms to which they are attached form heteroaryl, optionally substituted with one, two, three, or four substituents $Q^a$. In certain embodiments, $R^{7a}$ and $R^{7b}$ together with the carbon atoms to which they are attached form monocyclic heteroaryl, optionally substituted with one, two, three, or four substituents $Q^a$. In certain embodiments, $R^{7a}$ and $R^{7b}$ together with the carbon atoms to which they are attached form 5- or 6-membered heteroaryl, optionally substituted with one, two, three, or four substituents Q. In certain embodiments, $R^{7a}$ and $R^{7b}$ together with the carbon atoms to which they are attached form bicyclic heteroaryl, optionally substituted with one, two, three, or four substituents $Q^a$. In certain embodiments, $R^{7a}$ and $R^{7b}$ together with the carbon atoms to which they are attached form heterocyclyl, optionally substituted with one, two, three, or four substituents $Q^a$. In certain embodiments, $R^{7a}$ and $R^{7b}$ together with the carbon atoms to which they are attached form monocyclic heterocyclyl, optionally substituted with one, two, three, or four substituents $Q^a$. In certain embodiments, $R^{7a}$ and $R^{7b}$ together with the carbon atoms to which they are attached form 5- or 6-membered heterocyclyl, optionally substituted with one, two, three, or four substituents $Q^a$. In certain embodiments, $R^{7a}$ and $R^{7b}$ together with the carbon atoms to which they are attached form bicyclic heterocyclyl, optionally substituted with one, two, three, or four substituents $Q^a$.

In certain embodiments, $R^{7b}$ and $R^{7c}$ together with the carbon atoms to which they are attached form $C_{3-10}$ cycloalkenyl, $C_{6-14}$ aryl, heteroaryl, or heterocyclyl, each optionally substituted with one, two, three, or four substituents $Q^a$. In certain embodiments, $R^{7b}$ and $R^{7c}$ together with the carbon atoms to which they are attached form $C_{3-10}$ cycloalkenyl, optionally substituted with one, two, three, or four substituents $Q^a$. In certain embodiments, $R^{7b}$ and $R^{7c}$ together with the carbon atoms to which they are attached form cyclohexenyl, optionally substituted with one, two, three, or four substituents $Q^a$. In certain embodiments, $R^{7b}$ and $R^{7c}$ together with the carbon atoms to which they are attached form $C_{6-14}$ aryl, optionally substituted with one, two, three, or four substituents $Q^a$. In certain embodiments, $R^{7b}$ and $R^{7c}$ together with the carbon atoms to which they are attached form phenyl, optionally substituted with one, two, three, or four substituents $Q^a$. In certain embodiments, $R^{7b}$ and $R^{7c}$ together with the carbon atoms to which they are attached form heteroaryl, optionally substituted with one, two, three, or four substituents $Q^a$. In certain embodiments, $R^{7b}$ and $R^{7c}$ together with the carbon atoms to which they are attached form monocyclic heteroaryl, optionally substituted with one, two, three, or four substituents $Q^a$. In certain embodiments, $R^{7b}$ and $R^{7c}$ together with the carbon atoms to which they are attached form 5- or 6-membered heteroaryl, optionally substituted with one, two, three, or four substituents $Q^a$. In certain embodiments, $R^{7b}$ and $R^{7c}$ together with the carbon atoms to which they are attached form bicyclic heteroaryl, optionally substituted with one, two, three, or four substituents $Q^a$. In certain embodiments, $R^{7b}$ and $R^{7c}$ together with the carbon atoms to which they are attached form heterocyclyl, optionally substituted with one, two, three, or four substituents $Q^a$. In certain embodiments, $R^{7b}$ and $R^{7c}$ together with the carbon atoms to which they are attached form monocyclic heterocyclyl, optionally substituted with one, two, three, or four substituents $Q^a$. In certain embodiments, $R^{7b}$ and $R^{7c}$ together with the carbon atoms to which they are attached form 5- or 6-membered heterocyclyl, optionally substituted with one, two, three, or four substituents $Q^a$. In certain embodiments, $R^{7b}$ and $R^{7c}$ together with the carbon atoms to which they are attached form bicyclic heterocyclyl, optionally substituted with one, two, three, or four substituents $Q^a$.

In certain embodiments, $R^{7c}$ and $R^{7d}$ together with the carbon atoms to which they are attached form $C_{3-10}$ cycloalkenyl, $C_{6-14}$ aryl, heteroaryl, or heterocyclyl, each optionally substituted with one, two, three, or four substituents $Q^a$. In certain embodiments, $R^{7c}$ and $R^{7d}$ together with the carbon atoms to which they are attached form $C_{3-10}$ cycloalkenyl, optionally substituted with one, two, three, or four substituents $Q^a$. In certain embodiments, $R^{7c}$ and $R^{7d}$ together with the carbon atoms to which they are attached form cyclohexenyl, optionally substituted with one, two, three, or four substituents $Q^a$. In certain embodiments, $R^{7c}$ and $R^{7d}$ together with the carbon atoms to which they are attached form $C_{6-14}$ aryl, optionally substituted with one, two, three, or four substituents $Q^a$. In certain embodiments, $R^{7c}$ and $R^{7d}$ together with the carbon atoms to which they are attached form phenyl, optionally substituted with one, two, three, or four substituents $Q^a$. In certain embodiments, $R^{7c}$ and $R^{7d}$ together with the carbon atoms to which they are attached form heteroaryl, optionally substituted with one, two, three, or four substituents $Q^a$. In certain embodiments, $R^{7c}$ and $R^{7d}$ together with the carbon atoms to which they are attached form monocyclic heteroaryl, optionally substituted with one, two, three, or four substituents $Q^a$. In certain embodiments, $R^{7c}$ and $R^{7d}$ together with the carbon atoms to which they are attached form 5- or 6-membered heteroaryl, optionally substituted with one, two, three, or four substituents $Q^a$. In certain embodiments, $R^{7c}$ and $R^{7d}$ together with the carbon atoms to which they are attached form bicyclic heteroaryl, optionally substituted with one, two, three, or four substituents $Q^a$. In certain embodiments, $R^{7c}$ and $R^{7d}$ together with

the carbon atoms to which they are attached form heterocyclyl, optionally substituted with one, two, three, or four substituents $Q^a$. In certain embodiments, $R^{7c}$ and $R^{7d}$ together with the carbon atoms to which they are attached form monocyclic heterocyclyl, optionally substituted with one, two, three, or four substituents $Q^a$. In certain embodiments, $R^{7c}$ and $R^{7d}$ together with the carbon atoms to which they are attached form 5- or 6-membered heterocyclyl, optionally substituted with one, two, three, or four substituents $Q^a$. In certain embodiments, $R^{7c}$ and $R^{7d}$ together with the carbon atoms to which they are attached form bicyclic heterocyclyl, optionally substituted with one, two, three, or four substituents $Q^a$.

In certain embodiments, $R^{7d}$ and $R^{7e}$ together with the carbon atoms to which they are attached form $C_{3-10}$ cycloalkenyl, $C_{6-14}$ aryl, heteroaryl, or heterocyclyl, each optionally substituted with one, two, three, or four substituents $Q^a$. In certain embodiments, $R^{7d}$ and $R^{7e}$ together with the carbon atoms to which they are attached form $C_{3-10}$ cycloalkenyl, optionally substituted with one, two, three, or four substituents $Q^a$. In certain embodiments, $R^{7d}$ and $R^{7e}$ together with the carbon atoms to which they are attached form cyclohexenyl, optionally substituted with one, two, three, or four substituents $Q^a$. In certain embodiments, $R^{7d}$ and $R^{7e}$ together with the carbon atoms to which they are attached form $C_{6-14}$ aryl, optionally substituted with one, two, three, or four substituents $Q^a$. In certain embodiments, $R^{7d}$ and $R^{7e}$ together with the carbon atoms to which they are attached form phenyl, optionally substituted with one, two, three, or four substituents $Q^a$. In certain embodiments, $R^{7d}$ and $R^{7e}$ together with the carbon atoms to which they are attached form heteroaryl, optionally substituted with one, two, three, or four substituents $Q^a$. In certain embodiments, $R^{7d}$ and $R^{7e}$ together with the carbon atoms to which they are attached form monocyclic heteroaryl, optionally substituted with one, two, three, or four substituents $Q^a$. In certain embodiments, $R^{7d}$ and $R^{7e}$ together with the carbon atoms to which they are attached form 5- or 6-membered heteroaryl, optionally substituted with one, two, three, or four substituents $Q^a$. In certain embodiments, $R^{7d}$ and $R^{7e}$ together with the carbon atoms to which they are attached form bicyclic heteroaryl, optionally substituted with one, two, three, or four substituents $Q^a$. In certain embodiments, $R^{7d}$ and $R^{7e}$ together with the carbon atoms to which they are attached form heterocyclyl, optionally substituted with one, two, three, or four substituents $Q^a$. In certain embodiments, $R^{7d}$ and $R^{7e}$ together with the carbon atoms to which they are attached form monocyclic heterocyclyl, optionally substituted with one, two, three, or four substituents $Q^a$. In certain embodiments, $R^{7d}$ and $R^{7e}$ together with the carbon atoms to which they are attached form 5- or 6-membered heterocyclyl, optionally substituted with one, two, three, or four substituents $Q^a$. In certain embodiments, $R^{7d}$ and $R^{7e}$ together with the carbon atoms to which they are attached form bicyclic heterocyclyl, optionally substituted with one, two, three, or four substituents $Q^a$.

In certain embodiments, m is 0. In certain embodiments, m is 1.

In certain embodiments, n is 0. In certain embodiments, n is 1. In certain embodiments, n is 2. In certain embodiments, n is 3. In certain embodiments, n is 4. In certain embodiments, n is 0, 1, or 2. In certain embodiments, n is 0, 1, 2, or 3. In certain embodiments, n is 1, 2, or 3. In certain embodiments, n is 1 or 2.

In certain embodiments, m is 0, and n is 0, 1, 2, or 3. In certain embodiments, m is 0, n is 0, 1, or 2. In certain embodiments, m is 0, n is 0 or 1. In certain embodiments, m is 0, n is 0. In certain embodiments, m is 0 and n is 1. In certain embodiments, m is 1, n is 0, 1, 2, or 3. In certain embodiments, m is 1, n is 0, 1, or 2. In certain embodiments, m is 1, n is 0 or 1. In certain embodiments, m is 1, n is 0. In certain embodiments, m is 1, n is 1.

In specific embodiments, m is 0, n is 1, and $R^{5a}$ and $R^{5b}$ are each methyl.

In certain embodiments, X is N In certain embodiments, X is $CR^x$, wherein $R^x$ is as defined herein. In certain embodiments, X is CH.

In certain embodiments, Y is N In certain embodiments, Y is $CR^x$, wherein $R^x$ is as defined herein. In certain embodiments, Y is CH.

In certain embodiments, Z is N In certain embodiments, Z is $CR^x$, wherein $R^x$ is as defined herein. In certain embodiments, Z is CH.

In certain embodiments, X, Y, and Z are N. In certain embodiments, X and Y are N, and Z is CH. In certain embodiments, X and Z are N, and Y is CH. In certain embodiments, Y and Z are N, and X is CH.

In certain embodiments, the compound described herein is not 4-(2-(difluoromethyl)-1*H*-benzo[*d*]imidazol-1-yl)-6-morpholino-*N*-(2-phenyl-2-(pyrrolidin-1-yl)ethyl)-1,3,5-triazin-2-amine. In certain embodiments, the compound described herein is not 6-(2-(difluoromethyl)-1*H*-benzo[*d*]imidazol-1-yl)-*N*-(1-(4-((*R*)-3-(methoxymethyl)morpholino)phenyl)ethyl)-2-morpholinopyrimidin-4-amine.

In certain embodiments, when X, Y, and Z are N, and $R^{5a}$ is hydrogen, $R^{5b}$ is not heterocyclyl. In certain embodiments, when X, Y, and Z are N, and $R^{5a}$ is hydrogen, $R^{5b}$ is not 5-membered heterocyclyl. In certain embodiments, when X, Y, and Z are N, and $R^{5a}$ is hydrogen, $R^{5b}$ is not pyrrolidinyl. In certain embodiments, when X, Y, and Z are N, and $R^{5a}$ is hydrogen, $R^{5b}$ is not pyrrolidin-1-yl.

In certain embodiments, when X and Z are N, Y is CH, and $R^{5a}$ is hydrogen, $R^{5b}$ is morpholino-substituted phenyl. In certain embodiments, when X and Z are N, Y is CH, and $R^{5a}$ is hydrogen, $R^{5b}$ is not 4-((*R*)-3-(methoxymethyl)morpholino)phenyl.

In one embodiment, described herein is a compound selected from:

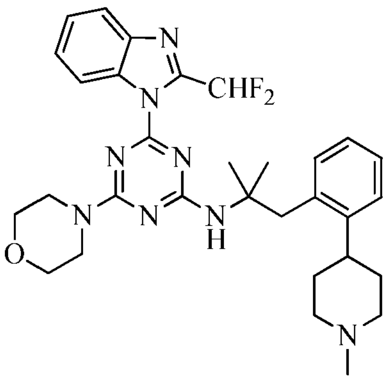,

**Compound I**

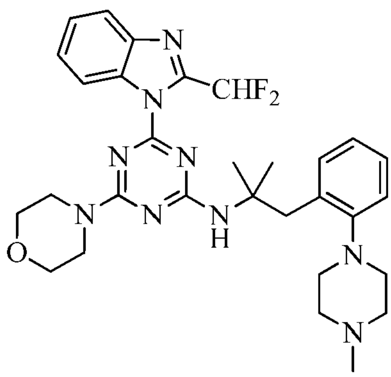,

**Compound II**

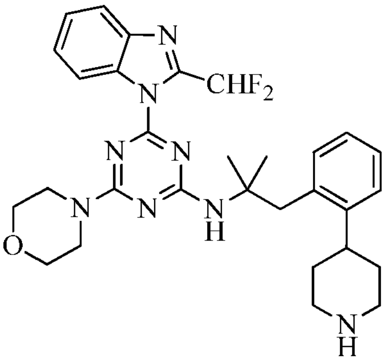,

**Compound III**

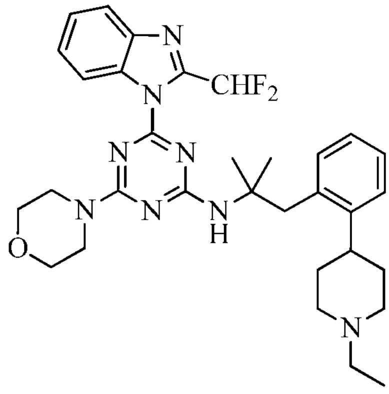,

**Compound IV**

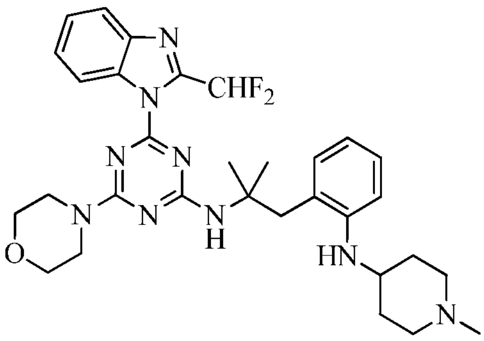,

**Compound V**

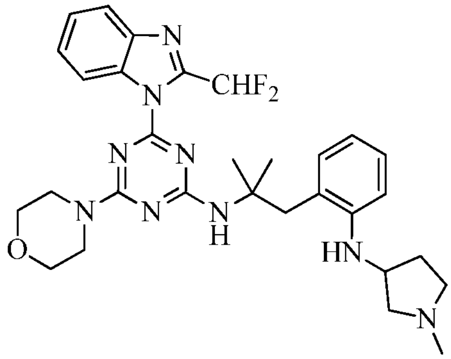,

**Compound VI**

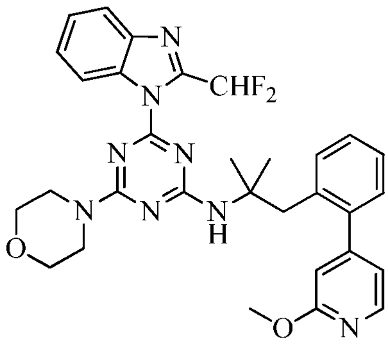,

**Compound VII**

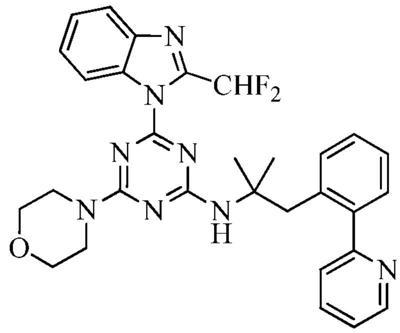,

**Compound VIII**

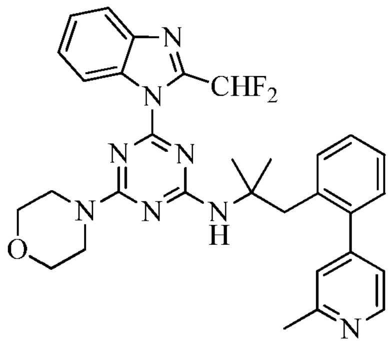,

**Compound IX**

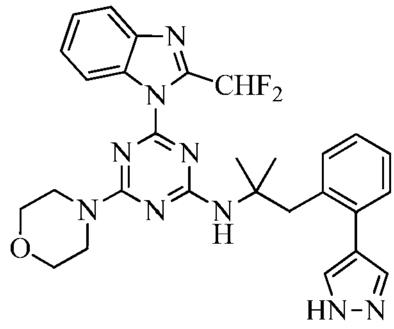,

**Compound X**

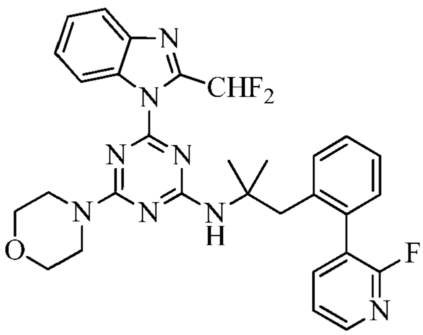,

**Compound XI**

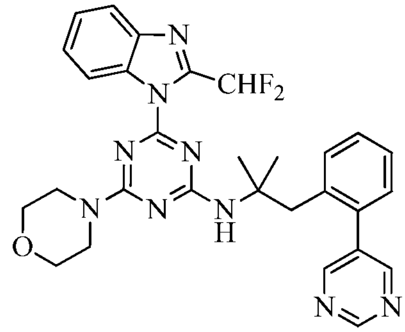,

**Compound XII**

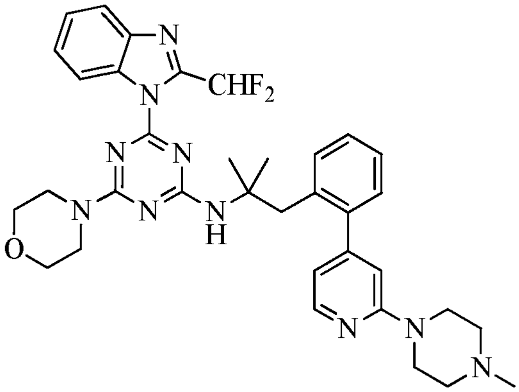,

**Compound XIII**

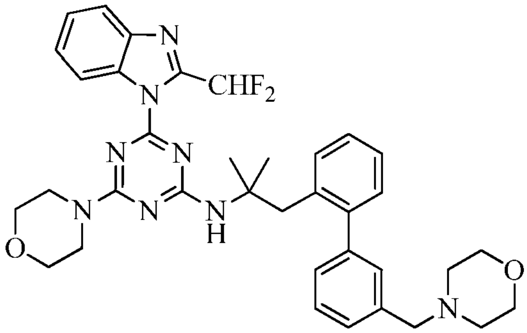,

**Compound XIV**

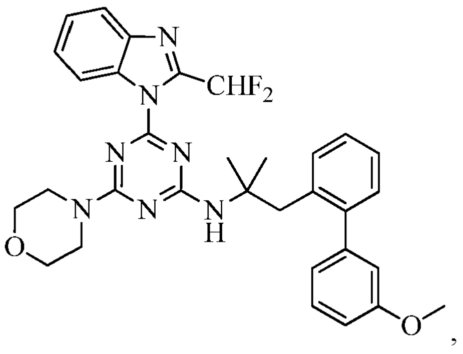

Compound XV

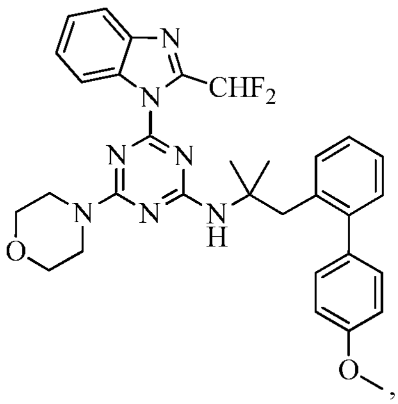

Compound XVI

In one embodiment, the PI3K inhibitor is Compound I, or isotopic variants, pharmaceutically acceptable salts, solvates, or hydrates thereof. In one embodiment, the PI3K inhibitor is Compound II, or isotopic variants, pharmaceutically acceptable salts, solvates, or hydrates thereof. In one embodiment, the PI3K inhibitor is Compound III, or isotopic variants, pharmaceutically acceptable salts, solvates, or hydrates thereof. In one embodiment, the PI3K inhibitor is Compound IV, or isotopic variants, pharmaceutically acceptable salts, solvates, or hydrates thereof. In one embodiment, the PI3K inhibitor is Compound V, or isotopic variants, pharmaceutically acceptable salts, solvates, or hydrates thereof. In one embodiment, the PI3K inhibitor is Compound VI, isotopic variants, pharmaceutically acceptable salts, solvates, or hydrates thereof. In one embodiment, the PI3K inhibitor is Compound VII, or isotopic variants, pharmaceutically acceptable salts, solvates, or hydrates thereof. In one embodiment, the PI3K inhibitor is Compound VIII, or isotopic variants, pharmaceutically acceptable salts, solvates, or hydrates thereof. In one embodiment, the PI3K inhibitor is Compound IX, or isotopic variants, pharmaceutically acceptable salts, solvates, or hydrates thereof. In one embodiment, the PI3K inhibitor is Compound X, or isotopic variants, pharmaceutically acceptable salts, solvates, or hydrates thereof. In one embodiment, the PI3K inhibitor is Compound XI, or isotopic variants, pharmaceutically acceptable salts, solvates, or hydrates thereof. In one embodiment, the PI3K inhibitor is Compound XII, or isotopic variants, pharmaceutically acceptable salts, solvates, or hydrates thereof. In one embodiment, the PI3K inhibitor is Compound XIII, or isotopic variants, pharmaceutically acceptable salts, solvates, or hydrates thereof. In one embodiment, the PI3K inhibitor is Compound XIV, or isotopic variants, pharmaceutically acceptable salts, solvates, or hydrates thereof. In one embodiment, the PI3K inhibitor is Compound XV, or isotopic variants, pharmaceutically acceptable salts, solvates, or hydrates thereof. In one embodiment, the PI3K inhibitor is Compound XVI, or isotopic variants, pharmaceutically acceptable salts, solvates, or hydrates thereof.

## SECOND AGENTS

Also described herein are pharmaceutical compositions or methods for using the pharmaceutical compositions comprising a PI3K inhibitor described herein in combination with a second agent, wherein the second agent is a BTK inhibitor, a Bcl-2 inhibitor, an EZH2 inhibitor, or any combination thereof.

In some embodiments, the second agent is a BTK inhibitor. Any suitable BTK inhibitor may be used in combination with a PI3K inhibitor described herein. In some embodiments, the BTK inhibitor is ibrutinib, BGB3111, CC-292, ACP 196, CNX-774, CGI1746, LFM-A13, CNX-774, ONO-4059, RN486 CPI-0610, DUAL946, GSK525762, I-BET151, JQ1, OTX015, PFI-1, RVX-208, RVX2135, TEN-010, or pharmaceutically acceptable salts thereof. In some embodiments, the BTK inhibitor is ibrutinib, or a pharmaceutically acceptable salt thereof, or BGB3111, or a pharmaceutically acceptable salt thereof. In another embodiment, the BTK inhibitor is ibrutinib or a pharmaceutically acceptable salt thereof. In yet another embodiment, the BTK inhibitor is BGB3111 or a pharmaceutically acceptable salt thereof.

In some embodiments, the second agent is a Bc1-2 inhibitor. Any suitable Bc1-2 inhibitor may be used in combination with a PI3K inhibitor described herein. In some embodiments, the Bcl-2 inhibitor is ABT-199 (venetoclax), ABT-737, ABT-263 (navitoclax), or PNT2258 or pharmaceutically acceptable salts thereof. In some embodiments, the Bc1-2 inhibitor is venetoclax, or a pharmaceutically acceptable salt thereof, or PNT2258, or a pharmaceutically acceptable salt thereof. In another embodiment, the Bcl-2 inhibitor is venetoclax or a pharmaceutically acceptable salt thereof. In another embodiment, the Bcl-2 inhibitor is PNT2258 or a pharmaceutically acceptable salt thereof.

In some embodiments, the second agent is an EZH2 inhibitor. Any suitable EZH2 inhibitor may be used in combination with a PI3K inhibitor described herein. In some embodiments, the EZH2 inhibitor is EPZ-6438 (tazemetostat, E7438), DNZeP (3-Deazaneplanocin), GSK2816126, EPZ005687, EI1, or pharmaceutically acceptable salts thereof. In

some embodiments, the EZH2 inhibitor is tazemetostat (EPZ-6438) or a pharmaceutically acceptable salt thereof. In other embodiments, the EZH2 inhibitor is tazemetostat or a pharmaceutically acceptable salt thereof.

**METHODS OF USE**

Also described herein are methods for treating a hematological cancer comprising administering an effective amount of a compound of Formula (I), or an isotopic variant thereof or a pharmaceutically acceptable salt, solvate, or hydrate thereof and an effective amount of a second agent selected from a BTK inhibitor, a Bcl-2 inhibitor, an EZH2 inhibitor or a combination thereof. In some embodiments, the second agent is ibrutinib, BGB3111, venetoclax, or tazemetostat, or pharmaceutically acceptable salts thereof. In some embodiments, the compound of Formula (I) is Compound I or an isotopic variant, pharmaceutically acceptable salt, solvate, or hydrate thereof. In some embodiments, the compound of Formula (I) is Compound II or an isotopic variant, pharmaceutically acceptable salt, solvate, or hydrate thereof. In some embodiments, the compound of Formula (I) is Compound III or an isotopic variant, pharmaceutically acceptable salt, solvate, or hydrate thereof. In some embodiments, the compound of Formula (I) is Compound IV or an isotopic variant, pharmaceutically acceptable salt, solvate, or hydrate thereof. In some embodiments, the compound of Formula (I) is Compound V or an isotopic variant, pharmaceutically acceptable salt, solvate, or hydrate thereof. In some embodiments, the compound of Formula (I) is Compound VI or an isotopic variant, pharmaceutically acceptable salt, solvate, or hydrate thereof. In some embodiments, the compound of Formula (I) is Compound VII or an isotopic variant, pharmaceutically acceptable salt, solvate, or hydrate thereof. In some embodiments, the compound of Formula (I) is Compound VIII or an isotopic variant, pharmaceutically acceptable salt, solvate, or hydrate thereof. In some embodiments, the compound of Formula (I) is Compound IX or an isotopic variant, pharmaceutically acceptable salt, solvate, or hydrate thereof. In some embodiments, the compound of Formula (I) is Compound X or an isotopic variant, pharmaceutically acceptable salt, solvate, or hydrate thereof. In some embodiments, the compound of Formula (I) is Compound XI or an isotopic variant, pharmaceutically acceptable salt, solvate, or hydrate thereof. In some embodiments, the compound of Formula (I) is Compound XII or an isotopic variant, pharmaceutically acceptable salt, solvate, or hydrate thereof. In some embodiments, the compound of Formula (I) is Compound XIII or an isotopic variant, pharmaceutically acceptable salt, solvate, or hydrate thereof. In some embodiments, the compound of Formula (I) is Compound XIV or an isotopic variant, pharmaceutically acceptable salt, solvate, or hydrate thereof. In some embodiments, the compound of Formula (I) is Compound XV or an isotopic variant, pharmaceutically acceptable salt, solvate, or hydrate thereof. In some embodiments, the compound of Formula (I) is Compound XVI or an isotopic variant, pharmaceutically acceptable salt, solvate, or hydrate thereof.

Also described herein are methods for treating a hematological cancer comprising administering an effective amount of a compound of Formula (I), or an isotopic variant thereof; or a pharmaceutically acceptable salt, solvate, or hydrate thereof and an effective amount of a BTK inhibitor to a patient. In one embodiment, the BTK inhibitor is ibrutinib or BGB3111, or pharmaceutically acceptable salts thereof. In some embodiments, the compound of Formula (I) is Compound I. In some embodiments, the compound of Formula (I) is Compound II. In some embodiments, the compound of Formula (I) is Compound III. In some embodiments, the compound of Formula (I) is Compound IV. In some embodiments, the compound of Formula (I) is Compound V. In some embodiments, the compound of Formula (I) is Compound VI. In some embodiments, the compound of Formula (I) is Compound VII. In some embodiments, the compound of Formula (I) is Compound VIII. In some embodiments, the compound of Formula (I) is Compound IX. In some embodiments, the compound of Formula (I) is Compound X. In some embodiments, the compound of Formula (I) is Compound XI. In some embodiments, the compound of Formula (I) is Compound XII. In some embodiments, the compound of Formula (I) is Compound XIII. In some embodiments, the compound of Formula (I) is Compound XIV. In some embodiments, the compound of Formula (I) is Compound XV. In some embodiments, the compound of Formula (I) is Compound XVI.

Also described herein are methods for treating a hematological cancer comprising administering an effective amount of a compound of Formula (I), or an isotopic variant thereof; or a pharmaceutically acceptable salt, solvate, or hydrate thereof and an effective amount of a Bcl-2 inhibitor to a patient. In one embodiment, the Bcl-2 inhibitor is venetoclax (ABT-199) or PNT2258, or pharmaceutically acceptable salts thereof. In some embodiments, the compound of Formula (I) is Compound I or an isotopic variant, pharmaceutically acceptable salt, solvate, or hydrate thereof. In some embodiments, the compound of Formula (I) is Compound II or an isotopic variant, pharmaceutically acceptable salt, solvate, or hydrate thereof. In some embodiments, the compound of Formula (I) is Compound III or an isotopic variant, pharmaceutically acceptable salt, solvate, or hydrate thereof. In some embodiments, the compound of Formula (I) is Compound IV or an isotopic variant, pharmaceutically acceptable salt, solvate, or hydrate thereof. In some embodiments, the compound of Formula (I) is Compound V or an isotopic variant, pharmaceutically acceptable salt, solvate, or hydrate thereof. In some embodiments, the compound of Formula (I) is Compound VI or an isotopic variant, pharmaceutically acceptable salt, solvate, or hydrate thereof. In some embodiments, the compound of Formula (I) is Compound VII or an isotopic variant, pharmaceutically acceptable salt, solvate, or hydrate thereof. In some embodiments, the compound of Formula (I) is Compound VIII or an isotopic variant, pharmaceutically acceptable salt, solvate, or hydrate

thereof. In some embodiments, the compound of Formula (I) is Compound IX or an isotopic variant, pharmaceutically acceptable salt, solvate, or hydrate thereof. In some embodiments, the compound of Formula (I) is Compound X or an isotopic variant, pharmaceutically acceptable salt, solvate, or hydrate thereof. In some embodiments, the compound of Formula (I) is Compound XI or an isotopic variant, pharmaceutically acceptable salt, solvate, or hydrate thereof. In some embodiments, the compound of Formula (I) is Compound XII or an isotopic variant, pharmaceutically acceptable salt, solvate, or hydrate thereof. In some embodiments, the compound of Formula (I) is Compound XIII or an isotopic variant, pharmaceutically acceptable salt, solvate, or hydrate thereof. In some embodiments, the compound of Formula (I) is Compound XIV or an isotopic variant, pharmaceutically acceptable salt, solvate, or hydrate thereof. In some embodiments, the compound of Formula (I) is Compound XV or an isotopic variant, pharmaceutically acceptable salt, solvate, or hydrate thereof. In some embodiments, the compound of Formula (I) is Compound XVI or an isotopic variant, pharmaceutically acceptable salt, solvate, or hydrate thereof.

Also described herein herein are methods for treating or preventing a hematological cancer comprising administering an effective amount of a compound of Formula (I), or an isotopic variant thereof; or a pharmaceutically acceptable salt, solvate, or hydrate thereof and an effective amount of a EZH2 inhibitor to a patient. In one embodiment, the EZH2 inhibitor is tazemetostat (EPZ-6438) or a pharmaceutically acceptable salt thereof. In some embodiments, the compound of Formula (I) is Compound I or an isotopic variant, pharmaceutically acceptable salt, solvate, or hydrate thereof. In some embodiments, the compound of Formula (I) is Compound II or an isotopic variant, pharmaceutically acceptable salt, solvate, or hydrate thereof. In some embodiments, the compound of Formula (I) is Compound III or an isotopic variant, pharmaceutically acceptable salt, solvate, or hydrate thereof. In some embodiments, the compound of Formula (I) is Compound IV or an isotopic variant, pharmaceutically acceptable salt, solvate, or hydrate thereof. In some embodiments, the compound of Formula (I) is Compound V or an isotopic variant, pharmaceutically acceptable salt, solvate, or hydrate thereof. In some embodiments, the compound of Formula (I) is Compound VI or an isotopic variant, pharmaceutically acceptable salt, solvate, or hydrate thereof. In some embodiments, the compound of Formula (I) is Compound VII or an isotopic variant, pharmaceutically acceptable salt, solvate, or hydrate thereof. In some embodiments, the compound of Formula (I) is Compound VIII or an isotopic variant, pharmaceutically acceptable salt, solvate, or hydrate thereof. In some embodiments, the compound of Formula (I) is Compound IX or an isotopic variant, pharmaceutically acceptable salt, solvate, or hydrate thereof. In some embodiments, the compound of Formula (I) is Compound X or an isotopic variant, pharmaceutically acceptable salt, solvate, or hydrate thereof. In some embodiments, the compound of Formula (I) is Compound XI or an isotopic variant, pharmaceutically acceptable salt, solvate, or hydrate thereof. In some embodiments, the compound of Formula (I) is Compound XII or an isotopic variant, pharmaceutically acceptable salt, solvate, or hydrate thereof. In some embodiments, the compound of Formula (I) is Compound XIII or an isotopic variant, pharmaceutically acceptable salt, solvate, or hydrate thereof. In some embodiments, the compound of Formula (I) is Compound XIV or an isotopic variant, pharmaceutically acceptable salt, solvate, or hydrate thereof. In some embodiments, the compound of Formula (I) is Compound XV or an isotopic variant, pharmaceutically acceptable salt, solvate, or hydrate thereof. In some embodiments, the compound of Formula (I) is Compound XVI or an isotopic variant, pharmaceutically acceptable salt, solvate, or hydrate thereof.

The hematological cancer treatable with the methods described herein includes, but is not limited to, (1) leukemias, including, but not limited to, acute leukemia, acute lymphocytic leukemia, acute myelocytic leukemias such as myeloblastic, promyelocytic, myelomonocytic, monocytic, erythroleukemia leukemias and myelodysplastic syndrome or a symptom thereof (such as anemia, thrombocytopenia, neutropenia, bicytopenia or pancytopenia), refractory anemia (RA), RA with ringed sideroblasts (RARS), RA with excess blasts (RAEB), RAEB in transformation (RAEB-T), preleukemia, and chronic myelomonocytic leukemia (CMML), (2) chronic leukemias, including, but not limited to, chronic myelocytic (granulocytic) leukemia, chronic lymphocytic leukemia, and hairy cell leukemia; (3) polycythemia vera; (4) lymphomas, including, but not limited to, Hodgkin's disease and non-Hodgkin's disease; (5) multiple myelomas, including, but not limited to, smoldering multiple myeloma, nonsecretory myeloma, osteosclerotic myeloma, plasma cell leukemia, solitary plasmacytoma, and extramedullary plasmacytoma; (6) Waldenstrom's macroglobulinemia; (7) monoclonal gammopathy of undetermined significance; (8) benign monoclonal gammopathy *(See* Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

Also described herein are methods of treating hematological malignancy with a combination of an effective amount of a compound of Formula (I), or an isotopic variant thereof; or a pharmaceutically acceptable salt, solvate, or hydrate thereof and an effective amount of BTK inhibitor in a patient. In certain embodiments, the hematological malignancy is a leukemia, a lymphoma, a myeloma, a non-Hodgkin's lymphoma, a Hodgkin's lymphoma, T-cell malignancy, or a B-cell malignancy. In some embodiments, the hematological malignancy is chronic lymphocytic leukemia, follicular lymphoma, diffuse large B-cell lymphoma, or non-Hodgkin's lymphoma. In some embodiments, the hematological malignancy is chronic lymphocytic leukemia or non-Hodgkin's lymphoma. In some embodiments, the hematological malignancy is chronic lymphocytic leukemia. In other embodiments, the hematological malignancy is non-Hodgkin's lymphoma. In some embodiments, the hematological malignancy is follicular lymphoma. In other embodiments, the hematological

malignancy is diffuse large B-cell lymphoma. In some embodiments, the compound of Formula (I) is Compound I or an isotopic variant, pharmaceutically acceptable salt, solvate, or hydrate thereof. In some embodiments, the compound of Formula (I) is Compound II or an isotopic variant, pharmaceutically acceptable salt, solvate, or hydrate thereof. In some embodiments, the compound of Formula (I) is Compound III or an isotopic variant, pharmaceutically acceptable salt, solvate, or hydrate thereof. In some embodiments, the compound of Formula (I) is Compound IV or an isotopic variant, pharmaceutically acceptable salt, solvate, or hydrate thereof. In some embodiments, the compound of Formula (I) is Compound V or an isotopic variant, pharmaceutically acceptable salt, solvate, or hydrate thereof. In some embodiments, the compound of Formula (I) is Compound VI or an isotopic variant, pharmaceutically acceptable salt, solvate, or hydrate thereof. In some embodiments, the compound of Formula (I) is Compound VII or an isotopic variant, pharmaceutically acceptable salt, solvate, or hydrate thereof. In some embodiments, the compound of Formula (I) is Compound VIII or an isotopic variant, pharmaceutically acceptable salt, solvate, or hydrate thereof. In some embodiments, the compound of Formula (I) is Compound IX or an isotopic variant, pharmaceutically acceptable salt, solvate, or hydrate thereof. In some embodiments, the compound of Formula (I) is Compound X or an isotopic variant, pharmaceutically acceptable salt, solvate, or hydrate thereof. In some embodiments, the compound of Formula (I) is Compound XI or an isotopic variant, pharmaceutically acceptable salt, solvate, or hydrate thereof. In some embodiments, the compound of Formula (I) is Compound XII or an isotopic variant, pharmaceutically acceptable salt, solvate, or hydrate thereof. In some embodiments, the compound of Formula (I) is Compound XIII or an isotopic variant, pharmaceutically acceptable salt, solvate, or hydrate thereof. In some embodiments, the compound of Formula (I) is Compound XIV or an isotopic variant, pharmaceutically acceptable salt, solvate, or hydrate thereof. In some embodiments, the compound of Formula (I) is Compound XV or an isotopic variant, pharmaceutically acceptable salt, solvate, or hydrate thereof. In some embodiments, the compound of Formula (I) is Compound XVI or an isotopic variant, pharmaceutically acceptable salt, solvate, or hydrate thereof.

In certain embodiments, the hematological malignancy is a T-cell malignancy. In certain embodiments, T-cell malignancies include peripheral T-cell lymphoma not otherwise specified (PTCL-NOS), anaplastic large cell lymphoma, angioimmunoblastic lymphoma, cutaneous T-cell lymphoma, adult T-cell leukemia/lymphoma (ATLL), blastic NK-cell lymphoma, enteropathy-type T-cell lymphoma, hematosplenic gamma-delta T-cell lymphoma, lymphoblastic lymphoma, nasal NK/T-cell lymphomas, or treatment-related T-cell lymphomas.

In certain embodiments, the hematological malignancy is a B-cell malignancy. In certain embodiments, B-cell malignancies include acute lymphoblastic leukemia (ALL), acute myelogenous leukemia (AML), chronic myelogenous leukemia (CML), acute monocytic leukemia (AMoL), chronic lymphocytic leukemia (CLL), high-risk chronic lymphocytic leukemia (CLL), small lymphocytic lymphoma (SLL), high-risk small lymphocytic lymphoma (SLL), follicular lymphoma (FL), diffuse large B-cell lymphoma (DLBCL), mantle cell lymphoma (MCL), Waldenstrom's macroglobulinemia, multiple myeloma, extranodal marginal zone B cell lymphoma, nodal marginal zone B cell lymphoma, Burkitfs lymphoma, non-Burkitt high grade B cell lymphoma, primary mediastinal B-cell lymphoma (PMBL), immunoblastic large cell lymphoma, precursor B-lymphoblastic lymphoma, B cell prolymphocytic leukemia, lymphoplasmacytic lymphoma, splenic marginal zone lymphoma, plasma cell myeloma, plasmacytoma, mediastinal (thymic) large B cell lymphoma, intravascular large B cell lymphoma, primary effusion lymphoma, or lymphomatoid granulomatosis. In certain embodiments, the B-cell malignancy is diffuse large B-cell lymphoma (DLBCL). In certain embodiments, the hematological malignancy is diffuse large B-cell lymphoma (DLBCL). In certain embodiments, the DLBCL is an activated B-cell DLBCL (ABC-DLBCL), a germinal center B-cell like DLBCL (GBC-DLBCL), a double hit DLBCL (DH-DLBCL), or a triple hit DLBCL (TH-DLBCL).

In certain embodiments, the hematological malignancy is a relapsed or refractory hematological malignancy. In certain embodiments, the relapsed or refractory hematological malignancy is a relapsed or refractory T-cell malignancy. In certain embodiments, the relapsed or refractory hematological malignancy is a relapsed or refractory B-cell malignancy.

Also described herein is a method for treating or preventing a hematological cancer comprising administering a PI3K inhibitor, as described herein, in combination with a second agent, as described herein. In some embodiments, the combination therapy of a PI3K inhibitor described herein (e.g., a compound of Formula (I)) and the second agent provides a synergistic effect. In some embodiments, the combination therapy of a PI3K inhibitor described herein (e.g., a compound of Formula (I)) and a second agent provides a synergistic antitumor or anti-cancer activity. In certain embodiments, the combination therapy described herein permits the use of lower dosages of the PI3K inhibitor and/or the second agent. In some embodiments, the combination therapy described herein permits less frequent administration of the PI3K inhibitor and/or the second agent to a subject. In some embodiments, the combination therapy described herein reduces the toxicity associated with the administration of the PI3K inhibitor and/or the second agent to a subject without reducing the efficacy in the prevention, management, treatment, or amelioration of cancer, such as chronic lymphocytic leukemia. In some embodiments, the synergistic effect observed with the combination therapy described herein results in improved efficacy of therapies in the prevention, management, treatment, or amelioration of cancer, such as chronic lymphocytic leukemia.

In some embodiments, the combination therapy described herein avoids or reduces adverse or unwanted side effects associated with the use of the PI3K inhibitor and/or the second agent. In some embodiments, the combination

therapy described herein avoids, reduces, or minimizes infections, neutropenia, diarrhea, pneumonia, anemia, thrombocytopenia, nausea, vomiting, swelling in extremities, or a combination thereof in patients receiving the combination therapy. In certain embodiments, the combination therapy described herein avoids, reduces, or minimizes the incidence of infection. In certain embodiments, the combination therapy described herein avoids, reduces, or minimizes the incidence of neutropenia. In certain embodiments, the combination therapy described herein avoids, reduces, or minimizes the incidence of diarrhea. In certain embodiments, the combination therapy described herein avoids, reduces, or minimizes the incidence of pneumonia. In certain embodiments, the combination therapy described herein avoids, reduces, or minimizes the incidence of anemia. In certain embodiments, the combination therapy described herein avoids, reduces, or minimizes the incidence of thrombocytopenia. In certain embodiments, the combination therapy described herein avoids, reduces, or minimizes the incidence of nausea. In certain embodiments, the combination therapy described herein avoids, reduces, or minimizes the incidence of vomiting. In certain embodiments, the combination therapy described herein avoids, reduces, or minimizes the incidence of swelling in the extremities.

Depending on the disorder, disease, or condition to be treated, and the subject's condition, the compounds or pharmaceutical compositions described herein can be administered by oral, parenteral (e.g., intramuscular, intraperitoneal, intravenous, ICV, intracistemal injection or infusion, subcutaneous injection, or implant), inhalation, nasal, vaginal, rectal, sublingual, or topical (e.g., transdermal or local) routes of administration and can be formulated, alone or together, in suitable dosage unit with pharmaceutically acceptable excipients, carriers, adjuvants, and vehicles appropriate for each route of administration as described elsewhere herein.

## DOSAGES AND DOSING REGIMENTS

In certain embodiments, the methods described herein comprise administering a compound of Formula (I), or an isotopic variant thereof; or a pharmaceutically acceptable salt, solvate, or hydrate thereof and a second agent selected from a BTK inhibitor, a Bcl-2 inhibitor, and a EZH2 inhibitor to a patient simultaneously or sequentially by the same or different routes of administration. In certain embodiments, the methods described herein comprise administering a compound of Formula (I), or an isotopic variant thereof; or a pharmaceutically acceptable salt, solvate, or hydrate thereof and a second agent selected from ibrutinib, BGB3111, venetoclax, and tazemetostat to a patient simultaneously or sequentially by the same or different routes of administration. In some embodiments, the compound of Formula (I) is Compound I or an isotopic variant, pharmaceutically acceptable salt, solvate, or hydrate thereof. In some embodiments, the compound of Formula (I) is Compound II or an isotopic variant, pharmaceutically acceptable salt, solvate, or hydrate thereof. In some embodiments, the compound of Formula (I) is Compound III or an isotopic variant, pharmaceutically acceptable salt, solvate, or hydrate thereof. In some embodiments, the compound of Formula (I) is Compound IV or an isotopic variant, pharmaceutically acceptable salt, solvate, or hydrate thereof. In some embodiments, the compound of Formula (I) is Compound V or an isotopic variant, pharmaceutically acceptable salt, solvate, or hydrate thereof. In some embodiments, the compound of Formula (I) is Compound VI or an isotopic variant, pharmaceutically acceptable salt, solvate, or hydrate thereof. In some embodiments, the compound of Formula (I) is Compound VII or an isotopic variant, pharmaceutically acceptable salt, solvate, or hydrate thereof. In some embodiments, the compound of Formula (I) is Compound VIII or an isotopic variant, pharmaceutically acceptable salt, solvate, or hydrate thereof. In some embodiments, the compound of Formula (I) is Compound IX or an isotopic variant, pharmaceutically acceptable salt, solvate, or hydrate thereof. In some embodiments, the compound of Formula (I) is Compound X or an isotopic variant, pharmaceutically acceptable salt, solvate, or hydrate thereof. In some embodiments, the compound of Formula (I) is Compound XI or an isotopic variant, pharmaceutically acceptable salt, solvate, or hydrate thereof. In some embodiments, the compound of Formula (I) is Compound XII or an isotopic variant, pharmaceutically acceptable salt, solvate, or hydrate thereof. In some embodiments, the compound of Formula (I) is Compound XIII or an isotopic variant, pharmaceutically acceptable salt, solvate, or hydrate thereof. In some embodiments, the compound of Formula (I) is Compound XIV or an isotopic variant, pharmaceutically acceptable salt, solvate, or hydrate thereof. In some embodiments, the compound of Formula (I) is Compound XV or an isotopic variant, pharmaceutically acceptable salt, solvate, or hydrate thereof. In some embodiments, the compound of Formula (I) is Compound XVI or an isotopic variant, pharmaceutically acceptable salt, solvate, or hydrate thereof.

The suitability of a particular route of administration employed for a particular active agent will depend on the active agent itself (e.g., whether it can be administered orally without decomposing prior to entering the blood stream) and the disease being treated. Recommended routes of administration for the second active agents are known to those of ordinary skill in the art. See, e.g., Physicians' Desk Reference, 1755-1760 (56th ed., 2002).

In certain embodiments, the compound of Formula (I), or an isotopic variant thereof; or a pharmaceutically acceptable salt, solvate, or hydrate thereof and a second agent selected from a BTK inhibitor, a Bcl-2 inhibitor, and a EZH2 inhibitor are administered simultaneously, at essentially the same time, or sequentially. If administration takes place sequentially, the second agent may be administered before or after administration of a compound of Formula (I), or an isotopic variant thereof; or a pharmaceutically acceptable salt, solvate, or hydrate thereof. In some embodiments,

the second agent is administered before administration of a compound of Formula (I), or an isotopic variant thereof; or a pharmaceutically acceptable salt, solvate, or hydrate thereof. In some embodiments, the second agent is administered simultaneously with administration of a compound of Formula (I), an isotopic variant thereof; or a pharmaceutically acceptable salt, solvate, or hydrate thereof. In some embodiments, the second agent is administered after the administration of a compound of Formula (I), an isotopic variant thereof; or a pharmaceutically acceptable salt, solvate, or hydrate thereof. In some embodiments, a compound of Formula (I), or an isotopic variant thereof; or a pharmaceutically acceptable salt, solvate, or hydrate thereof and the second agent need not be administered by means of the same vehicle. In some embodiments, the second agent and a compound of Formula (I), or an isotopic variant thereof; or a pharmaceutically acceptable salt, solvate, or hydrate thereof are administered in different vehicles. The second agent may be administered one or more times, and the number of administrations of each component of the combination may be the same or different. In addition, a compound of Formula (I), or an isotopic variant thereof, or a pharmaceutically acceptable salt, solvate, or hydrate thereof and the second agent need not be administered at the same site.

In certain embodiments, a compound of Formula (I), or an isotopic variant thereof; or a pharmaceutically acceptable salt, solvate, or hydrate thereof and the second agent selected from a BTK inhibitor, a Bcl-2 inhibitor, and a EZH2 inhibitor are cyclically administered to a patient. Cycling therapy involves the administration of an active agent or a combination of active agents for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

In certain embodiments, in the treatment, prevention, or amelioration of one or more symptoms of the disorders, diseases, or conditions described herein, an appropriate dosage level of a compound of Formula (I), or an isotopic variant thereof; or a pharmaceutically acceptable salt, solvate, or hydrate thereof generally is ranging from about 1 to 1000 mg, from about 1 to about 500 mg, from about 5 to about 500 mg, from about 5 to about 200 mg, from about 5 to about 250 mg or from about 10 to about 150 mg which can be administered in single or multiple doses. In certain embodiments, the compound of Formula (I), or an isotopic variant thereof; or a pharmaceutically acceptable salt, solvate, or hydrate thereof is administered in an amount of about 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 225, 250, 275, 300, 325, 350, 375, 400, 450 or 500 mg. In certain embodiments, the compound of Formula (I), or an isotopic variant thereof; or a pharmaceutically acceptable salt, solvate, or hydrate thereof is administered in an amount of about 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 225, 250, 275, 300, 325, 350, 375, 400, 450 or 500 mg/day. In certain embodiments, the compound of Formula (I), or an isotopic variant thereof; or a pharmaceutically acceptable salt, solvate, or hydrate thereof is administered in an amount of about 60 mg. In certain embodiments, the compound of Formula (I), or an isotopic variant thereof; or a pharmaceutically acceptable salt, solvate, or hydrate thereof is administered in an amount of about 1, about 5, about 10, about 15, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 225, about 250, about 275, about 300, about 325, about 350, about 375, about 400, about 450, or about 500 mg. In certain embodiments, the compound of Formula (I), or an isotopic variant thereof; or a pharmaceutically acceptable salt, solvate, or hydrate thereof is administered in an amount of about 45 mg. In certain embodiments, the compound of Formula (I), or an isotopic variant thereof; or a pharmaceutically acceptable salt, solvate, or hydrate thereof is administered in an amount of about 60 mg. In certain embodiments, the compound of Formula (I), or an isotopic variant thereof; or a pharmaceutically acceptable salt, solvate, or hydrate thereof is administered in an amount of about 90 mg. In certain embodiments, the compound of Formula (I), or an isotopic variant thereof; or a pharmaceutically acceptable salt, solvate, or hydrate thereof is administered in an amount of about 120 mg. In certain embodiments, the compound of Formula (I), or an isotopic variant thereof; or a pharmaceutically acceptable salt, solvate, or hydrate thereof is administered in an amount of about 150 mg. In certain embodiments, the compound of Formula (I), or an isotopic variant thereof; or a pharmaceutically acceptable salt, solvate, or hydrate thereof is administered in an amount of about 180 mg.

For oral administration, the pharmaceutical compositions described herein can be formulated in the form of tablets containing from about 1.0 to about 1,000 mg of a compound of Formula (I), or an isotopic variant thereof; or a pharmaceutically acceptable salt, solvate, or hydrate thereof, in one embodiment, about 1, about 5, about 10, about 15, about 20, about 25, about 45, about 50, about 60, about 75, about 90, about 100, about 120, about 150, about 180, about 200, about 250, about 300, about 400, about 500, about 600, about 750, about 800, about 900, and about 1,000 mg of the a compound of Formula (I), or an isotopic variant thereof; or a pharmaceutically acceptable salt, solvate, or hydrate thereof for the symptomatic adjustment of the dosage to the patient to be treated. The pharmaceutical compositions can be administered on a regimen of 1 to 4 times per day, including once, twice, three times, and four times per day.

In the methods described herein, an appropriate dosage level of a second agent generally is ranging from about 0.1 to 2000 milligrams per day. For example, 1-500 milligrams once or multiple times per day may be effective to obtain

the desired results.

In certain embodiments, the second agent is a BTK inhibitor ibrutinib and the amount of ibrutinib that is administered is from about 10 mg/day up to, and including, 1000 mg/day. In certain embodiments, the amount of ibrutinib that is administered is from about 10 mg/day to 600 mg/day. In certain embodiments, the amount of ibrutinib that is administered is from about 100 mg/day to 600 mg/day. In certain embodiments, the amount of ibrutinib that is administered per day is about 10 mg, about 50 mg, about 100 mg, about 140 mg, about 280 mg, about 420 mg or about 560 mg.

In certain embodiments, the second agent is venetoclax and the amount of venetoclax that is administered is from about 10 mg/day up to, and including, 1000 mg/day. In certain embodiments, the amount of venetoclax that is administered is from about 10 mg/day to 600 mg/day. In certain embodiments, the amount of venetoclax that is administered is from about 50 mg/day to 500 mg/day. In certain embodiments, the amount of venetoclax that is administered per day is about 100 mg-400 mg.

In certain embodiments, the second agent is a tazemetostat and the amount of tazemetostat that is administered is from about 10 mg up to, and including, 2000 mg. In certain embodiments, the amount of tazemetostat that is administered is from about 100 mg to 1000 mg. In certain embodiments, the amount of tazemetostat that is administered is about 800 mg. In certain embodiments, the amount of tazemetostat that is administered is about 800 mg b.i.d.

In certain embodiments, a second agent is administered once per day, twice per day, or three times per day. In certain embodiments, the second agent is administered once per day. In certain embodiments, the second agent is administered once per day, twice per day, or three times per day. In certain embodiments, the second agent is administered once per day. In certain embodiments, the second agent is co-administered (e.g., in a single dosage form), once per day.

It will be understood, however, that the specific dose level and frequency of dosage for any particular patient can be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

## ADDITIONAL COMBINATION THERAPY

As described herein, certain embodiments, the methods of combination therapy comprising a compound of Formula (I) an isotopic variant thereof; or a pharmaceutically acceptable salt, solvate, or hydrate thereof and the second agent selected from a BTK inhibitor, a Bcl-2 inhibitor, and a EZH2 inhibitor can also be combined or used in combination with a third agent or therapies useful in the treatment, prevention, or amelioration of one or more symptoms of a proliferative disorders, diseases, or conditions.

Suitable third agent of therapies include, but are not limited to, (1) alpha-adrenergic agents; (2) antiarrhythmic agents; (3) anti-atherosclerotic agents, such as ACAT inhibitors; (4) antibiotics, such as anthracyclines, bleomycins, mitomycin, dactinomycin, and plicamycin; (5) anticancer agents and cytotoxic agents, e.g., alkylating agents, such as nitrogen mustards, alkyl sulfonates, nitrosoureas, ethylenimines, and triazenes; (6) anticoagulants, such as acenocoumarol, argatroban, bivalirudin, lepirudin, fondaparinux, heparin, phenindione, warfarin, and xirnelagatran, (7) anti-diabetic agents, such as biguanides (e.g., metformin), glucosidase inhibitors (e.g., acarbose), insulins, meglitinides (e.g., repaglinide), sulfonylureas (e.g., glimepiride, glyburide, and glipizide), thiozolidinediones (e.g., troglitazone, rosiglitazone, and pioglitazone), and PPAR-gamma monists; (8) antifungal agents, such as amorolfine, amphotericin B, anidulafungin, bifonazole, butenafine, butoconazole, caspofungin, ciclopirox, clotrimazole, econazole, fenticonazole, filipin, fluconazole, isoconazole, itraconazole, ketoconazole, micafungin, miconazole, naftifine, natamycin, nystatin, oxyconazole, ravuconazole, posaconazole, rimocidin, sertaconazole, sulconazole, terbinafine, terconazole, tioconazole, and voriconazole; (9) antiinflammatories, e.g., non-steroidal anti-inflammatory agents, such as aceclofenac, acemetacin, amoxiprin, aspirin, azapropazone, benorilate, bromfenac, carprofen, celecoxib, choline magnesium salicylate, diclofenac, diflunisal, etodolac, etoricoxib, faislamine, fenbufen, fenoprofen, flurbiprofen, ibuprofen, indometacin, ketoprofen, ketorolac, lornoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinpyrazone, suprofen, tenoxicam, tiaprofenic acid, and tolmetin; (10) antimetabolites, such as folate antagonists, purine analogues, and pyrimidine analogues; (11) anti-platelet agents, such as GPIIb/IIIa blockers (e.g., abciximab, eptifibatide, and tirofiban), P2Y(AC) antagonists (e.g., clopidogrel, ticlopidine and CS-747), cilostazol, dipyridamole, and aspirin; (12) antiproliferatives, such as methotrexate, FK506 (tacrolimus), and mycophenolate mofetil; (13) anti-TNF antibodies or soluble TNF receptor, such as etanercept, rapamycin, and leflunimide; (14) aP2 inhibitors; (15) beta-adrenergic agents, such as carvedilol and metoprolol; (16) bile acid secjuestrants, such as questran; (17) calcium channel blockers, such as amlodipine besylate; (18) chemotherapeutic agents; (19) cyclooxygenase-2 (COX-2) inhibitors, such as celecoxib and rofecoxib; (20) cyclosporins; (21) cytotoxic drugs, such as azathioprine and cyclophosphamide; (22) diuretics, such as chlorothiazide, hydrochlorothiazide, flumethiazide, hydroflumethiazide, bendroflumethiazide, methylchlorothiazide, trichloromethiazide, polythiazide, benzothiazide, ethacrynic acid, ticrynafen, chlorthalidone, furosenide, muzolimine, bumetanide, triamterene, amiloride, and spironolactone; (23) endothelin converting enzyme (ECE) inhibitors, such as phosphoramidon; (24) enzymes, such as L-asparaginase; (25) Factor VIIa Inhibitors and Factor Xa Inhibitors; (26) famesyl-protein transferase inhibitors; (27) fibrates; (28) growth factor inhibitors, such as modulators of PDGF activity; (29) growth hormone secretagogues; (30) HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, atavastatin, or visastatin); neutral endopeptidase (NEP) inhibitors; (31) hormonal agents, such as glucocorticoids (e.g., cortisone), estrogens/antiestrogens, androgens/antiandrogens, progestins, and luteinizing hormone-releasing hormone antagonists, and octreotide acetate; (32) immunosuppressants; (33) mineralcorticoidreceptor antagonists, such as spironolactone and eplerenone; (34) microtubule-disruptor agents, such as ecteinascidins; (35) microtubule-stabilizing agents, such as pacitaxel, docetaxel, and epothilones A-F; (36) MTP Inhibitors; (37) niacin; (38) phosphodiesterase inhibitors, such as PDE III inhibitors (e.g., cilostazol) and PDE V inhibitors (e.g., sildenafil, tadalafil, and vardenafil); (39) plant-derived products, such as vinca alkaloids, epipodophyllotoxins, and taxanes; (40) platelet activating factor (PAF) antagonists; (41) platinum coordination complexes, such as cisplatin, satraplatin, and carboplatin; (42) potassium channel openers; (43) prenyl-protein transferase inhibitors; (44) protein tyrosine kinase inhibitors; (45) renin inhibitors; (46) squalene synthetase inhibitors; (47) steroids, such as aldosterone, beclometasone, betamethasone, deoxycorticosterone acetate, fludrocortisone, hydrocortisone (cortisol), prednisolone, prednisone, methylprednisolone, dexamethasone, and triamcinolone; (48) TNF-alpha inhibitors, such as tenidap; (49) thrombin inhibitors, such as hirudin; (50) thrombolytic agents, such as anistreplase, reteplase, tenecteplase, tissue plasminogen activator (tPA), recombinant tPA, streptokinase, urokinase, prourokinase, and anisoylated plasminogen streptokinase activator complex (APSAC); (51) thromboxane receptor antagonists, such as ifetroban; (52) topoisomerase inhibitors; (53) vasopeptidase inhibitors (dual NEP-ACE inhibitors), such as omapatrilat and gemopatrilat, and (54) other miscellaneous agents, such as, hydroxyurea, procarbazine, mitotane, hexamethylmelamine, and gold compounds.

In certain embodiments, the third therapies that may be used in combination with the methods described herein include, but are not limited to, surgery, endocrine therapy, biologic response modifiers (e.g., interferons, interleukins, and tumor necrosis factor (TNF)), hyperthermia and cryotherapy, and agents to attenuate any adverse effects (e.g., antiemetics).

In certain embodiments, the third therapeutic agents that may be used in combination with the compounds described herein include, but are not limited to, alkylating drugs (mechlorethamine, chlorambucil, cyclophosphamide, melphalan, and ifosfamide), antimetabolites (cytarabine (also known as cytosine arabinoside or Ara-C), and methotrexate), purine antagonists and pyrimidine antagonists (6-mercaptopurine, 5-fluorouracil, cytarbine, and gemcitabine), spindle poisons (vinblastine, vincristine, and vinorelbine), podophyllotoxins (etoposide, irinotecan, and topotecan), antibiotics (daunorubicin, doxorubicin, bleomycin, and mitomycin), nitrosoureas (carmustine and lomustine), enzymes (asparaginasc), and hormones (tamoxifen, leuprolide, flutamide, and megestrol), imatinib, adriamycin, dexamethasone, and cyclophosphamide. For a more comprehensive discussion of updated cancer therapies; *See,* http://www.nci.nih.gov/, a list of the FDA approved oncology drugs at http://www.fda.gov/cder/cancer/dniglistframe.htm, and The Merck Manual, Seventeenth Ed. 1999.

In another embodiment, the method described herein comprises administration of a compound of Formula (I), or an isotopic variant thereof, or a pharmaceutically acceptable salt, solvate, or hydrate thereof and a second agent selected from a BTK inhibitor, Bcl-2 inhibitor and EZH2 inhibitor, together with administering one or more chemotherapeutic agents and/or therapies selected from: alkylation agents (e.g., cisplatin, carboplatin); antimetabolites (e.g., methotrexate and 5-FU); antitumor antibiotics (e.g., adriamymycin and bleomycin); antitumor vegetable alkaloids (e.g., taxol and etoposide); antitumor hormones (e.g., dexamethasone and tamoxifen); antitumor immunological agents (e.g., interferon $\alpha$, $\beta$, and $\gamma$); radiation therapy; and surgery. In certain embodiments, the one or more chemotherapeutic agents and/or therapies are administered to the subject before, during, or after the administration of a compound of Formula (I), or an isotopic variant thereof, or a pharmaceutically acceptable salt, solvate, or hydrate thereof and a BTK inhibitor.

Such other agents, or drugs, can be administered, by a route and in an amount commonly used therefor, simultaneously or sequentially with a compound of Formula (I), or an isotopic variant thereof; or a pharmaceutically acceptable salt, solvate, or hydrate thereof and a BTK inhibitor. When a compound of Formula (I) and a BTK inhibitor are used contemporaneously with one or more other drugs, a pharmaceutical composition containing such other drugs in addition to the a compound of Formula (I), or an isotopic variant thereof; or a pharmaceutically acceptable salt, solvate, or hydrate thereof and a BTK inhibitor can be utilized, but is not required. Accordingly, the pharmaceutical compositions described herein include those that also contain one or more other active ingredients or therapeutic agents, in addition to a compound of Formula (I).

## PHARMACEUTICAL COMPOSITIONS AND ROUTES OF ADMINISTRATION

Also described herein is a pharmaceutical composition comprising a compound described herein (a compound

of Formula (I), a BTK inhibitor, a Bcl-2 inhibitor, or a EZH2 inhibitor) and a pharmaceutically acceptable excipient, adjuvant, carrier, buffer, or stabilizer. In some embodiments, the compound of Formula (I) and the second agent selected from BTK inhibitor, a Bcl-2 inhibitor, and an EZH2 inhibitor are present in the same pharmaceutical composition. In some embodiments, the compound of Formula (I) and the second agent selected from BTK inhibitor, a Bcl-2 inhibitor, and an EZH2 inhibitor are in different pharmaceutical compositions.

In one embodiment, the pharmaceutical compositions are provided in a dosage form for oral administration, which comprise a compound described herein, and one or more pharmaceutically acceptable excipients or carriers. The pharmaceutical compositions described herein that are formulated for oral administration may be in tablet, capsule, powder, or liquid form. In some embodiments, a tablet comprises a solid carrier or an adjuvant. Liquid pharmaceutical compositions generally comprise a liquid carrier such as water, petroleum, animal or vegetable oils, mineral oil, or synthetic oil. Physiological saline solution, dextrose or other saccharide solution, or glycols such as ethylene glycol, propylene glycol, or polyethylene glycol may be included. In some embodiments, a capsule comprises a solid carrier such as gelatin.

In another embodiment, the pharmaceutical compositions are provided in a dosage form for parenteral administration, which comprise a compound described herein, and one or more pharmaceutically acceptable excipients or carriers. Where pharmaceutical compositions may be formulated for intravenous, cutaneous or subcutaneous injection, the active ingredient will be in the form of a parenterally acceptable aqueous solution, which is pyrogen-free and has a suitable pH, isotonicity, and stability. Those of relevant skill in the art are well able to prepare suitable solutions using, for example, isotonic vehicles, such as Sodium Chloride injection, Ringer's injection, or Lactated Ringer's injection. In some embodiments, preservatives, stabilisers, buffers, antioxidants, and/or other additives are included as required.

In yet another embodiment, the pharmaceutical compositions are provided in a dosage form for topical administration, which comprise a compound described herein, and one or more pharmaceutically acceptable excipients or carriers.

The pharmaceutical compositions can also be formulated as modified release dosage forms, including delayed-, extended-, prolonged-, sustained-, pulsatile-, controlled-, accelerated-, fast-, targeted-, and programmed-release, and gastric retention dosage forms. These dosage forms can be prepared according to conventional methods and techniques known to those skilled in the art (*see, Remington: The Science and Practice of Pharmacy,* supra; *Modified-Release Drug Delivery Technology,* 2nd Edition, Rathbone *et al.,* Eds., Marcel Dekker, Inc.: New York, NY, 2008).

The pharmaceutical compositions described herein can be provided in a unit-dosage form or multiple-dosage form. A unit-dosage form, as used herein, refers to physically discrete a unit suitable for administration to a human and animal subject, and packaged individually as is known in the art. Each unit-dose contains a predetermined quantity of an active ingredient(s) sufficient to produce the desired therapeutic effect, in association with the required pharmaceutical carriers or excipients. Examples of a unit-dosage form include an ampoule, syringe, and individually packaged tablet and capsule. A unit-dosage form may be administered in fractions or multiples thereof. A multiple-dosage form is a plurality of identical unit-dosage forms packaged in a single container to be administered in segregated unit-dosage form. Examples of a multiple-dosage form include a vial, bottle of tablets or capsules, or bottle of pints or gallons.

The pharmaceutical compositions described herein can be administered at once, or multiple times at intervals of time. It is understood that the precise dosage and duration of treatment may vary with the age, weight, and condition of the patient being treated, and may be determined empirically using known testing protocols or by extrapolation from *in vivo* or *in vitro* test or diagnostic data. It is further understood that for any particular individual, specific dosage regimens should be adjusted over time according to the individual need and the professional judgment of the person administering or supervising the administration of the formulations.

In certain embodiments, the pharmaceutical compositions described herein further comprise one or more chemotherapeutic agents as defined herein.

A. Oral Administration

The pharmaceutical compositions described herein for oral administration can be provided in solid, semisolid, or liquid dosage forms for oral administration. As used herein, oral administration also includes buccal, lingual, and sublingual administration. Suitable oral dosage forms include, but are not limited to, tablets, fastmelts, chewable tablets, capsules, pills, strips, troches, lozenges, pastilles, cachets, pellets, medicated chewing gum, bulk powders, effervescent or non-effervescent powders or granules, oral mists, solutions, emulsions, suspensions, wafers, sprinkles, elixirs, and syrups. In addition to the active ingredient(s), the pharmaceutical compositions can contain one or more pharmaceutically acceptable carriers or excipients, including, but not limited to, binders, fillers, diluents, disintegrants, wetting agents, lubricants, glidants, coloring agents, dye-migration inhibitors, sweetening agents, flavoring agents, emulsifying agents, suspending and dispersing agents, preservatives, solvents, non-aqueous liquids, organic acids, and sources of carbon dioxide.

Binders or granulators impart cohesiveness to a tablet to ensure the tablet remaining intact after compression.

Suitable binders or granulators include, but are not limited to, starches, such as corn starch, potato starch, and pre-gelatinized starch (e.g., STARCH 1500); gelatin; sugars, such as sucrose, glucose, dextrose, molasses, and lactose; natural and synthetic gums, such as acacia, alginic acid, alginates, extract of Irish moss, panwar gum, ghatti gum, mucilage of isabgol husks, carboxymethylcellulose, methylcellulose, polyvinylpyrrolidone (PVP), Veegum, larch arabogalactan, powdered tragacanth, and guar gum; celluloses, such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC); microcrystalline celluloses, such as AVICEL-PH-101, AVICEL-PH-103, AVICEL RC-581, AVICEL-PH-105 (FMC Corp., Marcus Hook, PA); and mixtures thereof. Suitable fillers include, but are not limited to, talc, calcium carbonate, microcrystalline cellulose, powdered cellulose, dextrates, kaolin, mannitol, silicic acid, sorbitol, starch, pre-gelatinized starch, and mixtures thereof. The amount of a binder or filler in the pharmaceutical compositions described herein varies upon the type of formulation, and is readily discernible to those of ordinary skill in the art. The binder or filler may be present from about 50 to about 99% by weight in the pharmaceutical compositions described herein.

Suitable diluents include, but are not limited to, dicalcium phosphate, calcium sulfate, lactose, sorbitol, sucrose, inositol, cellulose, kaolin, mannitol, sodium chloride, dry starch, and powdered sugar. Certain diluents, such as mannitol, lactose, sorbitol, sucrose, and inositol, when present in sufficient quantity, can impart properties to some compressed tablets that permit disintegration in the mouth by chewing. Such compressed tablets can be used as chewable tablets. The amount of a diluent in the pharmaceutical compositions described herein varies upon the type of formulation, and is readily discernible to those of ordinary skill in the art.

Suitable disintegrants include, but are not limited to, agar; bentonite; celluloses, such as methylcellulose and carboxymethylcellulose; wood products; natural sponge; cation-exchange resins; alginic acid; gums, such as guar gum and Veegum HV; citrus pulp; cross-linked celluloses, such as croscarmellose; cross-linked polymers, such as crospovidone; cross-linked starches; calcium carbonate; microcrystalline cellulose, such as sodium starch glycolate; polacrilin potassium; starches, such as corn starch, potato starch, tapioca starch, and pre-gelatinized starch; clays; aligns; and mixtures thereof. The amount of a disintegrant in the pharmaceutical compositions described herein varies upon the type of formulation, and is readily discernible to those of ordinary skill in the art. The amount of a disintegrant in the pharmaceutical compositions described herein varies upon the type of formulation, and is readily discernible to those of ordinary skill in the art. The pharmaceutical compositions described herein may contain from about 0.5 to about 15% or from about 1 to about 5% by weight of a disintegrant.

Suitable lubricants include, but are not limited to, calcium stearate; magnesium stearate; mineral oil; light mineral oil; glycerin; sorbitol; mannitol; glycols, such as glycerol behenate and polyethylene glycol (PEG); stearic acid; sodium lauryl sulfate; talc; hydrogenated vegetable oil, including peanut oil, cottonseed oil, sunflower oil, sesame oil, olive oil, corn oil, and soybean oil; zinc stearate; ethyl oleate; ethyl laureate; agar; starch; lycopodium; silica or silica gels, such as AEROSIL® 200 (W.R. Grace Co., Baltimore, MD) and CAB-O-SIL® (Cabot Co. of Boston, MA); and mixtures thereof. The pharmaceutical compositions described herein may contain about 0.1 to about 5% by weight of a lubricant.

Suitable glidants include, but are not limited to, colloidal silicon dioxide, CAB-O-SIL® (Cabot Co. of Boston, MA), and asbestos-free talc. Suitable coloring agents include, but are not limited to, any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Suitable flavoring agents include, but are not limited to, natural flavors extracted from plants, such as fruits, and synthetic blends of compounds which produce a pleasant taste sensation, such as peppermint and methyl salicylate. Suitable sweetening agents include, but are not limited to, sucrose, lactose, mannitol, syrups, glycerin, and artificial sweeteners, such as saccharin and aspartame. Suitable emulsifying agents include, but are not limited to, gelatin, acacia, tragacanth, bentonite, and surfactants, such as polyoxyethylene sorbitan monooleate (TWEEN® 20), polyoxyethylene sorbitan monooleate 80 (TWEEN® 80), and triethanolamine oleate. Suitable suspending and dispersing agents include, but are not limited to, sodium carboxymethylcellulose, pectin, tragacanth, Veegum, acacia, sodium carbomethylcellulose, hydroxypropyl methylcellulose, and polyvinylpyrrolidone. Suitable preservatives include, but are not limited to, glycerin, methyl and propylparaben, benzoic add, sodium benzoate and alcohol. Suitable wetting agents include, but are not limited to, propylene glycol monostearate, sorbitan monooleate, diethylene glycol monolaurate, and polyoxyethylene lauryl ether. Suitable solvents include, but are not limited to, glycerin, sorbitol, ethyl alcohol, and syrup. Suitable non-aqueous liquids utilized in emulsions include, but are not limited to, mineral oil and cottonseed oil. Suitable organic acids include, but are not limited to, citric and tartaric acid. Suitable sources of carbon dioxide include, but are not limited to, sodium bicarbonate and sodium carbonate.

It should be understood that many carriers and excipients may serve several functions, even within the same formulation.

The pharmaceutical compositions described herein for oral administration can be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that

resist the action of stomach acid but dissolve or disintegrate in the intestine, thus protecting the active ingredients from the acidic environment of the stomach. Enteric-coatings include, but are not limited to, fatty acids, fats, phenyl salicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms can be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions described herein for oral administration can be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms described herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions described herein for oral administration can be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquid or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxyl groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) described herein, and a dialkylated mono- or poly-alkylene glycol, including, 1,2-dimetboxymethane, diglyme, triglyme, tetraglyme, polyethylene glycol-350-dimethyl ether, polyethylene glycol-550-dimcthyl ether, polyethylene glycol-750-dimethyl ether, wherein 350, 550, and 750 refer to the approximate average molecular weight of the polyethylene glycol. These formulations can further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

The pharmaceutical compositions described herein for oral administration can be also provided in the forms of liposomes, micelles, microspheres, or nanosystems. Micellar dosage forms can be prepared as described in U.S. Pat. No. 6,350,458.

The pharmaceutical compositions described herein for oral administration can be provided as non-effervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms.

The pharmaceutical compositions described herein for oral administration can be formulated as immediate or modified release dosage forms, including delayed-, sustained, pulsed-, controlled, targeted-, and programmed-release forms.

B. Parenteral Administration

The pharmaceutical compositions described herein can be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, intravesical, and subcutaneous administration.

The pharmaceutical compositions described herein for parenteral administration can be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (*see, Remington: The Science and Practice of Pharmacy,* supra).

The pharmaceutical compositions intended for parenteral administration can include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Suitable non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Suitable water-miscible vehicles include, but are not limited to, ethanol, 1,3-butanediol, liquid polyethylene glycol (e.g., polyethylene glycol 300 and polyethylene glycol 400), propylene glycol, glycerin, *N*-methyl-2-pyrrolidone, *N,N*-dimethylacetamide, and dimethyl sulfoxide.

Suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (e.g., benzethonium chloride), methyl-and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents are those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including $\alpha$-cyclodextrin, $\beta$-cyclodextrin, hydroxypropyl-$\beta$-cyclodextrin, sulfobutylether-$\beta$-cyclodextrin, and sulfobutylether 7-$\beta$-cyclodextrin (CAPTISOL®, CyDex, Lenexa, KS).

When the pharmaceutical compositions described herein are formulated for multiple dosage administration, the multiple dosage parenteral formulations must contain an antimicrobial agent at bacteriostatic or fungi static concentrations. All parenteral formulations must be sterile, as known and practiced in the art.

In one embodiment, the pharmaceutical compositions for parenteral administration are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions described herein for parenteral administration can be formulated as immediate or modified release dosage forms, including delayed-, sustained, pulsed-, controlled, targeted-, and programmed-release forms.

The pharmaceutical compositions described herein for parenteral administration can be formulated as a suspension, solid, semi-solid, or thixotropic liquid, for administration as an implanted depot. In one embodiment, the pharmaceutical compositions described herein are dispersed in a solid inner matrix, which is surrounded by an outer polymeric membrane that is insoluble in body fluids but allows the active ingredient in the pharmaceutical compositions diffuse through.

Suitable inner matrixes include, but are not limited to, polymethylmethacrylate, polybutylmethacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and metliacrylic acid, collagen, cross-linked polyvinyl alcohol, and cross-linked partially hydrolyzed polyvinyl acetate.

Suitable outer polymeric membranes include but are not limited to, polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

C. Topical Administration

The pharmaceutical compositions described herein can be administered topically to the skin, orifices, or mucosa. The topical administration, as used herein, includes (intra)dermal, conjunctival, intracorneal, intraocular, ophthalmic, auricular, transdermal, nasal, vaginal, urethral, respiratory, and rectal administration.

The pharmaceutical compositions described herein can be formulated in any dosage forms that are suitable for topical administration for local or systemic effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, and dermal patches. The topical formulation of the pharmaceutical compositions described herein can also comprise liposomes, micelles, microspheres, nanosystems, and mixtures thereof.

Pharmaceutically acceptable carriers and excipients suitable for use in the topical formulations described herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions can also be administered topically by electroporation, iontophoresis, phonophoresis, sonophoresis, or microneedle or needle-free injection, such as POWDERJECT™ (Chiron Corp., Emeryville, CA), and BIOJECT™ (Bioject Medical Technologies Inc., Tualatin, OR).

The pharmaceutical compositions described herein can be provided in the forms of ointments, creams, and gels. Suitable ointment vehicles include oleaginous or hydrocarbon vehicles, including lard, benzoinated lard, olive oil, cottonseed oil, and other oils, white petrolatum; emulsifiable or absorption vehicles, such as hydrophilic petrolatum, hydroxystearin sulfate, and anhydrous lanolin; water-removable vehicles, such as hydrophilic ointment; water-soluble ointment vehicles, including polyethylene glycols of varying molecular weight; emulsion vehicles, either water-in-oil (W/O) emulsions or oil-in-water (O/W) emulsions, including cetyl alcohol, glyceryl monostearate, lanolin, and stearic acid (*see, Remington: The Science and Practice of Pharmacy,* supra). These vehicles are emollient but generally require addition of antioxidants and preservatives.

Suitable cream base can be oil-in-water or water-in-oil. Suitable cream vehicles may be water-washable, and contain an oil phase, an emulsifier, and an aqueous phase. The oil phase is also called the "internal" phase, which is generally comprised of petrolatum and a fatty alcohol such as cetyl or stearyl alcohol. The aqueous phase usually, although not necessarily, exceeds the oil phase in volume, and generally contains a humectant. In some embodiments, the emulsifier in a cream formulation is a nonionic, anionic, cationic, or amphoteric surfactant.

Gels are semisolid, suspension-type systems. Single-phase gels contain organic macromolecules distributed substantially uniformly throughout the liquid carrier. Suitable gelling agents include, but are not limited to, crosslinked acrylic acid polymers, such as carbomers, carboxypolyalkylenes, and CARBOPOL®; hydrophilic polymers, such as polyethylene oxides, polyoxyethylene-polyoxypropylene copolymers, and polyvinyl alcohol; cellulosic polymers, such as hydroxypropyl cellulose, hydroxyethyl cellulose, hydroxypropyl methylcellulose, hydroxypropyl methylcellulose phthalate, and methylcellulose; gums, such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

The pharmaceutical compositions described herein can be administered rectally, urethrally, vaginally, or perivaginally in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in *Remington: The Science and Practice of Pharmacy,* supra.

Rectal, urethral, and vaginal suppositories are solid bodies for insertion into body orifices, which are solid at ordinary temperatures but melt or soften at body temperature to release the active ingredient(s) inside the orifices. Pharmaceutically acceptable carriers utilized in rectal and vaginal suppositories include bases or vehicles, such as stiffening agents, which produce a melting point in the proximity of body temperature, when formulated with the pharmaceutical compositions described herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, and hydrogels, such as polyvinyl alcohol, hydroxyethyl methacrylate, and polyacrylic acid;. Combinations of the various vehicles can also be used. Rectal and vaginal suppositories may be prepared by compressing or molding. The typical weight of a rectal and vaginal suppository is about 2 to about 3 g.

The pharmaceutical compositions described herein can be administered ophthalmically in the forms of solutions, suspensions, ointments, emulsions, gel-forming solutions, powders for solutions, gels, ocular inserts, and implants.

The pharmaceutical compositions described herein can be administered intranasally or by inhalation to the respiratory tract. The pharmaceutical compositions can be provided in the form of an aerosol or solution for delivery using a pressurized container, pump, spray, atomizer, such as an atomizer using electrohydrodynamics to produce a fine mist, or nebulizer, alone or in combination with a suitable propellant, such as 1,1,1,2-tetrafluoroethane or 1,1,1,2,3,3,3-heptafluoropropane. The pharmaceutical compositions can also be provided as a dry powder for insufflation, alone or in combination with an inert carrier such as lactose or phospholipids; and nasal drops. For intranasal use, the powder can comprise a bioadhesive agent, including chitosan or cyclodextrin.

Solutions or suspensions for use in a pressurized container, pump, spray, atomizer, or nebulizer can be formulated to contain ethanol, aqueous ethanol, or a suitable alternative agent for dispersing, solubilizing, or extending release of the active ingredient described herein; a propellant as solvent; and/or a surfactant, such as sorbitan trioleate, oleic acid, or an oligolactic acid.

The pharmaceutical compositions described herein can be micronized to a size suitable for delivery by inhalation, such as about 50 micrometers or less, or about 10 micrometers or less. Particles of such sizes can be prepared using a comminuting method known to those skilled in the art, such as spiral jet milling, fluid bed jet milling, supercritical fluid processing to form nanoparticles, high pressure homogenization, or spray drying.

Capsules, blisters, and cartridges for use in an inhaler or insufflator can be formulated to contain a powder mix of the pharmaceutical compositions described herein; a suitable powder base, such as lactose or starch; and a performance modifier, such as *l*-leucine, mannitol, or magnesium stearate. The lactose may be anhydrous or in the form of the monohydrate. Other suitable excipients or carriers include, but are not limited to, dextran, glucose, maltose, sorbitol, xylitol, fructose, sucrose, and trehalose. The pharmaceutical compositions described herein for inhaled/intranasal administration can further comprise a suitable flavor, such as menthol and levomenthol; and/or sweeteners, such as saccharin and saccharin sodium.

The pharmaceutical compositions described herein for topical administration can be formulated to be immediate release or modified release, including delayed-, sustained-, pulsed-, controlled-, targeted, and programmed release.

D. Modified Release

The pharmaceutical compositions described herein can be formulated as a modified release dosage form. As used herein, the term "modified release" refers to a dosage form in which the rate or place of release of the active ingredient(s) is different from that of an immediate dosage form when administered by the same route. Modified release dosage forms include, but are not limited to, delayed-, extended-, prolonged-, sustained-, pulsatile-, controlled-, accelerated- and fast-, targeted-, programmed-release, and gastric retention dosage forms. The pharmaceutical compositions in modified release dosage forms can be prepared using a variety of modified release devices and methods known to those skilled in the art, including, but not limited to, matrix controlled release devices, osmotic controlled release devices, multiparticulate controlled release devices, ion-exchange resins, enteric coatings, multilayered coatings, microspheres, liposomes, and combinations thereof. The release rate of the active ingredient(s) can also be modified by varying the particle sizes and polymorphism of the active ingredient(s).

Examples of modified release include, but are not limited to, those described in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; and 6,699,500.

1. Matrix Controlled Release Devices

The pharmaceutical compositions described herein in a modified release dosage form can be fabricated using a matrix controlled release device known to those skilled in the art (*see,* Takada et al. in "Encyclopedia of Controlled Drug Delivery," Vol. 2, Mathiowitz Ed., Wiley, 1999).

In certain embodiments, the pharmaceutical compositions described herein in a modified release dosage form is formulated using an erodible matrix device, which is water-swellable, erodible, or soluble polymers, including, but not

limited to, synthetic polymers, and naturally occurring polymers and derivatives, such as polysaccharides and proteins.

Materials useful in forming an erodible matrix include, but are not limited to, chitin, chitosan, dextran, and pullulan; gum agar, gum arabic, gum karaya, locust bean gum, gum tragacanth, carrageenans, gum ghatti, guar gum, xanthan gum, and scleroglucan; starches, such as dextrin and maltodextrin; hydrophilic colloids, such as pectin; phosphatides, such as lecithin; alginates; propylene glycol alginate; gelatin; collagen; cellulosics, such as ethyl cellulose (EC), methylethyl cellulose (MEC), carboxymethyl cellulose (CMC), CMEC, hydroxyethyl cellulose (HEC), hydroxypropyl cellulose (HPC), cellulose acetate (CA), cellulose propionate (CP), cellulose butyrate (CB), cellulose acetate butyrate (CAB), CAP, CAT, hydroxypropyl methyl cellulose (HPMC), HPMCP, HPMCAS, hydroxypropyl methyl cellulose acetate trimellitate (HPMCAT), and ethyl hydroxyethyl cellulose (EHEC); polyvinyl pyrrolidone; polyvinyl alcohol; polyvinyl acetate; glycerol fatty acid esters; polyacrylamide; polyacrylic acid; copolymers of ethacrylic acid or methacrylic acid (EUDRAGIT®, Rohm America, Inc., Piscataway, NJ); poly(2-hydroxyethyl-methacrylate), polylactides; copolymers of L-glutamic acid and ethyl-L-glutamate; degradable lactic acid-glycolic acid copolymers; poly-D-(-)-3-hydroxybutyric acid; and other acrylic acid derivatives, such as homopolymers and copolymers of butylmethacrylate, methyl methacrylate, ethyl methacrylate, ethylacrylate, (2-dimethylaminoethyl)methacrylate, and (trimethylaminoethyl)methacrylate chloride.

In certain embodiments, the pharmaceutical compositions described herein are formulated with a non-erodible matrix device. The active ingredient(s) is dissolved or dispersed in an inert matrix and is released primarily by diffusion through the inert matrix once administered. Materials suitable for use as a non-erodible matrix device include, but are not limited to, insoluble plastics, such as polyethylene, polypropylene, polyisoprene, polyisobutylene, polybutadiene, polymethylmethacrylate, polybutylmethacrylate, chlorinated polyethylene, polyvinylchloride, methyl acrylate-methyl methacrylate copolymers, ethylene-vinyl acetate copolymers, ethylene/propylene copolymers, ethylene/ethyl acrylate copolymers, vinyl chloride copolymers with vinyl acetate, vinylidene chloride, ethylene and propylene, ionomer polyethylene terephthalate, butyl rubbers, epichlorohydrin rubbers, ethylene/vinyl alcohol copolymer, ethylene/vinyl acetate/vinyl alcohol terpolymer, ethylene/vinyloxyethanol copolymer, polyvinyl chloride, plasticized nylon, plasticized polyethylene terephthalate, natural rubber, silicone rubbers, polydimethylsiloxanes, and silicone carbonate copolymers; hydrophilic polymers, such as ethyl cellulose, cellulose acetate, crospovidone, and cross-linked partially hydrolyzed polyvinyl acetate; and fatty compounds, such as carnauba wax, microcrystalline wax, and triglycerides.

In a matrix controlled release system, the desired release kinetics can be controlled, for example, *via* the polymer type employed, the polymer viscosity, the particle sizes of the polymer and/or the active ingredient(s), the ratio of the active ingredient(s) versus the polymer, and other excipients or carriers in the compositions.

The pharmaceutical compositions described herein in a modified release dosage form can be prepared by methods known to those skilled in the art, including direct compression, dry or wet granulation followed by compression, and melt-granulation followed by compression.

2. Osmotic Controlled Release Devices

The pharmaceutical compositions described herein in a modified release dosage form can be fabricated using an osmotic controlled release device, including, but not limited to, one-chamber system, two-chamber system, asymmetric membrane technology (AMT), and extruding core system (ECS). In general, such devices have at least two components: (a) a core which contains an active ingredient; and (b) a semipermeable membrane with at least one delivery port, which encapsulates the core. The semipermeable membrane controls the influx of water to the core from an aqueous environment of use so as to cause drug release by extrusion through the delivery port(s).

In addition to the active ingredient(s), the core of the osmotic device optionally includes an osmotic agent, which creates a driving force for transport of water from the environment of use into the core of the device. One class of osmotic agents is water-swellable hydrophilic polymers, which are also referred to as "osmopolymers" and "hydrogels." Suitable water-swellable hydrophilic polymers as osmotic agents include, but are not limited to, hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP), crosslinked PVP, polyvinyl alcohol (PVA), PVA/PVP copolymers, PVA/PVP copolymers with hydrophobic monomers such as methyl methacrylate and vinyl acetate, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl, cellulose (CEC), sodium alginate, polycarbophil, gelatin, xanthan gum, and sodium starch glycolate.

The other class of osmotic agents is osmogens, which are capable of imbibing water to affect an osmotic pressure gradient across the barrier of the surrounding coating. Suitable osmogens include, but are not limited to, inorganic salts, such as magnesium sulfate, magnesium chloride, calcium chloride, sodium chloride, lithium chloride, potassium sulfate, potassium phosphates, sodium carbonate, sodium sulfite, lithium sulfate, potassium chloride, and sodium sulfate; sugars, such as dextrose, fructose, glucose, inositol, lactose, maltose, mannitol, raffinose, sorbitol, sucrose, trehalose, and xylitol; organic acids, such as ascorbic acid, benzoic acid, fumaric acid, citric acid, maleic acid,

sebacic acid, sorbic acid, adipic acid, edetic acid, glutamic acid, p-toluenesulfonic acid, succinic acid, and tartaric acid; urea; and mixtures thereof.

Osmotic agents of different dissolution rates can be employed to influence how rapidly the active ingredient(s) is initially delivered from the dosage form. For example, amorphous sugars, such as MANNOGEM™ EZ (SPI Pharma, Lewes, DE) can be used to provide faster delivery during the first couple of hours to promptly produce the desired therapeutic effect, and gradually and continually release of the remaining amount to maintain the desired level of therapeutic or prophylactic effect over an extended period of time. In this case, the active ingredient(s) is released at such a rate to replace the amount of the active ingredient metabolized and excreted.

The core can also include a wide variety of other excipients and carriers as described herein to enhance the performance of the dosage form or to promote stability or processing.

Materials useful in forming the semipermeable membrane include various grades of acrylics, vinyls, ethers, polyamides, polyesters, and cellulosic derivatives that are water-permeable and water-insoluble at physiologically relevant pHs, or are susceptible to being rendered water-insoluble by chemical alteration, such as crosslinking. Examples of suitable polymers useful in forming the coating, include plasticized, unplasticized, and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxylated ethylene-vinyl acetate, EC, PEG, PPG, PEG/PPG copolymers, PVP, HEC, HPC, CMC, CMEC, HPMC, HPMCP, HPMCAS, HPMCAT, poly(acrylic) acids and esters and poly-(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes, and synthetic waxes.

Semipermeable membrane can also be a hydrophobic microporous membrane, wherein the pores are substantially filled with a gas and are not wetted by the aqueous medium but are permeable to water vapor, as disclosed in U.S. Pat. No. 5,798,119. Such hydrophobic but water-vapor permeable membrane are typically composed of hydrophobic polymers such as polyalkenes, polyethylene, polypropylene, polytetrafluoroethylene, polyacrylic acid derivatives, polyethers, polysulfones, polyethersulfones, polystyrenes, polyvinyl halides, polyvinylidene fluoride, polyvinyl esters and ethers, natural waxes, and synthetic waxes

The delivery port(s) on the semipermeable membrane can be formed post-coating by mechanical or laser drilling. Delivery port(s) can also be formed in situ by erosion of a plug of water-soluble material or by rupture of a thinner portion of the membrane over an indentation in the core. In addition, delivery ports can be formed during coating process, as in the case of asymmetric membrane coatings of the type disclosed in U.S. Pat. Nos. 5,612,059 and 5,698,220.

The total amount of the active ingredient(s) released and the release rate can substantially by modulated *via* the thickness and porosity of the semipermeable membrane, the composition of the core, and the number, size, and position of the delivery ports

The pharmaceutical compositions in an osmotic controlled-release dosage form can further comprise additional conventional excipients or carriers as described herein to promote performance or processing of the formulation.

The osmotic controlled-release dosage forms can be prepared according to conventional methods and techniques known to those skilled in the art (*see, Remington: The Science and Practice of Pharmacy,* supra; Santus and Baker, J. Controlled Release 1995, 35, 1-21; Verma et al., Drug Development and Industrial Pharmacy 2000, 26, 695-708; Verma et al., J. Controlled Release 2002, 79, 7-27).

In certain embodiments, the pharmaceutical compositions described herein are formulated as AMT controlled-release dosage form, which comprises an asymmetric osmotic membrane that coats a core comprising the active ingredient(s) and other pharmaceutically acceptable excipients or carriers. *See,* U.S. Pat. No. 5,612,059 and WO 2002/17918. The AMT controlled-release dosage forms can be prepared according to conventional methods and techniques known to those skilled in the art, including direct compression, dry granulation, wet granulation, and a dip-coating method.

In certain embodiments, the pharmaceutical compositions described herein are formulated as ESC controlled-release dosage form, which comprises an osmotic membrane that coats a core comprising the active ingredient(s), a hydroxylethyl cellulose, and other pharmaceutically acceptable excipients or carriers.

3. Multiparticulate Controlled Release Devices

The pharmaceutical compositions described herein in a modified release dosage form can be fabricated as a multiparticulate controlled release device, which comprises a multiplicity of particles, granules, or pellets, ranging from about 10 μm to about 3 mm, about 50 μm to about 2.5 mm, or from about 100 μm to about 1 mm in diameter. Such multiparticulates can be made by the processes known to those skilled in the art, including wet-and dry-granulation, extrusion/spheronization, roller-compaction, melt-congealing, and by spray-coating seed cores. *See,* for example, Multiparticulate Oral Drug Delivery; Marcel Dekker: 1994; and Pharmaceutical Pelletization Technology; Marcel Dekker: 1989.

Other excipients or carriers as described herein can be blended with the pharmaceutical compositions to aid in processing and forming the multiparticulates. The resulting particles can themselves constitute the multiparticulate device or can be coated by various film-forming materials, such as enteric polymers, water-swellable, and water-soluble polymers. The multiparticulates can be further processed as a capsule or a tablet.

4. Targeted Delivery

The pharmaceutical compositions described herein can also be formulated to be targeted to a particular tissue, receptor, or other area of the body of the subject to be treated, including liposome-, resealed erythrocyte-, and antibody-based delivery systems. Examples include, but are not limited to, those disclosed in U.S. Pat. Nos. 6,316,652; 6,274,552; 6,271,359; 6,253,872; 6,139,865; 6,131,570; 6,120,751; 6,071,495; 6,060,082; 6,048,736; 6,039,975; 6,004,534; 5,985,307; 5,972,366; 5,900,252; 5,840,674; 5,759,542; and 5,709,874.

5. Articles of Manufacture

The compounds described herein can also be provided as an article of manufacture using packaging materials well known to those of skill in the art. *See, e.g.,* U.S. Pat. Nos. 5,323,907; 5,052,558; and 5,033,252. Examples of pharmaceutical packaging materials include, but are not limited to, blister packs, bottles, tubes, inhalers, pumps, bags, vials, containers, syringes, and any packaging material suitable for a selected formulation and intended mode of administration and treatment.

Described herein also are kits which, when used by the medical practitioner, can simplify the administration of appropriate amounts of active ingredients to a subject. In certain embodiments, the kit described herein includes one or more containers and a dosage form of a compound of Formula (I), or an isotopic variant thereof; or a pharmaceutically acceptable salt, solvate, or hydrate thereof and a second agent selected from a BTK inhibitor, a Bcl-2 inhibitor and an EZH2 inhibitor.

In certain embodiments, the kit described herein includes one or more containers and a dosage form of a compound of Formula (I), or an isotopic variant thereof; or a pharmaceutically acceptable salt, solvate, or hydrate thereof and a BTK inhibitor. Kits described herein can further include devices that are used to administer the active ingredients. Examples of such devices include, but are not limited to, syringes, needle-less injectors drip bags, patches, and inhalers.

Kits described herein can further include pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: aqueous vehicles, including, but not limited to, Water for Injection USP, Sodium Chloride Injection, Ringer's Injection, Dextrose Injection, Dextrose and Sodium Chloride Injection, and Lactated Ringer's Injection; water-miscible vehicles, including, but not limited to, ethyl alcohol, polyethylene glycol, and polypropylene glycol; and non-aqueous vehicles, including, but not limited to, corn oil, cottonseed oil, peanut oil, sesame oil, ethyl oleate, isopropyl myristate, and benzyl benzoate.

The disclosure will be further understood by the following non-limiting examples.

## EXAMPLES

As used herein, the symbols and conventions used in these processes, schemes and examples, regardless of whether a particular abbreviation is specifically defined, are consistent with those used in the contemporary scientific literature, for example, the Journal of the American Chemical Society or the Journal of Biological Chemistry. Specifically, but without limitation, the following abbreviations may be used in the examples and throughout the specification: g (grams); mg (milligrams); mL (milliliters); $\mu$L, (microliters); M (molar); mM (millimolar), $\mu$M (micro molar); eq. (equivalent); mmol (millimoles), Hz (Hertz), MHz (megahertz); hr or hrs (hour or hours); min (minutes); and MS (mass spectrometry).

For all of the following examples, standard work-up and purification methods known to those skilled in the art can be utilized. Unless otherwise indicated, all temperatures are expressed in °C (degrees Centigrade). All reactions conducted at room temperature unless otherwise noted. Synthetic methodologies illustrated herein are intended to exemplify the applicable chemistry through the use of specific examples and are not indicative of the scope of the disclosure.

Synthesis of compound I is described in US Patent No. 9,056,852 B2.

**Example 1:** Synthesis of 4-(2-(difluoromethyl)-1*H*-benzo[*d*]imidazol-1-yl)-N-(2-methyl-1-(2-(1-methylpiperidin-4-yl)phenyl)propan-2-yl)-6-morpholino-1,3,5-triazin-2-amine, Compound I

A mixture of 4-(2-(difluoromethyl)-1H-benzo[d]imidazol-1-yl)-N-(2-methyl-1-(2-(piperidin-4-yl)phenyl)propan-2-yl)-6-morpholino-1,3,5-triazin-2-amine (80 mg, 0.14 mmol), *aq.* formaldehyde (37%, 23 mg), and sodium cyanoborohydride (11 mg, 0.17 mmol) in methanol (2 mL) was stirred at room temperature for 1 hr. The crude product was purified by prep-HPLC to give compound I (11 mg, 13% yield) as a white solid: 99% purity (HPLC); MS *m/z:* 577.3 (M+1); $^1$H NMR ($CDCl_3$, 500 MHz) $\delta$ 8.37 (d, 1H), 7.90 (d, 1H), 7.64 (t, 1H), 7.42 (m, 2H), 7.32 (d, 1H), 7.24 (1, 1H), 7.13 (t, 1H), 7.07 (d, 1H), 5.15 (s, 1H), 4.00-3.70 (m, 8H), 3.28 (s, 2H), 2.94 (m, 2H), 2.78 (m, 2H), 2.28 (s, 3H), 1.89-1.60 (m, 6H), 1.53 (s, 6H) ppm.

**Example 2:** Combination Studies

The activity of compound I was examined in combination with PCI-32765 (ibrutinib), ABT-199 (venetoclax) and EPZ-6438 (Tazemetostat) across a panel of 8 DLBCL cell lines indicated below (7 GCB subtype cell lines and 1 ABC subtype cell line).

Table I

| Cell Lines | Subtype |
|---|---|
| DB | GCB |
| DOHH-2 | GCB |
| HT | GCB |
| NU-DHL-1 | GCB |
| Pfeiffer | GCB |
| SU-DHL-10 | GCB |
| OCI-Ly19 | GCB |
| OCI-Ly3 | ABC |

**ATPLite assay**

The effect of test compounds and combinations on growth inhibition as a measure of cell viability was determined in an ATPLite assay. The endpoint readout of the assay was based upon quantitation of ATP as an indicator of viable cells.

Cells were thawed from a liquid nitrogen preserved state. Once cells were expanded and divided at their expected doubling times, screening begins. Cells were seeded in growth media in black 384-well tissue culture treated plates at 500 cells per well (except where noted in Analyzer). Cells were equilibrated in assay plates via centrifugation and placed in incubators attached to the Dosing Modules at 37°C for 24 hours before treatment. At the time of treatment, a set of assay plates (which do not receive treatment) were collected and ATP levels were measured by adding ATPLite (Perkin Elmer). These Tzero ($T_0$) plates were read using ultra-sensitive luminescence on Envision Plate Readers. Treated assay plates were incubated with compound for 72 hours. All data points were collected via automated processes; quality controlled; and analyzed using Horizon Discovery proprietary software. Assay plates were accepted if they pass the following quality control standards: relative luciferase values were consistent throughout the entire experiment, Z-factor scores were greater than 0.6, untreated/vehicle controls behaved consistently on the plate.

Growth Inhibition (GI) was used as a measure of cell viability. The cell viability of vehicle was measured at the time of dosing ($T_0$) and after seventy two hours ($T_{72}$). A GI reading of 0% represented no growth inhibition - cells treated with compound and $T_{72}$ or $T_{120}$ vehicle signals were matched. A GI 100% represented complete growth inhibition - cells treated by compound and $T_o$ vehicle signals were matched. Cell numbers did not increase during the treatment period in wells with GI 100% and may suggest a cytostatic effect for compounds reaching a plateau at this effect level. A GI 200% represents complete death of all cells in the culture well. Compounds reaching an activity plateau of GI 200% were considered cytotoxic. GI was calculated by applying the following test and equation:

$$\text{If } T < V_0 : 100 \bullet (1 - \frac{T - V_0}{V_0})$$

$$\text{If } T \geq V_0 : 100 \bullet (1 - \frac{T-V_0}{V-V_0})$$

where T is the signal measure for a test article, V is the vehicle-treated control measure, and $V_0$ is the vehicle control measure at time zero. This formula is derived from the Growth Inhibition calculation used in the National Cancer Institute's[sic] NCI[sic]-60 high throughput screen.

FIGs. 1-8 provide signle[sic] agent dose response curves for growth inhibition by compound I in the cell lines indicated in Table 1.

As illustrated in FIG. 9, compound I had varying activity levels across cell line panel. All cell lines achieved growth inhibition levels of >50% with median $GI_{50}$ of 1.73μM.

As illustrated in FIG. 10, compound I induced cytotoxic effects in 3 cell lines (DOHH-2, OCI-Ly3 and OCI-Ly19) and sub-cytostatic effects in 3 cell lines (SU-DIAL-10, Pfeiffer and NU-DHL-1).

**Loewe Additivity model**

Loewe Additivity model was used for measuring the effects of drug combinations. The Loewe additivity model is dose-based and applies only to the activity levels achieved by the single agents. Loewe Volume was used to assess the overall magnitude of the combination interaction in excess of the Loewe additivity model. Loewe Volume is particularly useful when distinguishing synergistic increases in a phenotypic activity (positive Loewe Volume) versus synergistic antagonisms (negative Loewe Volume). When antagonisms are observed, the Loewe Volume should be assessed to examine if there is any correlation between antagonism and a particular drug target-activity or cellular genotype. This model defines additivity as a non-synergistic combination interaction where the combination dose matrix surface should be indistinguishable from either drug crossed with itself. The calculation for additivity is:

$$I_{\text{Loewe}} \text{ that satisfies } (X/X_I) + (Y/Y_I) = 1$$

where $X_I$ and $Y_I$ are the single agent effective concentrations for the observed combination effect *I*. For example, if 50% inhibition is achieved separately by 1mM of drug A or 1mM of drug B, a combination of 0.5mM of A and 0.5mM of B should also inhibit by 50%.

**Synergy Score**

To measure combination effects in excess of Loewe additivity, a scalar measure to characterize the strength of synergistic interaction was devised and termed the Synergy Score. The Synergy Score was calculated as:

$$\text{Synergy Score} = \log f_X \log f_Y \Sigma \max(0, I_{\text{data}})(I_{\text{data}} - I_{\text{Loewe}})$$

The fractional inhibition for each component agent and combination point in the matrix was calculated relative to the median of all vehicle-treated control wells. The Synergy Score equation integrates the experimentally-observed activity volume at each point in the matrix in excess of a model surface numerically derived from the activity of the component agents using the Loewe model for additivity. Additional terms in the Synergy Score equation (above) were used to normalize for various dilution factors used for individual agents and to allow for comparison of synergy scores across an entire experiment. The inclusion of positive inhibition gating or an $I_{data}$ multiplier removed noise near the zero effect level, and biases resulted for synergistic interactions at that occur at high activity levels.

Potency shifting was evaluated using an isobologram, which demonstrates how much less drug is required in combination to achieve a desired effect level, when compared to the single agent doses needed to reach that effect. The isobologram was drawn by identifying the locus of concentrations that correspond to crossing the indicated inhibition level. This was done by finding the crossing point for each single agent concentration in a dose matrix across the concentrations of the other single agent. Practically, each vertical concentration Cy was held fixed while a bisection algorithm was used to identify the horizontal concentration Cx in combination with that vertical dose that gave the chosen effect level in the response surface Z(CX,CY). These concentrations were then connected by linear interpolation to generate the isobologram display. For synergistic interactions, the isobologram contour fall below the additivity threshold and approaches the origin, and an antagonistic interaction would lie above the additivity threshold. The error bars represent the uncertainty arising from the individual data points used to generate the isobologram. The uncertainty for each crossing point was estimated from the response errors using bisection to find the concentrations where $Z-\sigma z(C_X,C_Y)$

and Z+$\sigma_Z$($C_X$,$C_Y$) cross $I_{cut}$, where $\sigma_Z$ is the standard deviation of the residual error on the effect scale.

Synergy scores for the tested combinations are provided below in Table 2.

In certain embodiments, synergy score of 0 is an additive result. In certain embodiments, synergy score from 0-2 can be considered Additive or just above Additive. In certain embodiments, the higher the synergy score value, the stronger is the synergistic result for the two agents.

Table 2:

| | DLBCL-GCB | | | | | | | DLBCL-ABC |
|---|---|---|---|---|---|---|---|---|
| | DB | DOHH-2 | HT | NU-DHL-1 | Pfeiffer | SU-DHL-10 | OCI-Ly19 | OCI-Ly3 |
| Compound I + Venetoclax | 12.50 | 32.10 | 3.09 | 20.10 | 1.11 | 0.18 | 5.58 | 4.79 |
| Venetoclax +PCI-32765 | 3.35 | 36.40 | 0.56 | 8.88 | 0.39 | 0.81 | 2.44 | 0.33 |
| Tazemetostat +compound I | 1.1.5 | 8.49 | 4.66 | 10.10 | 19.30 | 5.40 | 1.77 | 0.09 |
| Compound I+Ibrutinib | 0.39 | 34.70 | 9.94 | 10.40 | 30.10 | 20.70 | 1.32 | 0.25 |

As seen from the data, strong breadth of activity for combinations of compound I with ibrutinib in GCB-DLBCL cell lines were observed. Strong synergy for ibrutinib with venetoclax in DOHE-2 cell line and for compound I with venetoclax in DOM-I-2 cell line was observed.

The examples set forth above are provided to give those of ordinary skill in the art with a complete disclosure and description of how to make and use the claimed embodiments, and are not intended to limit the scope of what is disclosed herein.

**Example 3:** Study of a Combination of a PI3K Inhibitor and Ibrutinib in Patients with Chronic Lymphocyctic Leukemia (CLL)

The purpose of this study is to evaluate the safety and effectiveness of Compound I, II, III, or IV (three does: 60 mg, 120 mg, and 150 mg/day) and ibrutinib, in patients with CLL.

Primary Outcome Measures: Determine Acceptable Adverse Events That Are Related to Treatment [Time Frame: 6 months of therapy]. To determine the incidence of adverse events, any potential abnormal laboratory results and any dose-limiting toxicities.

Secondary Outcome Measures: Overall Response Rate [Time Frame: Up to 1 year]. The overall response rate (ORR) in patients with CLL treated with a combination of Compound I, II, III, or IV and ibrutinib.

| Arms | Assigned Interventions |
|---|---|
| Experimental: ibrutinib + Compound I, II, III, or IV<br>Ibrutinib oral daily dose - 420 mg, 280 mg, or 140 mg<br>Compound I, II, III, or IV oral daily dose - 60 mg | PI3K Inhibitor: Compound I, II, III, or IV<br>A once daily oral agent<br>Ibrutinib<br>A once daily oral agent |
| Experimental: ibrutinib + Compound I, II, III, or IV<br>Ibrutinib oral daily dose - 420 mg, 280 mg, or 140 mg<br>Compound I, II, III, or IV oral daily dose - 120 mg | PI3K Inhibitor: Compound I, II, III, or IV<br>A once daily oral agent<br>Ibrutinib<br>A once daily oral agent |
| Experimental: ibrutinib + Compound I, II, III, or IV<br>Ibrutinib oral daily dose - 420 mg, 280 mg, or 140 mg<br>Compound I, II, III, or IV oral daily dose - 150 mg | PI3K Inhibitor: Compound I, II, III, or IV<br>A once daily oral agent<br>Ibrutinib<br>A once daily oral agent |

Patients should not have had exposure to the compounds prior to the study entry. Patients must not have

received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Doses of the compounds may be held or modified for toxicity based on assessments as outlined below. Treatment repeats every 28 days in the absence of unacceptable toxicity. Dose limiting toxicities are determined according to the definitions and standards set by the National Cancer Institute (NCI) Common Terminology for Adverse Events (CTCAE) Version 3.0 (August 9, 2006).

Blood Sampling Serial blood is drawn by direct vein puncture before and after administration of the compound. Venous blood samples (5 mL) for determination of serum concentrations are obtained at about 10 minutes prior to dosing and at approximately the following times after dosing: days 1, 8, and 15. Each serum sample is divided into two aliquots. All serum samples are stored at - 20°C. Serum samples are shipped on dry ice.

Pharmacokinetics: Patients undergo plasma/serum sample collection for pharmacokinetic evaluation before beginning treatment and at days 1, 8, and 15. Pharmacokinetic parameters are calculated by model independent methods on a Digital Equipment Corporation VAX 8600 computer system using the latest version of the BIOAVL software. The following pharmacokinetics parameters are determined: peak serum concentration ($C_{max}$); time to peak serum concentration (tmax); area under the concentration-time curve (AUC) from time zero to the last blood sampling time ($AUC_{0-72}$) calculated with the use of the linear trapezoidal rule; and terminal elimination half-life ($t_{1/2}$), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/nonpreserved formulation) is calculated.

Patient Response to combination therapy: Patient response is assessed via imaging with X-ray, CT scans, and MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Patient response is also assessed via complete blood cell count and/or marrow biopsy. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). After completion of study treatment, patients are followed periodically for 4 weeks.

**Example 4:** Study of a Combination of a PI3K Inhibitor and Venetoclax in Patients with Chronic Lymphocytic Leukemia (CLL)

The purpose of this study is to evaluate the safety and effectiveness of Compound I, II, III, or IV (three does: 60 mg, 120 mg, and 150 mg/day) and Venetoclax, in patients with CLL.

Primary Outcome Measures: Determine Acceptable Adverse Events That Are Related to Treatment [Time Frame: 6 months of therapy]. To determine the incidence of adverse events, any potential abnormal laboratory results and any dose-limiting toxicities.

Secondary Outcome Measures: Overall Response Rate [Time Frame: Up to 1 year]. The overall response rate (ORR) in patients with CLL treated with a combination of Compound I, II, III, or IV and Venetoclax.

| Arms | Assigned Interventions |
|---|---|
| Experimental: Venetoclax + Compound I, II, III, or IV<br>Venetoclax oral daily dose -<br>Week 1: 20 mg/day<br>Week 2: 50 mg/day<br>Week 3: 100 mg/day<br>Week 4: 200 mg/day<br>Week 5: 400 mg/day; continue with daily dose of 400 mg until disease progression or unacceptable toxicity.<br>Compound I, II, III, or IV oral daily dose - 60 mg | PI3K Inhibitor: Compound I, II, III, or IV<br><br>A once daily oral agent<br>Venetoclax<br>A once daily oral agent |
| Experimental: Venetoclax + Compound I, II, III, or IV<br>Venetoclax oral daily dose - | PI3K Inhibitor: Compound I, II, III, or IV |

(continued)

| Arms | Assigned Interventions |
|---|---|
| Week 1: 20 mg/day<br>Week 2: 50 mg/day<br>Week 3: 100 mg/day<br>Week 4: 200 mg/day<br>Week 5: 400 mg/day; continue with daily dose of 400 mg until disease progression or unacceptable toxicity.<br>Compound I, II, III, or IV oral daily dose - 120 mg | A once daily oral agent<br>Venetoclax<br>A once daily oral agent |
| Experimental: Venetoclax + Compound I, II, III, or IV<br>Venetoclax oral daily dose -<br>Week 1: 20 mg/day<br>Week 2: 50 mg/day<br>Week 3: 100 mg/day<br>Week 4: 200 mg/day<br>Week 5: 400 mg/day; continue with daily dose of 400 mg until disease progression or unacceptable toxicity.<br>Compound I, II, III, or IV oral daily dose - 150 mg | PI3K Inhibitor: Compound I, II, III, or IV<br><br>A once daily oral agent<br>Venetoclax<br>A once daily oral agent |

Patients should not have had exposure to the compounds prior to the study entry. Patients must not have received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Doses of the compounds may be held or modified for toxicity based on assessments as outlined below. Treatment repeats every 28 days in the absence of unacceptable toxicity. Dose limiting toxicities are determined according to the definitions and standards set by the National Cancer Institute (NCI) Common Terminology for Adverse Events (CTCAE) Version 3.0 (August 9, 2006).

Blood Sampling Serial blood is drawn by direct vein puncture before and after administration of the compound. Venous blood samples (5 mL) for determination of serum concentrations are obtained at about 10 minutes prior to dosing and at approximately the following times after dosing: days 1, 8, and 15. Each serum sample is divided into two aliquots. All serum samples are stored at - 20°C. Serum samples are shipped on dry ice.

Pharmacokinetics: Patients undergo plasma/serum sample collection for pharmacokinetic evaluation before beginning treatment and at days 1, 8, and 15. Pharmacokinetic parameters are calculated by model independent methods on a Digital Equipment Corporation VAX 8600 computer system using the latest version of the BIOAVL software. The following pharmacokinetics parameters are determined: peak serum concentration ($C_{max}$); time to peak serum concentration (tmax); area under the concentration-time curve (AUC) from time zero to the last blood sampling time ($AUC_{0-72}$) calculated with the use of the linear trapezoidal rule; and terminal elimination half-life ($t_{1/2}$), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/nonpreserved formulation) is calculated.

Patient Response to combination therapy: Patient response is assessed via imaging with X-ray, CT scans, and MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Patient response is also assessed via complete blood cell count and/or marrow biopsy. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). After completion of study treatment, patients are followed periodically for 4 weeks.

**Example 5:** Study of a Combination of a PI3K Inhibitor and Tazemetostat in Patients with Non-Hodgkin's Lymphoma

The purpose of this study is to evaluate the safety and effectiveness of Compound I, II, III, or IV (three does: 60 mg, 120 mg, and 150 mg/day) and Tazemetostat, in patients with Non-Hodgkin's Lymphoma.

Primary Outcome Measures: Determine Acceptable Adverse Events That Are Related to Treatment [Time Frame: 6 months of therapy]. To determine the incidence of adverse events, any potential abnormal laboratory results and any dose-limiting toxicities.

Secondary Outcome Measures: Overall Response Rate [Time Frame: Up to 1 year]. The overall response rate (ORR) in patients with Non-Hodgkin's Lymphoma treated with a combination of Compound I, II, III, or IV and Tazemetostat.

| Arms | Assigned Interventions |
|---|---|
| Experimental: Tazemetostat + Compound I, II, III, or IV<br>Tazemetostat oral BID dose - 800 mg<br>Compound I, II, III, or IV oral daily dose - 60 mg | PI3K Inhibitor: Compound I, II, III, or IV<br>A once daily oral agent<br>Tazemetostat<br>A twice daily oral agent |
| Experimental: Tazemetostat + Compound I, II, III, or IV<br>Tazemetostat oral BID dose - 800 mg<br>Compound I, II, III, or IV oral daily dose - 120 mg | PI3K Inhibitor: Compound I, II, III, or IV<br>A once daily oral agent<br>Tazemetostat<br>A twice daily oral agent |
| Experimental: Tazemetostat + Compound I, II, III, or IV<br>Tazemetostat oral BID dose - 800 mg<br>Compound I, II, III, or IV oral daily dose - 150 mg | PI3K Inhibitor: Compound I, II, III, or IV<br>A once daily oral agent<br>Tazemetostat<br>A twice daily oral agent |

Patients should not have had exposure to the compounds prior to the study entry. Patients must not have received treatment for their cancer within 2 weeks of beginning the trial. Treatments include the use of chemotherapy, hematopoietic growth factors, and biologic therapy such as monoclonal antibodies. Patients must have recovered from all toxicities (to grade 0 or 1) associated with previous treatment. All subjects are evaluated for safety and all blood collections for pharmacokinetic analysis are collected as scheduled. All studies are performed with institutional ethics committee approval and patient consent.

Doses of the compounds may be held or modified for toxicity based on assessments as outlined below. Treatment repeats every 28 days in the absence of unacceptable toxicity. Dose limiting toxicities are determined according to the definitions and standards set by the National Cancer Institute (NCI) Common Terminology for Adverse Events (CTCAE) Version 3.0 (August 9, 2006).

Blood Sampling Serial blood is drawn by direct vein puncture before and after administration of the compound. Venous blood samples (5 mL) for determination of serum concentrations are obtained at about 10 minutes prior to dosing and at approximately the following times after dosing: days 1, 8, and 15. Each serum sample is divided into two aliquots. All serum samples are stored at - 20°C. Serum samples are shipped on dry ice.

Pharmacokinetics: Patients undergo plasma/serum sample collection for pharmacokinetic evaluation before beginning treatment and at days 1, 8, and 15. Pharmacokinetic parameters are calculated by model independent methods on a Digital Equipment Corporation VAX 8600 computer system using the latest version of the BIOAVL software. The following pharmacokinetics parameters are determined: peak serum concentration ($C_{max}$); time to peak serum concentration (tmax); area under the concentration-time curve (AUC) from time zero to the last blood sampling time ($AUC_{0-72}$) calculated with the use of the linear trapezoidal rule; and terminal elimination half-life ($t_{1/2}$), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/nonpreserved formulation) is calculated.

Patient Response to combination therapy: Patient response is assessed via imaging with X-ray, CT scans, and MRI, and imaging is performed prior to beginning the study and at the end of the first cycle, with additional imaging performed every four weeks or at the end of subsequent cycles. Imaging modalities are chosen based upon the cancer type and feasibility/availability, and the same imaging modality is utilized for similar cancer types as well as throughout each patient's study course. Patient response is also assessed via complete blood cell count and/or marrow biopsy. Response rates are determined using the RECIST criteria. (Therasse et al, J. Natl. Cancer Inst. 2000 Feb 2; 92(3):205-16; http://ctep.cancer.gov/forms/TherasseRECISTJNCI.pdf). After completion of study treatment, patients are followed periodically for 4 weeks.

## Claims

**1.** A compound of Formula (I):

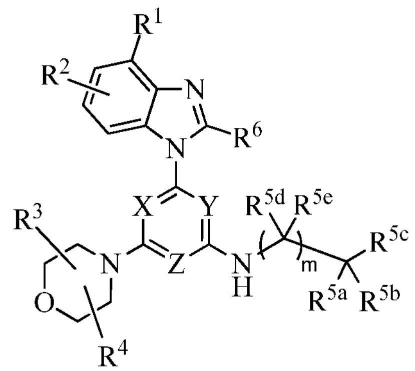

Formula (I),

or an enantiomer, a mixture of enantiomers, a mixture of two or more diastereomers;
or an isotopic variant thereof;
or a pharmaceutically acceptable salt, solvate, or hydrate thereof;
wherein:

X, Y, and Z are each independently N or $CR^X$, with the proviso that at least two of X, Y, and Z are nitrogen atoms; where $R^X$ is hydrogen or $C_{1-6}$ alkyl;
$R^1$ and $R^2$ are each independently

(a) hydrogen, cyano, halo, or nitro;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)$-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

wherein each $R^{1a}$, $R^{1b}$, $R^{1c}$, and $R^{1d}$ is independently

(i) hydrogen;
(ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(iii) $R^{1b}$ and $R^{1c}$ together with the N atom to which they are attached form heterocyclyl;

$R^3$ and $R^4$ are each independently hydrogen or $C_{1-6}$ alkyl;
or $R^3$ and $R^4$ are linked together to form a bond, $C_{1-6}$ alkylene, $C_{1-6}$ heteroalkylene, $C_{2-6}$ alkenylene, or $C_{2-6}$ heteroalkenylene;
$R^{5a}$ is

(a) hydrogen or halo;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)$-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$,

$-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5b}$ is

(a) halo;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)$-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5c}$ is $-(CR^{5f}R^{5g})_n-(C_{6-14}$ aryl) or $-(CR^{5f}R^{5g})_n$-heteroaryl;
$R^{5d}$ and $R^{5e}$ are each independently

(a) hydrogen or halo;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)$-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$;

$R^{5f}$ and $R^{5g}$ are each independently

(a) hydrogen or halo;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)$-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$; or $-S(O)_2NR^{1b}R^{1c}$; or
(d) when one occurrence of $R^{5f}$ and one occurrence of $R^{5g}$ are attached to the same carbon atom, the $R^{5f}$ and $R^{5g}$ together with the carbon atom to which they are attached form a $C_{3-10}$ cycloalkyl or heterocyclyl;

$R^6$ is hydrogen, $C_{1-6}$ alkyl, $-S-C_{1-6}$ alkyl, $-S(O)-C_{1-6}$ alkyl, or $-SO_2-C_{1-6}$ alkyl;
m is 0 or 1; and
n is 0, 1, 2, 3, or 4;
wherein each alkyl, alkylene, heteroalkylene, alkenyl, alkenylene, heteroalkenylene, alkynyl, cycloalkyl, aryl, aralkyl, heteroaryl, and heterocyclyl in $R^1$, $R^2$, $R^3$, R4, R6, $R^X$, $R^{1a}$, $R^{1b}$, $R^{1c}$, $R^{1d}$, $R^{5a}$, $R^{5b}$, $R^{5c}$, $R^{5d}$, $R^{5e}$, $R^{5f}$, and $R^{5g}$ is optionally substituted with one, two, three, four, or five substituents Q,
wherein each substituent Q is independently selected from

(a) oxo, cyano, halo, and nitro;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl, each of which is further optionally substituted with one, two, three, or four, substituents $Q^a$; and
(c)$-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, -S(O)2$R^a$, $-S(O)NR^bR^c$, and $-S(O)_2NR^bR^c$,

wherein each $R^a$, $R^b$, $R^c$, and $R^d$ is independently

(i) hydrogen;
(ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is further optionally substituted with one, two, three, or four substituents $Q^a$; or
(iii) $R^b$ and $R^c$ together with the N atom to which they are attached form heterocyclyl, which is further optionally substituted with one, two, three, or four substituents $Q^a$;

wherein each $Q^a$ is independently selected from the group consisting of

(a) oxo, cyano, halo, and nitro;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, and heterocyclyl; and
(c)-$C(O)R^e$, $-C(O)OR^e$, $-C(O)NR^fR^g$, $-C(NR^e)NR^fR^g$, $-OR^e$, $-OC(O)R^e$, $-OC(O)OR^e$, $-OC(O)NR^fR^g$, $-OC(=NR^e)NR^fR^g$, $-OS(O)R^e$, $-OS(O)_2R^e$, $-OS(O)NR^fR^g$, $-OS(O)_2NR^fR^g$, $-NR^fR^g$, $-NR^eC(O)R^h$, $-NR^eC(O)OR^h$, $-NR^eC(O)NR^fR^g$, $-NR^eC(=NR^h)NR^fR^g$, $-NR^eS(O)R^h$, $-NR^eS(O)_2R^h$, $-NR^eS(O)NR^fR^g$, $-NR^eS(O)_2NR^fR^g$, $-SR^e$, $-S(O)R^e$, $-S(O)_2R^e$, $-S(O)NR^fR^g$, and $-S(O)_2NR^fR^g$;

wherein each $R^e$, $R^f$, $R^g$, and $R^h$ is independently

(i) hydrogen;
(ii) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(iii) $R^f$ and $R^g$ together with the N atom to which they are attached form heterocyclyl;

wherein two substituents Q that are adjacent to each other optionally form a $C_{3-10}$ cycloalkenyl, $C_{6-14}$ aryl, heteroaryl, or heterocyclyl, each optionally substituted with one, two, three, or four substituents $Q^a$;
for use in a method of treating a hematological cancer in combination with a second agent selected from a BTK inhibitor, a Bcl-2 inhibitor, an EZH2 inhibitor and any combination thereof.

**2.** The compound for use according to claim 1, wherein:
$R^{5a}$ and $R^{5b}$ are each independently:

(a) halo;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl; or
(c)-$C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$, or $-S(O)_2NR^{1b}R^{1c}$.

**3.** The compound for use according to claim 1 or 2, wherein:
$R^{5a}$ and $R^{5b}$ are each methyl, optionally substituted with one, two, or three halo(s).

**4.** The compound for use according to any one of claims 1-3, wherein:

n is 1; and
$R^{5f}$ and $R^{5g}$ are each hydrogen.

**5.** The compound for use according to any one of claims 1-4, wherein the compound of Formula (I) is a compound of Formula (XI):

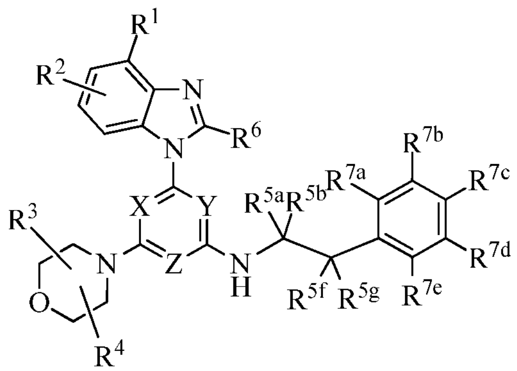

Formula (XI),

or an enantiomer, a mixture of enantiomers, a mixture of two or more diastereomers;
or an isotopic variant thereof;
or a pharmaceutically acceptable salt, solvate, or hydrate thereof;
wherein:

$R^{7a}$, $R^{7b}$, $R^{7c}$, $R^{7d}$, and $R^{7e}$ are each independently

(a) hydrogen, cyano, halo, or nitro;
(b) $C_{1-6}$ alkyl, $C_{2-6}$ alkenyl, $C_{2-6}$ alkynyl, $C_{3-10}$ cycloalkyl, $C_{6-14}$ aryl, $C_{7-15}$ aralkyl, heteroaryl, or heterocyclyl, each of which is optionally substituted with one, two, three, or four substituents $Q^a$; or
(c)-C(O)$R^a$, -C(O)O$R^a$, -C(O)N$R^bR^c$, -C(N$R^a$)N$R^bR^c$, -O$R^a$, -OC(O)$R^a$, -OC(O)O$R^a$, -OC(O)N$R^bR^c$, -OC(=N$R^a$)N$R^bR^c$, -OS(O)$R^a$, -OS$(O)_2R^a$, -OS(O)N$R^bR^c$, -OS$(O)_2$N$R^bR^c$, -N$R^bR^c$, -N$R^a$C(O)$R^d$, -N$R^a$C(O)O$R^d$, -N$R^a$C(O)N$R^bR^c$, -N$R^a$C(=N$R^d$)N$R^bR^c$, -N$R^a$S(O)$R^d$, -N$R^a$S$(O)_2R^d$, -N$R^a$S(O)N$R^bR^c$, -N$R^a$S$(O)_2$N$R^bR^c$, -S$R^a$, -S(O)$R^a$, -S$(O)_2R^a$, -S(O)N$R^bR^c$, or -S$(O)_2$N$R^bR^c$; or

two of $R^{7a}$, $R^{7b}$, $R^{7c}$, $R^{7d}$, and $R^{7e}$ that are adjacent to each other form $C_{3-10}$ cycloalkenyl, $C_{6-14}$ aryl, heteroaryl, or heterocyclyl, each optionally substituted with one, two, three, or four substituents $Q^a$.

**6.** The compound for use according to claim 1, wherein the compound of Formula (I) is:

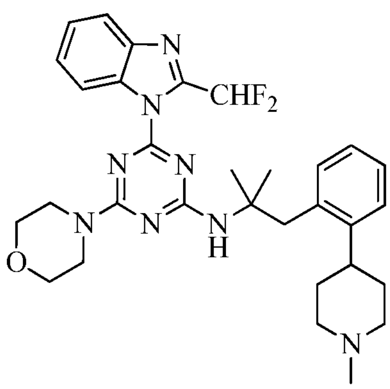

,

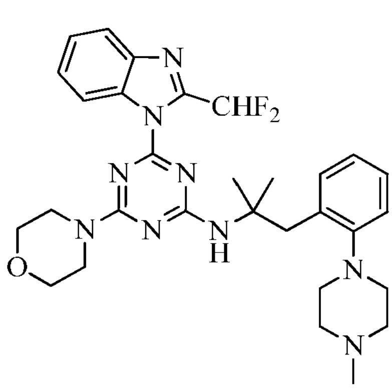

,

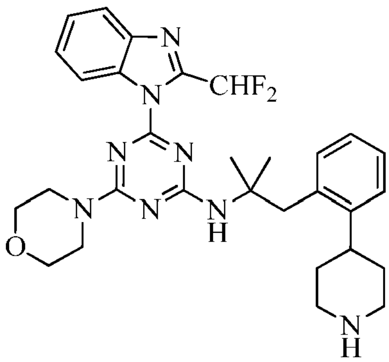 , 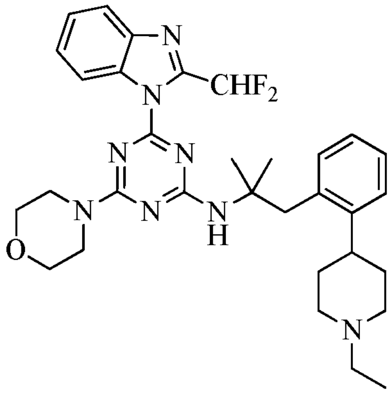 ,

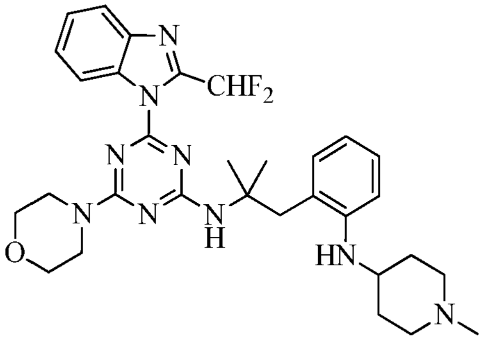 , 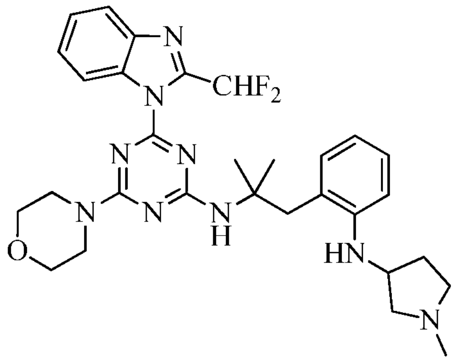 ,

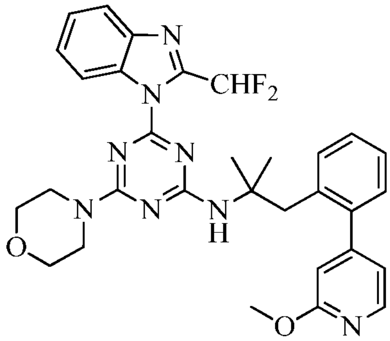 , 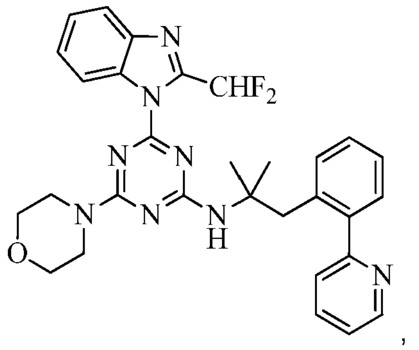 ,

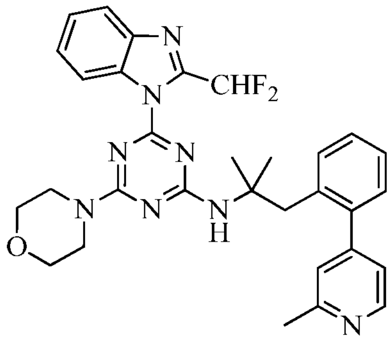 , or 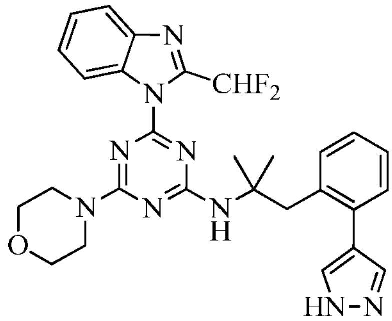 ,

or an isotopic variant thereof;
or a pharmaceutically acceptable salt, solvate, or hydrate thereof.

**7.** The compound for use according to claim 1, wherein the compound of Formula (I) is

a compound of the following formula:

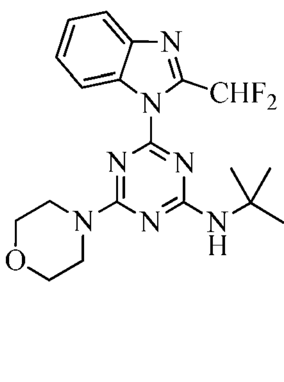

,

or an isotopic variant thereof;
or a pharmaceutically acceptable salt, solvate, or hydrate thereof.

**8.** The compound for use according to any one of claims 1-7, wherein:
the second agent is a BTK inhibitor.

**9.** The compound for use according to claim 8, wherein:
the BTK inhibitor is selected from ibrutinib, BGB3111, CC-292, ACP 196, CNX-774, CG11746, LFM-A13, CNX-774, ONO-4059, RN486 CPI-0610, DUAL946, GSK525762, I-BET151, JQ1, OTX015, PFI-1, RVX-208, RVX2135, TEN-010, and a combination thereof.

**10.** The compound for use according to claim 9, wherein:
the BTK inhibitor is ibrutinib or BGB3111.

**11.** The compound for use according to any one of claims 1-7, wherein:
the second agent is a Bcl-2 inhibitor.

**12.** The compound for use according to claim 11, wherein:
the Bcl-2 inhibitor is venetoclax or PNT2258.

**13.** The compound for use according to any one of claims 1-12, wherein:
the hematological cancer is relapsed or refractory.

**14.** The compound for use according to any one of claims 1-13, wherein:
the hematological cancer is chronic lymphocytic leukemia, follicular lymphoma, diffuse large β-cell lymphoma, or non-Hodgkin's lymphoma.

**15.** The compound for use according to any one of claims 1-14, wherein:
the compound is used in combination with a third agent or therapy useful in the treatment of the hematological cancer.

## Patentansprüche

**1.** Verbindung der Formel (I):

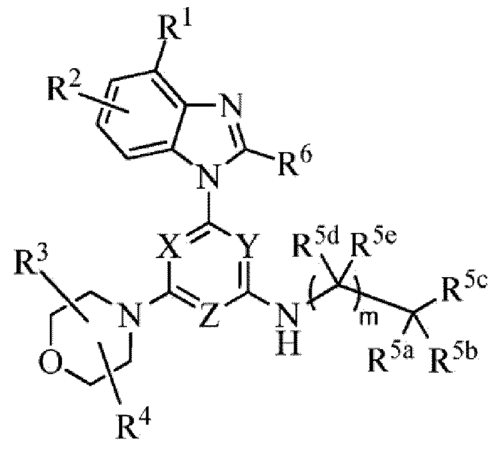

Formel (I)

oder ein Enantiomer, ein Enantiomerengemisch, ein Gemisch aus zwei oder mehr Diastereomeren davon; oder eine Isotopenvariante davon;
oder ein pharmazeutisch annehmbares Salz, Solvat oder Hydrat davon;
worin:

X, Y und Z jeweils unabhängig N oder $CR^x$ sind, mit der Maßgabe, dass zumindest zwei von X, Y und Z Stickstoffatome sind; worin $R^x$ Wasserstoff oder $C_{1-6}$-Alkyl ist;
$R^1$ und $R^2$ jeweils unabhängig Folgendes sind:

(a) Wasserstoff, Cyano, Halogen oder Nitro;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(c)-$C(O)R^{1a}$, -$C(O)OR^{1a}$, -$C(O)NR^{1b}R^{1c}$, -$C(NR^{1a})NR^{1b}R^{1c}$, -$OR^{1a}$, -$OC(O)R^{1a}$, -$OC(O)$-$OR^{1a}$, -$OC(O)NR^{1b}R^{1c}$, -$OC(=NR^{1a})NR^{1b}R^{1c}$, -$OS(O)R^{1a}$, -$OS(O)_2R^{1a}$, -$OS(O)NR^{1b}R^{1c}$, -$OS(O)_2NR^{1b}R^{1c}$, -$NR^{1b}R^{1c}$, -$NR^{1a}C(O)R^{1d}$, -$NR^{1a}C(O)OR^{1d}$, -$NR^{1a}C(O)NR^{1b}R^{1c}$, -$NR^{1a}$-$C(=NR^{1d})NR^{1b}R^{1c}$, -$NR^{1a}S(O)R^{1d}$, -$NR^{1a}S(O)_2R^{1d}$, -$NR^{1a}S(O)NR^{1b}R^{1c}$, -$NR^{1a}S(O)_2NR^{1b}R^{1c}$, -$SR^{1a}$, -$S(O)R^{1a}$, -$S(O)_2R^{1a}$, -$S(O)NR^{1b}R^{1c}$ oder -$S(O)_2NR^{1b}R^{1c}$; worin

$R^{1a}$, $R^{1b}$, $R^{1c}$ und $R^{1d}$ jeweils unabhängig Folgendes sind:

(i) Wasserstoff;
(ii) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(iii) $R^{1b}$ und $R^{1c}$ zusammen mit dem N-Atom, an das sie gebunden sind, ein Heterocyclyl bilden;

$R^3$ und $R^4$ jeweils unabhängig Wasserstoff oder $C_{1-6}$-Alkyl sind;
oder $R^3$ und $R^4$ verbunden sind und eine Bindung, $C_{1-6}$-Alkylen, $C_{1-6}$-Heteroalkylen, $C_{2-6}$-Alkenylen oder $C_{2-6}$-Heteroalkenylen bilden;
$R^{5a}$ Folgendes ist:

(a) Wasserstoff oder Halogen;
(b) $C_{1-6}$-Alkyl, $C_{2-6}$-Alkenyl, $C_{2-6}$-Alkinyl, $C_{3-10}$-Cycloalkyl, $C_{6-14}$-Ary), $C_{7-15}$-Aralkyl, Heteroaryl oder Heterocyclyl; oder
(c)-$C(O)R^{1a}$, -$C(O)OR^{1a}$, -$C(O)NR^{1b}R^{1c}$, -$C(NR^{1a})NR^{1b}R^{1c}$, -$OR^{1a}$, -$OC(O)R^{1a}$, -$OC(O)OR^{1a}$, -$OC(O)NR^{1b}R^{1c}$, -$OC(=NR^{1a})NR^{1b}R^{1c}$, -$OS(O)R^{1a}$, -$OS(O)_2R^{1a}$, -$OS(O)NR^{1b}R^{1c}$, -$OS(O)_2NR^{1b}R^{1c}$, -$NR^{1b}R^{1c}$, -$NR^{1a}C(O)R^{1d}$, -$NR^{1a}C(O)OR^{1d}$, - $NR^{1a}C(O)NR^{1b}R^{1c}$, -$NR^{1a}C(=NR^{1d})NR^{1b}R^{1c}$, -$NR^{1b}S(O)R^{1d}$, -$NR^{1a}S(O)_2R^{1d}$, - $NR^{1a}S(O)NR^{1b}R^{1c}$, -$NR^{1a}S(O)_2NR^{1b}R^{1c}$, -$SR^{1a}$, -$S(O)R^{1a}$, -$S(O)_2R^{1a}$, -$S(O)NR^{1b}R^{1c}$ oder -$S(O)_2NR^{1b}R^{1c}$;

$R^{5b}$ Folgendes ist:

(a) Halogen;
(b) $C_{1-6}$-A)ky), $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ oder $-S(O)_2NR^{1b}R^{1c}$;

$R^{5c}$ Folgendes ist: $-(CR^{5f}R^{5g})_n-(C_{6-14}$-Aryl) oder $-(CR^{5f}R^{5g})_n$-Heteroaryl;
$R^{5d}$ und $R^{5e}$ jeweils unabhängig Folgendes sind:

(a) Wasserstoff oder Halogen;
(b) $C_{1-6}$-Alky), $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ oder $-S(O)_2NR^{1b}R^{1c}$;

worin $R^{5f}$ und $R^{5g}$ jeweils unabhängig Folgendes sind:

(a) Wasserstoff oder Halogen;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ oder $-S(O)_2NR^{1b}R^{1c}$; oder
(d) wenn ein $R^{5f}$ und ein $R^{5g}$ an das gleiche Kohlenstoffatom gebunden sind, $R^{5f}$ und $R^{5g}$ zusammen mit dem Kohlenstoffatom, an das sie gebunden sind, ein $C_{3-10}$-Cycloalkyl oder Heterocyclyl bilden;

$R^6$ Wasserstoff, $C_{1-6}$-Alkyl, $-S-C_{1-6}$-Alkyl, $-S(O)-C_{1-6}$-Alkyl oder $-SO_2-C_{1-6}$-Alkyl ist;
m = 0 oder 1 ist; und
worin n = 0, 1, 2, 3 oder 4 ist;
worin jedes Alkyl, Alkylen, Heteroalkylen, Alkenyl, Alkenylen, Heteroalkenylen, Alkinyl, Cycloalkyl, Aryl, Aralkyl, Heteroaryl und Heterocyclyl in $R^1$, $R^2$, $R^3$, $R^4$, $R^6$, $R^x$, $R^{1a}$, $R^{1b}$, $R^{1c}$, $R^{1d}$, $R^{5a}$, $R^{5b}$, $R^{5c}$, $R^{5d}$, $R^{5e}$, $R^{5f}$ und $R^{5g}$ gegebenenfalls mit einem, zwei, drei, vier oder fünf Substituenten Q substituiert ist,

worin jeder Substituent Q unabhängig aus Folgendem ausgewählt ist:

(a) Oxo, Cyano, Halogen und Nitro;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl, die jeweils gegebenenfalls mit einem, zwei, drei oder vier Substituenten $Q^a$ weiter substituiert sind; und
(c) $-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$ oder $-S(O)_2NR^bR^c$,

worin $R^a$, $R^b$, $R^c$ und $R^d$ jeweils unabhängig Folgendes sind:

(i) Wasserstoff;
(ii) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl, die jeweils gegebenenfalls mit einem, zwei, drei oder vier Substituenten $Q^a$ weiter substituiert sind; oder

(iii) $R^b$ und $R^c$ zusammen mit dem N-Atom, an das sie gebunden sind, ein Heterocyclyl bilden, das gegebenenfalls mit einem, zwei, drei oder vier Substituenten $Q^a$ weiter substituiert ist;

worin die $Q^a$ jeweils unabhängig aus der aus Folgendem bestehenden Gruppe ausgewählt sind:

(a) Oxo, Cyano, Halogen und Nitro;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; und
(c) $-C(O)R^e$, $-C(O)OR^e$, $-C(O)NR^fR^g$, $-C(NR^e)NR^fR^g$, $-OR^e$, $-OC(O)R^e$, $-OC(O)OR^a$, $-OC(O)NR^fR^g$, $-OC(=NR^e)NR^fR^g$, $-OS(O)R^e$, $-OS(O)_2R^e$, $-OS(O)NR^fR^g$, $-OS(O)_2NR^fR^g$, $-NR^fR^g$, $-NR^eC(O)R^h$, $-NR^eC(O)OR^h$, $-NR^eC(O)NR^fR^g$, $-NR^eC(=NR^h)NR^fR^g$, $-NR^eS(O)R^h$, $-NR^eS(O)_2R^h$, $-NR^eS(O)NR^fR^g$, $-NR^eS(O)_2NR^fR^g$, $-SR^e$, $-S(O)R^e$, $-S(O)_2R^a$, $-S(O)NR^fR^g$ oder $-S(O)_2NR^fR^g$;

worin $R^e$, $R^f$, $R^g$ und $R^h$ jeweils unabhängig Folgendes sind:

(i) Wasserstoff;
(ii) $C_{1-6}$-Alkyl, $C_{2-6}$-Alkenyl, $C_{2-6}$-Alkinyl, $C_{3-10}$-Cycloalkyl, $C_{6-14}$-Ary), $C_{7-15}$-Aralkyl, Heteroaryl oder Heterocyclyl; oder
(iii) $R^f$ und $R^g$ zusammen mit dem N-Atom, an das sie gebunden sind, ein Heterocyclyl bilden;

wobei zwei benachbarte Substituenten Q gegebenenfalls ein $C_{3-10}$-Cycloalkenyl, $C_{6-14}$-Aryl, Heteroaryl oder Heterocyclyl bilden, die jeweils gegebenenfalls mit einem, zwei, drei oder vier Substituenten $Q^a$ substituiert sind;

zur Verwendung in einem Verfahren zur Behandlung einer hämatologischen Krebserkrankung in Kombination mit einem zweiten Mittel, das aus einem BTK-Inhibitor, einem Bcl-2-Inhibitor, einem EZH2-Inhibitor und einer beliebigen Kombination davon ausgewählt ist.

**2.** Verbindung zur Verwendung nach Anspruch 1, wobei:
$R^{5a}$ und $R^{5b}$ jeweils unabhängig Folgendes sind:

(a) Halogen;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl; oder
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ oder $-S(O)_2NR^{1b}R^{1c}$.

**3.** Verbindung zur Verwendung nach Anspruch 1 oder 2, worin $R^{5a}$ und $R^{5b}$ jeweils Methyl sind, das gegebenenfalls mit einem, zwei oder drei Halogen(en) substituiert ist.

**4.** Verbindung zur Verwendung nach einem der Ansprüche 1 bis 3, worin:

n =1 ist; und
$R^{5f}$ und $R^{5g}$ jeweils Wasserstoff sind.

**5.** Verbindung zur Verwendung nach einem der Ansprüche 1 bis 4, worin die Verbindung der Formel (I) eine Verbindung der Formel (XI) ist:

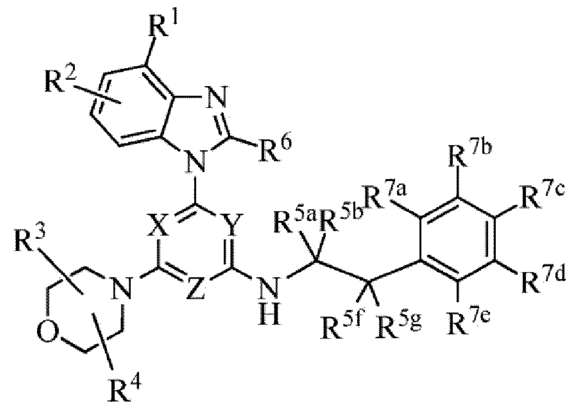

Formel (XI)

oder ein Enantiomer, ein Enantiomerengemisch, ein Gemisch aus zwei oder mehr Diastereomeren; oder eine Isotopenvariante davon; oder ein pharmazeutisch annehmbares Salz, Solvat oder Hydrat davon; worin:

$R^{7a}$, $R^{7b}$, $R^{7c}$, $R^{7d}$ und $R^{7e}$ jeweils unabhängig Folgendes sind:

(a) Wasserstoff, Cyano, Halogen oder Nitro;
(b) $C_{1-6}$-Alkyl, $C_{2-6}$-Alkenyl, $C_{2-6}$-Alkinyl, $C_{3-10}$-Cycloalkyl, $C_{6-14}$-Aryl, $C_{7-15}$-Aralkyl, Heteroaryl oder Heterocyclyl, die jeweils gegebenenfalls weiter mit einem, zwei, drei oder vier Substituenten $Q^a$ substituiert sind; oder
(c) $-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$ oder $-S(O)_2NR^bR^c$; oder

zwei benachbarte von $R^{7a}$, $R^{7b}$, $R^{7c}$, $R^{7d}$ und $R^{7e}$ ein $C_{3-10}$-Cycloalkenyl, $C_{6-14}$-Aryl, Heteroaryl oder Heterocyclyl bilden, die jeweils gegebenenfalls mit einem, zwei, drei oder vier Substituenten $Q^a$ substituiert sind.

**6.** Verbindung zur Verwendung nach Anspruch 1, wobei die Verbindung der Formel (I) die folgende ist:

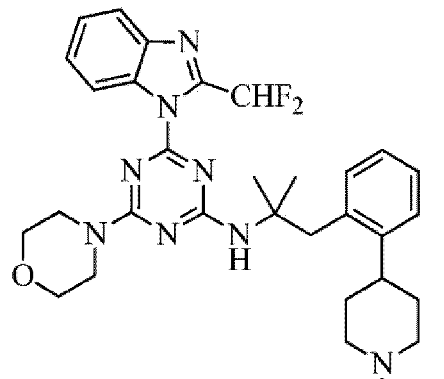
,
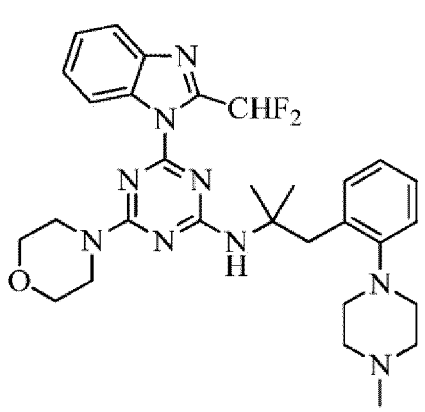
,

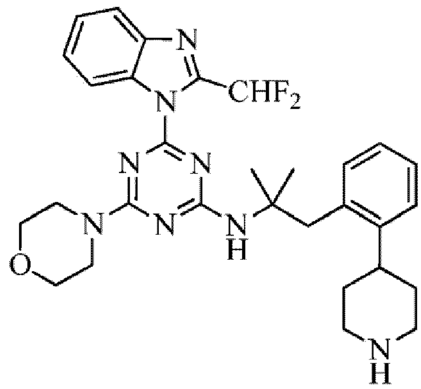
,
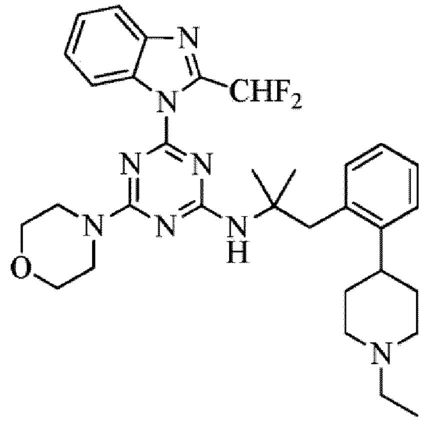
,
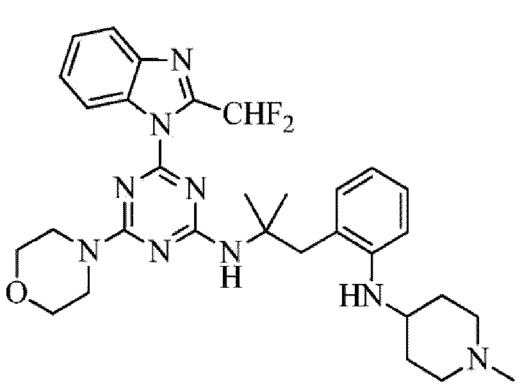
,
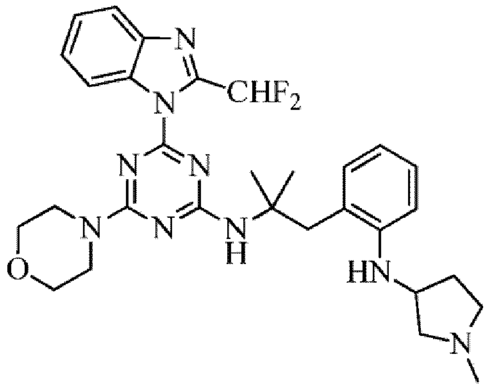
,
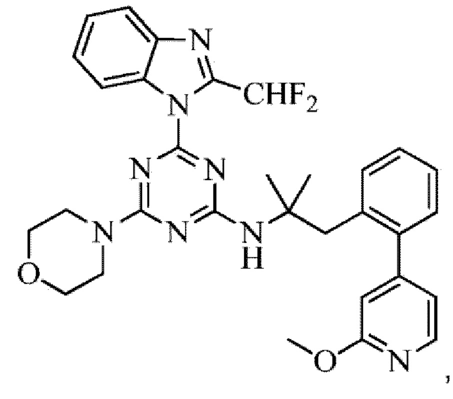
,
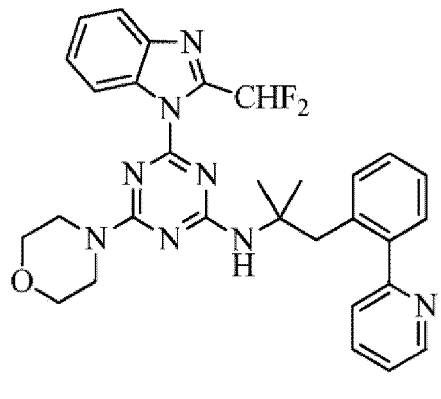
,
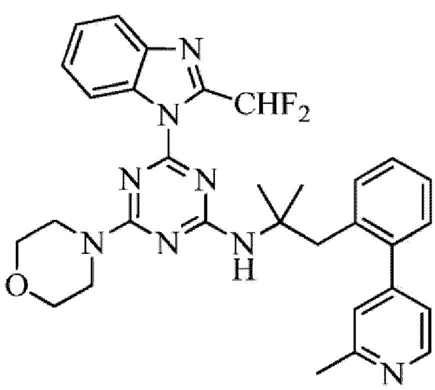
oder
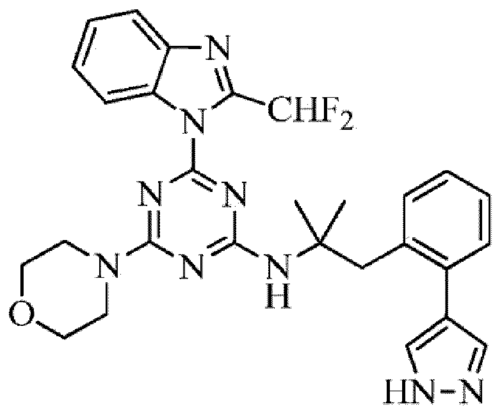

oder eine Isotopenvariante davon;
oder ein pharmazeutisch annehmbares Salz, Solvat oder Hydrat davon.

7. Verbindung zur Verwendung nach Anspruch 1, wobei die Verbindung der Formel (I) eine Verbindung der folgenden Formel ist:

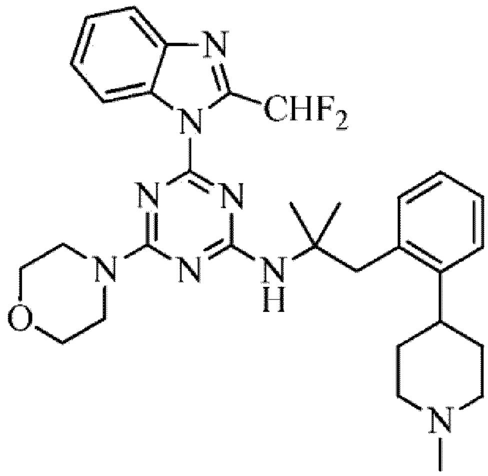

oder eine Isotopenvariante davon;
oder ein pharmazeutisch annehmbares Salz, Solvat oder Hydrat davon.

8. Verbindung zur Verwendung nach einem der Ansprüche 1 bis 7, wobei das zweite Mittel ein BTK-Inhibitor ist.

9. Verbindung zur Verwendung nach Anspruch 8, wobei der BTK-Inhibitor aus Ibrutinib, BGB3111, CC-292, ACP 196, CNX-774, CGI1746, LFM-A13, CNX-774, ONO-4059, RN486 CPI-0610, DUAL946, GSK525762, I-BET151, JQ1, OTX015, PFI-1, RVX-208, RVX2135, TEN-010 und einer Kombination davon ausgewählt ist.

10. Verbindung zur Verwendung nach Anspruch 9, wobei der BTK-Inhibitor Ibrutinib oder BGB3111 ist.

11. Verbindung zur Verwendung nach einem der Ansprüche 1 bis 7, wobei das zweite Mittel ein Bcl-2-Inhibitor ist.

12. Verbindung zur Verwendung nach Anspruch 11, wobei der Bcl-2-Inhibitor Venetoclax oder PNT2258 ist.

13. Verbindung zur Verwendung nach einem der Ansprüche 1 bis 12, wobei die hämatologische Krebserkrankung rezidiviert oder refraktär ist.

14. Verbindung zur Verwendung nach einem der Ansprüche 1 bis 13, wobei die hämatologische Krebserkrankung chronische lymphatische Leukämie, follikuläre Leukämie, ein diffuses großzelliges B-Zell-Lymphom oder ein Non-Hodgkin-Lymphom ist.

15. Verbindung zur Verwendung nach einem der Ansprüche 1 bis 14, wobei die Verbindung in Kombination mit einem dritten Mittel oder einer Therapie verwendet wird, das bzw. die zur Behandlung der hämatologischen Krebserkrankung von Nutzen ist.

## Revendications

1. Composé de Formule (I) :

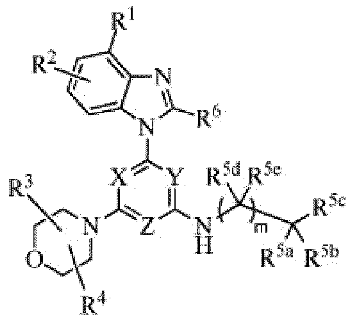

Formule (I),

ou un énantiomère, un mélange d'énantiomères, un mélange de deux diastéréomères ou plus ; ou un variant isotopique de celui-ci ; ou un sel, solvate ou hydrate pharmaceutiquement acceptable de celui-ci ; dans lequel :

X, Y et Z sont chacun indépendamment N ou $CR^x$, à condition qu'au moins deux de X, Y et Z soient des atomes d'azote ; où $R^x$ est un hydrogène ou un alkyle en $C_{1-6}$ ;
$R^1$ et $R^2$ sont chacun indépendamment

(a) un hydrogène, un cyano, un halogéno ou un nitro ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ou $-S(O)_2NR^{1b}R^{1c}$ ;

dans lequel chaque $R^{1a}$, $R^{1b}$, $R^{1c}$ et $R^{1d}$ est indépendamment

(i) un hydrogène ;
(ii) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(iii) $R^{1b}$ et $R^{1c}$ conjointement avec l'atome de N auquel ils sont fixés forment un hétérocyclyle ;

$R^3$ et $R^4$ sont chacun indépendamment un hydrogène ou un alkyle en $C_{1-6}$ ;
ou $R^3$ et $R^4$ sont reliés pour former une liaison, un alkylène en $C_{1-6}$, un hétéroalkylène en $C_{1-6}$, un alcényle en $C_{2-6}$ ou un hétéroalcénylène en $C_{2-6}$ ;
$R^{5a}$ est

(a) un hydrogène ou un halogéno ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ou $-S(O)_2NR^{1b}R^{1c}$ ;

$R^{5b}$ est

(a) un halogéno ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$,

$-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ou $-S(O)_2NR^{1b}R^{1c}$ ;

$R^{5c}$ est un $-(CR^{5f}R^{5g})_n-$ (aryle en $C_{6-14}$) ou un $-(CR^{5f}R^{5g})_n$-hétéroaryle ;
$R^{5d}$ et $R^{5e}$ sont chacun indépendamment

(a) un hydrogène ou un halogéno ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ou $-S(O)_2NR^{1b}R^{1c}$ ;

$R^{5f}$ et $R^{5g}$ sont chacun indépendamment

(a) un hydrogène ou un halogéno ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou
(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ; ou $-S(O)_2NR^{1b}R^{1c}$ ; ou (d) lorsqu'une occurrence de $R^{5f}$ et une occurrence de $R^{5g}$ sont fixées au même atome de carbone, les $R^{5f}$ et $R^{5g}$ conjointement avec l'atome de carbone auquel ils sont fixés forment un cycloalkyle en $C_{3-10}$ ou un hétérocyclyle ;

$R^6$ est un hydrogène, un alkyle en $C_{1-6}$, un -S-alkyle en $C_{1-6}$, un -S(O)-alkyle en $C_{1-6}$ ou un $-SO_2$-alkyle en $C_{1-6}$ ;
m vaut 0 ou 1 ; et
n vaut 0, 1, 2, 3 ou 4 ;
dans lequel chaque alkyle, alkylène, hétéroalkylène, alcényle, alcénylène, hétéroalcénylène, alcynyle, cycloalkyle, aryle, aralkyle, hétéroaryle et hétérocyclyle dans $R^1$, $R^2$, $R^3$, $R^4$, $R^6$, $R^X$, $R^{1a}$, $R^{1b}$, $R^{1c}$, $R^{1d}$, $R^{5a}$, $R^{5b}$, $R^{5c}$, $R^{5d}$, $R^{5e}$, $R^{5f}$ et $R^{5g}$ est optionnellement substitué par un, deux, trois, quatre ou cinq substituants Q, dans lequel chaque substituant Q est indépendamment choisi parmi

(a) un oxo, un cyano, un halogéno et un nitro ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle et un hétérocyclyle, chacun étant en outre optionnellement substitué par un, deux, trois ou quatre substituants $Q^a$ ; et
(c) $-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$ et $-S(O)_2NR^bR^c$,

dans lequel chaque $R^a$, $R^b$, $R^c$ et $R^d$ est indépendamment

(i) un hydrogène ;
(ii) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle, chacun étant en outre optionnellement substitué par un, deux, trois ou quatre substituants $Q^a$ ; ou
(iii) $R^b$ et $R^c$ conjointement avec l'atome de N auquel ils sont fixés forment un hétérocyclyle, qui est en outre optionnellement substitué par un, deux, trois ou quatre substituants $Q^a$ ;

dans lequel chaque $Q^a$ est indépendamment choisi dans le groupe constitué de

(a) un oxo, un cyano, un halogéno et un nitro ;

(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle et un hétérocyclyle ; et

(c) $-C(O)R^e$, $-C(O)OR^e$, $-C(O)NR^fR^g$, $-C(NR^e)NR^fR^g$, $-OR^e$, $-OC(O)R^e$, $-OC(O)OR^e$, $-OC(O)NR^fR^g$, $-OC(=NR^e)NR^fR^g$, $-OS(O)R^e$, $-OS(O)_2R^e$, $-OS(O)NR^fR^g$, $-OS(O)_2NR^fR^g$, $-NR^fR^g$, $-NR^eC(O)R^h$, $-NR^eC(O)OR^h$, $-NR^eC(O)NR^fR^g$, $-NR^eC(=NR^h)NR^fR^g$, $-NR^eS(O)R^h$, $-NR^eS(O)_2R^h$, $-NR^eS(O)NR^fR^g$, $-NR^eS(O)_2NR^fR^g$, $-SR^e$, $-S(O)R^e$, $-S(O)_2R^e$, $-S(O)NR^fR^g$ et $-S(O)_2NR^fR^g$ ;

dans lequel chaque $R^e$, $R^f$, $R^g$ et $R^h$ est indépendamment

(i) un hydrogène ;

(ii) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou

(iii) $R^f$ et $R^g$ conjointement avec l'atome de N auquel ils sont fixés forment un hétérocyclyle ;

dans lequel deux substituants Q qui sont adjacents l'un à l'autre forment optionnellement un cycloalcényle en $C_{3-10}$, un aryle en $C_{6-14}$, un hétéroaryle ou un hétérocyclyle, chacun optionnellement substitué par un, deux, trois ou quatre substituants $Q^a$ ;

pour une utilisation dans un procédé de traitement d'un cancer hématologique en combinaison avec un deuxième agent choisi parmi un inhibiteur de BTK, un inhibiteur de Bcl-2, un inhibiteur d'EZH2 et toute combinaison de ceux-ci.

**2.** Composé pour une utilisation selon la revendication 1, dans lequel :
$R^{5a}$ et $R^{5b}$ sont chacun indépendamment

(a) un halogéno ;

(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle ; ou

(c) $-C(O)R^{1a}$, $-C(O)OR^{1a}$, $-C(O)NR^{1b}R^{1c}$, $-C(NR^{1a})NR^{1b}R^{1c}$, $-OR^{1a}$, $-OC(O)R^{1a}$, $-OC(O)OR^{1a}$, $-OC(O)NR^{1b}R^{1c}$, $-OC(=NR^{1a})NR^{1b}R^{1c}$, $-OS(O)R^{1a}$, $-OS(O)_2R^{1a}$, $-OS(O)NR^{1b}R^{1c}$, $-OS(O)_2NR^{1b}R^{1c}$, $-NR^{1b}R^{1c}$, $-NR^{1a}C(O)R^{1d}$, $-NR^{1a}C(O)OR^{1d}$, $-NR^{1a}C(O)NR^{1b}R^{1c}$, $-NR^{1a}C(=NR^{1d})NR^{1b}R^{1c}$, $-NR^{1a}S(O)R^{1d}$, $-NR^{1a}S(O)_2R^{1d}$, $-NR^{1a}S(O)NR^{1b}R^{1c}$, $-NR^{1a}S(O)_2NR^{1b}R^{1c}$, $-SR^{1a}$, $-S(O)R^{1a}$, $-S(O)_2R^{1a}$, $-S(O)NR^{1b}R^{1c}$ ou $-S(O)_2NR^{1b}R^{1c}$.

**3.** Composé pour une utilisation selon la revendication 1 ou 2, dans lequel :
$R^{5a}$ et $R^{5b}$ sont chacun un méthyle, optionnellement substitué par un, deux ou trois halogéno(s).

**4.** Composé pour une utilisation selon l'une quelconque des revendications 1 à 3, dans lequel :

n vaut 1 ; et
$R^{5f}$ et $R^{5g}$ sont chacun un hydrogène.

**5.** Composé pour une utilisation selon l'une quelconque des revendications 1 à 4, dans lequel le composé de Formule (I) est un composé de Formule (XI) :

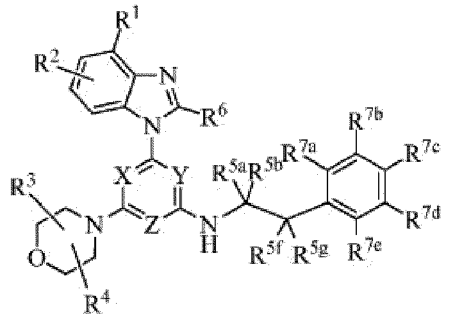

Formule (XI),

ou un énantiomère, un mélange d'énantiomères, un mélange de deux diastéréomères ou plus ;

ou un variant isotopique de celui-ci ;
ou un sel, solvate ou hydrate pharmaceutiquement acceptable de celui-ci ;
dans lequel :

$R^{7a}$, $R^{7b}$, $R^{7c}$, $R^{7d}$, et $R^{7e}$ sont chacun indépendamment

(a) un hydrogène, un cyano, un halogéno ou un nitro ;
(b) un alkyle en $C_{1-6}$, un alcényle en $C_{2-6}$, un alcynyle en $C_{2-6}$, un cycloalkyle en $C_{3-10}$, un aryle en $C_{6-14}$, un aralkyle en $C_{7-15}$, un hétéroaryle ou un hétérocyclyle, chacun étant optionnellement substitué par un, deux, trois ou quatre substituants $Q^a$ ; ou
(c) $-C(O)R^a$, $-C(O)OR^a$, $-C(O)NR^bR^c$, $-C(NR^a)NR^bR^c$, $-OR^a$, $-OC(O)R^a$, $-OC(O)OR^a$, $-OC(O)NR^bR^c$, $-OC(=NR^a)NR^bR^c$, $-OS(O)R^a$, $-OS(O)_2R^a$, $-OS(O)NR^bR^c$, $-OS(O)_2NR^bR^c$, $-NR^bR^c$, $-NR^aC(O)R^d$, $-NR^aC(O)OR^d$, $-NR^aC(O)NR^bR^c$, $-NR^aC(=NR^d)NR^bR^c$, $-NR^aS(O)R^d$, $-NR^aS(O)_2R^d$, $-NR^aS(O)NR^bR^c$, $-NR^aS(O)_2NR^bR^c$, $-SR^a$, $-S(O)R^a$, $-S(O)_2R^a$, $-S(O)NR^bR^c$, ou $-S(O)_2NR^bR^c$ ; ou

deux de $R^{7a}$, $R^{7b}$, $R^{7c}$, $R^{7d}$ et $R^{7e}$ qui sont adjacents l'un à l'autre forment un cycloalcényle en $C_{3-10}$, un aryle en $C_{6-14}$, un hétéroaryle ou un hétérocyclyle, chacun optionnellement substitué par un, deux, trois ou quatre substituants $Q^a$.

**6.** Composé pour une utilisation selon la revendication 1, dans lequel le composé de Formule (I) est :

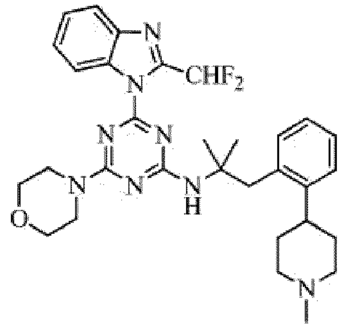
,
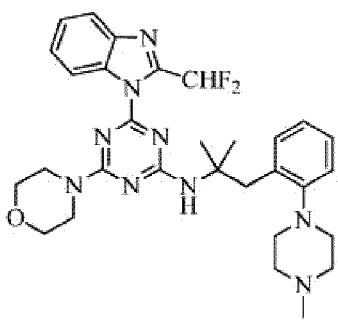
,
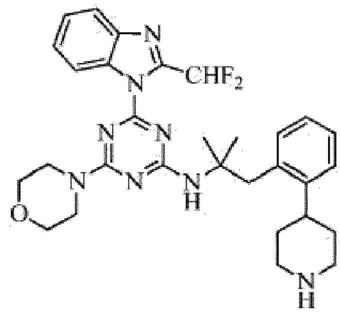
,
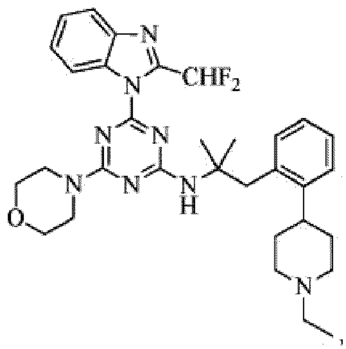
,
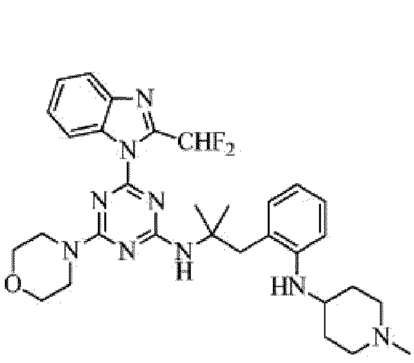
,
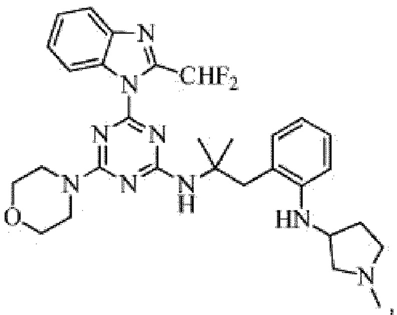
,

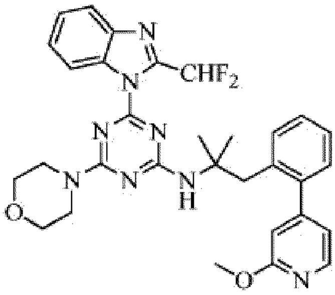,

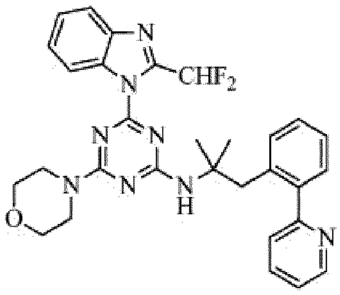,

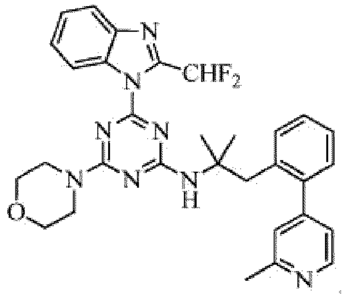 ou

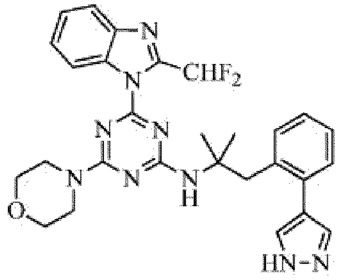,

ou un variant isotopique de celui-ci ;
ou un sel, solvate ou hydrate pharmaceutiquement acceptable de celui-ci.

**7.** Composé pour une utilisation selon la revendication 1, dans lequel le composé de Formule (I) est un composé de la formule suivante :

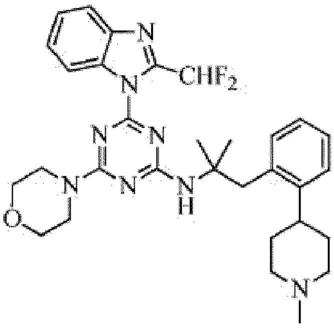,

ou un variant isotopique de celui-ci ;
ou un sel, solvate ou hydrate pharmaceutiquement acceptable de celui-ci.

**8.** Composé pour une utilisation selon l'une quelconque des revendications 1 à 7, dans lequel :
le deuxième agent est un inhibiteur de BTK.

**9.** Composé pour une utilisation selon la revendication 8, dans lequel :
l'inhibiteur de BTK est choisi parmi l'ibrutinib, BGB3111, CC-292, ACP 196, CNX-774, CGI1746, LFM-A13, CNX-774, ONO-4059, RN486 CPI-0610, DUAL946, GSK525762, I-BET151, JQ1, OTX015, PFI-1, RVX-208, RVX2135, TEN-010, et une combinaison de ceux-ci.

**10.** Composé pour une utilisation selon la revendication 9, dans lequel :
l'inhibiteur de BTK est de l'ibrutinib ou BGB3111.

**11.** Composé pour une utilisation selon l'une quelconque des revendications 1 à 7, dans lequel :
le deuxième agent est un inhibiteur de Bcl-2.

**12.** Composé pour une utilisation selon la revendication 11, dans lequel :

l'inhibiteur de Bcl-2 est du vénétoclax ou PNT2258.

**13.** Composé pour une utilisation selon l'une quelconque des revendications 1 à 12, dans lequel : le cancer hématologique est en rechute ou réfractaire.

**14.** Composé pour une utilisation selon l'une quelconque des revendications 1 à 13, dans lequel : le cancer hématologique est une leucémie lymphocytaire chronique, un lymphome folliculaire, un lymphome diffus à grandes cellules B ou un lymphome non hodgkinien.

**15.** Composé pour une utilisation selon l'une quelconque des revendications 1 à 14, dans lequel : le composé est utilisé en combinaison avec un troisième agent ou une thérapie utile dans le traitement du cancer hématologique.

FIG. 1

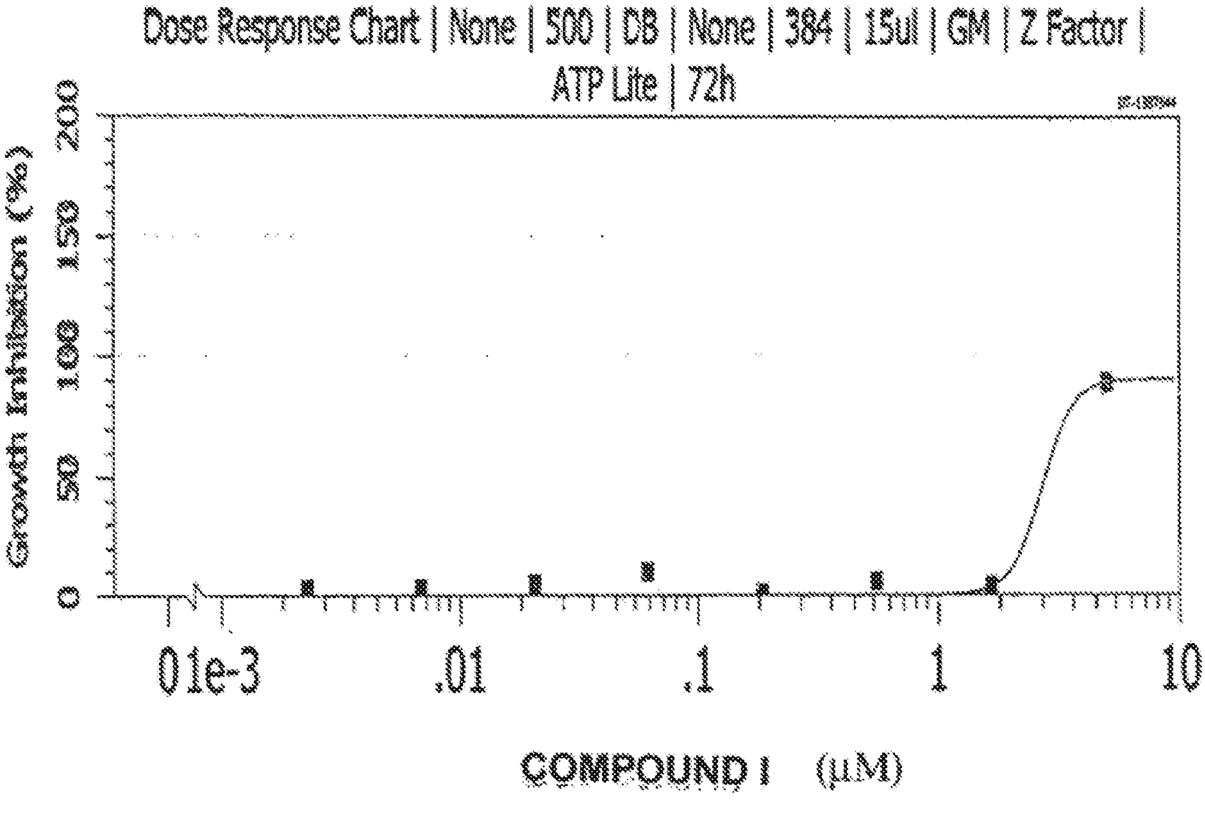
Dose Response Chart | None | 500 | DB | None | 384 | 15ul | GM | Z Factor |
ATP Lite | 72h
Growth Inhibition (%)
0
50
100
150
200
01e-3
.01
.1
1
10
COMPOUND I (μM)

FIG. 2

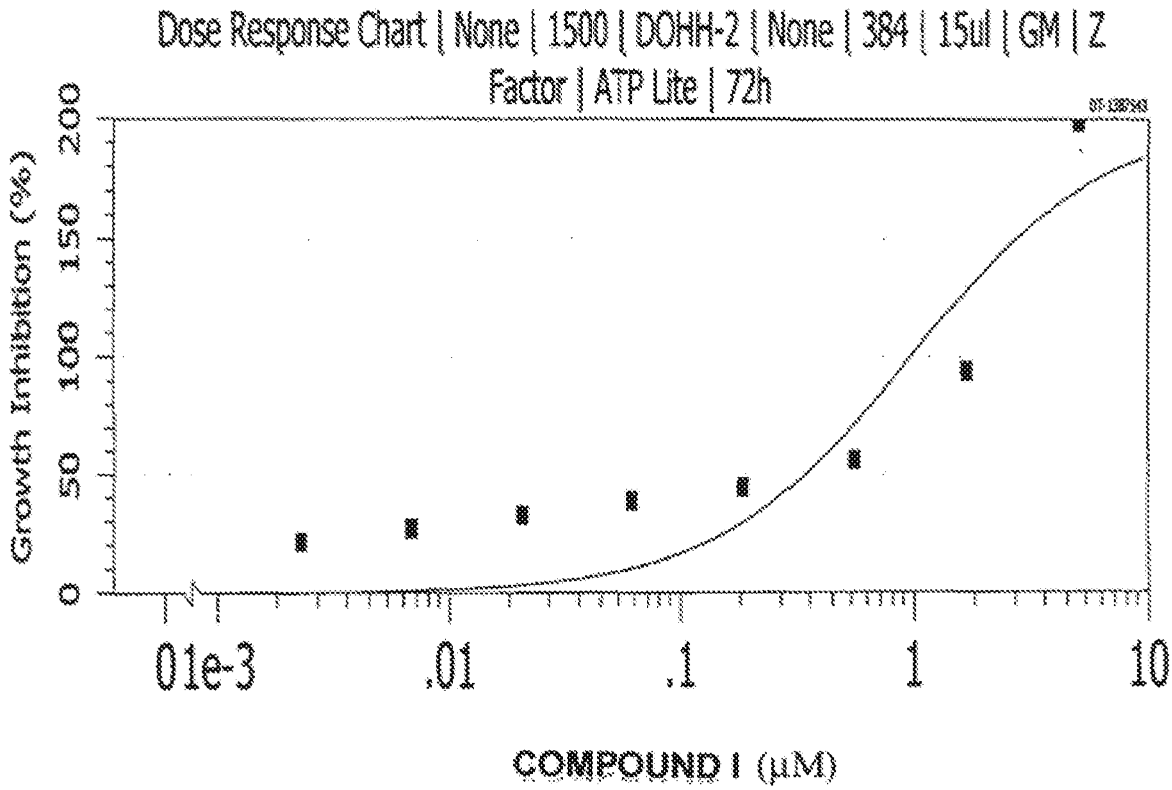
Dose Response Chart | None | 1500 | DOHH-2 | None | 384 | 15ul | GM | Z Factor | ATP Lite | 72h
Growth Inhibition (%)
0
50
100
150
200
01e-3
.01
.1
1
10
COMPOUND I (µM)

FIG. 3

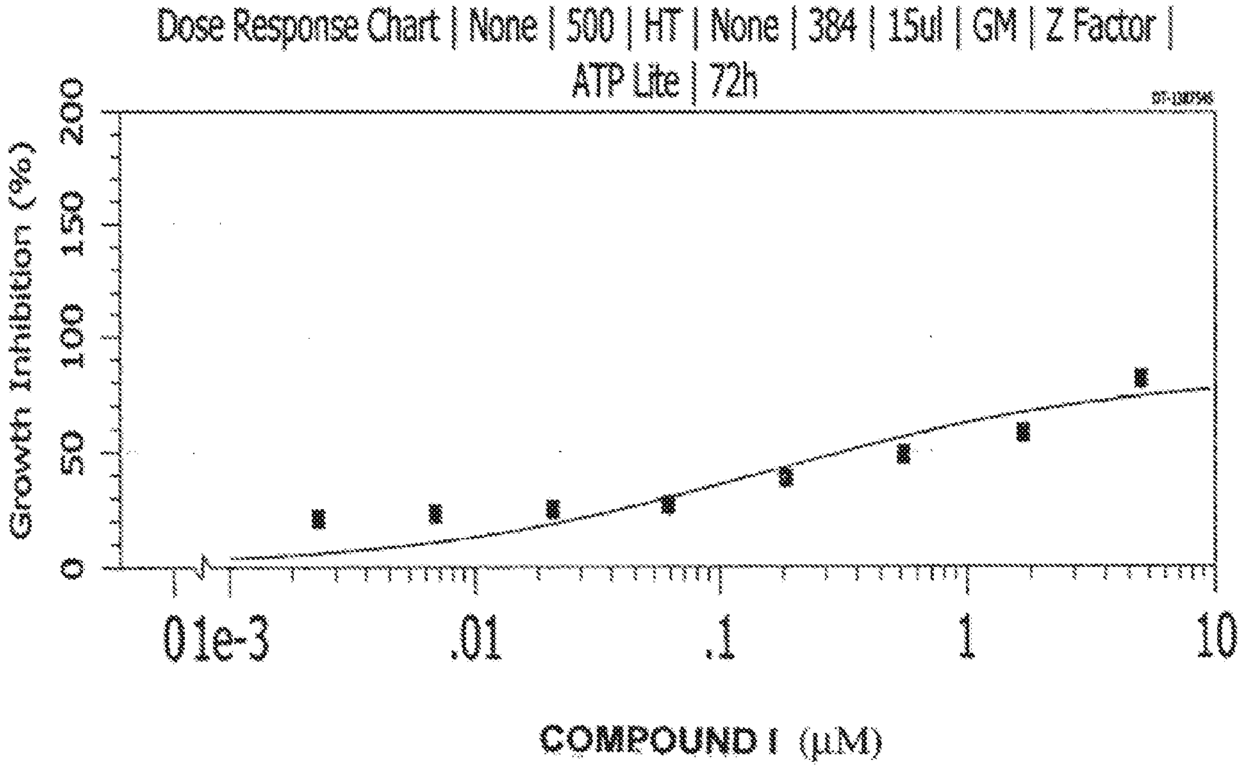
Dose Response Chart | None | 500 | HT | None | 384 | 15ul | GM | Z Factor |
ATP Lite | 72h
Growth Inhibition (%)
0
50
100
150
200
01e-3
.01
.1
1
10
COMPOUND I (μM)

FIG. 4

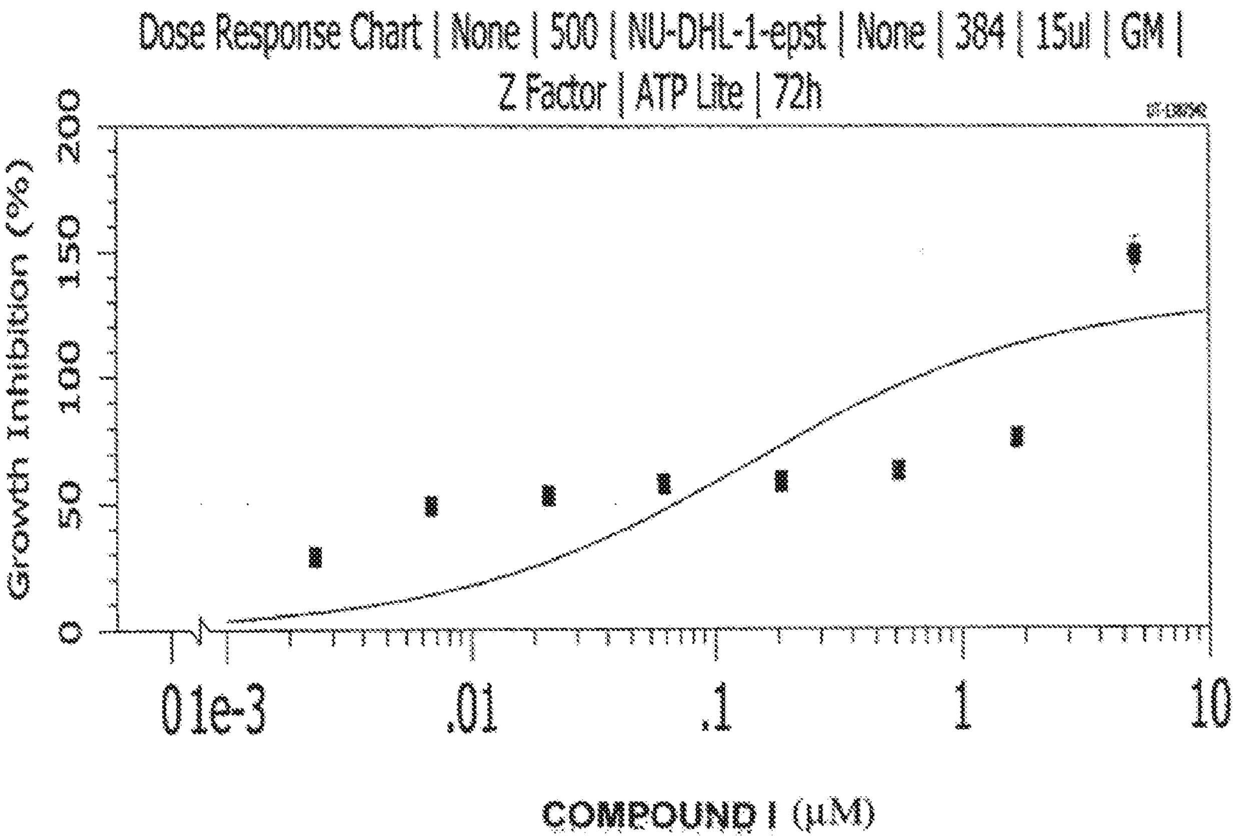
Dose Response Chart | None | 500 | NU-DHL-1-epst | None | 384 | 15ul | GM |
Z Factor | ATP Lite | 72h
Growth Inhibition (%)
0
50
100
150
200
01e-3
.01
.1
1
10
COMPOUND I (μM)

FIG. 5

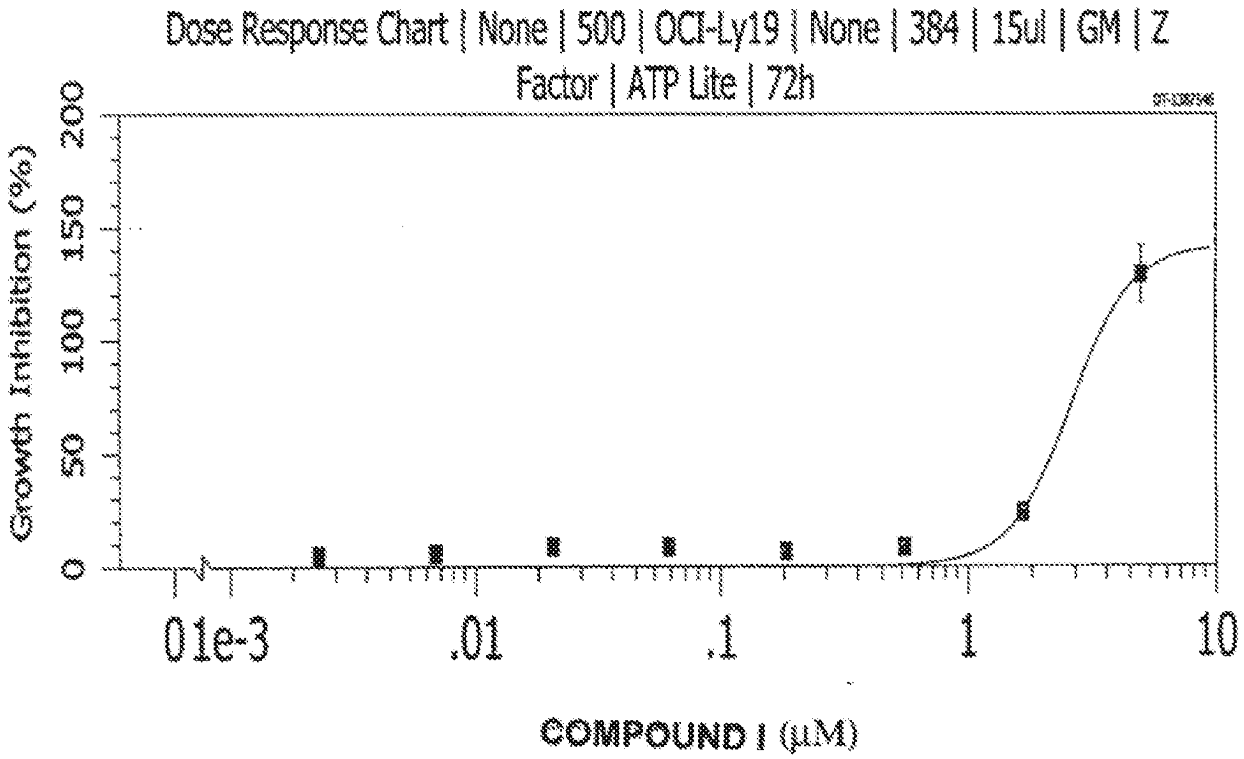
Dose Response Chart | None | 500 | OCI-Ly19 | None | 384 | 15ul | GM | Z Factor | ATP Lite | 72h
Growth Inhibition (%)
0
50
100
150
200
01e-3
.01
.1
1
10
COMPOUND I (μM)

FIG. 6

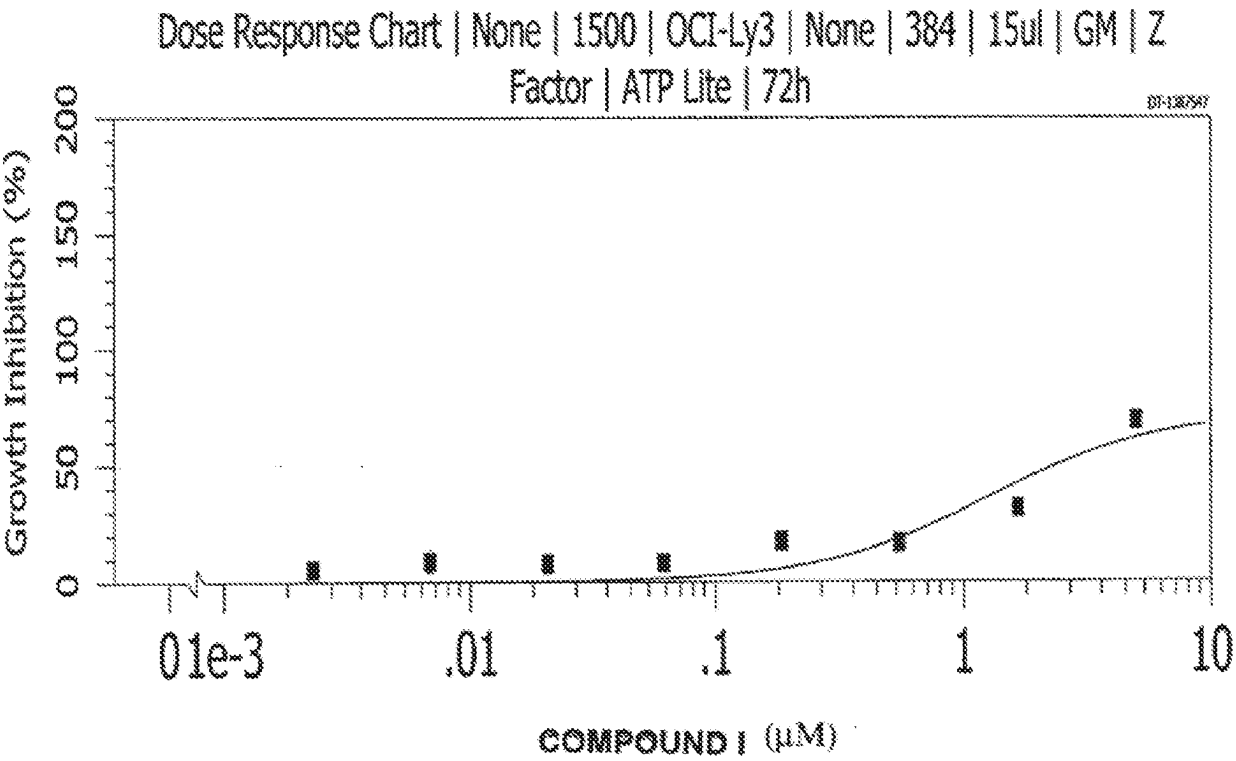
Dose Response Chart | None | 1500 | OCI-Ly3 | None | 384 | 15ul | GM | Z Factor | ATP Lite | 72h
Growth Inhibition (%)
0
50
100
150
200
01e-3
.01
.1
1
10
COMPOUND I (µM)

FIG. 7

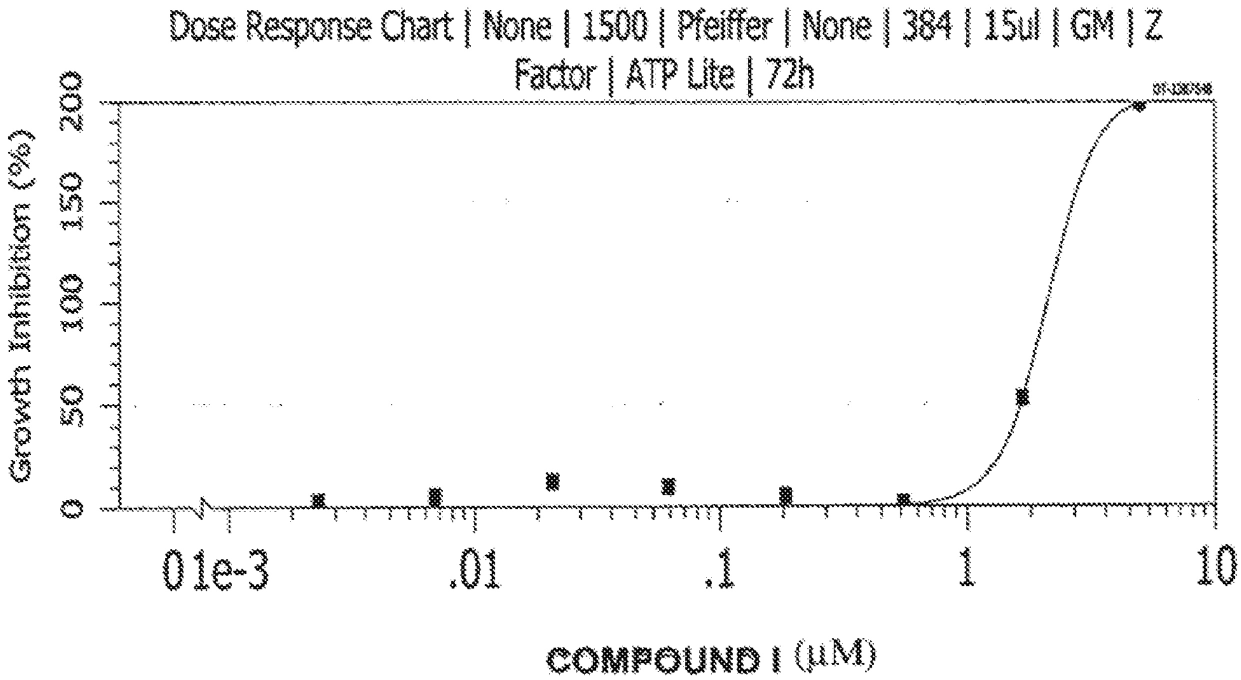
Dose Response Chart | None | 1500 | Pfeiffer | None | 384 | 15ul | GM | Z Factor | ATP Lite | 72h
Growth Inhibition (%)
0
50
100
150
200
01e-3
.01
.1
1
10
COMPOUND I (μM)

FIG. 8

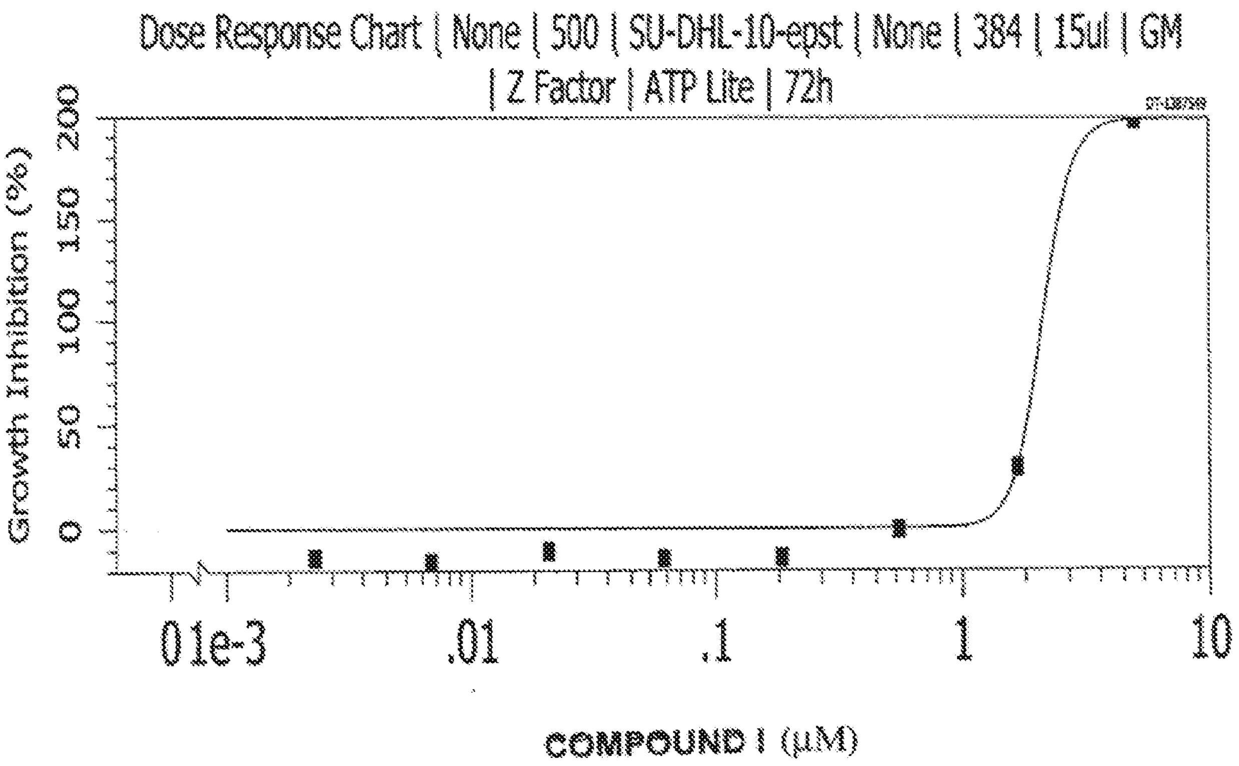
Dose Response Chart | None | 500 | SU-DHL-10-epst | None | 384 | 15ul | GM
| Z Factor | ATP Lite | 72h
Growth Inhibition (%)
0
50
100
150
200
01e-3
.01
.1
1
10
COMPOUND I (μM)

FIG. 9

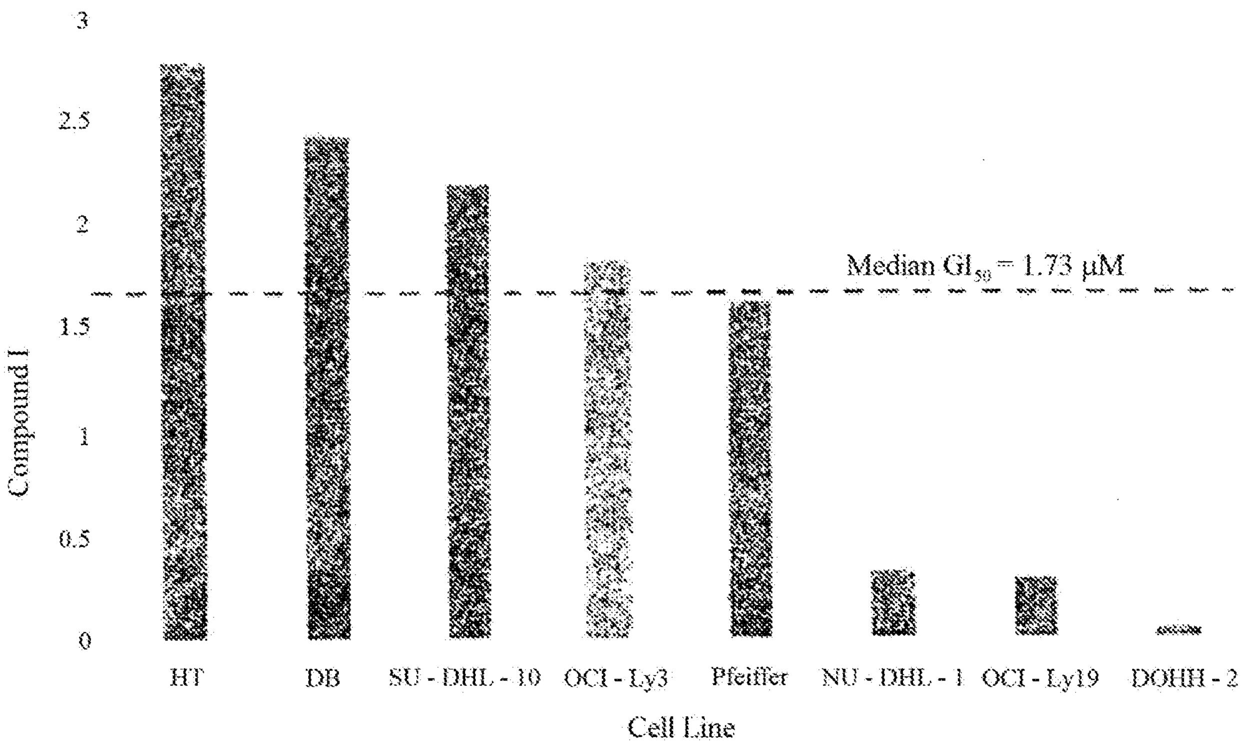

3
2.5
2
1.5
1
0.5
0
Compound I
Median $GI_{50}$ = 1.73 μM
HT
DB
SU - DHL - 10
OCI - Ly3
Pfeiffer
NU - DHL - 1
OCI - Ly19
DOHH - 2
Cell Line

FIG. 10

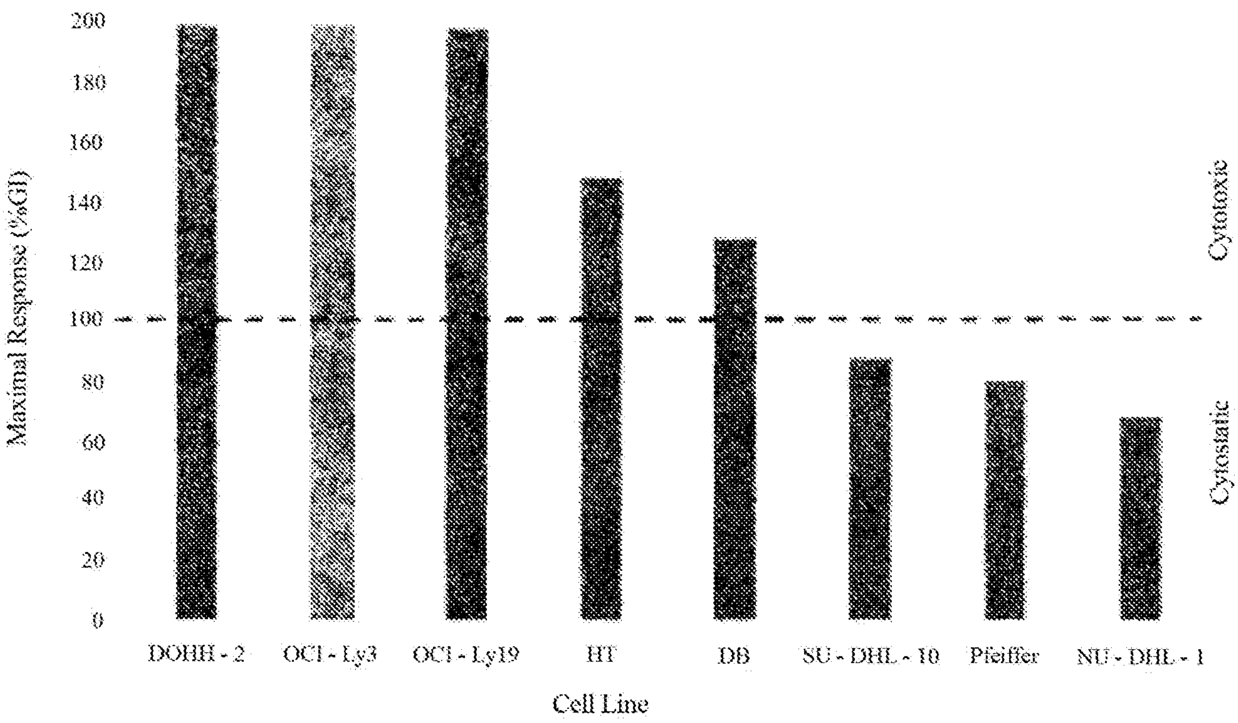

200
180
160
140
120
100
80
60
40
20
0
Maximal Response (%GI)
Cytotoxic
Cytostatic
DOHH - 2
OCI - Ly3
OCI - Ly19
HT
DB
SU - DHL - 10
Pfeiffer
NU - DHL - 1
Cell Line

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015083008 A1
- US 20120252802 A1
- US 20160193211 A1
- WO 2014055647 A1
- US 20140088102 A1
- US 20130150364 A1
- US 20120165309 A1
- US 9056852 B2
- US 8084620 B2
- US 7514444 B2
- US 7718662 B1
- US 7393848 B1
- US 20160083392 A1
- US 20150005277 A1
- US 20150259354 A1
- US 2012053189 A1
- US 2010254905 A1
- US 20080139582 A1
- US 20120077832 A1
- US 20120232054 A1
- US 2012082702 A1
- US 20100160303 A1
- US 2012129852 A1
- US 20060178367 A1
- US 20060183746 A1
- US 2012040961 A1
- US 2010144705 A1
- US 20120028981 A1
- US 2012058996 A1
- US 20090318448 A1
- US 20100016301 A1
- US 2009105209 A1
- US 20100222325 A1
- US 20100004231 A1
- WO 2011153514 A
- WO 2011046964 A
- WO 2010009342 A
- WO 2008121742 A
- WO 2008054827 A
- WO 2007087068 A
- WO 2011090760 A
- WO 2010028236 A
- WO 2009158571 A
- WO 2009051822 A
- WO 2010123870 A
- WO 2010126960 A
- WO 2011162515 A
- WO 2012135801 A
- WO 2011152351 A
- WO 2007136790 A2
- WO 2002050071 A
- WO 2008116064 A
- WO 2010011837 A
- WO 2011159857 A
- WO 2011019780 A
- WO 2011029043 A
- WO 2011029046 A
- WO 2005005429 A
- WO 2005014599 A
- WO 2005047290 A
- WO 2006053121 A
- WO 2008033834 A
- WO 2008033858 A
- WO 2006099075 A
- WO 2008033854 A
- WO 2008033857 A
- WO 2009039397 A
- WO 2009137596 A
- WO 2010056875 A
- WO 2010068788 A
- WO 2010068806 A
- WO 2010068810 A
- WO 2011140488 A
- WO 2012030990 A
- WO 2012031004 A
- WO 2005011597 A
- WO 2008045627 A
- WO 2008144253 A
- WO 2007140222 A
- WO 2013008095 A
- WO 2012170976 A2
- WO 2012135944 A1
- WO 2010065898 A2
- WO 2012158795 A1
- WO 2012158764 A1
- WO 2012158810 A1
- WO 2012156334 A1
- WO 2012020008 A
- WO 2010122038 A
- WO 2010006970 A
- WO 2010006947 A
- WO 2010000633 A
- WO 2009077334 A
- WO 2009098144 A
- WO 2006065946 A
- WO 2007027594 A

- WO 2007027729 A
- EP 2068849 A
- US 4328245 A
- US 4409239 A
- US 4410545 A
- US 6350458 B
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5639480 A
- US 5733566 A
- US 5739108 A
- US 5891474 A
- US 5922356 A
- US 5972891 A
- US 5980945 A
- US 5993855 A
- US 6045830 A
- US 6087324 A
- US 6113943 A
- US 6197350 B
- US 6248363 B
- US 6264970 B
- US 6267981 B
- US 6376461 B
- US 6419961 B
- US 6589548 B
- US 6613358 B
- US 6699500 B
- US 5798119 A
- US 5612059 A
- US 5698220 A
- WO 200217918 A
- US 6316652 B
- US 6274552 B
- US 6271359 B
- US 6253872 B
- US 6139865 A
- US 6131570 A
- US 6120751 A
- US 6071495 A
- US 6060082 A
- US 6048736 A
- US 6039975 A
- US 6004534 A
- US 5985307 A
- US 5972366 A
- US 5900252 A
- US 5840674 A
- US 5759542 A
- US 5709874 A
- US 5323907 A
- US 5052558 A
- US 5033252 A

**Non-patent literature cited in the description**


- **FOUKAS ; SHEPHERD.** *Biochem. Soc. Trans.,* 2004, vol. 32, 330
- **SHEPHERD.** *Acta Physiol. Scand.,* 2005, vol. 183, 3
- **JACKSON et al.** *Nat. Med.,* 2005, vol. 11, 507
- **BARBER et al.** *Nat. Med.,* 2005, vol. 11, 933
- **CAMPS et al.** *Nat. Med.,* 2005, vol. 11, 936
- **ROMMEL et al.** *Nat. Rev.,* 2007, vol. 7, 191
- **ITO et al.** *. Pharm. Exp. Therap.,* 2007, vol. 321, 1
- Remington: The Science and Practice of Pharmacy. Lippincott Williams & Wilkins, 2005
- Handbook of Pharmaceutical Excipients. The Pharmaceutical Press
- American Pharmaceutical Association. 2005
- Handbook of Pharmaceutical Additives. Gower Publishing Company, 2007
- Pharmaceutical Preformulation and Formulation. CRC Press LLC, 2009
- **FRY.** *Biochem. Biophys. Acta,* 1994, vol. 1226, 237-268
- **VANHAESEBROECK ; WATERFIELD.** *Exp. Cell. Res.,* 1999, vol. 253, 239-254
- **FRY.** *Breast Cancer Res.,* 2001, vol. 3, 304-312
- **IKENOUE et al.** *Cancer Res.,* 2005, vol. 65, 4562-4567
- **GYMNOPOULOS et al.** *Proc. Natl. Acad Sci.,* 2007, vol. 104, 5569-5574
- **VETRIE et al.** *Nature,* 1993, vol. 361, 226-233
- **BRADSHAW.** *Cell Signal.,* 2010, vol. 22, 1175-84
- **SMITH et al.** *J. Immunol.,* 1994, vol. 152, 557-565
- **KHAN.** *Immunol. Res.,* 2001, vol. 23, 147
- **TING-CHAO CHOU.** Theoretical Basis, Experimental Design, and Computerized Simulation of Synergism and Antagonism in Drug Combination Studies. *Pharmacol Rev,* 2006, vol. 58, 621-681
- **BREAST.** *Cancer Research and Treatment,* 1997, vol. 46, 255-278
- **FISHMAN et al.** Medicine. J.B. Lippincott Co, 1985

- **PHILADELPHIA ; MURPHY et al.** Informed Decisions: The Complete Book of Cancer Diagnosis, Treatment, and Recovery. Viking Penguin, Penguin Books U.S.A., Inc, 1997
- Physicians' Desk Reference. 2002, 1755-1760
- **TAKADA et al.** Encyclopedia of Controlled Drug Delivery. Wiley, 1999, vol. 2
- **SANTUS ; BAKER.** *J. Controlled Release,* 1995, vol. 35, 1-21
- **VERMA et al.** *Drug Development and Industrial Pharmacy,* 2000, vol. 26, 695-708
- **VERMA et al.** *J. Controlled Release,* 2002, vol. 79, 7-27
- Multiparticulate Oral Drug Delivery. Marcel Dekker, 1994
- d Pharmaceutical Pelletization Technology. Marcel Dekker, 1989
- **THERASSE et al.** *J. Natl. Cancer Inst.,* 02 February 2000, vol. 92 (3), 205-16
- **THERASSE et al.** *J. Natl. Cancer Inst.,* 02 February 2000, vol. 92 (3), 205-16, http://ctep.cancer.gov/forms/TherasseRECISTJNCI.pdf
- National Cancer Institute (NCI) Common Terminology for Adverse Events (CTCAE). 09 August 2006